# EUROPEAN PATENT APPLICATION

(11) **EP 4 215 608 A1**
(43) Date of publication of application: **26.07.2023**
(21) Application number: 21869807.4
(22) Date of filing: 17.09.2021
(51) Int. Cl.: C12N 9/78, C12N 15/52, C12N 9/22, C12N 15/10, C12N 15/90, C12N 15/82, C12N 15/62

(54) **TARGETED DEAMINASE AND BASE EDITING USING SAME**

(30) Priority: 18.09.2020 KR 20200120399; 25.11.2020 KR 20200159920; 29.01.2021 KR 20210013263; 05.02.2021 KR 20210016788; 15.04.2021 KR 20210049348; 19.04.2021 KR 20210050497; 30.06.2021 KR 20210085473; 30.06.2021 KR 20210085474; 14.07.2021 KR 20210092056; 30.08.2021 KR 20210114750
(71) Applicant: Institute for Basic Science, Yuseong-gu Daejeon 34126 (KR)
(72) Inventor: KIM, Jin-Soo, Seoul 08831 (KR); LIM, Kayeong, Seoul 05502 (KR); CHO, Sung Ik, Seoul 05334 (KR); KANG, Beum-Chang, Daejeon 34125 (KR); LEE, Seonghyun, Incheon 22695 (KR); LEE, Hyunji, Daejeon 34140 (KR); MOK, Young Geun, Seoul 08833 (KR); LEE, Ji Min, Seoul 08607 (KR); CHUNG, Eugene, Seoul 05642 (KR)
(74) Representative: Mewburn Ellis LLP
(86) International application number: PCT/KR2021/012872
(87) International publication number: WO 2022/060185

(57) **Abstract**

The present invention relates to a cytosine or adenine deaminase in an isolated form or a variant thereof, a non-toxic full-length cytosine deaminase or a variant thereof, a fusion protein comprising same, a composition for base editing, and a method for editing a base by using same.

## Description

### [Technical Field]

The present invention relates to a cytosine or adenine deaminase in an isolated form or a variant thereof, a non-toxic full-length cytosine deaminase or a variant thereof, a fusion protein including the same, a composition for base editing, and a method of editing a base using the same.

### [Background Art]

A fusion protein in which a DNA-binding protein and a deaminase are fused to each other enables targeted nucleotide substitution or base editing in the genome without produing DNA double-strand breaks (DSBs), corrects point mutations that cause genetic disorders, or makes single nucleotide conversions in a targeted manner to introduce desired single nucleotide mutations into prokaryotic cells and human and other eukaryotic cells.

Unlike nucleases such as CRISPR-Cas9 that induce small insertions or deletions (indels) at the target site, deaminase fusion proteins convert single bases within a window of several nucleotides at the target site. Thus, it is possible to edit point mutations that cause genetic diseases in cultured cells, animals, and plants or to create single nucleotide polymorphisms (SNPs).

Examples of the fusion protein in which a DNA-binding protein and a deaminase are fused to each other may include 1) base editors (BEs) including catalytically deficient Cas9 (dCas9) derived from *S. pyogenes* or D10A Cas9 nickase (nCas9), and rAPOBEC1, which is a cytosine deaminase derived from the rat; 2) target-AIDs including dCas9 or nCas9 and PmCDA1, an activation-induced cytidine deaminase (AID) ortholog derived from sea lamprey, or human AID; 3) CRISPR-X including MS2 RNA hairpin-linked sgRNA and dCas9 to recruit a hyperactive AID variant fused to an MS2-binding protein, and the like.

Programmable genome editing tools such as ZFNs (zinc finger nucleases), TALENs (transcription activator-like effector nucleases), CRISPR (clustered regularly interspaced short palindromic repeat) systems, and base editors composed of CRISPR-associated protein 9 (Cas9) variants and nucleobase deaminase proteins have been developed for plant genetic research and crop trait improvement through changes in base sequences. However, these tools are not suitable for editing the DNA sequences of plant organelles, including mitochondria and chloroplasts, mainly because of difficulty in delivering guide RNAs to organelles or co-expressing two compounds in organelles. Plant organelles encode essential genes required for photosynthesis. Methods or tools for editing the genes of organelles are essential for functional study of organellar genes or improvement in crop productivity and traits. For example, targeted mutations in the mitochondrial atp6 gene may lead to male sterility, which is a useful trait for seed production, and specific point mutations in the 16S rRNA gene of the chloroplast genome may lead to antibiotic resistance.

The bacterial toxin DddAₜₒₓ is an enzymatic domain of a bacterial toxin derived from *Burkholderia cenocepacia,* and is able to deaminate cytosine in double-stranded DNA. As an example of a deaminase, DddAₜₒₓ is cytotoxic, and thus, in order to avoid toxicity in host cells, DddAₜₒₓ is split into two inactive halves, each of which is fused to a DNA-binding protein in a DddA-derived cytosine base editor (DdCBE). A functional deaminase is reassembled at a target DNA site, when two inactive halves are brought together by the DNA-binding protein.

In principle, this deaminase reaction is activated only when two inactive halves are in close proximity to the target DNA by a DNA-binding protein. Thus, cytosine to thymine (C-to-T) base editing is induced at a spacer region between the binding sites of two DNA-binding proteins. Two inactive forms fused to TALE (transcription activator-like effector) DNA-binding arrays become functional when they are brought together by TALE-DNA interactions. C-to-T editing is induced typically in the region of 14-18 bases between the two TALE binding sites. However, the DddAₜₒₓ split system has many limitations in experiments.

The gene encoding the full-length DddAₜₒₓ cannot be cloned in *E. coli* due to toxicity. Cloning is possible only when a DddA inhibitor gene is co-expressed in *E*. *coli.*

On the other hand, mitochondrial DNA plays a very important role in cellular respiration, which is achieved through a mitochondrial oxidative phosphorylation (OXPHOS) mechanism. Because the OXPHOS mechanism is essential for survival, mutations in mitochondrial DNA may cause severe malfunctions in many organs and muscles, particularly in high-energy-demanding tissues. In many human mitochondrial diseases, wild-type mitochondrial DNA coexists with mutant mitochondrial DNA having single base mutations, resulting in a heteroplasmic state of mitochondrial DNA. The balance between mutant and wild-type mitochondrial DNAs determines the development of clinically symptomatic mitochondrial diseases. *In vitro* and *in vivo,* programmable nucleases have been used for cleaving and, thereby, removing mutant mitochondrial DNA without cleaving wild-type mitochondrial DNA. However, these nucleases cannot induce or revert specific mutations in mitochondria: DNA double-strand breaks are not efficiently repaired in mitochondria through non-homologous end joining or homologous recombination, unlike those in the nucleus.

Mitochondrial base editing can be used for creating models for various diseases or for producing therapeutic agents to treat such diseases. In this regard, there is an increasing need for the development of highly efficient mitochondrial base editing enzymes.

With this technical background, we have completed this invention by confirming that DNA can be corrected by using a desired CBE (cytosine base editor) or ABE (adenine base editor) created by reducing non-selective base editing through substitution of the residues of a deaminase or by using a novel full-length deaminase that is not cytotoxic.

### [Disclosure]

It is an object of the present invention to provide a fusion protein including a DNA-binding protein, and a cytosine or adenine deaminase in an isolated form or a variant thereof, or a non-toxic full-length cytosine deaminase or a variant thereof.

It is another object of the present invention to provide a nucleic acid encoding the fusion protein.

It is still another object of the present invention to provide a composition for base editing including the fusion protein or the nucleic acid.

It is yet another object of the present invention to provide a base editing method including treating cells with the composition.

In order to accomplish the above objects, the present invention provides a fusion protein including (i) a DNA-binding protein and (ii) a first split and a second split derived from a cytosine deaminase or a variant thereof, in which each of the first split and the second split is fused to the DNA-binding protein.

In addition, the present invention provides a fusion protein including (i) a DNA-binding protein and (ii) a non-toxic full-length cytosine deaminase derived from a cytosine deaminase or a variant thereof.

In addition, the present invention provides a fusion protein including (i) a DNA-binding protein, (ii) a cytosine deaminase or a variant thereof, and (iii) an adenine deaminase, in which the cytosine deaminase or the variant thereof includes (a) a non-toxic full-length cytosine deaminase or (b) a first split and a second split derived from a cytosine deaminase or a variant thereof, each of the first split and the second split fused to the DNA-binding protein.

In addition, the present invention provides a nucleic acid encoding the fusion protein.

In addition, the present invention provides a composition for base editing including the fusion protein or the nucleic acid.

In addition, the present invention provides a composition for base editing in eukaryotic cells including the fusion protein or the nucleic acid.

In addition, the present invention provides a composition for base editing in plant cells including the fusion protein or the nucleic acid and a nuclear localization signal (NLS) peptide or a nucleic acid encoding the same.

In addition, the present invention provides a composition for base editing in plant cells including the fusion protein or the nucleic acid and a chloroplast transit peptide or a nucleic acid encoding the same.

In addition, the present invention provides a composition for base editing in plant cells including the fusion protein or the nucleic acid and a mitochondrial targeting signal (MTS) or a nucleic acid encoding the same.

In some cases, the present invention also provides a composition for base editing in plant cells, further including a nuclear export signal or a nucleic acid encoding the same.

In addition, the present invention provides a method for base editing in plant cells including treating plant cells with the composition.

In addition, the present invention provides a method for base editing in plant cells including treating plant cells with the fusion protein or the nucleic acid, containing a nuclear localization signal (NLS) peptide, or a nucleic acid encoding the same.

In addition, the present invention provides a method for base editing in plant cells including treating plant cells with the fusion protein or the nucleic acid, containing a chloroplast transit peptide, or a nucleic acid encoding the same.

In addition, the present invention provides a method for base editing in plant cells including treating plant cells with the fusion protein or the nucleic acid, containing a mitochondrial targeting signal (MTS), or a nucleic acid encoding the same.

In addition, the present invention provides a composition for base editing in animal cells including the fusion protein or the nucleic acid, containing a nuclear localization signal (NLS) peptide, or a nucleic acid encoding the same.

In addition, the present invention provides a composition for base editing in animal cells including the fusion protein or the nucleic acid and a mitochondrial targeting signal (MTS) or a nucleic acid encoding the same.

In some cases, the present invention also provides a composition for base editing in animal cells, further including a nuclear export signal or a nucleic acid encoding the same.

In addition, the present invention provides a method for base editing in animal cells including treating animal cells with the composition.

In addition, the present invention provides a method for base editing in animal cells including treating animal cells with the fusion protein or the nucleic acid, containing a nuclear localization signal (NLS) peptide, or a nucleic acid encoding the same.

In addition, the present invention provides a method for base editing in animal cells including treating animal cells with the fusion protein or the nucleic acid, containing a mitochondrial targeting signal (MTS), or a nucleic acid encoding the same.

In addition, the present invention provides a composition for A-to-G base editing in prokaryotic or eukaryotic cells including the fusion protein or a nucleic acid encoding the same, in which the DNA-binding protein is a zinc finger protein, a TALE protein, or a CRISPR-associated protein, and a cytosine deaminase or a variant thereof, which is derived from bacteria and is specific to double-stranded DNA.

In addition, the present invention provides a composition for A-to-G base editing in prokaryotic or eukaryotic cells including the fusion protein or a nucleic acid encoding the same, in which the DNA-binding protein is a zinc finger protein, a TALE protein, or a CRISPR-associated protein, a cytosine deaminase of the fusion protein or a variant thereof is derived from bacteria and is specific to double-stranded DNA. The DNA-binding protein is fused to both the N-terminus and the C-terminus of the cytosine deaminase or the variant thereof. Similarly, the DNA-binding protein also is fused both the N-terminus and the C-terminus of the adenine deaminase of the fusion protein. In the context of the fusion protein including a DNA-binding protein, a cytosine deaminase or variant thereof, and an adenine deaminase, the adenine deaminase may be located at the N-terminus or C-terminus of the cytosine deaminase within the fusion protein, or may be present as a separate protein independent of other DNA-binding proteins.

In addition, the present invention provides a composition for C-to-T base editing in prokaryotic or eukaryotic cells including the fusion protein or a nucleic acid encoding the same and a uracil glycosylase inhibitor (UGI), in which the DNA-binding protein is a zinc finger protein, a TALE protein, or a CRISPR-associated protein, and a cytosine deaminase or a variant thereof, which is a non-toxic full-length cytosine deaminase, and the cytosine deaminase in the fusion protein or a variant thereof is derived from bacteria and is specific to double-stranded DNA.

In addition, the present invention provides a composition for C-to-T base editing in prokaryotic or eukaryotic cells including the fusion protein or a nucleic acid encoding the same and a UGI, in which the DNA-binding protein is a zinc finger protein, a TALE protein, or a CRISPR-associated nuclease, and the cytosine deaminase of the fusion protein or a variant thereof is a split cytosine deaminase including a first split and a second split, and the cytosine deaminase of the fusion protein or a variant thereof is derived from bacteria and is specific to double-stranded DNA.

In addition, the present invention provides a method for A-to-G base editing in prokaryotic or eukaryotic cells including treating prokaryotic or eukaryotic cells with the fusion protein or a nucleic acid encoding the same, in which the DNA-binding protein is a zinc finger protein, a TALE protein, or a CRISPR-associated nuclease, and the cytosine deaminase of the fusion protein or a variant thereof is derived from bacteria and is specific to double-stranded DNA.

In addition, the present invention provides a method for A-to-G base editing in prokaryotic or eukaryotic cells including treating prokaryotic or eukaryotic cells with the fusion protein or a nucleic acid encoding the same, in which the DNA-binding protein is a zinc finger protein, a TALE protein, or a CRISPR-associated nuclease,
the cytosine deaminase of the fusion protein or a variant thereof is derived from bacteria and is specific to double-stranded DNA, and
a cytosine deaminase of the fusion protein or a variant thereof is derived from bacteria and is specific to double-stranded DNA. The DNA-binding protein is fused to both the N-terminus and the C-terminus of the cytosine deaminase or the variant thereof. Similarly, the DNA-binding protein also is fused both the N-terminus and the C-terminus of the adenine deaminase of the fusion protein. In the context of the fusion protein including a DNA-binding protein, a cytosine deaminase or variant thereof, and an adenine deaminase, the adenine deaminase may be located at the N-terminus or C-terminus of the cytosine deaminase within the fusion protein, or may be present as a separate protein independent of other DNA-binding proteins.

In addition, the present invention provides a method for C-to-T base editing in prokaryotic or eukaryotic cells including treating prokaryotic or eukaryotic cells with the fusion protein or a nucleic acid encoding the same and a UGI, in which the DNA-binding protein is a zinc finger protein, a TALE protein, or a CRISPR-associated nuclease, and the cytosine deaminase of the fusion protein or a variant thereof is derived from bacteria and is specific to double-stranded DNA.

### [Description of Drawings]

FIG. 1 shows results of optimization of a ZFD using pTarget plasmids,

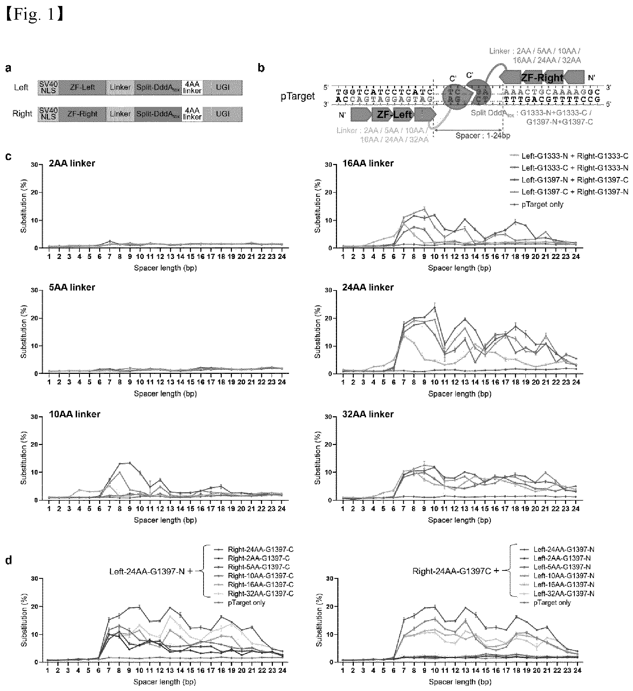

FIG. 1a showing a ZFD construct, in which split-DddAtox halves are fused to the C-terminus of ZFPs (zinc finger proteins: C type), FIG. 1b showing optimization of a ZFD platform using pTarget libraries, in which the pTarget plasmids include a spacer region ranging in size of 1 to 24 bps (represented in red) and ZFP DNA-binding sites (represented in green) and ZFD constructs include AA linkers of various lengths (represented in yellow and orange) and different DddAtox split sites and orientations (represented in blue), and FIGs. 1c and 1d showing ZFD activities measured at target sites in the pTarget library to examine the effects of the variables described in FIG. 1b, in which ZFD pairs with linkers of the same (c) or different (d) lengths in the left and right ZFDs were tested, base editing frequencies were measured by targeted deep sequencing of the relevant region of pTarget plasmids, and data are represented as mean ± standard error of the mean (s.e.m.) from n = 2 biologically independent samples;
FIG. 2 shows results confirming the ZFD efficiencies with various linkers in the pTarget plasmids,

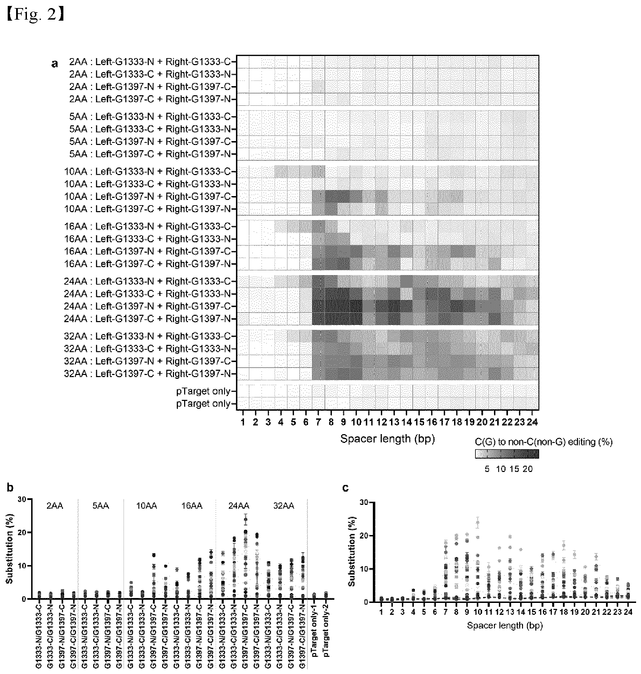

FIG. 2a showing editing frequencies from C/G to non-C/G in various pTarget spacers with lengths of 1-24 bps depicted in the heat map, in which various ZFD configurations were tested, including various types of linkers between ZFP and split DddAtox and regions where DddAtox was split, FIG. 2b showing overall activity of each ZFD pair, in which the nomenclature used for the x-axis shows the left ZFD on the bottom and the right ZFD on the top, and FIG. 2c showing base editing efficiency depending on the spacer length, in which "AA" indicates the number of amino acids in the linker, data being represented as mean ± standard error of the mean (s.e.m.) from n = 2 biologically independent samples;
FIG. 3 shows results confirming the efficiencies of ZFDs having 24AA linkers and different linkers,
   in which the effect of the ZFD linker length on the editing efficiency from C/G to non-C/G in the heat map is shown, in which the left ZFD of a ZFD pair is fixed with 24AA linkers and the right ZFD includes a linker of variable length, or *vice versa,* error bars being standard error of the mean (s.e.m.) for n = 2 biologically independent samples;

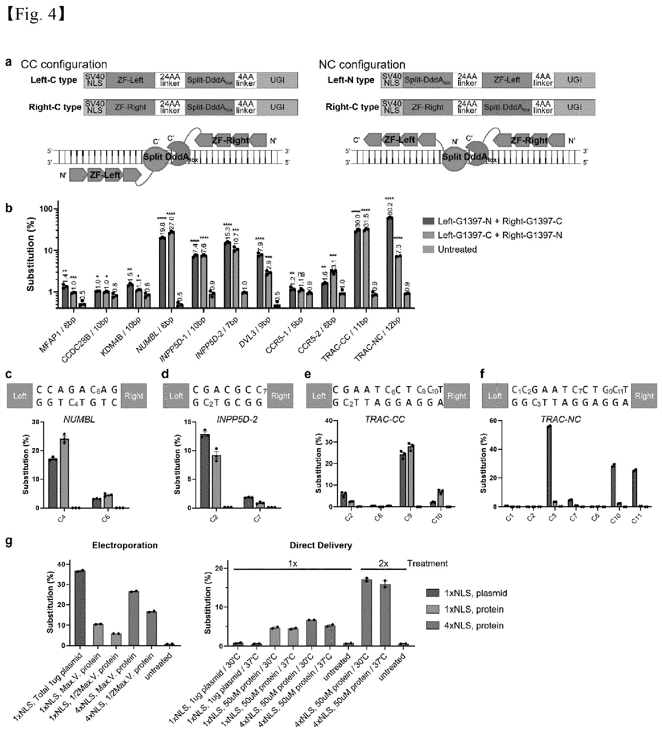

FIG. 4 shows results confirming the activity of ZFDs targeting the nucleus *in vivo,*
FIG. 4a showing the configurations of nuclear DNA-targeting ZFDs, in which split-DddAtox halves are fused to the C-terminus (C-type) or N-terminus (N-type) of ZFPs, ZFD pairs are designed in CC or NC configurations, which are composed of a C-type left ZFD and a C-type right ZFD or an N-type left ZFD and a C-type right ZFD, respectively, FIG. 4b showing base editing frequencies induced by ZFDs at nuclear DNA target sites in HEK 293T cells, data being represented as mean ± s.e.m. from n = 3 biologically independent samples, FIGs. 4c-4f showing ZFD-induced base editing efficiencies at each base position within the spacer at *NUMBL* (c), *INPP5D-2* (d), *TRAC-CC* (e), and *TRAC-NC* (f) target sites in HEK 293T cells, data being represented as mean ± s.e.m. from n = 3 biologically independent samples, FIG. 4g showing ZFD-induced base editing frequencies in K562 cells after electroporation or direct delivery of ZFD proteins or ZFD-encoding plasmids, in which ZFD proteins with one or four NLSs were tested and left and right ZFDs were used equimolarly, electroporation was performed using an Amaxa 4D-Nucleofector, and for direct delivery, K562 cells were incubated with cell medium containing left and right ZFD proteins, and cells were treated either once (1x) or twice (2x) in the same manner, data being represented as mean ± s.e.m. from n = 2 biologically independent samples;

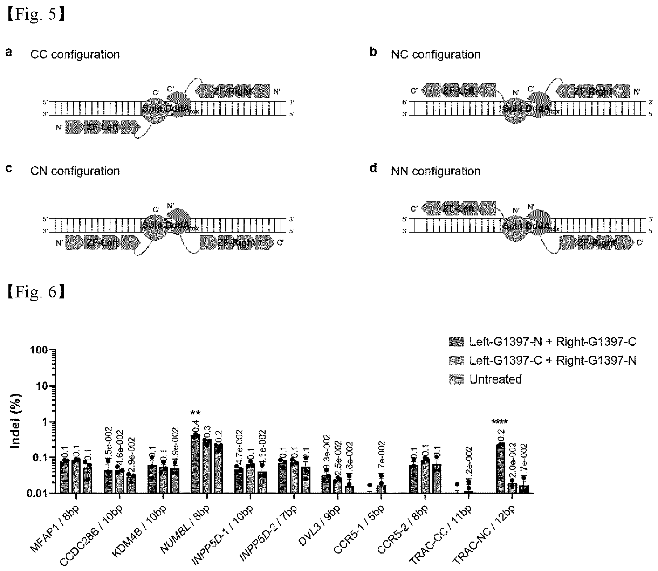

FIG. 5 schematically shows the configurations of nuclear DNA-targeting ZFDs, FIGs. 5a-5d showing four possible ZFD configurations, in which the NC and CN configurations are structurally identical, but the types of left and right ZFD configurations are different;
FIG. 6 shows results confirming the indel rates of ZFDs targeting the nucleus *in vivo,* in which all ZFDs tested produced indels with a frequency of less than 0.4%, and data are represented as mean ± s.e.m. from n = 3 biologically independent samples;

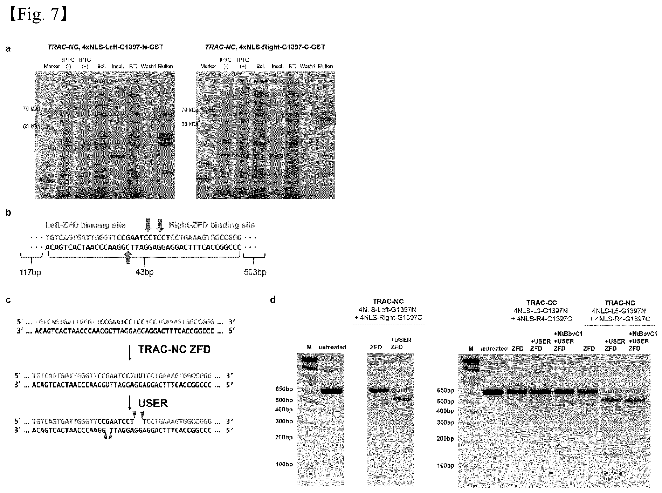

FIG. 7 shows results of testing the activities of *in-vitro* recombinant ZFD proteins,
FIG. 7a showing purification of ZFD pairs targeting TRAC sites, in which GST-tagged proteins were purified from *E. coli* cell lysates using glutathione Sepharose beads, the purification step was monitored using polyacrylamide gel electrophoresis, gels were stained with Coomassie blue, Lane 1 showing a molecular-weight marker, Lane 2 showing a sample of cells, protein expression of which was not induced with IPTG, Lane 3 showing a sample of cells, protein expression of which was induced with IPTG, Lane 4 showing a soluble fraction after sonication, Lane 5 showing an insoluble fraction after sonication, Lane 6 showing a column flow-through fraction, Lane 6 showing a washing fraction, and Lane 7 showing an elution fraction, the size of a representative marker is represented on the left, and the red box indicates a ZFD protein, FIG. 7b showing left and right ZFD binding sites, in which the red arrow indicate a possible site for ZFD-induced deamination, FIG. 7c showing ZFD activity for PCR amplicons containing TRAC sites, in which TRAC-NC ZFD pairs deaminate cytosine to produce uracil (represented in red) and then a USER enzyme cleaves uracil to form a cleft (represented as a red triangle), and FIG. 7d showing untreated PCR amplicons (left) analyzed by agarose gel electrophoresis and PCR amplicons treated with ZFD pairs (right);

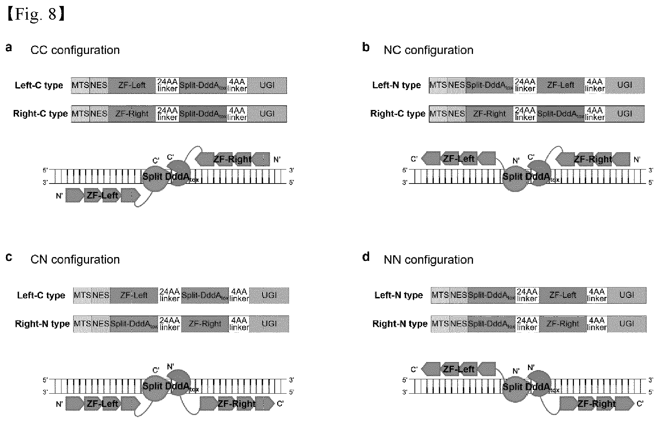

FIG. 8 schematically shows various configurations of mitochondrial DNA-targeting ZFDs, FIGs. 8a-8d showing four possible mitoZFD configurations, in which the NLS of existing ZFD is replaced with MTS and NES, and the NC and CN configurations are structurally identical, but the types of left and right ZFD configurations are different;

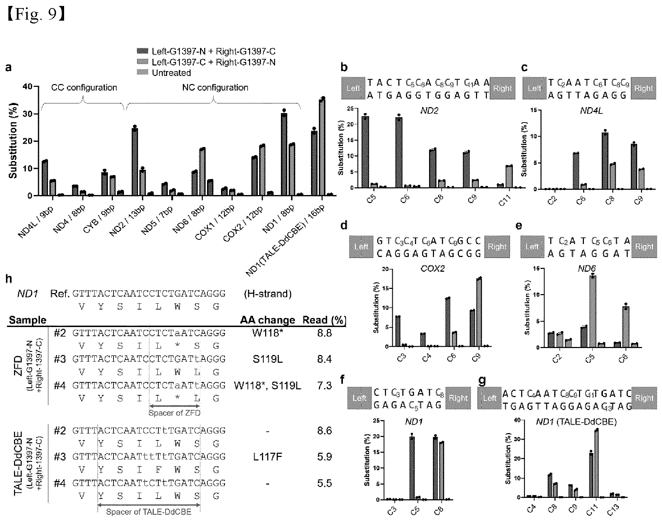

FIG. 9 shows results confirming the mitochondrial gene base editing efficiencies of mitoZFDs,
FIG. 9a showing base editing frequencies in mtDNA induced by mitoZFD and TALE-DdCBE in HEK 293T cells, data being represented as mean ± standard error of the mean (s.e.m.) from n = 2 biologically independent samples, FIGs. 9b-9g showing mitoZFD-induced base editing efficiencies at each base position within the spacer at ND2 (b), ND4L (c), COX2 (d), ND6 (e), and ND1 (f) target sites, and TALE-DdCBE-induced base editing efficiencies at the ND1 (g) target site, in HEK 293T cells, data being represented as mean ± standard error of the mean (s.e.m.) from n = 2 biologically independent samples, and FIG. 9h showing comparison of DNA changes and amino acid changes in the ND1 gene introduced by mitoZFD and TALE-DdCBE, in which the frequency (%) of sequencing reads for each mutant allele was measured by targeted deep sequencing, and spacer regions for ZFD pairs and TALE-DdCBE pairs are represented as blue dotted lines;

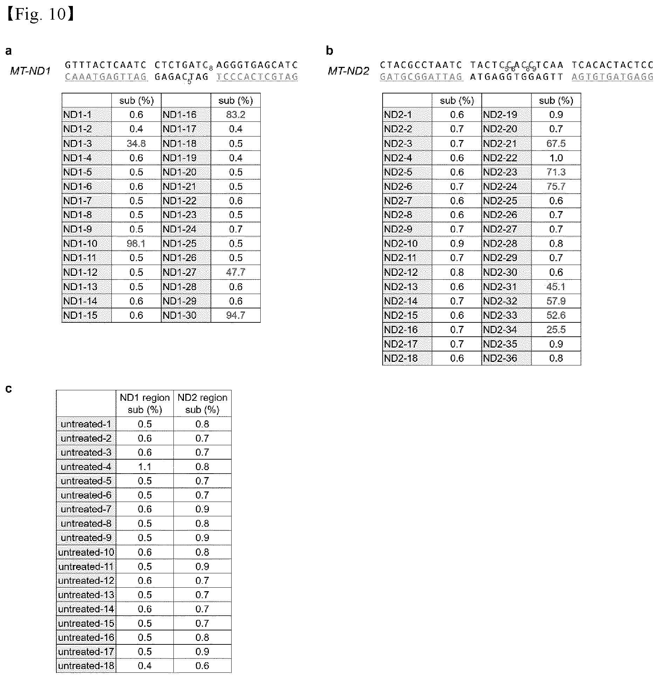

FIG. 10 shows results confirming the base editing efficiencies of single-cell-derived clonal populations isolated from HEK293T cells treated with MT-ZFDs,
   in which single-cell-derived clones were obtained for allele analysis, C/G-to-non-C/G editing frequencies in individual single-cell-derived clones were determined by targeted deep sequencing, FIG. 10a showing single-cell-derived clones of HEK 293T cell populations treated with ND1-targeted mitoZFD, FIG. 10b showing single-cell-derived clones of HEK 293T cell populations treated with ND2-targeted mitoZFD, and FIG. 10c showing single-cell-derived clones of untreated HEK 293T cell populations, in which the ZFP binding site is represented in green, and the high editing frequency of clones subjected to mitoZFD-induced editing is represented in red;

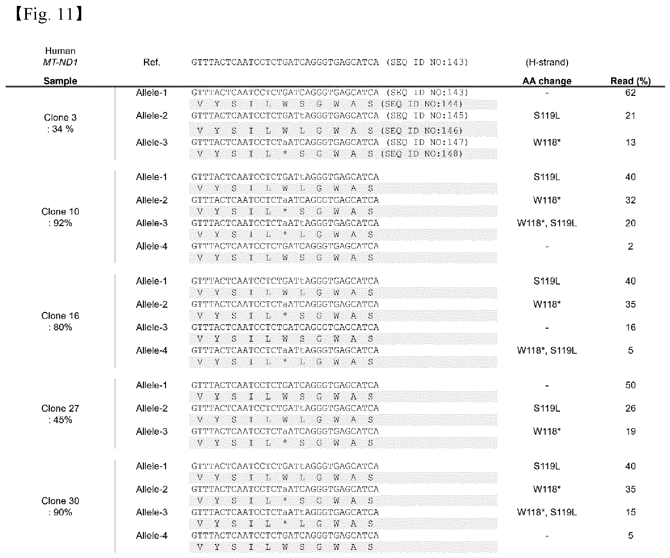

FIG. 11 shows results confirming the base editing efficiencies of single-cell-derived clonal populations isolated from HEK293T cells treated with MT-ZFD,
   in which there is provided allele analysis of single-cell-derived clones with high base editing frequency, the table shows amino acids changed by base editing in ND1, and in the top reference sequence, the red letter indicates a spacer, and in alleles, the red letter indicates a change in the amino acid sequence (* indicates a stop codon);

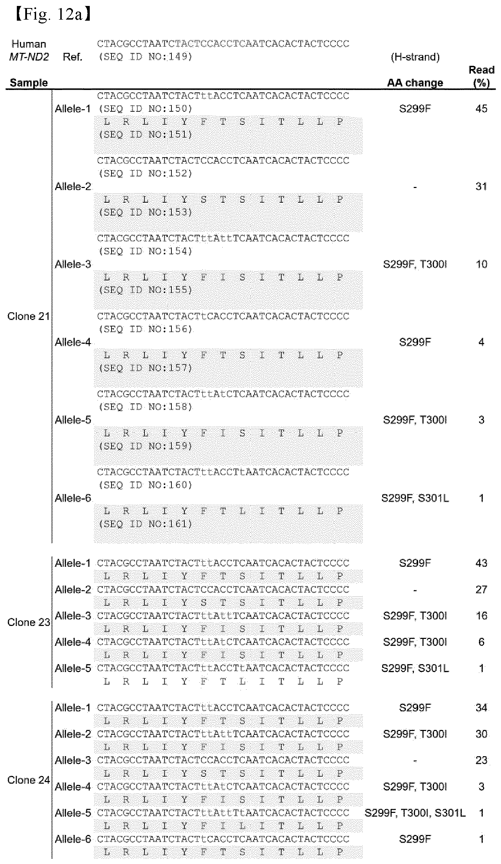

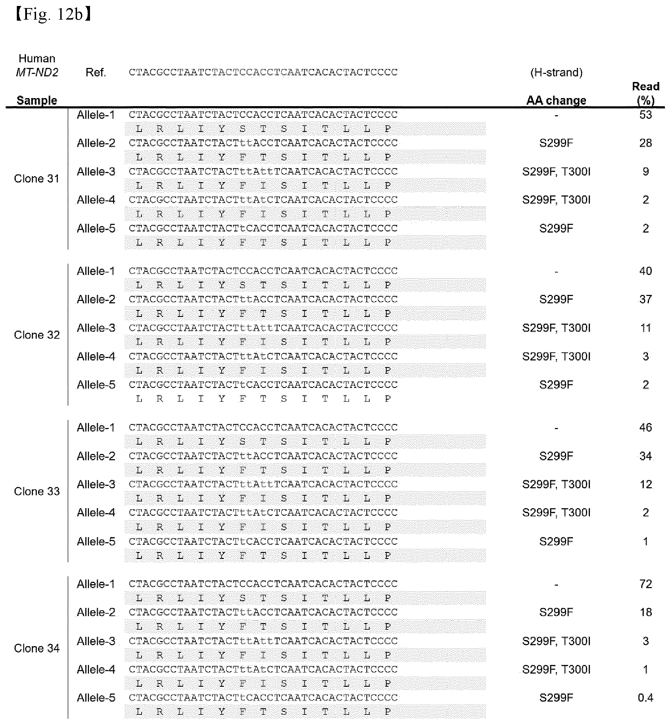

FIGs. 12a and 12b show results confirming the base editing efficiencies of single-cell-derived clonal populations isolated from HEK293T cells treated with MT-ZFD, in which there is provided allele analysis of single-cell-derived clones with high base editing frequency, the table shows amino acids changed by base editing in ND2, and in the top reference sequence, the red letter indicates a spacer, and in alleles, the red letter indicates a change in the amino acid sequence;

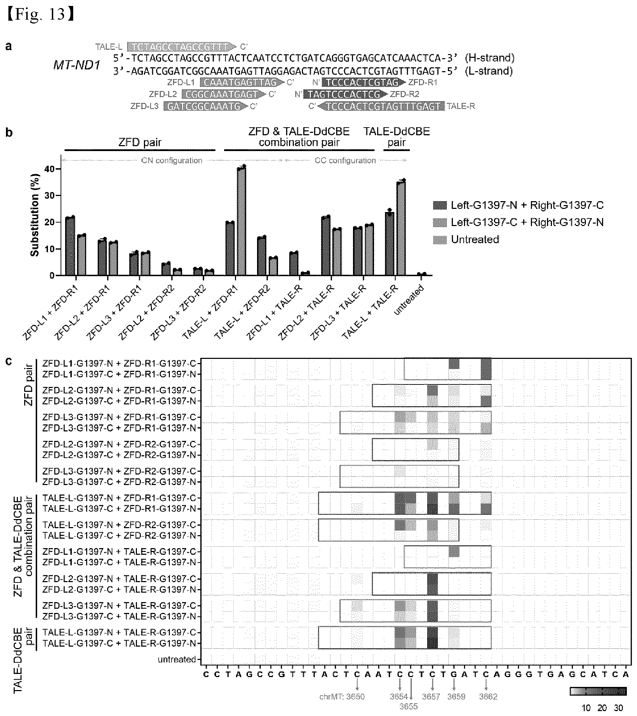

FIG. 13 shows results confirming the base editing efficiency by a combination of ZFD and TALE-DdCBE,
FIG. 13a showing DNA sequences of the binding regions of mitoZFD and TALE-DdCBE pairs, in which sites recognized by TALE-DdCBE are highlighted in green and for mitoZFD in blue, and the upper sequence represents the mtDNA heavy strand and the lower sequence represents the mtDNA light strand, FIG. 13b showing frequencies of cytosines edited by ZFD, TALE-DdCBE, and ZFD/DdCBE hybrid pairs, data being obtained using targeted deep sequencing, and data being represented as mean ± standard error of the mean (s.e.m.) from n = 2 biologically independent samples, and FIG. 13c showing a heat map of base editing activity at each base position, the red box representing the spacer region for each configuration, and the blue arrow representing the position of mtDNA;

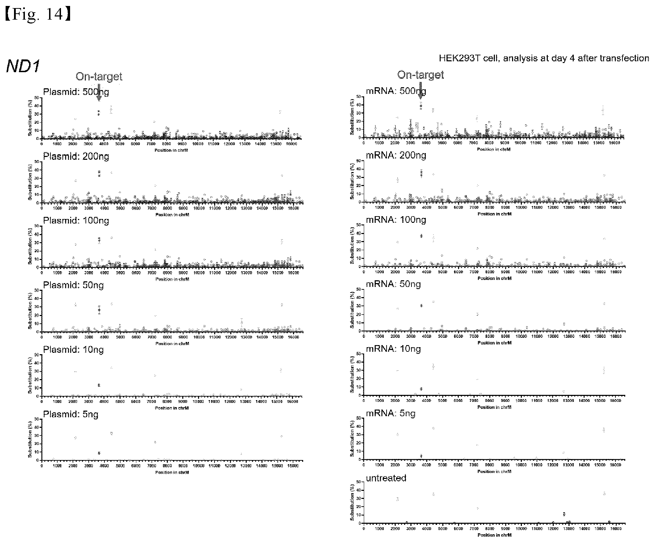

FIG. 14 shows results confirming the editing efficiencies depending on mRNA vs plasmid at different ZFD concentrations, in which mitochondrial genome-wide targeting specificity of ND1-targeted mitoZFD has specificity varying depending on the concentration of ZFD-encoding mRNA or plasmid, on- and off-target base editing frequencies determined by whole mtDNA sequencing are shown, results of HEK 293T cells transfected with the indicated concentrations of ND1-targeted mitoZFD-encoding plasmid or mRNA are plotted as dots in the graphs, the red arrow represents the target site, the red dot represents the base editing frequency of the target site, and the gray dot represents the SNP that is also present in the control, data being represented as mean ± standard error of the mean (s.e.m.) from n = 2 biologically independent samples;

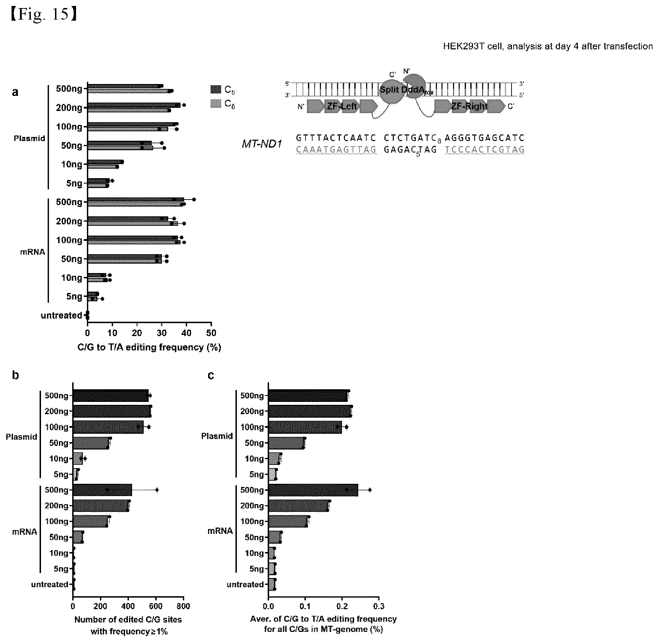

FIG. 15 shows results confirming the editing efficiencies depending on mRNA vs plasmid at different ZFD concentrations, FIG. 15a showing ZFD binding at the ND1 site on the top, in which the ZFD binding site is represented in green, target cytosine within the spacer is represented in red, and on-target activity determined from whole mtDNA sequencing data in FIG. 14 is shown, the activity decreasing with a decrease in the amount of plasmid or mRNA encoding transfected mitoZFD, FIG. 15b showing the number of C/G sites edited with a frequency of >1% for each plasmid or mRNA amount, and FIG. 15c showing average C/G-to-T/A editing frequency for all C/Gs in the mitochondrial genome depending on each plasmid or mRNA concentration, data being represented as mean ± standard error of the mean (s.e.m.) from n = 2 biologically independent samples;

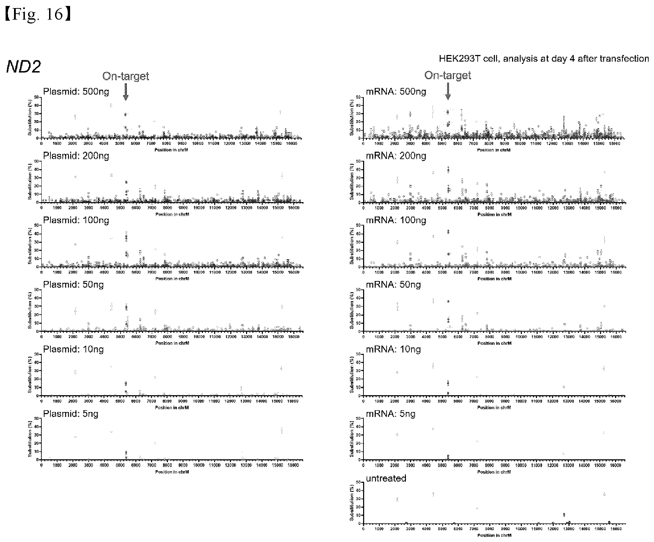

FIG. 16 shows results confirming the editing efficiencies depending on mRNA vs plasmid at different ZFD concentrations, in which on- and off-target base editing frequencies determined by whole mtDNA sequencing are shown, results of HEK 293T cells transfected with the indicated concentrations of ND2-targeted mitoZFD-encoding plasmid or mRNA are plotted as dots in graphs, the red arrow represents the target site, the red dot represents the base editing frequency of the target site, and the gray dot represents the SNP that is also present in the control, data being represented as mean ± standard error of the mean (s.e.m.) from n = 2 biologically independent samples;

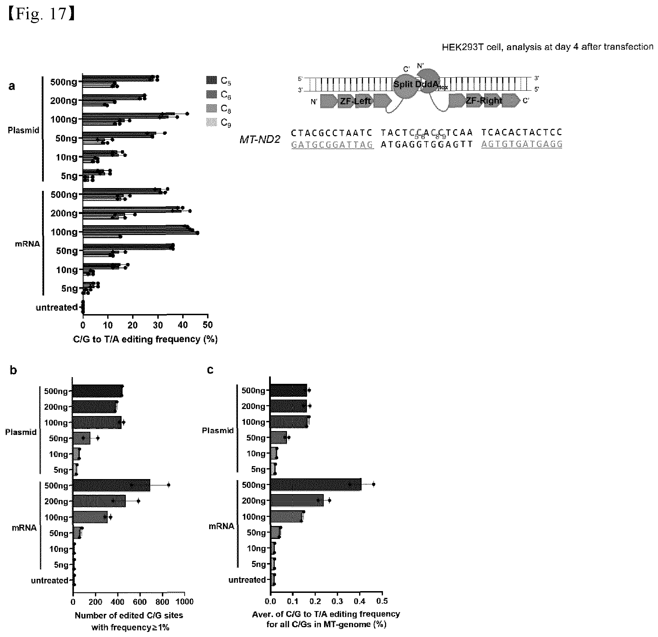

FIG. 17 shows results confirming the editing efficiencies depending on mRNA vs plasmid at different ZFD concentrations,
FIG. 17a showing ZFD binding at the ND2 site on the top, in which the ZFD binding site is represented in green, target cytosine within the spacer is represented in red, on-target activity determined from whole mtDNA sequencing data in FIG. 16 is shown, and the activity decreases with a decrease in the amount of plasmid or mRNA encoding transfected mitoZFD, FIG. 17b showing the number of C/G sites subjected to base editing with a frequency of >1% for each plasmid or mRNA amount, FIG. 17c showing average C/G-to-T/A editing frequency for all C/Gs in the mitochondrial genome depending on each plasmid or mRNA concentration, data being represented as mean ± standard error of the mean (s.e.m.) from n = 2 biologically independent samples;

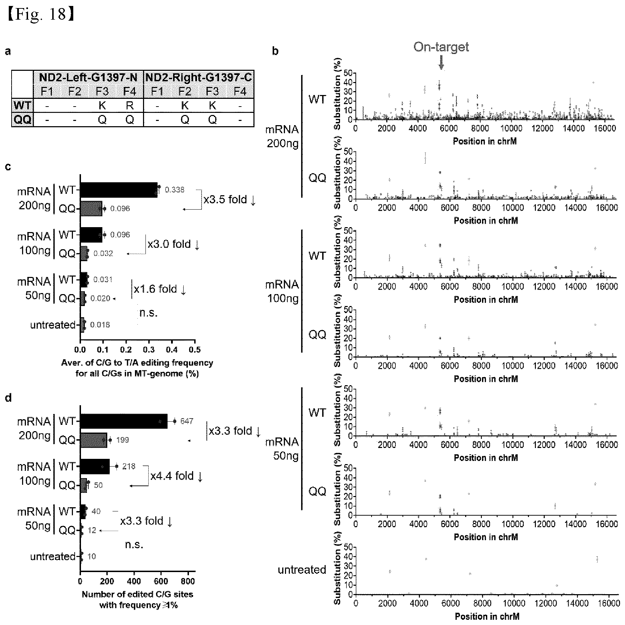

FIG. 18 shows results confirming the editing efficiency after constructing the whole mitochondrial sequencing/QQ variant,
FIG. 18a showing the QQ mitoZFD variant that includes R(-5)Q mutations in each zinc finger of ZFD to eliminate non-specific DNA contacts (if there is no R at position -5 of the zinc finger framework, nearby K or R is converted to Q), FIG. 18b showing whole mtDNA sequencing of mitoZFD-treated cells, in which the on- and off-site editing frequencies are represented as red and black dots, respectively, data being represented as mean ± standard error of the mean (s.e.m.) from n = 2 biologically independent samples, all base edits from C/G to T/A with an efficiency of >1% are shown, and FIGs. 18c and 18d showing editing efficiency and specificity varying depending on the capacity of ZFD-encoding mRNA delivered, FIG. 18c showing average C/G-to-T/A editing frequency for all C/Gs in the mitochondrial genome and FIG. 18d showing the number of C/Gs edited at a base editing frequency of >1%;

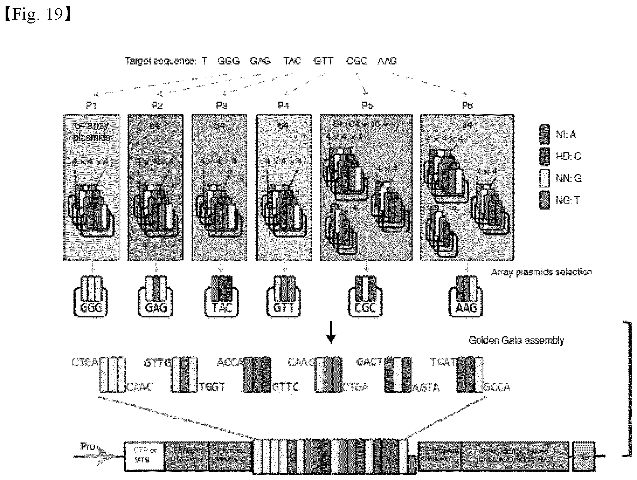

FIG. 19 shows a Golden Gate assembly system of base editors in plants and schematically shows the Golden Gate assembly for cp-DdCBE and mt-DdCBE constructs, in which for each position in a target sequence, a TALE subarray plasmid was selected from among a total set of 424 sequences (=6x64 tripartite + 2x16 bipartite + 2x4 monopartite) and mixed with a desired vector to obtain a plasmid encoding a DdCBE targeting a specific sequence;

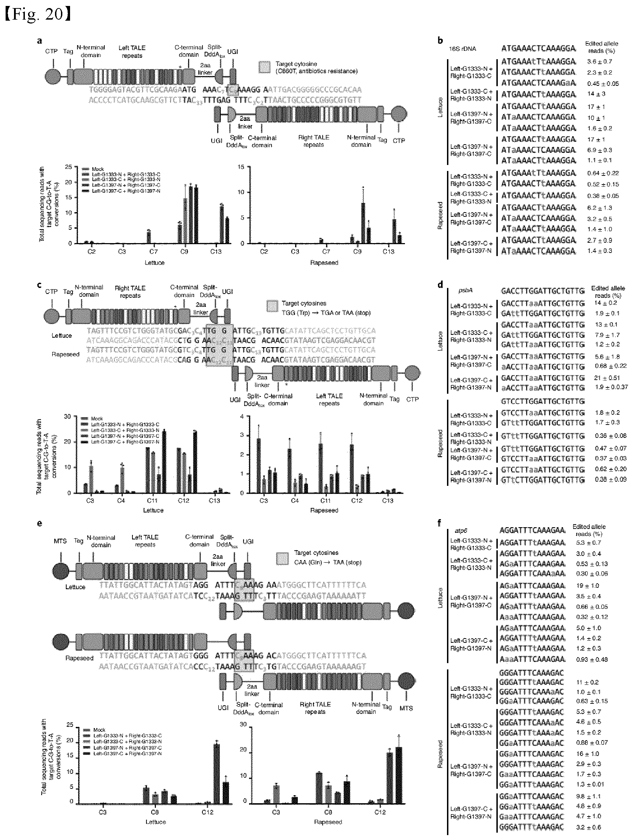

FIG. 20 shows plant chloroplast and mitochondrial base editing, FIGs. 20a, 20b, 20c, and 20d showing frequencies and patterns of chloroplast base editing induced by cp-DdCBE in 16s rDNA (a, b) and psbA (c, d), in which split DdCBE G1333 and G1397 pairs were transfected into lettuce and rapeseed protoplasts, FIG. 20e and 20f showing efficiencies and patterns of mitochondrial base editing induced by mt-DdCBE in the ATP6 gene, in which split DdCBE G1333 and G1397 pairs were transfected into lettuce and rapeseed protoplasts, in FIGs. 20a, 20c, and 20e, the TALE-binding region is represented in blue and the cytosine in the spacer is represented in orange, error bars in all graphs representing mean ± standard deviation of three independent biological replicates, and in FIGs. 20b, 20d, and 20f, the converted nucleotide is represented in red, edited alleles % (mean ± standard deviation) being obtained from three independent experiments;

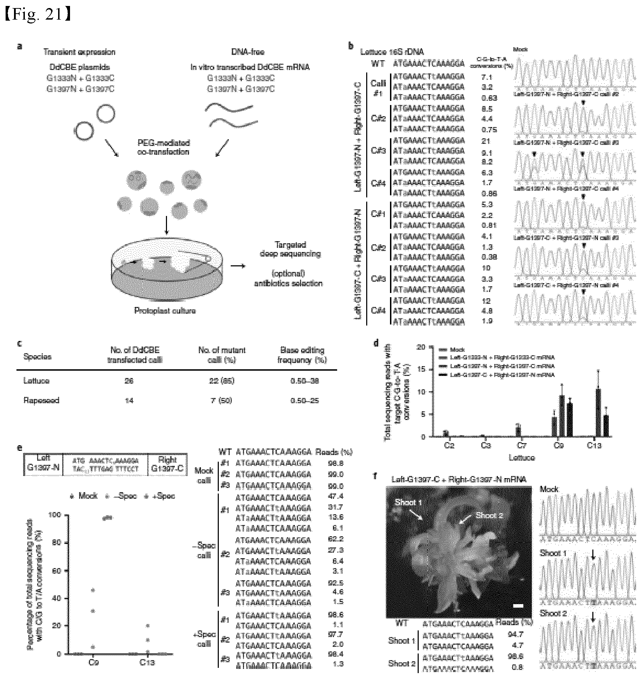

FIG. 21 shows plant organelle DNA editing via DdCBE, FIG. 21a schematically showing plant organelle mutagenesis, FIG. 21b showing C·G-to-T·A conversion efficiency in cultured cp-DdCBE transfected calli in the absence of spectinomycin, including representative Sanger sequencing chromatograms, in which the converted nucleotide is represented in red on the left, and the arrow represents the substituted nucleotide in the chromatograms, FIG. 21c showing DdCBE-driven plant organelle mutagenesis, in which the mutant calli appear to have much higher editing frequency than the frequency in simulated calli, FIG. 21d showing C-to-T conversion frequencies induced after transfection of cp-DdCBE-encoding mRNA targeting 16srDNA into lettuce protoplasts, error bars being mean ± s.d. of n = 3 independent biological replicates, FIG. 21e showing editing frequencies and patterns of spectinomycin-resistant calli at 2.5 months, FIG. 21f showing C·G-to-T·A conversion efficiencies in streptomycin-resistant plants transfected with DdCBE mRNA using representative Sanger sequencing chromatograms, the arrow representing the substituted nucleotide in the chromatograms, scale bar: 1 mm;

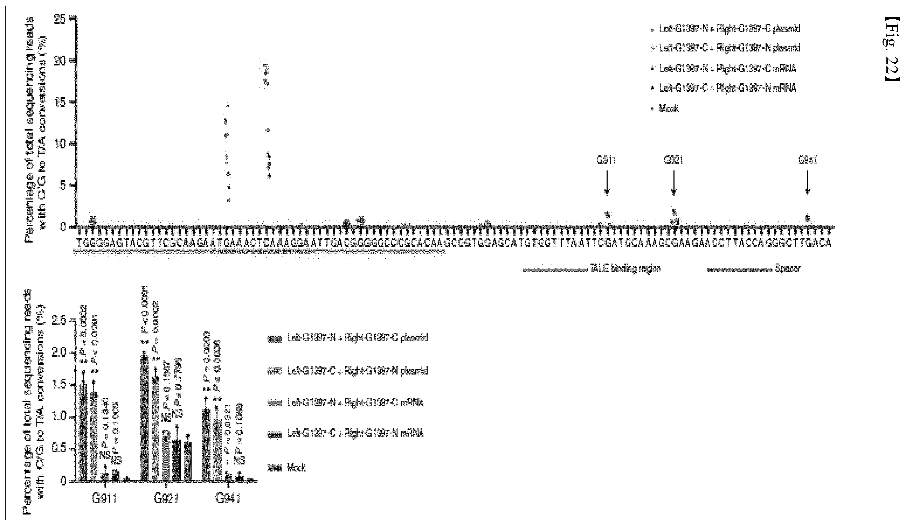

FIG. 22 shows comparison of off-target activity in the vicinity of the target site in DdCBE plasmid-transfected or DdCBE mRNA-transfected lettuce protoplasts. Plasmids or mRNAs encoding the cp-DdCBE pair targeted to the chloroplast 16S rRNA gene were transfected into lettuce protoplasts. Off-target TC-to-TT edits were detected in the immediate proximity of the target site. Editing efficiencies were measured by targeted deep sequencing seven days post-transfection. Frequencies (mean ± s.d.) were obtained from three independent experiments. Student's unpaired two-tailed *t*-test was applied. ^{∗∗}*P* < 0.01; ^{∗}*P* < 0.05; NS, not significant (*P* > 0.05);

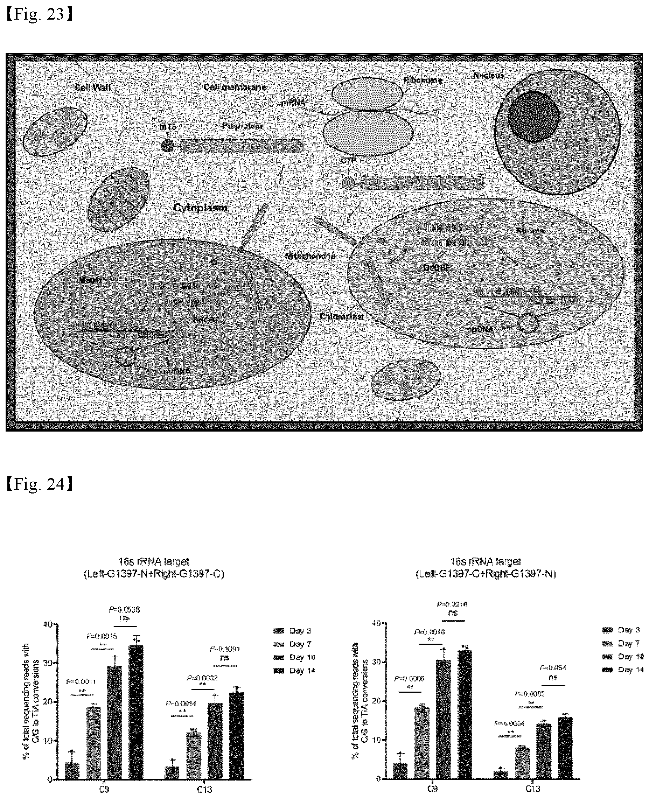

FIG. 23 shows a chloroplast and mitochondrial base editing strategy, in which each of the cp-DdCBE and mt-DdCBE preproteins contains a chloroplast transit peptide (CTP) or a mitochondrial targeting signal (MTS) and is thus translated in plant cells and then transported to chloroplasts and mitochondria, and the preproteins pass through the outer and inner membranes of organelles, CTP and MTS are cleaved by interstitial processing peptidase and mitochondrial processing peptidase, respectively, and then cp-DdCBE and mt-DdCBE (mature protein) form the final conformation;
FIG. 24 shows editing via DdCBE plasmids in lettuce protoplasts over time, in which transfected protoplasts were collected at each time point and editing efficiencies were analyzed by targeted deep sequencing, frequencies (mean ± s.d.) being obtained from three independent experiments;

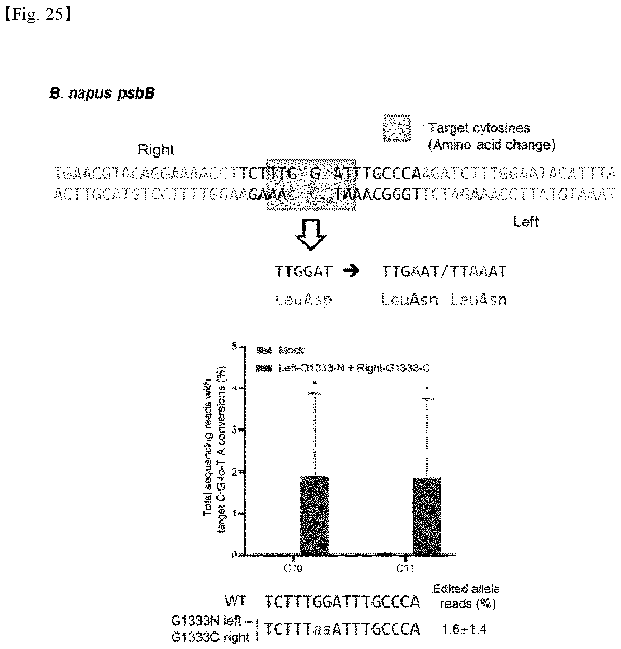

FIG. 25 shows base editing frequency of a psbB gene, in which plasmids encoding the cp-DdCBE pair left-G1333-N + right-G1333-C targeting the chloroplast psbB gene were transfected into rapeseed protoplasts, and then the base editing efficiency in the spacer was analyzed by targeted deep sequencing, the TALE binding region, target cytosine, and converted nucleotide being represented in blue, orange, and red, respectively, and frequencies (mean ± standard deviation) being calculated from n = 3 independent experiments;

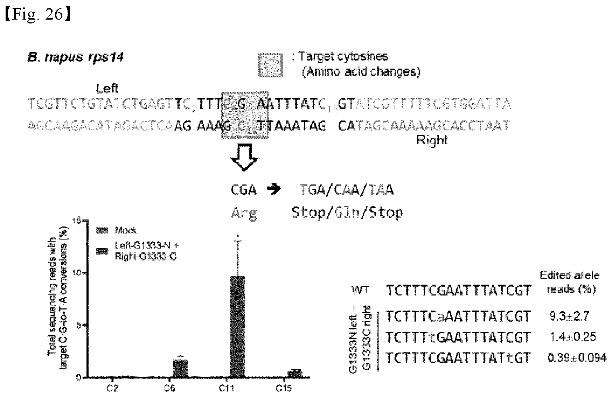

FIG. 26 shows base editing efficiency of a mitochondrial RPS14 gene, in which plasmids encoding the mt-DdCBE pair left-G1333-N + right-G1333-C targeting the RPS14 gene were transfected into rapeseed protoplasts, and then C-to-T conversion efficiencies were analyzed by targeted deep sequencing, the TALE binding region, target cytosine, and converted nucleotide being represented in blue, orange, and red, respectively, and frequencies (mean ± standard deviation) being calculated from n = 3 independent experiments;

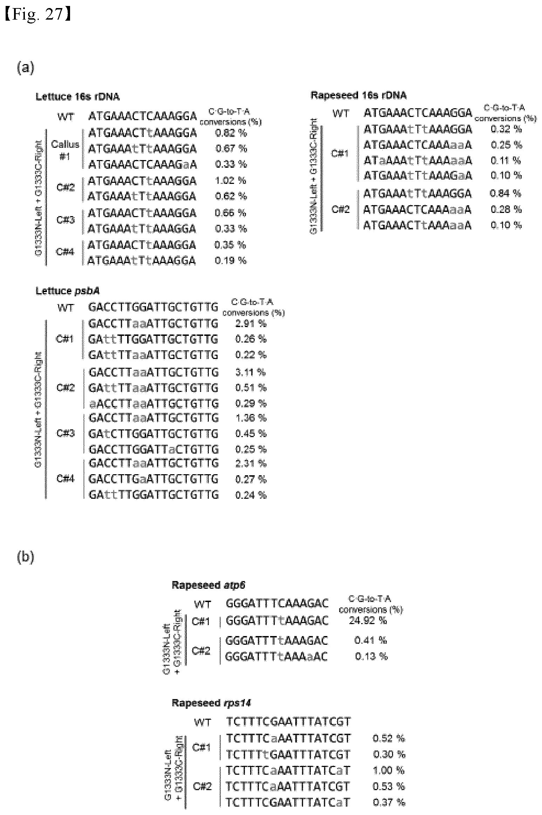

FIG. 27a shows base editing efficiencies targeting the chloroplast genome in calli, with base editing frequencies and patterns by DdCBE at the target sites of 16srDNA and psbA in lettuce and rapeseed calli after 4 weeks of culture, the converted nucleotide in the spacer being represented in red; FIG. 27b shows base editing efficiencies targeting the mitochondrial genome in calli, in which base editing frequencies and patterns by DdCBE at the target sites of ATP6 and RPS14 genes in rapeseed calli were confirmed by targeted deep sequencing, the converted nucleotide in the target spacer being represented in red;

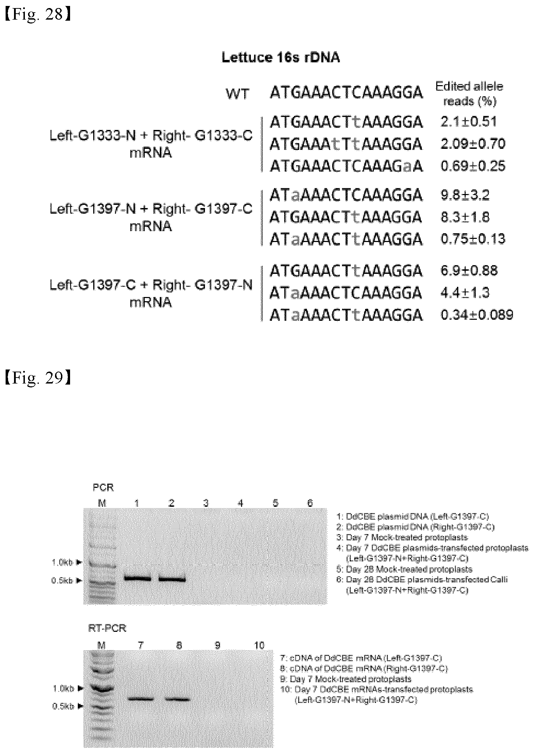

FIG. 28 shows DNA-free base editing, with chloroplast base editing frequencies and patterns at the target site of 16srDNA after transfection of DdCBE mRNA into lettuce protoplasts, in which the protoplasts were cultured for 7 days and then targeted deep sequencing was performed, the converted nucleotide in the spacer of interest being represented in red;
FIG. 29 shows results of gel electrophoresis showing the absence of DdCBE mRNA or DNA sequences in protoplasts and calli (in which M is a marker);

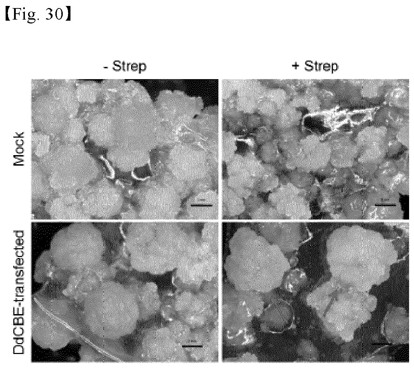

FIG. 30 shows 16srDNA mutation screening, in which the red arrow represents streptomycin-resistant green callus;

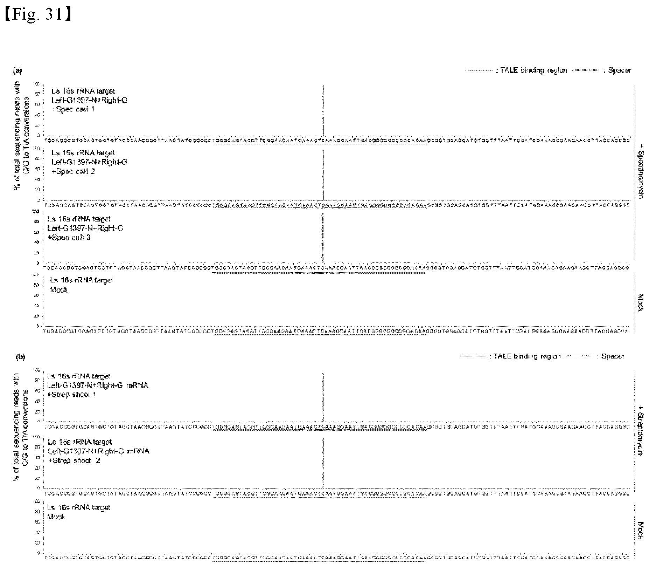

FIG. 31 shows no off-target mutations near the DdCBE target site in antibiotic-resistant calli or shoots, FIGs. 31(a) and 31(b) showing off-target activities analyzed by target deep sequencing, in which the TALE binding site and spacer region are underlined in green and red, respectively, FIG. 31(a) showing spectinomycin-resistant calli resulting from culture of lettuce protoplasts transfected with DdCBE plasmids, and FIG. 31(b) showing shoots obtained from streptomycin-resistant shoots;

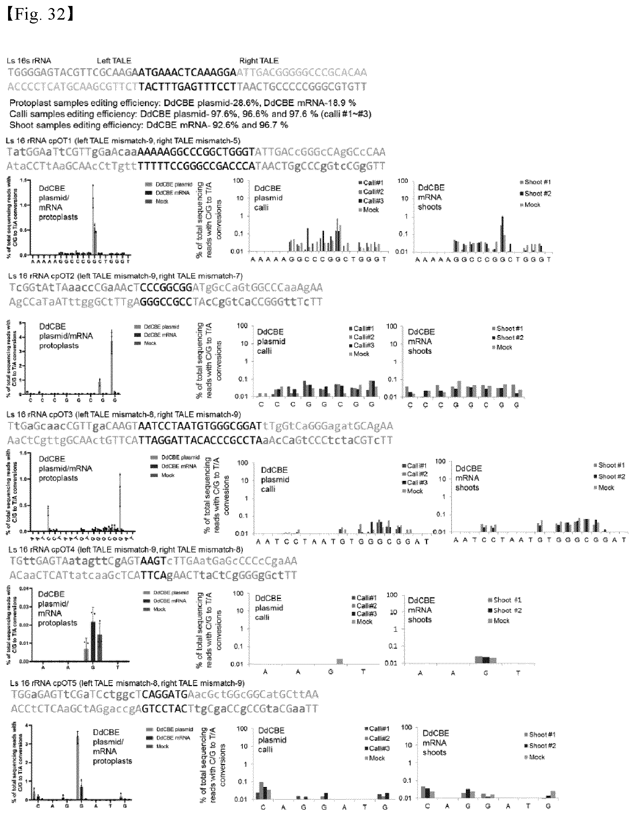

FIG. 32 shows results of analysis of off-target activity at the five most homologous sites with the on-target site, in which the top five candidate off-target sites of the 16s rRNA gene-specific DdCBE in the lettuce chloroplast genome were selected, including a maximum of nine mismatches in the TALE binding site, the TALE binding sequence and mismatched nucleotide are represented in blue and red, respectively, and off-target mutation frequencies were measured in protoplasts and drug-resistant calli or shoots transfected with DdCBE plasmids or DdCBE mRNAs using targeted deep sequencing, frequencies (mean ± standard deviation) being obtained from three independent experiments;

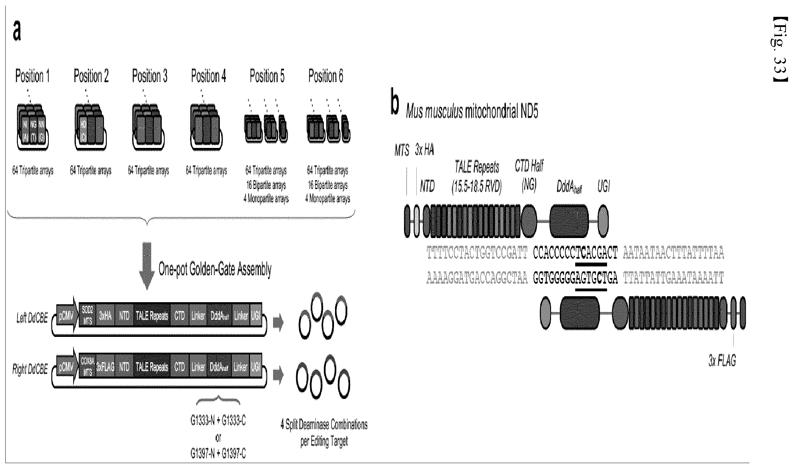

FIG. 33 schematically shows DdCBE assembly and mitochondrial DNA editing, FIG. 33a showing one-pot Golden Gate assembly for efficient DdCBE construction, in which a total of 424 sequences (64 tripartite arrays x 6 + 16 bipartite arrays x 2 + 4 monopartite arrays x 2) and expression vectors were mixed to construct the left and right modules for final plasmid construction, and FIG. 33b schematically showing interactions between the DdCBE and the target gene ND5 in mouse mitochondrial DNA, in which the TALE binding site is represented in gray, the base editing site is represented in black, and respective repeat variable diresidue modules are represented in orange, blue, green, and yellow: "NI", "NG", "NN", and "HD" for recogniztion of adenine, thymine, guanine, and cytosine, respectively;

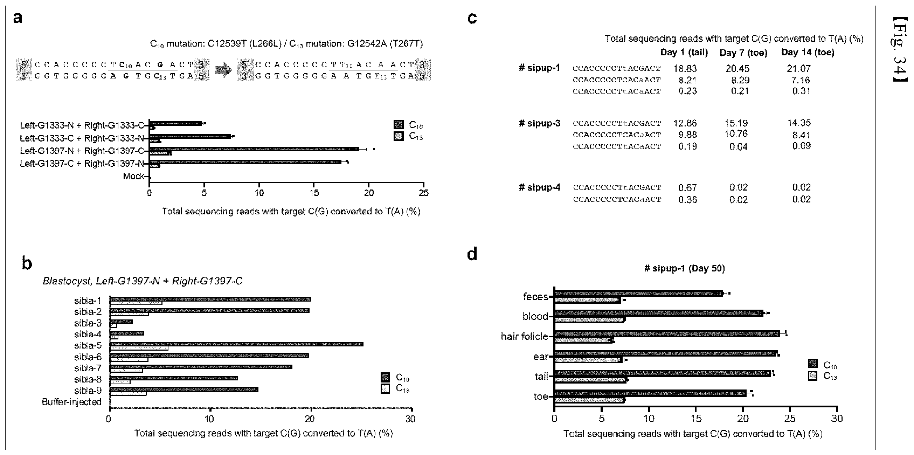

FIG. 34 shows mouse mitochondrial ND5 point mutations caused by base editing with DdCBE, FIG. 34a showing efficiencies in DdCBE deaminase-mediated cytosine-to-thymine base editing target sequences and in NIH3T3 cells, in which the translational codon in the target sequence is underlined, the editable site is represented in red, combinations for DdCBE transfection are represented as left or right, -G1333 or -G1397, and -N or -C, P values for C10 mutations of left-G1333-N + right-G1333-C, left-G1333-C + right-G1333-N, left-G1397-N + right-G1397-C, and left-G1397-C + right-G1397-N are 0.0012, 0.0003, 0.0014, and 0.0009, respectively, and p values for C13 mutations are 0.0116, 0.0076, 0.0030, and 0.0003, respectively (*p < 0.05 and **p < 0.01, Student's two-tailed t test), FIG. 34b showing base editing efficiencies in mouse blastocysts, in which sequencing data were obtained from blastocysts developed from zygotes microinjected with left-G1397-N and right-G1397-C DdCBE mRNA, FIG. 34c showing alignment of mutant sequences of newborn pups, in which targeted deep sequencing was performed by extracting genomic DNA from tissues obtained from the tails immediately after birth and the toes at 7 and 14 days after birth, the edited base is represented in red, and the frequencies of editing of mutant mitochondrial genomes are indicated, and FIG. 34d showing editing efficiencies in various tissues of adult F0 mice (sipup-1), in which sequencing data were obtained from each tissue at 50 days after birth, and in all graphs, dark and light gray bars represent respective editing frequencies of m.C12539T (C10) and m.G12542A (C13) mutations, error bars being standard error of the mean (s.e.m.) for n = 3 biologically independent samples;

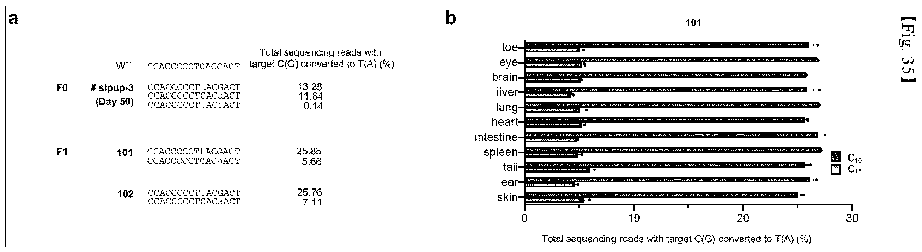

FIG. 35 shows transmission of mutant mitochondrial DNA into germ cells, FIG. 35a showing results of targeted deep sequencing performed after obtaining F1 offspring (101, 102) by crossing female F0 (sipup-3) mice with wild-type C57BL6/J males to observe germline transmission of mtDNA mutations, in which the edited base is represented in red, and the editing frequencies of mutant mitochondrial genomes are indicated, and FIG. 35b showing base editing efficiencies in various tissues of F1 pups (101) obtained using targeted deep sequencing of genomic DNA, in which dark and light gray bars represent respective frequencies of m.C12539T (C10) and m.G12542A (C13) mutations, error bars being standard error of the mean (s.e.m.) for n = 3 biologically independent samples;

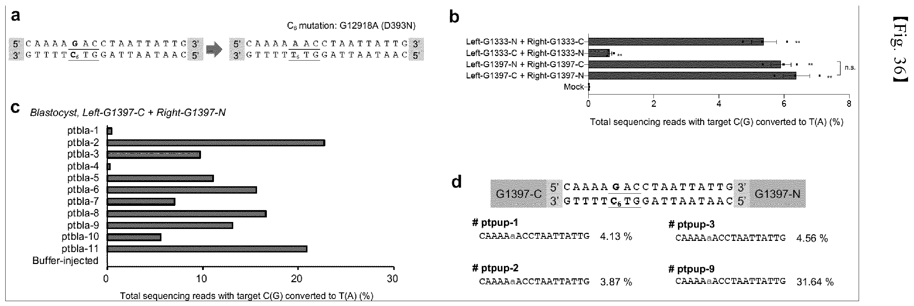

FIG. 36 shows mouse mitochondrial ND5 G12918A mutations caused by DdCBE, FIG. 36a showing a DdCBE target to make the m.G12918A point mutation causing a D393N change in the ND5 protein, in which the target codon is underlined and the editable site is represented in red, FIG. 36b showing cytosine-thymine base editing efficiencies using DdCBE in NIH3T3 cells, in which combinations of transfected DdCBE pairs are indicated, error bars are s.e.m of n = 3 biologically independent samples (n.s.: not significant, *p < 0.05, **p < 0.01 using Student's two-tailed t test), P values for C6 mutations of left-G1333-N + right-G1333-C, left-G1333-C + right-G1333-N, left-G1397-N + right-G1397-C, and left-G1397-C + right-G1397-N are 0.0052, 0.0099, 0.0027, and 0.0040, respectively, and the P value for n.s is 0.4971, FIG. 36c showing point mutation base editing efficiencies in m.G12918A mouse blastocysts, in which sequencing data were obtained from blastocysts developed by microinjecting mRNA encoding left-G1397-C and right-G1397-N DdCBE into 1-cell stage embryos and then culturing the same, and FIG. 36d showing mice with ND5 point mutations (F0), in which F0 pups with ND5 point mutations developed after microinjection of DdCBE mRNA and mutant sequence arrays identified in newborn pups are shown, the edited base is represented in red, and the editing frequencies of mutant mitochondrial genes are indicated on the right;

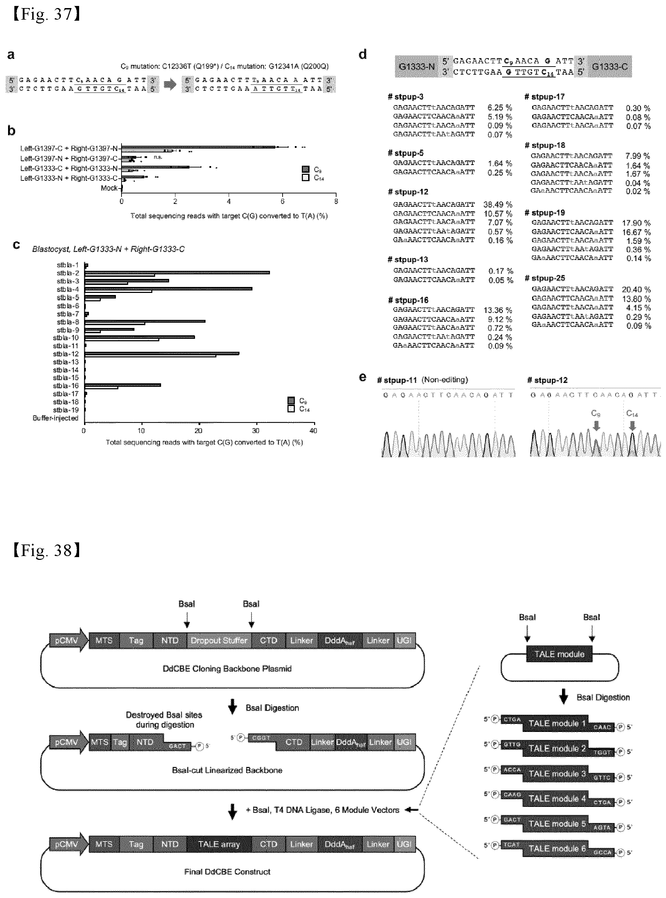

FIG. 37 shows mouse mitochondrial ND5 nonsense mutations generated through cytosine deaminase-mediated base editing, FIG. 37a showing DdCBE target sequences to generate m.C12336T nonsense mutations and m.G12341A silent mutations, in which the m.C12336T (C9) mutations create Q199stop mutations in the ND5 protein, whereas the m.G12341A (C14) causes silent Q200Q mutations, the transcription triplet is underlined, and the editable site is represented in red, FIG. 37b showing cytosine-thymine base editing efficiencies to generate nonsense mutations in NIH3T3 cells, in which combinations of transfected DdCBE pairs are indicated, dark and light gray bars represent respective frequencies of m.C12336T (C9) and m.G12341A (C14) mutations, error bars indicate s.e.m. of n = 3 biologically independent samples (n.s.: not significant, *p < 0.05, **p < 0.01 using Student's two-tailed t test), P values for C9 mutations of left-G1333-N + right-G1333-C, left-G1333-C + right-G1333-N, left-G1397-N + right-G1397-C, and left-G1397-C + right-G1397-N were 0.0065, 0.1143, 0.0266, and 0.0037, and respective P values for C14 mutations were 0.0077, 0.0144, 0.0406, and 0.0214, FIG. 37c showing editing efficiencies in mouse blastocysts, in which sequencing data were obtained from blastocysts developed after microinjection of zygotes with mRNA encoding left-G1333-N and right-G1333-C DdCBE, and dark and light gray bars represent the frequencies of C9 and C14 mutations, respectively, FIG. 37d showing mutant sequence arrays of newborn pups, in which the edited base is represented in red and the editing frequencies of mutant mitochondrial genomes are indicated on the right, and FIG. 37e showing Sanger sequencing chromatograms of wild-type and edited mice, the red arrow representing the substituted nucleotide;
FIG. 38 schematically shows Golden Gate cloning to create a DdCBE construct, in which all reactions occur simultaneously in one tube, the arrow does not indicate continuous reaction, the empty expression vector and the module vector were cleaved using a BsaI enzyme to eliminate the linearized backbone and TALE module insert, including compatible cohesive ends, the backbone and the six module inserts were ligated by a T4 DNA ligase to create a final DdCBE construct, eight DdCBE cloning backbone plasmids were used, and for SOD2MTS, there are provided left-G1333-N, left-G1333-C, left-G1397-N, and left-G1397-C, and for COX8A MTS, there are provided right-G1333-N, right-G1333-C, right-G1397-N, and right-G1397-C;

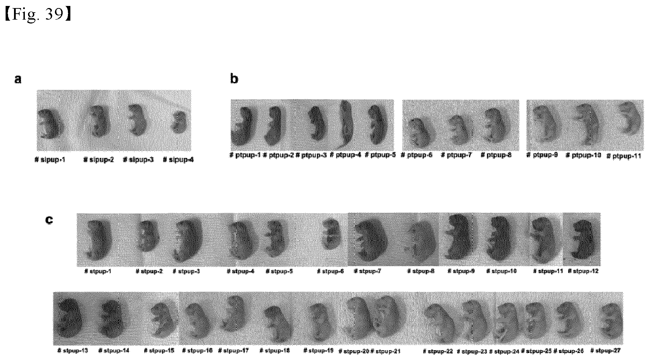

FIG. 39 shows ND5 mutant mice (F0), FIG. 39a showing ND5 silent mutant mice, FIG. 39b showing ND5 G12918A mutant mice, and FIG. 39c showing ND5 nonsense mutant mice resulting from DdCBEmRNA microinjection;

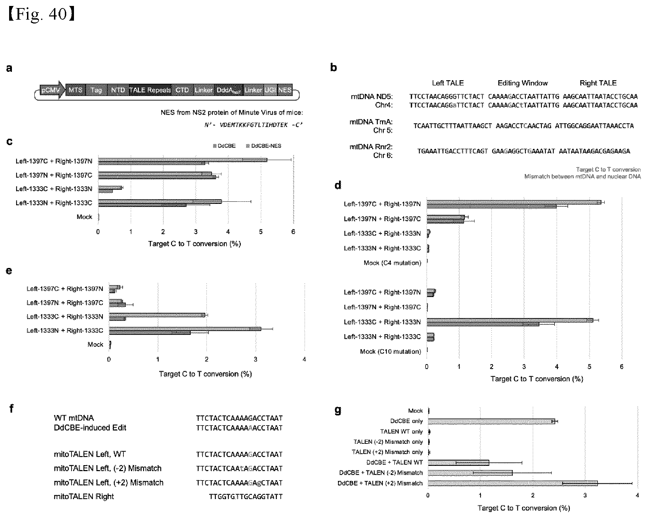

FIG. 40: FIG. 40a schematically shows a vector containing DdCBE-NES and an NES sequence, FIG. 40b shows a sequence of a mouse m.G12918 ND5 gene and a ND5-like gene in chromosome 4 in the nucleus, a sequence of mitochondrial TrnA and chromosome 5 in the nucleus, and a sequence of mitochondrial Rnr2 and chromosome 6 in the nucleus, FIG. 40c shows editing efficiencies in the ND5 gene by DdCBE and DdCBE-NES using a NIH3T3 cell line, FIG. 40d shows editing efficiencies in the TrnA gene by DdCBE and DdCBE-NES using a NIH3T3 cell line, FIG. 40e shows editing efficiencies in the Rnr2 gene by DdCBE and DdCBE-NES using a NIH3T3 cell line, in which the orange graph shows the editing efficiency of DdCBE, and the gray graph shows the editing efficiency of DdCBE-NES, FIG. 40f shows DNA recognition sequences of mitoTALEN TALE arrays, and FIG. 40g shows DdCBE base editing efficiencies in experimental groups treated with mitoTALEN or not treated therewith, all graphs being n=2 and error bars being standard error of the mean;

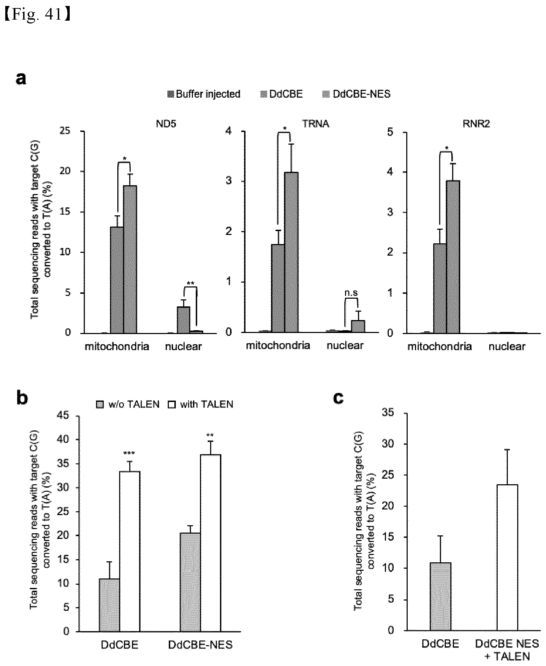

FIG. 41 shows improved editing efficiencies in mouse embryos and mice using DdCBE-NES and mitoTALEN, FIG. 41a showing base editing efficiencies of various mitochondrial DNA targets (mtND5, mtTrnA, and mtRNR2) in blastocysts using DdCBE and DdCBE-NES, FIG. 41b showing comparison of m.G12918A base editing efficiencies using DdCBE and DdCBE-NES with or without mitoTALEN, and FIG. 41c showing comparison of m.G12918A base editing efficiencies in mice, all graphs being n>=3 and error bars being standard error of the mean (n.s.: not significant, *p<0.05, **p<0.01, ***p<0.001 obtained using Student's two-tailed t-test);

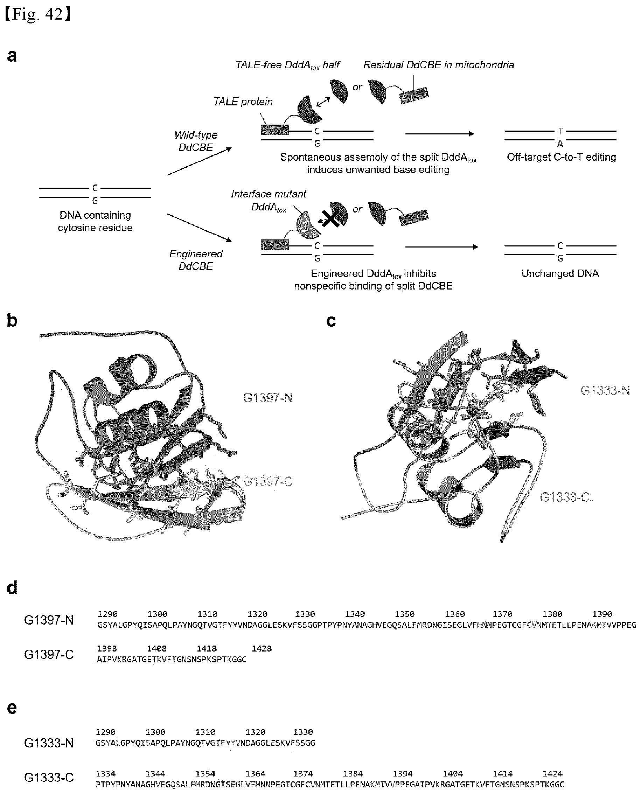

FIG. 42: FIG. 42a schematically shows an improvement in a DdCBE protein, FIGs. 42b and 42c show crystal structures of DddAtox deaminases, in which the residue at the split dimer interface is represented as a stick, FIG. 42b showing the G1397-N split and the G1397-C split represented in purple and light blue, respectively, FIG. 42c showing the G1333-N split and the G1333-C split represented in orange and green, respectively, and FIGs. 42d and 42e show amino acid sequences of G1397-N and G1397-C (d) and G1333-N and G1333-C (e), and interface residues represented in red;

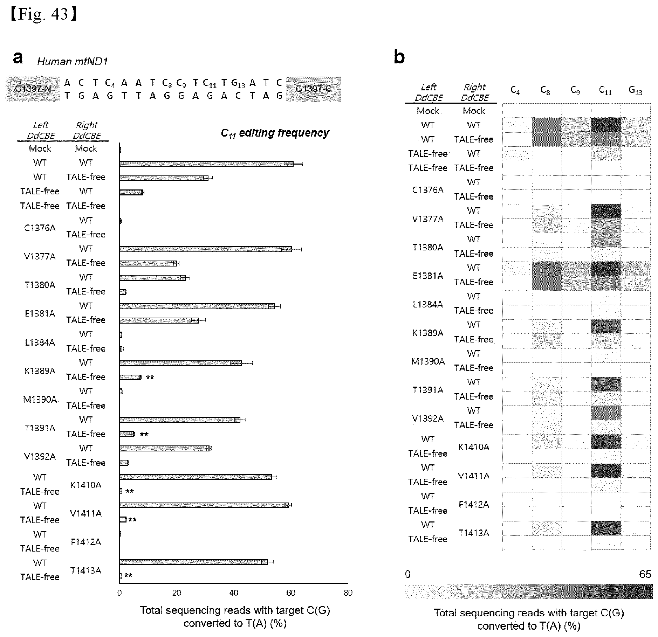

FIG. 43: FIG. 43a shows a graph of base editing efficiencies of G1397 interface mutants, in which editing ranges and target cytosines are represented on the top, and mutant and wild-type/TALE-free DddAtox proteins were co-transfected as indicated next thereto, and for left-DdCBE, the TALE-free DddAtox protein is G1397-N, and for right-DdCBE, the TALE-free DddAtox protein is G1397-C, and FIG. 43b shows a heat map with the target cytosine-thymine (guanine-adenine) base editing efficiencies of DdCBE and mutants;

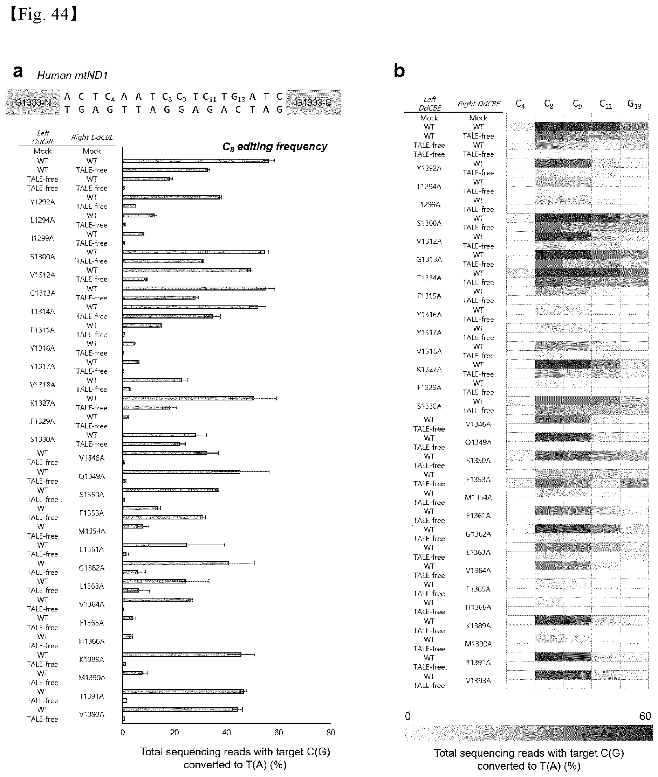

FIG. 44: FIG. 44a shows a graph of base editing efficiencies of G1333 interface mutants, in which editing ranges and target cytosines are represented on the top, and mutant and wild-type/TALE-free DddAtox proteins were co-transfected as indicated next thereto, and for left-DdCBE, the TALE-free DddAtox protein is G1333-N, and for right-DdCBE, the TALE-free DddAtox protein is G1333-C, and FIG. 44b shows a heat map with the target cytosine-thymine (guanine-adenine) base editing efficiencies of DdCBE and mutants;

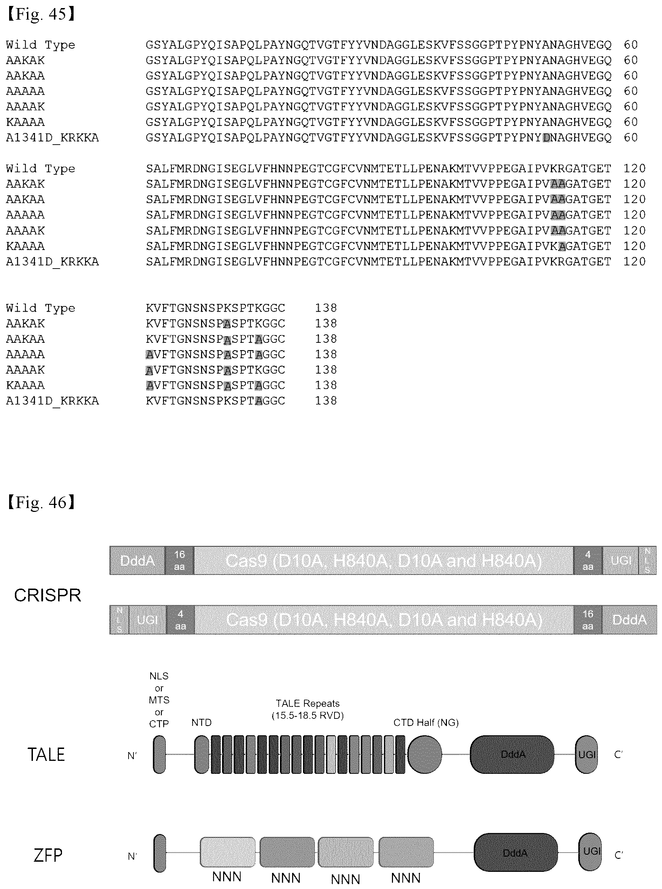

FIG. 45 shows results of comparing the amino acid sequences of wild-type and novel full-length DddAs;
FIG. 46 shows conformations in which full-length DddA is delivered to animal or plant cells;

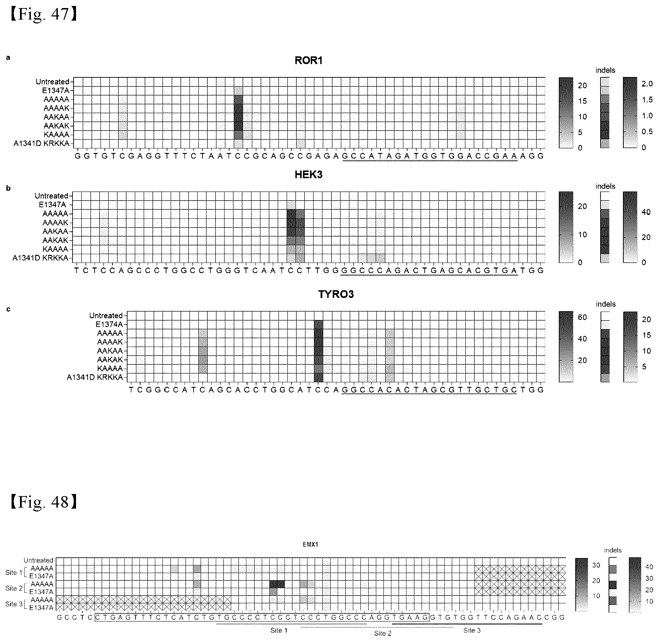

FIG. 47 shows results confirming the activities of cytosine-to-thymine conversions in TC motifs in the human cell genomic context ROR1 site (a), HEK3 site (b), and TYRO3 site (c);
FIG. 48 shows advantages of full-length DddA;

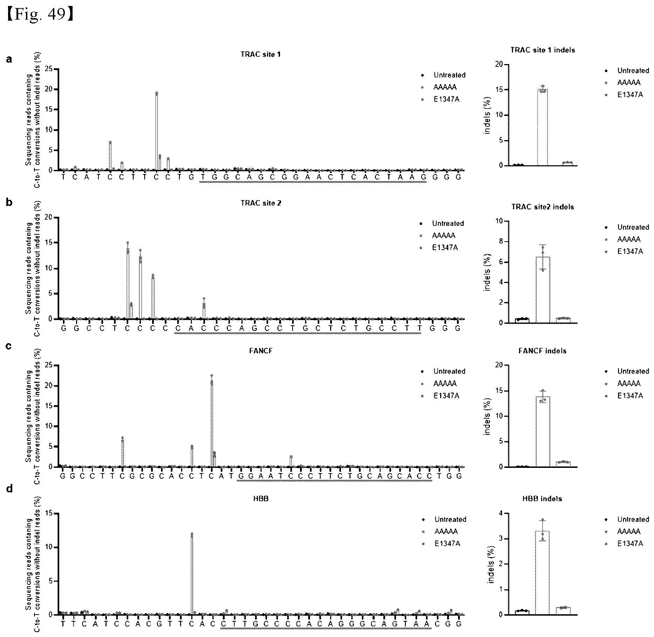

FIG. 49 shows results of measuring the activity of full-length DddA in the human cell genomic context TRAC site 1 (a), TRAC site 2 (b), FANCF (c), and HBB (d) ;

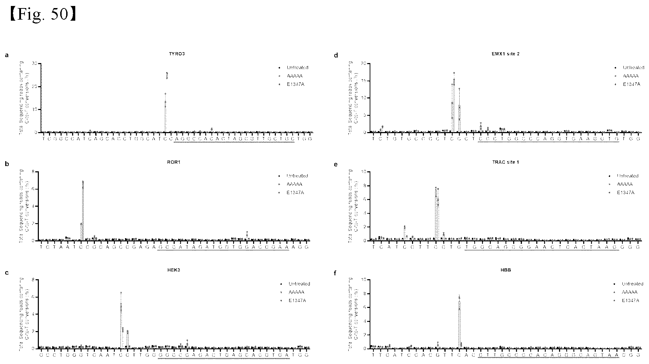

FIG. 50 shows results of measuring the activity of DddA in the human cell genomic context TYRO3 (a), ROR1 (b), HEK3 (c), EMX1 site 2 (d), TRAC site 1 (e), and HBB (f) using DddA-dCas9(D10A, H840A)-UGI;

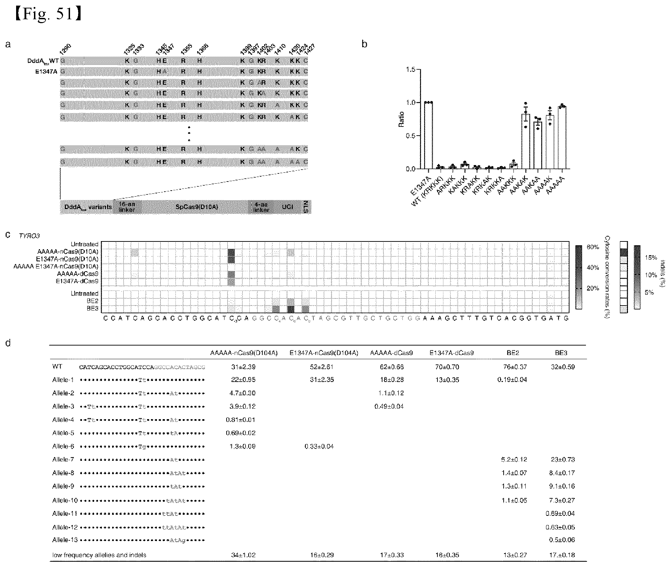

FIG. 51 shows base editing efficiencies of full-length DddAtox in HEK293T cells, FIG. 51a schematically showing screening of full-length DddAtox in a structure-based manner, red alanine indicating that a positively charged amino acid residue is substituted with alanine, FIG. 51b showing *E.coli* transformants of the DddA variant substituted with alanine, E1347A being used as an active site mutant in a control, FIG. 51c showing frequencies of editing and indels in DddA AAAAA and CBE at the TYRO3 site, and FIG. 51d showing allele frequency of the TYRO3 site, the C-to-T conversion being represented in red, the protospacer being represented in blue, and the protospacer-adjacent motif (PAM) being represented in orange;

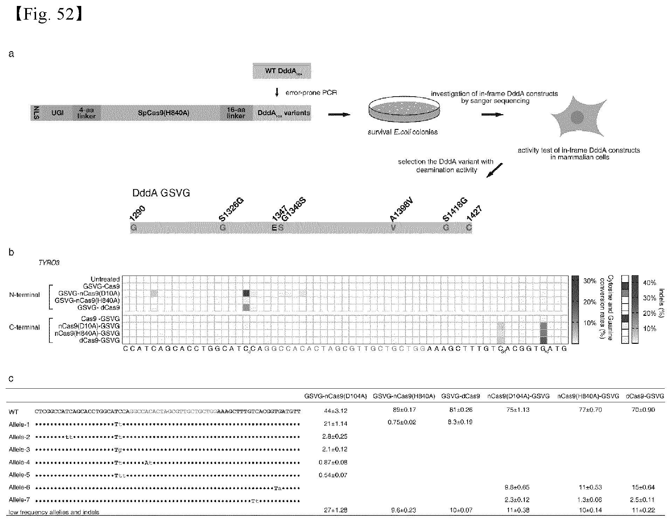

FIG. 52 shows non-toxic DddA GSVG, FIG. 52a schematically showing screening of non-toxic full-length DddAtox variants based on error-prone PCR, and FIGs. 52b and 52c showing editing frequencies of genes (b) and alleles (c) fused to the N-terminus and C-terminus of Cas9, nCas9(D10A), nCas9(H840A), and dCas9(D10A, H840A), the protospacer being represented in blue and the protospacer-adjacent motif (PAM) being represented in orange;

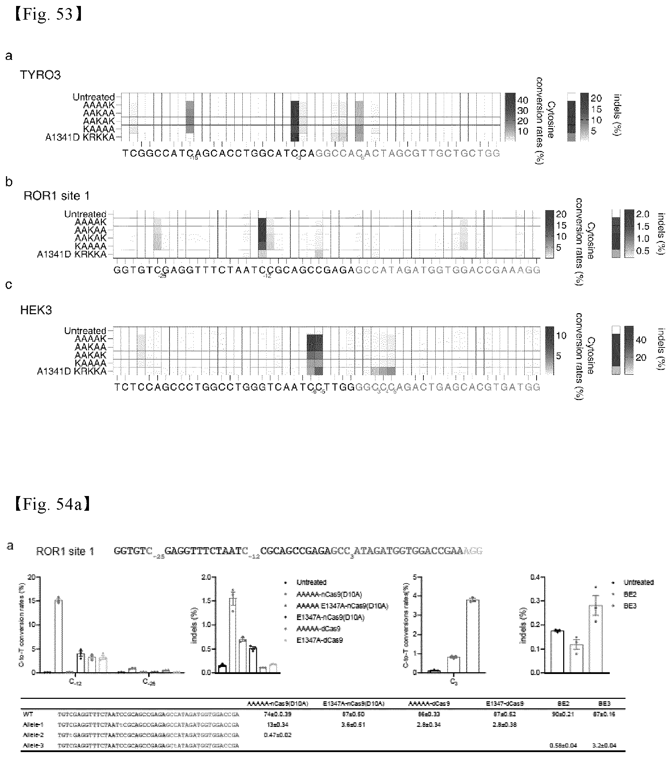

FIG. 53 shows editing frequencies of DddAtox variants in which positively charged amino acid residues in the TYRO3 site (a), ROR1 site 1 (b), and HEK3 site (c) are substituted with alanine, at the N-terminus of nCas9(D10A), the protospacer being represented in blue and the protospacer-adjacent motif (PAM) being represented in orange;

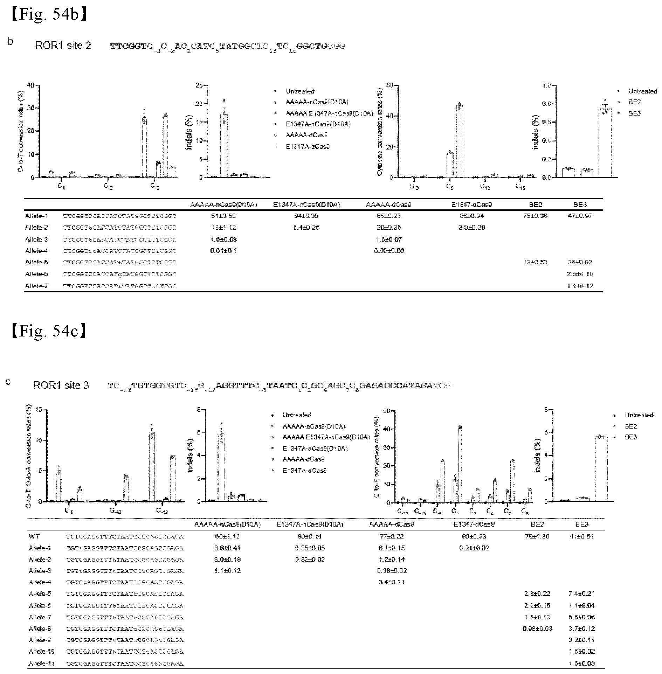

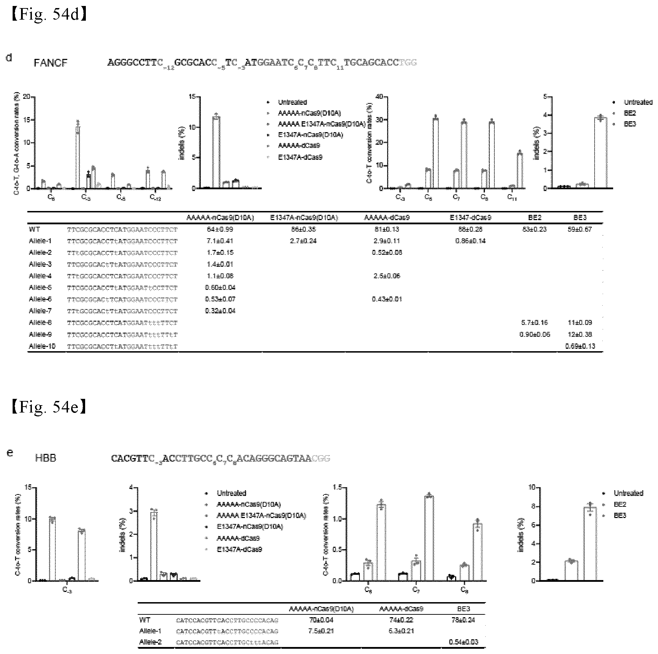

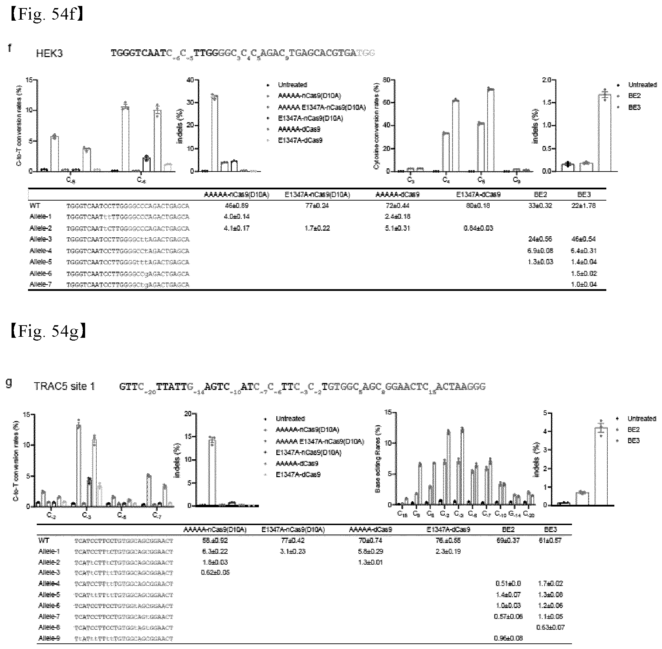

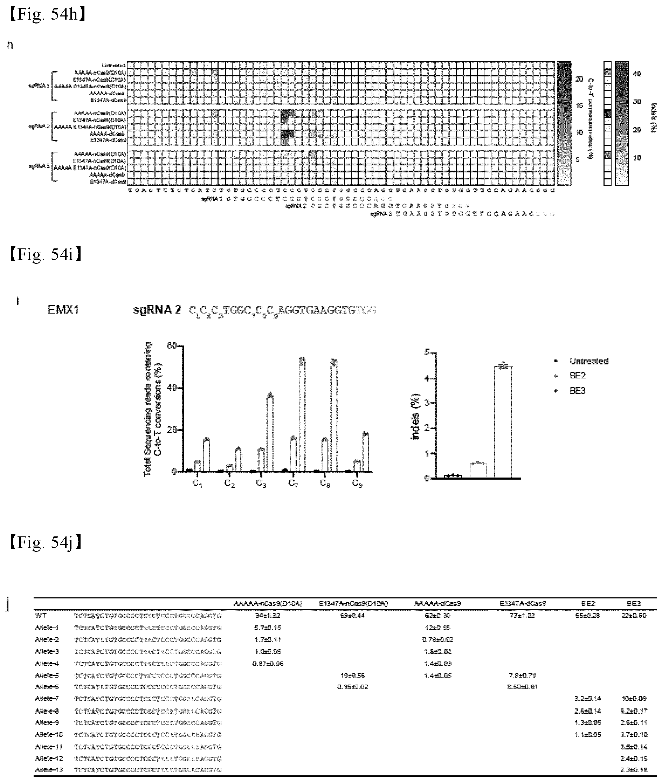

FIG. 54 shows editing frequencies at several sites. FIG. 54a, 54b, 54c, 54d, 54e, 54f, 54g, and 54h to 54j show the ROR1 site 1, ROR1 site 2, ROR1 site 3, FANCF site, HBB site, HEK3 site, TRAC5 site 1, and EMX1 sites, respectively, in which the protospacer is represented in blue, the protospacer-adjacent motif (PAMs) is represented in orange, the C-to-T conversion is represented in red, and the target window of DddA is indicated as a negative number by counting 5' upstream of the protospacer;

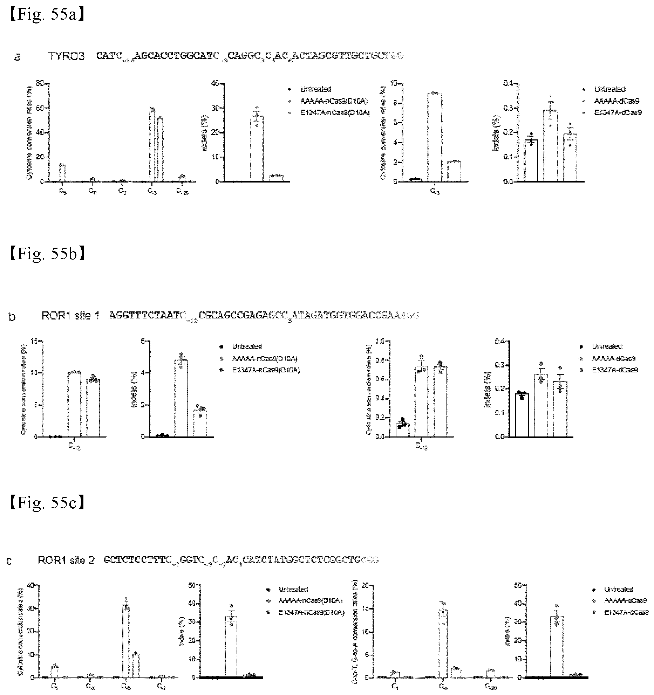

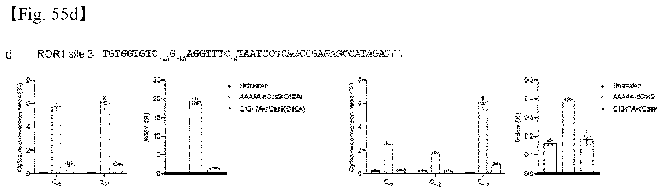

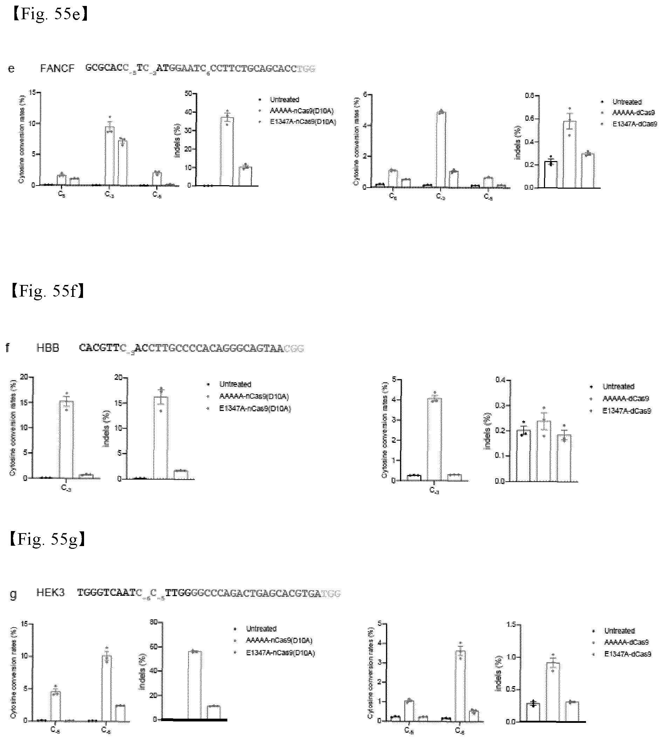

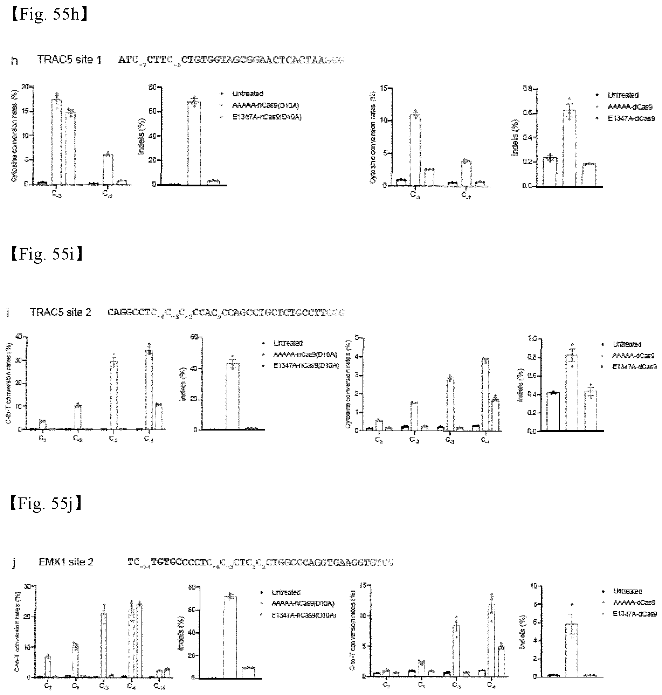

FIGs. 55a, 55b, 55c, 55d, 55e, 55f, 55g, 55h, 55i, and 55j show editing frequencies at the TYRO3 site, ROR1 site 1, ROR1 site 2, ROR1 site 3, FANCF site, HBB site, HEK3 site, TRAC5 site 1, TRAC5 site 2, and EMX1 site 2, respectively, in AAAAA and E1347A in HeLa cells, in which the protospacer is represented in blue, the protospacer-adjacent motif (PAM) is represented in orange, the target window of DddA is indicated as a negative number by counting 5' upstream of the protospacer, and the target cytosine is represented in red;

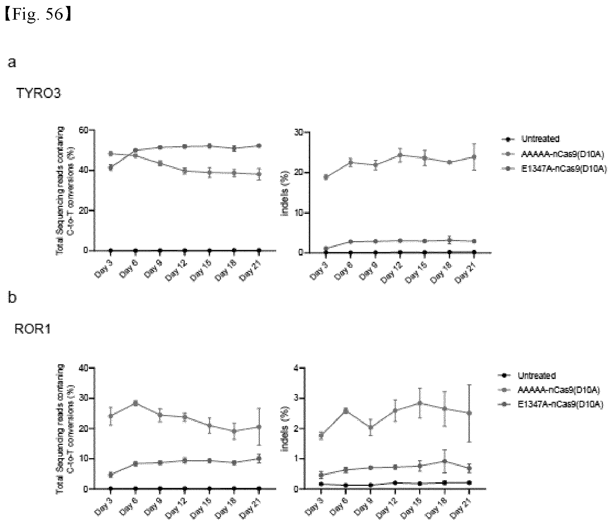

FIG. 56 shows time-dependent base editing and indel rates of AAAAA and E1347A at the TYRO3 site (a) and ROR1 site 1 (b);

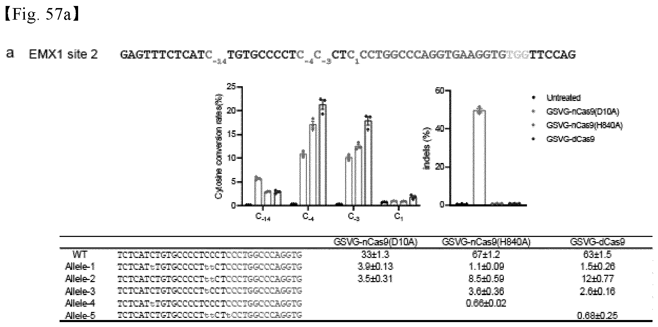

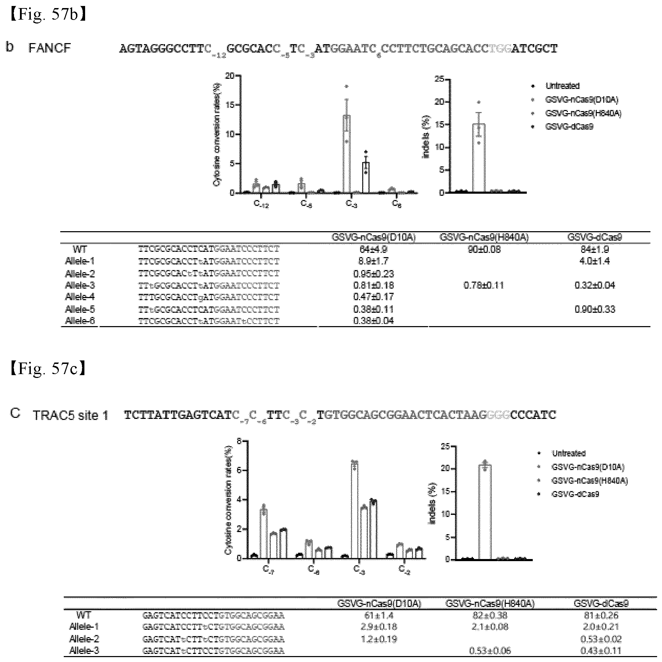

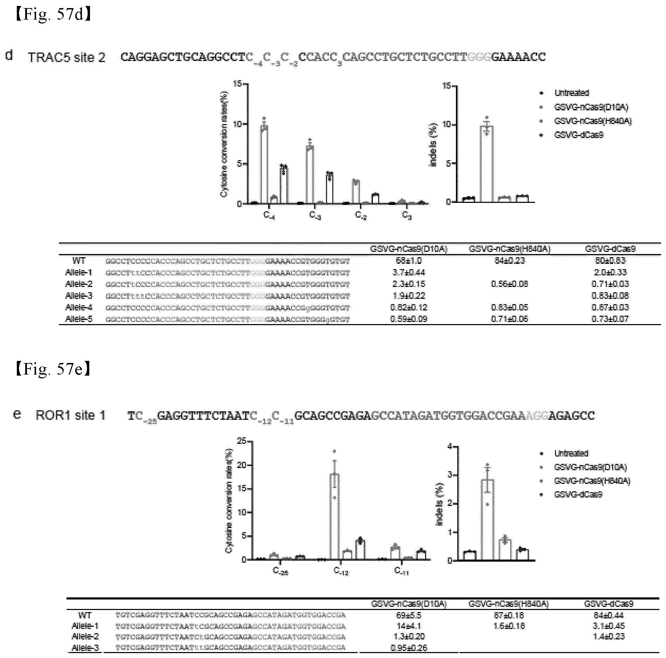

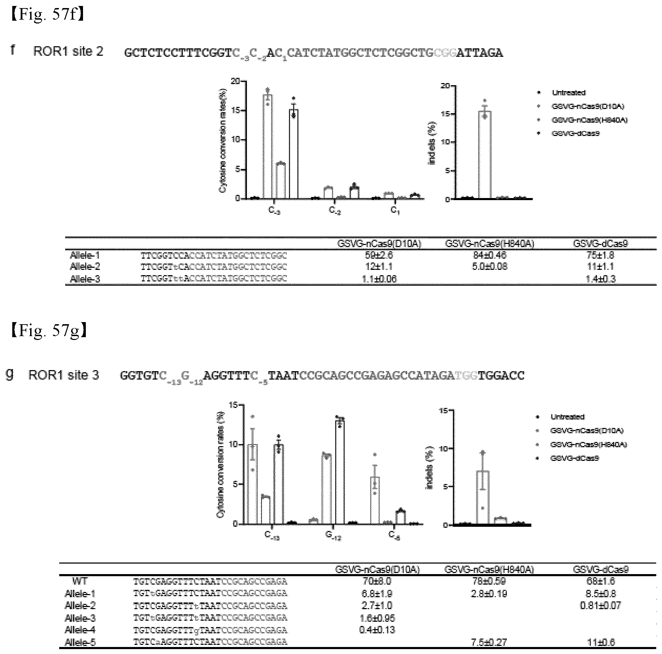

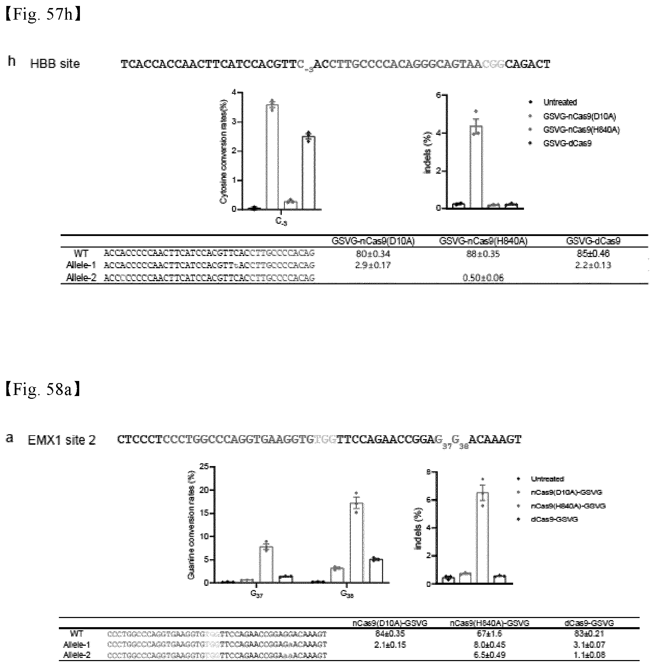

FIGs. 57a, 57b, 57c, 57d, 57e, 57f, 57g, and 57h show editing, insertion-deletion, and allele frequencies of GSVG fused to the N-terminus of nCas9(D10A), nCas9(H840A), and dCas9 at the EMX1 site 2, FANCF site, TRAC5 site 1, TRAC5 site 2, ROR1 site 1, ROR1 site 2, ROR1 site 3, and HBB site, respectively, in which the protospacer is represented in blue, the protospacer-adjacent motif (PAM) is represented in orange, the C-toT conversion is represented in red, and the target window of GSVG is indicated as a negative number by counting 5' upstream of the protospacer;

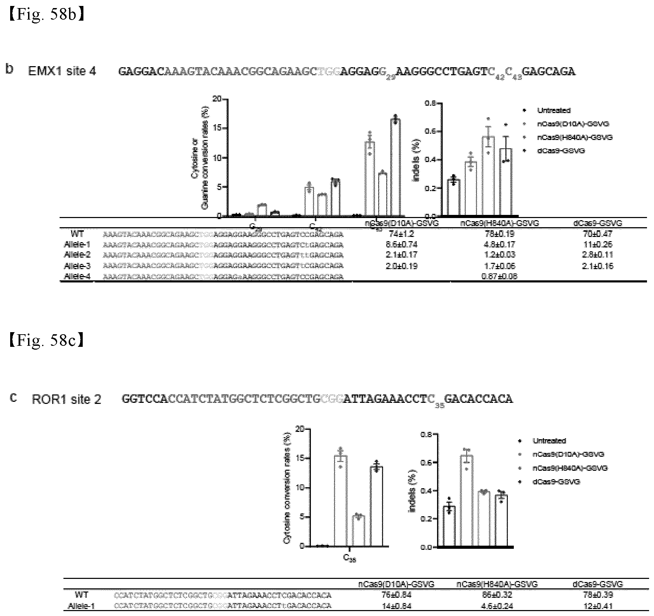

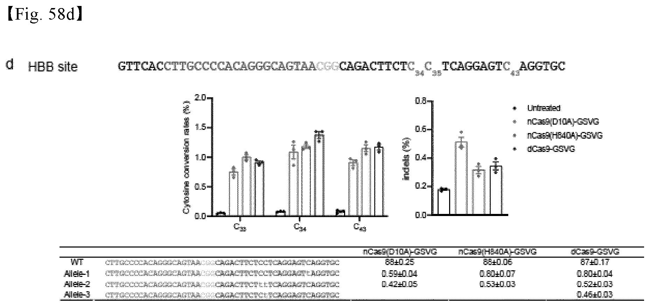

FIGs. 58a, 58b, 58c, and 58d show editing, insertion-deletion, and allele frequencies of GSVG fused to the C-terminus of nCas9(D10A), nCas9(H840A), and dCas9 at the EMX1 site 2, EMX1 site 4, ROR1 site 2, and HBB site, respectively, in which the protospacer is represented in blue, the protospacer-adjacent motif (PAM) is represented in orange, the G-to-A conversion is represented in red, and the target window of GSVG is indicated by counting 3' downstream from position 1 of the protospacer;

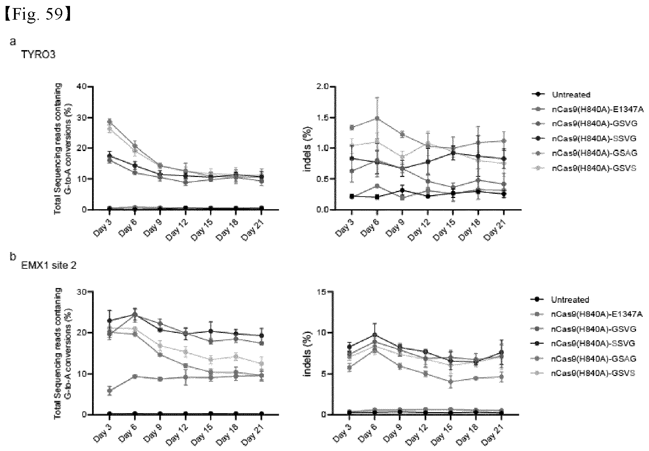

FIG. 59 shows time-dependent editing and insertion-deletion frequencies of E1347A, GSVG, SSVG, GSAG, and GSVS fused to the C-terminus of nCas9(H840A) at the TYRO3 (a) and EMX1 site 2 (b);

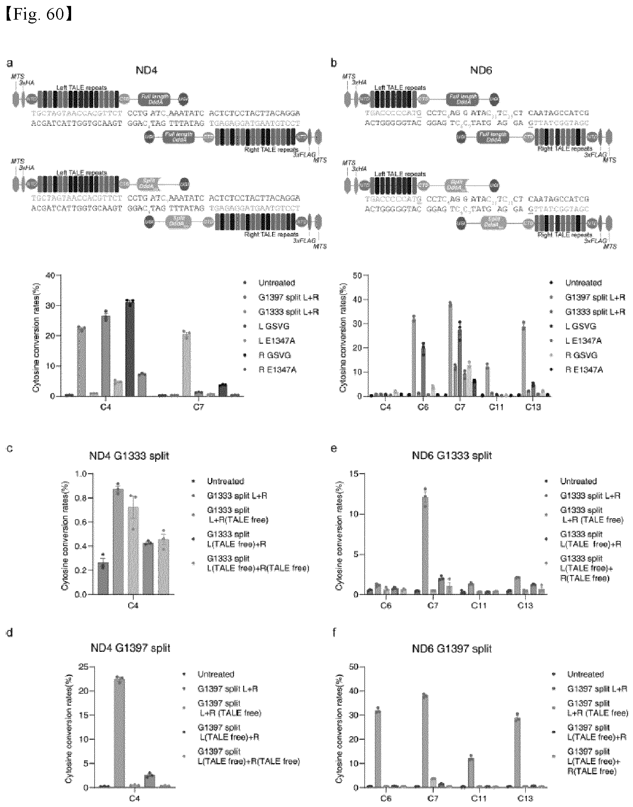

FIG. 60 shows mitochondrial base editing of mDdCBE in HEK293T cells, FIGs. 60a and 60b showing editing efficiencies of ND4 and ND6, respectively, in which the target cytosine and TALE binding site are represented in red and gray, respectively, FIGs. 60c to 60f showing editing efficiencies in ND4 (c, d) and ND6 (e, f) when only half of DddAtox is fused to the TALE array and the remaining half is free of TALE, in which the left TALE array and the right TALE array are represented as L and R, respectively, and the ND6 TALE array mismatch with the reference genome is underlined in purple;
FIG. 61:

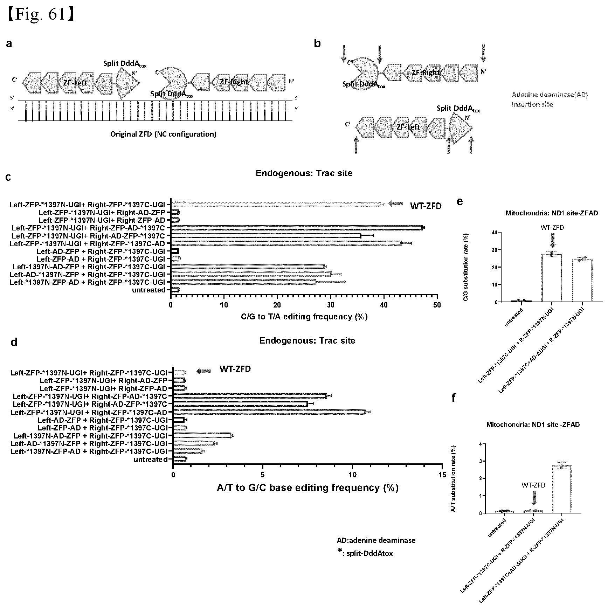

   FIG. 61a schematically shows a zinc finger cytosine deaminase (ZFD) using a conventional ZFP DNA-binding protein,
   FIG. 61b shows positions at which an adenine deaminase is inserted into the ZFD (in which the red arrow is the insertion site),
   FIG. 61c shows base editing efficiencies at nuclear DNA Trac sites of the constructed ZF-DdABE (C to T),
   FIG. 61d shows base editing efficiencies at nuclear DNA Trac sites of the constructed ZF-DdABE (A to G)
      (in which WT-ZFD is a C-to-T deaminase with split DddAtox alone in the absence of adenine deaminase),
   FIG. 61e shows efficiency at the ND1 site of ZF-DdABE targeting mitochondrial DNA (C to T), and
   FIG. 61f shows efficiency at the ND1 site of ZF-DdABE targeting mitochondrial DNA (A to G);
FIG. 62:

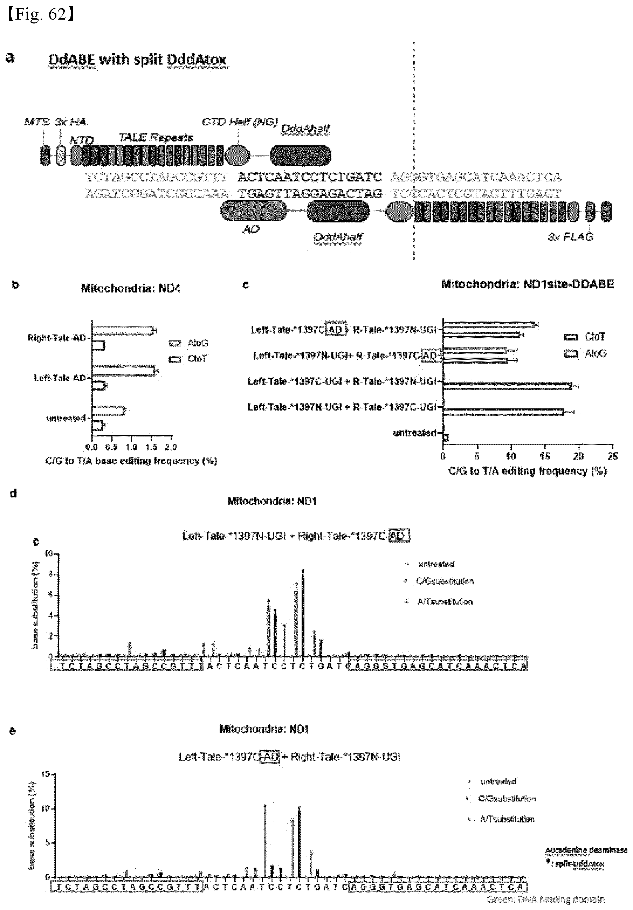

   FIG. 62a schematically shows DdABE using TALE and split DddAtox (in which components include split DddAtox, adenine deaminase, and TALE array),
   FIG. 62b shows base editing efficiency when an adenine deaminase alone is attached to TALE targeting the mitochondrial ND4 site,
   FIG. 62c shows base editing efficiency when an adenine deaminase is attached to TALE-split DddAtox targeting the mitochondrial ND1 site,
   FIG. 62d shows base editing efficiency in a single nucleotide unit when a DdCBE pair is used on the left and an adenine deaminase is attached to TALE-split DddAtox on the right (in which the green box is a portion to which TALE is attached), and
   FIG. 62e shows base editing efficiency in a single nucleotide unit when an adenine deaminase is attached to TALE-split DddAtox on the left and a DdCBE pair is used on the right (in which the green box is a portion to which TALE is attached);
FIG. 63:

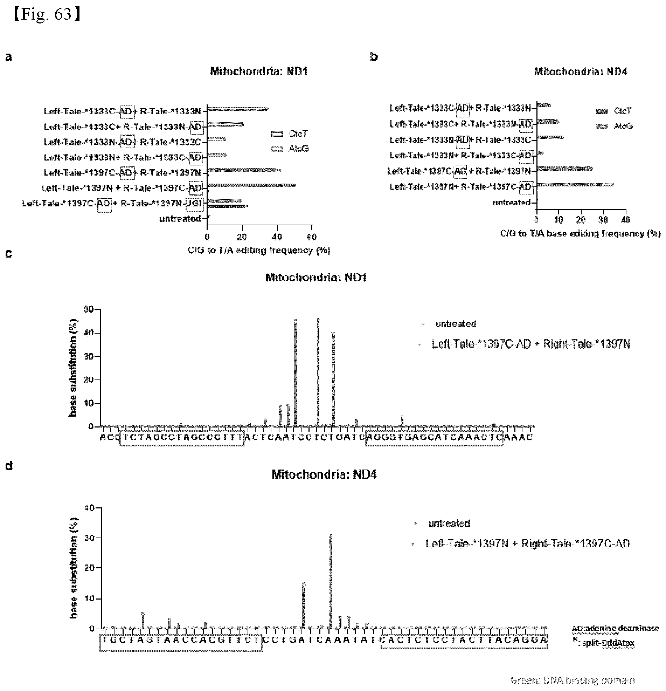

   FIG. 63a shows C-to-T and A-to-G base editing efficiencies of DdABE targeting the mitochondrial ND1 site in the absence or presence of UGI (in which the red box indicates an adenine deaminase),
   FIG. 63b shows C-to-T and A-to-G base editing efficiencies of DdABE targeting the mitochondrial ND4 site in the absence or presence of UGI (in which the red box indicates an adenine deaminase),
   FIG. 63c shows a configuration with the highest efficiency in a single nucleotide unit among DdABE configurations targeting the mitochondrial ND1 site (in which the green box is a portion to which TALE is attached), and
   FIG. 63d shows a configuration with the highest efficiency in a single nucleotide unit among DdABE configurations targeting the mitochondrial ND4 site (in which the green box is a portion to which TALE is attached);
FIG. 64:

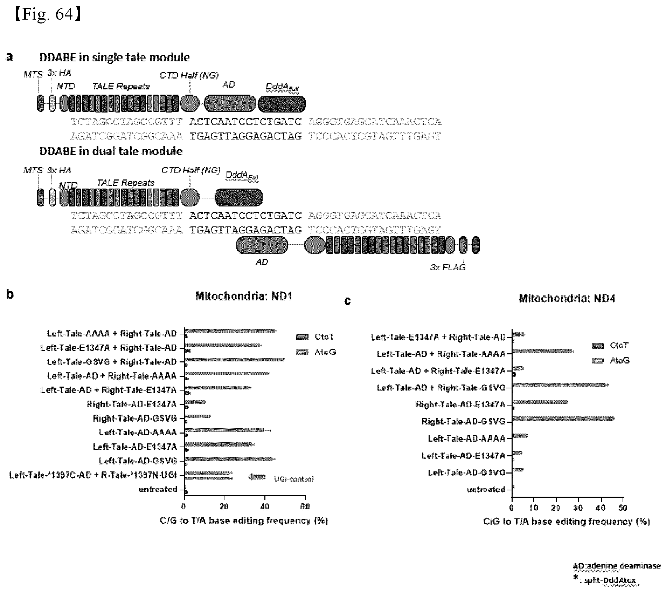

   FIG. 64a schematically shows a single TALE module with all constructs in one TALE module on the top (in which components include full-length DddAtox, adenine deaminase, and TALE array), and
      also shows a dual TALE module using two TALE modules on the bottom (in which components include full-length DddAtox and TALE array at one side, and adenine deaminase and TALE array at the other side),
   FIG. 64b shows base editing efficiencies of the single module and dual module DdABEs targeting the mitochondrial ND1 site, and
   FIG. 64c shows base editing efficiencies of the single module and dual module DdABEs targeting the mitochondrial ND4 site;

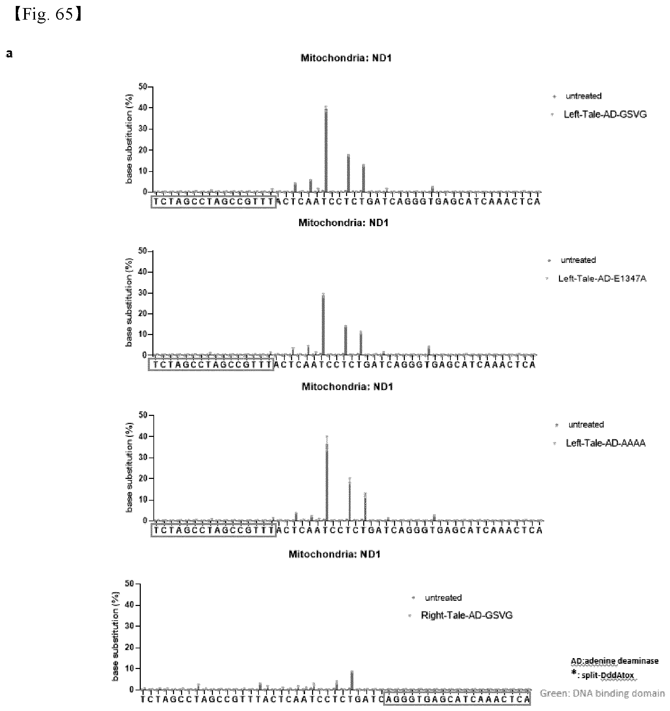

FIG. 65 shows results confirming the base editing efficiency of the single module targeting the ND1 site (in which components include TALE array, adenine deaminase (AD), and full-length DddAtox (GSVG, AAAA, and E1347A are variants));

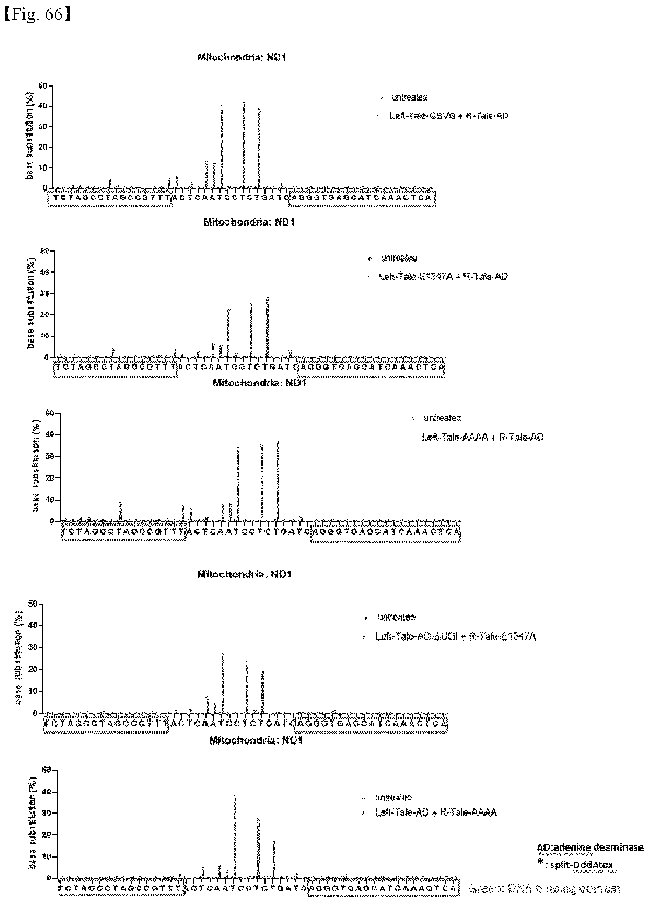

FIG. 66 shows results confirming the base editing efficiency of the dual module targeting the ND1 site (in which components include TALE array, adenine deaminase (AD), and full-length DddAtox (GSVG, AAAA, and E1347A are variants));

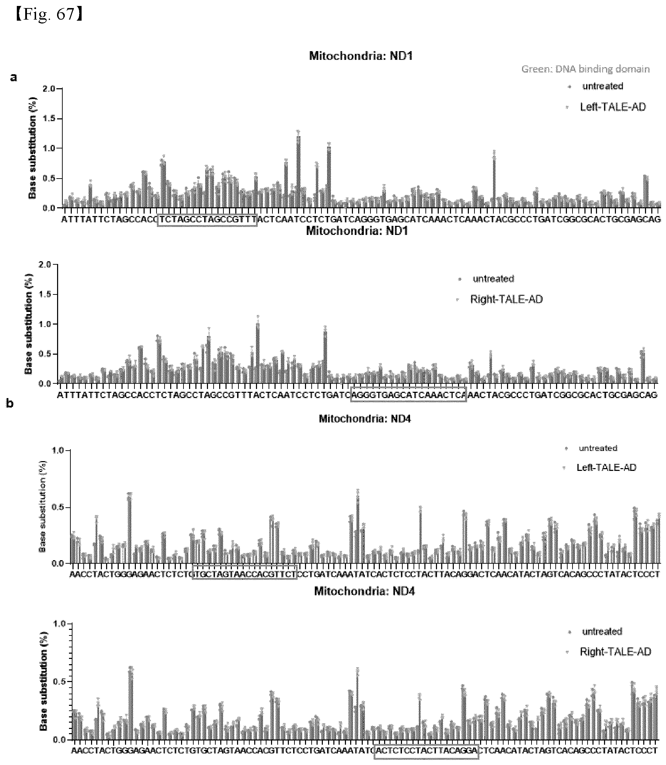

FIG. 67: FIG. 67a shows base editing efficiencies when a TadA(AD) adenine deaminase alone is attached to the TALE-binding protein targeting the ND1 site, and
FIG. 67b shows base editing efficiencies when a TadA(AD) adenine deaminase alone is attached to the TALE-binding protein targeting the ND4 site; and

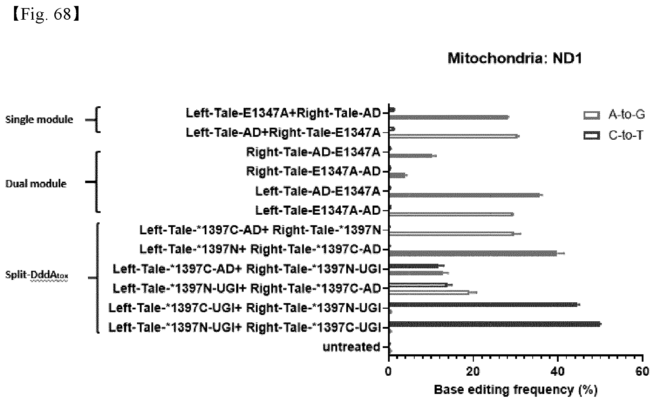

FIG. 68 shows adenine and cytosine base editing efficiencies for the dual module, single module, and split-DddA-AD in TALE targeting the ND1 site, in which (from the lowermost) when UGI is attached to both sides without AD, which is referred to as DdCBE, only cytosine base editing occurs, or when AD is replaced with either side of UGI, both cytosine base editing and adenine base editing occur, or when UGI is absent, only adenine base editing occurs selectively, and similarly, only adenine base editing occurs selectively even in the dual module and single module.

### [Mode for Invention]

Unless otherwise defined, all technical and scientific terms used herein have the same meanings as those typically understood by those skilled in the art to which the present invention belongs. In general, the nomenclature used herein is well known in the art and is typical.

As used herein, the term "editing" may be used interchangeably with "correcting" and refers to a method of altering a nucleic acid sequence at a specific genomic target site in cells. Such specific genomic targets include, but are not limited to, chromosomal regions, genes, promoters, open reading frames, or any nucleic acid sequence.

As used herein, the term "single base" refers to only one nucleotide in a nucleic acid sequence. When used in the context of single base editing, it means that a base at a specific position in a nucleic acid sequence is substituted with a different base. Such substitution may occur by a number of mechanisms, including substitution or modification without limitation.

As used herein, the term "target" or "target site" refers to a previously identified nucleic acid sequence of any composition and/or length. Such target sites include, but are not limited to, chromosomal regions, genes, promoters, open reading frames, or any nucleic acid sequence.

As used herein, the term "on-target" refers to a subsequence of a specific genomic target that is bound by a programmable DNA-binding protein or may be perfectly complementary with a single guide RNA sequence.

As used herein, the term "off-target" refers to a subsequence of a specific genomic target that may be partially complementary to a on-target sequence recognized by a programmable DNA-binding region and/or a single guide RNA sequence.

### 1. Split cytosine deaminase

A fusion protein according to an aspect of the present invention includes a cytosine deaminase or a variant thereof, in which the cytosine deaminase or the variant thereof includes a first split and a second split derived from a cytosine deaminase or a variant thereof, and each of the first split and the second split is fused to a DNA-binding protein.

The cytosine deaminase is an enzyme that removes an amino group in a cytosine base, and is able to convert cytosine (C) into uridine (U).

It may be the cytosine deaminase. Examples of the cytosine deaminase may include APOBEC1 (apolipoprotein B editing complex 1) and AID (activation-induced deaminase), but most DNA deaminases may act only on single-stranded DNA and may not be suitable for base editing through linkage to a DNA-binding protein. Specifically, the cytosine deaminase may be derived from a double-stranded DNA deaminase (DddA) or an orthologue thereof. More specifically, the cytosine deaminase may be a double-stranded DNA-specific bacterial cytosine deaminase.

The cytosine deaminase is provided in a split form, the cytosine deaminase includes a first split and a second split, and each of the first split and the second split has no deaminase activity.

The sequence of SEQ ID NO: 1 corresponding to the DddAtox split in a full-length cytosine deaminase may be included. The cytosine deaminase includes a first split and a second split, and each of the first split and the second split has no deaminase activity.

[SEQ ID NO: 1]

GSYALGPYQISAPQLPAYNGQTVGTFYYVNDAGGLESKVFSSGGPTPY

PNYANAGHVEGQSALFMRDNGISEGLVFHNNPEGTCGFCVNMTETLLPENAKMTVV

PPEGAIPVKRGATGETKVFTGNSNSPKSPTKGGC

In an embodiment, the first split or the second split of the cytosine deaminase may include a sequence from the N terminus to at least one selected from the group consisting of G33, G44, A54, N68, G82, N98, and G108 in the sequence of SEQ ID NO: 1. The first split or the second split of the cytosine deaminase may include a sequence from at least one selected from the group consisting of G34, P45, G55, N69, T83, A99, and A109 to the C-terminus in the sequence of SEQ ID NO: 1.

Specifically, the cytosine deaminase may include the first split of SEQ ID NO: 23 (G1333-N) and the second split of SEQ ID NO: 24 (G1333-C), the first split of SEQ ID NO: 25 (G1397-N) and the second split of SEQ ID NO: 26 (G1397-C), the first split of SEQ ID NO: 23 (G1333-N) and the second split of SEQ ID NO: 26 (G1397-C), or the first split of SEQ ID NO: 25 (G1397-N) and the second split of SEQ ID NO: 24 (G1333-C).
(SEQ ID NO: 23) Wild-type DddAtox G1333-N
   GSYALGPYQISAPQLPAYNGQTVGTFYYVNDAGGLESKVFSSGG
(SEQ ID NO: 27) GGCTCTGGTTCCTACGCCCTGGGTCCATATCAGATTAGTGCTCCCCAA 
CTCCCCGCCTACAACGGTCAGACAGTGGGGACCTTTTACTATGTCAACGACGCCGG

GGGATTGGAATCCAAGGTTTTCTCTAGCGGTGGG
(SEQ ID NO: 24) Wild-type G1333-C 
PTPYPNYANAGHVEGQSALFMRDNGISEGLVFHNNPEGTCGFCVNMTE
TLLPENAKMTVVPPEGAIPVKRGATGETKVFTGNSNSPKSPTKGGC
(SEQ ID NO: 28) 
CCAACACCTTATCCTAACTACGCTAACGCCGGGCACGTCGAGGGGCAG
TCAGCTCTTTTTATGAGAGATAACGGCATTAGCGAAGGGCTTGTGTTCCATAATAA
TCCTGAGGGCACCTGTGGCTTCTGTGTAAATATGACCGAAACACTTCTGCCTGAGA
ACGCTAAAATGACTGTCGTACCACCCGAAGGCGCAATCCCAGTTAAACGGGGCGCA
ACCGGCGAAACCAAAGTATTCACCGGAAACAGCAATAGTCCAAAGTCCCCCACCAA
GGGAGGTTGC
(SEQ ID NO: 25) Wild-type DddAtox G1397-N 
GSYALGPYQISAPQLPAYNGQTVGTFYYVNDAGGLESKVFSSGGPTPY
PNYANAGHVEGQSALFMRDNGISEGLVFHNNPEGTCGFCVNMTETLLPENAKMTVV
PPEG
(SEQ ID NO: 29) 
GGTAGCTACGCACTTGGTCCTTACCAGATTAGCGCACCCCAACTCCCC
GCCTATAATGGTCAAACCGTCGGGACCTTTTACTACGTAAACGATGCTGGTGGGCT
GGAATCCAAAGTATTCTCCTCAGGGGGCCCTACACCCTACCCCAACTACGCCAATG
CTGGTCATGTAGAAGGGCAGTCAGCACTGTTTATGCGCGATAATGGTATAAGCGAG
GGGTTGGTCTTCCATAACAACCCAGAGGGTACTTGTGGCTTCTGTGTGAATATGAC
TGAAACCCTTCTGCCCGAAAATGCCAAGATGACTGTCGTCCCACCTGAAGGC
(SEQ ID NO: 26) Wild-type DddAtox G1397-C
   AIPVKRGATGETKVFTGNSNSPKSPTKGGC
(SEQ ID NO: 30)

GCCATACCTGTGAAGCGGGGAGCAACAGGGGAGACAAAGGTGTTCACA
GGCAACTCTAACAGTCCAAAGAGCCCCACCAAAGGCGGGTGT

G1333N, G1333C, G1397N, and G1397C in combination may be used as deaminases in split forms. Specifically, the form of left-G1333-N + right-G133-C, left-G1397-N + right-G1397-C, left-G1397-N + right-G1333-C, or left-G1333-N + right-G1397-C may be used.

### 2. Variant

The inventors of the present application attempted to suppress unwanted base editing through DdCBE mutations in which amino acid residues are substituted. A high-precision DddA-derived cytosine base editor capable of reducing the off-target effect of DdCBE is presented. This off-target base editing effect is a phenomenon caused by spontaneous assembly of the DddAₜₒₓ deaminase splits, independent of interaction between TALE and DNA.

Here, the amino acid residue that is mutated is a contact site located on the surface where DddAₜₒₓ split dimers interact with each other. High Fidelity-DdCBE(HF-DdCBE) was constructed by substituting an amino acid residue located on the surface between DddAₜₒₓ splits with alanine. HF-DdCBE prevented a pair of two split deaminase halves linked to TALE from functioning properly when not bound to DNA. Through whole mitochondrial genome analysis, it was confirmed that HF-DdCBE is very efficient and precise, unlike conventional DdCBEs that causes numerous unwanted off-target C-to-T conversions in human mitochondrial DNA.

For DddAtox, base editing may be induced only when both split dimers are recruited to the target site of DNA in principle. Based on results of actual experiments, targeted base editing occurs even when using DdCBE, one half of which binds to DNA and the remaining half of which does not bind. In order to solve this problem, binding of DdCBE pairs at unwanted positions is prevented by substituting residues on the protein surface where split dimers interact with each other.

Based thereon, the present invention pertains to a new variant that reduces non-selective base editing by substituting an amino acid residue of the cytosine deaminase DddAtox.

The cytosine deaminase includes the first split of SEQ ID NO: 23 (G1333-N) and the second split of SEQ ID NO: 24 (G1333-C), or the first split of SEQ ID NO: 25 (G1397-N) and the second split of SEQ ID NO: 26 (G1397-C) .

The variant of the cytosine deaminase may be configured such that at least one amino acid selected from the group consisting of amino acids at positions 3, 5, 10, 11, 13, 14, 15, 16, 17, 18, 19, 28, 30, and 31 in the first split of SEQ ID NO: 23 is substituted with a different amino acid, or also that at least one amino acid selected from the group consisting of amino acids at positions 13, 16, 17, 20, 21, 28, 29, 30, 31, 32, 33, 56, 57, 58, and 60 in the second split of SEQ ID NO: 24 is substituted with a different amino acid.

The variant according to the present invention may be configured such that at least one amino acid selected from the group consisting of amino acids at positions 87, 88, 91, 92, 95, 100, 101, 102, and 103 in the first split of SEQ ID NO: 25 or at least one amino acid selected from the group consisting of amino acids at positions 13, 14, 15, and 16 in the second split of SEQ ID NO: 26 is substituted with a different amino acid.

Here, the "different amino acid" may be alanine, isoleucine, leucine, methionine, phenylalanine, proline, tryptophan, valine, asparagine, cysteine, glutamine, glycine, serine, threonine, tyrosine, aspartic acid, glutamic acid, arginine, histidine, or lysine, and may refer to an amino acid selected from among amino acids excluding amino acids at original mutation positions in a wild-type protein from all known variants of the amino acids described above. In an exemplary embodiment, the "different amino acid" may be alanine.

Specifically, amino acid substitution of at least one selected from the group consisting of Y3A, L5A, I10A, S11A, V13A, G14A, T15A, F16A, Y17A, Y18A, V19A, K28A, F30A, and S31A in the first split of SEQ ID NO: 23 (corresponding to Y1292A, L1294A, I1299A, S1300A, V1312A, G1313A, T1314A, F1315A, Y1316A, Y1317A, V1318A, K1327A, F1329A, and S1330A, respectively) may be included.

Also, amino acid substitution of at least one selected from the group consisting of V13A, Q16A, S17A, F20A, M21A, E28A, G29A, L30A, V31A, F32A, H33A, K56A, M57A, T58A, and V60A in the second split of SEQ ID NO: 24 (corresponding to V1346A, Q1349A, S1350A, F1353A, M1354A, E1361A, G1362A, L1363A, V1364A, F1365A, H1366A, K1389A, M1390A, T1391A, and V1393A, respectively) may be included.

Specifically, amino acid substitution of at least one selected from the group consisting of C87A, V88A, T91A, E92A, L95A, K100A, M101A, T102A, and V103A in the first split of SEQ ID NO: 25 (corresponding to C1376A, V1377A, T1380A, E1381A, L1384A, K1389A, M1390A, T1391A, and V1392A, respectively) may be included.

Also, amino acid substitution of at least one selected from the group consisting of K13A, V14A, F15A, and T16A in the second split of SEQ ID NO: 26 (corresponding to K1410A, V1411A, F1412A, and T1413A, respectively) may be included.

### [DddAtox G1333-N variant]

| Type | SEQ | No. |
|---|---|---|
| Y1292A | GSAALGPYQISAPQLPAYNGQTVGTFYYVNDAGGLESKVFSSGG | 31 |
| | GGCTCTGGTTCCgcaGCCCTGGGTCCATATCAGATTAGTGCTCCCCAACT CCCCGCCTACAACGGTCAGACAGTGGGGACCTTTTACTATGTCAACGAC GCCGGGGGATTGGAATCCAAGGTTTTCTCTAGCGGTGGG | 32 |
| L1294A | GSYAAGPYQISAPQLPAYNGQTVGTFYYVNDAGGLESKVFSSGG | 33 |
| | GGCTCTGGTTCCTACGCCgcaGGTCCATATCAGATTAGTGCTCCCCAACTC CCCGCCTACAACGGTCAGACAGTGGGGACCTTTTACTATGTCAACGACG CCGGGGGATTGGAATCCAAGGTTTTCTCTAGCGGTGGG | 34 |
| I1299A | GSYALGPYQASAPQLPAYNGQTVGTFYYVNDAGGLESKVFSSGG | 35 |
| | GGCTCTGGTTCCTACGCCCTGGGTCCATATCAGgcaAGTGCTCCCCAACT CCCCGCCTACAACGGTCAGACAGTGGGGACCTTTTACTATGTCAACGAC GCCGGGGGATTGGAATCCAAGGTTTTCTCTAGCGGTGGG | 36 |
| S1300A | GSYALGPYQIAAPQLPAYNGQTVGTFYYVNDAGGLESKVFSSGG | 37 |
| | GGCTCTGGTTCCTACGCCCTGGGTCCATATCAGATTgcaGCTCCCCAACTC CCCGCCTACAACGGTCAGACAGTGGGGACCTTTTACTATGTCAACGACG CCGGGGGATTGGAATCCAAGGTTTTCTCTAGCGGTGGG | 38 |
| V13 12A. | GSYALGPYQISAPOLPAYNGQTAGTFYYVNDAGGLESKVFSSGG | 39 |
| | GGCTCTGGTTCCTACGCCCTGGGTCCATATCAGATTAGTGCTCCCCAACT CCCCGCCTACAACGGTCAGACAgcaGGGACCTTTTACTATGTCAACGACG CCGGGGGATTGGAATCCAAGGTTTTCTCTAGCGGTGGG | 40 |
| G1313A | GSYALGPYQISAPQLPAYNGQTVATFYYVNDAGGLESKVFSSGG | 41 |
| | GGCTCTGGTTCCTACGCCCTGGGTCCATATCAGATTAGTGCTCCCCAACT CCCCGCCTACAACGGTCAGACAGTGgcaACCTTTTACTATGTCAACGACG CCGGGGGATTGGAATCCAAGGTTTTCTCTAGCGGTGGG | 42 |
| T1314A | GSYALGPYQISAPQLPAYNGQTVGAFYYNDAGGLESKVFSSGG | 43 |
| | GGCTCTGGTTCCTACGCCCTGGGTCCATATCAGATTAGTGCTCCCCAACT CCCCGCCTACAACGGTCAGACAGTGGGGgcaTTTTACTATGTCAACGACG CCGGGGGATTGGAATCCAAGGTTTTCTCTAGCGGTGGG | 44 |
| F1315A | GSYALGPYQISAPQLPAYNGQTVGTAYYVNDAGGLESKVFSSGG | 45 |
| | GGCTCTGGTTCCTACGCCCTGGGTCCATATCAGATTAGTGCTCCCCAACT CCCCGCCTACAACGGTCAGACAGTGGGGACCgcaTACTATGTCAACGACG CCGGGGGATTGGAATCCAAGGTTTTCTCTAGCGGTGGG | 46 |
| Y1316A | GSYALGPYQISAPQLPAYNGQTVGTFAYVNDAGGLESKVFSSGG | 47 |
| | GGCTCTGGTTCCTACGCCCTGGGTCCATATCAGATTAGTGCTCCCCAACT CCCCGCCTACAACGGTCAGACAGTGGGGACCTTTgcaTATGTCAACGACG CCGGGGGATTGGAATCCAAGGTTTTCTCTAGCGGTGGG | 48 |
| Y1317A | GSYALGPYQISAPQLPAYNGQTVGTFYAVNDAGGLESKVFSSGG | 49 |
| | GGCTCTGGTTCCTACGCCCTGGGTCCATATCAGATTAGTGCTCCCCAACT CCCCGCCTACAACGGTCAGACAGTGGGGACCTTTTACgcaGTCAACGACG CCGGGGGATTGGAATCCAAGGTTTTCTCTAGCGGTGGG | 50 |
| V1318A | GSYALGPYQISAPQLPAYNGQTVGTFYYANDAGGLESKVFSSGG | 51 |
| | Count line 1: GGCTCTGGTT (10) CCTACGCCCT (20) GGGTCCATAT (30) CAGATTAGTG (40) CTCCCCAACT (50). Good. Line 2: CCCCGCCTAC (10) AACGGTCAGA (20) CAGTGGGGAC (30) CTTTTACTAT (40) gcaAACGACG (50). Good.GGCTCTGGTTCCTACGCCCTGGGTCCATATCAGATTAGTGCTCCCCAACT CCCCGCCTACAACGGTCAGACAGTGGGGACCTTTTACTATgcaAACGACG CCGGGGGATTGGAATCCAAGGTTTTCTCTAGCGGTGGG | 52 |
| K1327A | GSYALGPYQISAPQLPAYNGQTVGTFYYVNDAGGLESAVFSSGG | 53 |
| | GGCTCTGGTTCCTACGCCCTGGGTCCATATCAGATTAGTGCTCCCCAACT CCCCGCCTACAACGGTCAGACAGTGGGGACCTTTTACTATGTCAACGAC GCCGGGGGATTGGAATCCgcaGTTTTCTCTAGCGGTGGG | 54 |
| F1329A | GSYALGPYQISAPQLPAYNGQTVGTFYYVNDAGGLESKVASSGG | 55 |
| | GGCTCTGGTTCCTACGCCCTGGGTCCATATCAGATTAGTGCTCCCCAACT CCCCGCCTACAACGGTCAGACAGTGGGGACCTTTTACTATGTCAACGAC GCCGGGGGATTGGAATCCAAGGTTgcaTCTAGCGGTGGG | 56 |
| S1330A | GSYALGPYQISAPQLPAYNGQTVGTFYYVNDAGGLESKVFASGG | 57 |
| | GGCTCTGGTTCCTACGCCCTGGGTCCATATCAGATTAGTGCTCCCCAACT CCCCGCCTACAACGGTCAGACAGTGGGGACCTTTTACTATGTCAACGAC GCCGGGGGATTGGAATCCAAGGTTTTCgcaAGCGGTGGG | 58 |

### [DddAtox G1333-C variant]

| Type | SEQ | No. |
|---|---|---|
| V1346A | PTPYPNYANAGH[?]EGQSALFMRDNGISEGLVFHNNPEGTCGFCVNMTETLL PENAKMTVVPPEGAIPVKRGATGETKVFTGNSNSPKSPTKGGC | 59 |
| | CCAACACCTTATCCTAACTACGCTAACGCCGGGCACgcaGAGGGGCAGTC AGCTCTTTTTATGAGAGATAACGGCATTAGCGAAGGGCTTGTGTTCCATAA TAATCCTGAGGGCACCTGTGGCTTCTGTGTAAATATGACCGAAACACTTCT GCCTGAGAACGCTAAAATGACTGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 60 |
| Q1349A | PTPYPNYANAGHVEGASALFMRDNGISEGLVFHNNPEGTCGFCVNMTETLL PENAKMTVVPPEGAIPVKRGATGETKVFTGNSNSPKSPTKGGC | 61 |
| | CCAACACCTTATCCTAACTACGCTAACGCCGGGCACGTCGAGGGGgcaTC AGCTCTTTTTATGAGAGATAACGGCATTAGCGAAGGGCTTGTGTTCCATAA TAATCCTGAGGGCACCTGTGGCTTCTGTGTAAATATGACCGAAACACTTCT GCCTGAGAACGCTAAAATGACTGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 62 |
| S1350A | PTPYPNYANAGHVEGQAALFMRDNGISEGLVFHNNPEGTCGFCVNMTETLL PENAKMTVVPPEGAIPVKRGATGETKVFTGNSNSPKSPTKGGC | 63 |
| | CCAACACCTTATCCTAACTACGCTAACGCCGGGCACGTCGAGGGGCAGgc aGCTCTTTTTATGAGAGATAACGGCATTAGCGAAGGGCTTGTGTTCCATAA TAATCCTGAGGGCACCTGTGGCTTCTGTGTAAATATGACCGAAACACTTCT GCCTGAGAACGCTAAAATGACTGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 61 |
| F1353A | PTPYPNYANAGHVEGQSALAMRDNGISEGLVFHNNPEGTCGFCVNMTETLL PENAKMTVVPPEGAIPVKRGATGETKVFTGNSNSPKSPTKGGC | 65 |
| | CCAACACCTTATCCTAACTACGCTAACGCCGGGCACGTCGAGGGGCAGT CAGCTCTTgcaATGAGAGATAACGGCATTAGCGAAGGGCTTGTGTTCCATA ATAATCCTGAGGGCACCTGTGGCTTCTGTGTAAATATGACCGAAACACTTC TGCCTGAGAACGCTAAAATGACTGTCGTACCACCCGAAGGCGCAATCCC AGTTAAACGGGGCGCAACCGGCGAAACCAAAGTATTCACCGGAAACAGC AATAGTCCAAAGTCCCCCACCAAGGGAGGTTGC | 66 |
| M1354A | PTPYPNYANAGHVEGQSALFARDNGISEGLVFHNNPEGTCGFCVNMTETLL PENAKMTVVPPEGAIPVKRGATGETKVFTGNSNSPKSPTKGGC | 67 |
| | CCAACACCTTATCCTAACTACGCTAACGCCGGGCACGTCGAGGGGCAGT CAGCTCTTTTTgcaAGAGATAACGGCATTAGCGAAGGGCTTGTGTTCCATA ATAATCCTGAGGGCACCTGTGGCTTCTGTGTAAATATGACCGAAACACTTC TGCCTGAGAACGCTAAAATGACTGTCGTACCACCCGAAGGCGCAATCCC AGTTAAACGGGGCGCAACCGGCGAAACCAAAGTATTCACCGGAAACAGC AATAGTCCAAAGTCCCCCACCAAGGGAGGTTGC | 68 |
| E1361A | PTPYPNYANAGHVEGQSALFMRDNGISAGLVFHNNPEGTCGFCVNMTETLL | 69 |
| | PENAKMTWPPEGAIPVKRGATGETKVFTGNSNSPKSPTKGGC | |
| | CCAACACCTTATCCTAACTACGCTAACGCCGGGCACGTCGAGGGGCAGT CAGCTCTTTTTATGAGAGATAACGGCATTAGCgcaGGGCTTGTGTTCCATAA TAATCCTGAGGGCACCTGTGGCTTCTGTGTAAATATGACCGAAACACTTCT GCCTGAGAACGCTAAAATGACTGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 70 |
| G1362A | PTPYPNYANAGHVEGQSALFMRDNGISEALVFHNNPEGTCGFCVNMTETLL PENAKMTVVPPEGAIPVKRGATGETKVFTGNSNSPKSPTKGGC | 71 |
| | CCAACACCTTATCCTAACTACGCTAACGCCGGGCACGTCGAGGGGCAGT CAGCTCTTTTTATGAGAGATAACGGCATTAGCGAAgcaCTTGTGTTCCATAA TAATCCTGAGGGCACCTGTGGCTTCTGTGTAAATATGACCGAAACACTTCT GCCTGAGAACGCTAAAATGACTGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 72 |
| L1363A | PTPYPNYANAGHVEGQSALFMRDNGISEGAVFHNNPEGTCGFCVNMTETLL PENAKMTVVPPEGAIPVKRGATGETKVFTGNSNSPKSPTKGGC | 73 |
| | CCAACACCTTATCCTAACTACGCTAACGCCGGGCACGTCGAGGGGCAGT CAGCTCTTTTTATGAGAGATAACGGCATTAGCGAAGGGgcaGTGTTCCATA ATAATCCTGAGGGCACCTGTGGCTTCTGTGTAAATATGACCGAAACACTTC TGCCTGAGAACGCTAAAATGACTGTCGTACCACCCGAAGGCGCAATCCC AGTTAAACGGGGCGCAACCGGCGAAACCAAAGTATTCACCGGAAACAGC AATAGTCCAAAGTCCCCCACCAAGGGAGGTTGC | 74 |
| V1364A | PTPYPNYANAGHVEGQSALFMRDNGISEGLAFHNNPEGTCGFCVNMTETLL PENAKMTVVPPEGAIPVKRGATGETKVFTGNSNSPKSPTKGGC | 75 |
| | CCAACACCTTATCCTAACTACGCTAACGCCGGGCACGTCGAGGGGCAGT CAGCTCTTTTTATGAGAGATAACGGCATTAGCGAAGGGCTTgcaTTCCATAA TAATCCTGAGGGCACCTGTGGCTTCTGTGTAAATATGACCGAAACACTTCT GCCTGAGAACGCTAAAATGACTGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 76 |
| F1365A | PTPYPNYANAGHVEGQSALFMRDNGISEGLVAHNNPEGTCGFCVNMTETLL PENAKMTVVPPEGAIPVKRGATGETKVFTGNSNSPKSPTKGGC | 77 |
| | CCAACACCTTATCCTAACTACGCTAACGCCGGGCACGTCGAGGGGCAGT CAGCTCTTTTTATGAGAGATAACGGCATTAGCGAAGGGCTTGTGgcaCATA ATAATCCTGAGGGCACCTGTGGCTTCTGTGTAAATATGACCGAAACACTTC TGCCTGAGAACGCTAAAATGACTGTCGTACCACCCGAAGGCGCAATCCC AGTTAAACGGGGCGCAACCGGCGAAACCAAAGTATTCACCGGAAACAGC AATAGTCCAAAGTCCCCCACCAAGGGAGGTTGC | 78 |
| H1366A | PTPYPNYANAGHVEGQSALFMRDNGISEGLVFANNPEGTCGFCVNMTETLL PENAKMTVVPPEGAIPVKRGATGETKVFTGNSNSPKSPTKGGC | 79 |
| | CCAACACCTTATCCTAACTACGCTAACGCCGGGCACGTCGAGGGGCAGT CAGCTCTTTTTATGAGAGATAACGGCATTAGCGAAGGGCTTGTGTTCgcaA ATAATCCTGAGGGCACCTGTGGCTTCTGTGTAAATATGACCGAAACACTTC TGCCTGAGAACGCTAAAATGACTGTCGTACCACCCGAAGGCGCAATCCC | 80 |
| | AGTTAAACGGGGCGCAACCGGCGAAACCAAAGTATTCACCGGAAACAGC AATAGTCCAAAGTCCCCCACCAAGGGAGGTTGC | |
| K1389A | PTPYPNYANAGHVEGQSALFMRDNGISEGLVFHNNPEGTCGFCVNMTETLL PENAAMTVVPPEGAIPVKRGATGETKVFTGNSNSPKSPTKGGC | 81 |
| | CCAACACCTTATCCTAACTACGCTAACGCCGGGCACGTCGAGGGGCAGT CAGCTCTTTTTATGAGAGATAACGGCATTAGCGAAGGGCTTGTGTTCCATA ATAATCCTGAGGGCACCTGTGGCTTCTGTGTAAATATGACCGAAACACTTC TGCCTGAGAACGCTgcaATGACTGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 82 |
| M1390A | PTPYPNYANAGHVEGQSALFMRDNGISEGLVFHNNPEGTCGFCVNMTETLL PENAKATVVPPEGAIPVKRGATGETKVFTGNSNSPKSPTKGGC | 83 |
| | CCAACACCTTATCCTAACTACGCTAACGCCGGGCACGTCGAGGGGCAGT CAGCTCTTTTTATGAGAGATAACGGCATTAGCGAAGGGCTTGTGTTCCATA ATAATCCTGAGGGCACCTGTGGCTTCTGTGTAAATATGACCGAAACACTTC TGCCTGAGAACGCTAAAgcaACTGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 84 |
| T1391A | PTPYPNYANAGHVEGQSALFMRDNGISEGLVFHNNPEGTCGFCVNMTETLL PENAKMAVVPPEGAIPVKRGATGETKVFTGNSNSPKSPTKGGC | as |
| | CCAACACCTTATCCTAACTACGCTAACGCCGGGCACGTCGAGGGGCAGT CAGCTCTTTTTATGAGAGATAACGGCATTAGCGAAGGGCTTGTGTTCCATA ATAATCCTGAGGGCACCTGTGGCTTCTGTGTAAATATGACCGAAACACTTC TGCCTGAGAACGCTAAAATGgcaGTCGTACCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 86 |
| V1393A | PTPYPNYANAGHVEGQSALFMRDNGISEGLVFHNNPEGTCGFCVNMTETLL PENAKMTVAPPEGAIPVKRGATGETKVFTGNSNSPKSPTKGGC | 87 |
| | CCAACACCTTATCCTAACTACGCTAACGCCGGGCACGTCGAGGGGCAGT CAGCTCTTTTTATGAGAGATAACGGCATTAGCGAAGGGCTTGTGTTCCATA ATAATCCTGAGGGCACCTGTGGCTTCTGTGTAAATATGACCGAAACACTTC TGCCTGAGAACGCTAAAATGACTGTCgcaCCACCCGAAGGCGCAATCCCA GTTAAACGGGGCGCAACCGGCGAAACCAAAGTATTCACCGGAAACAGCA ATAGTCCAAAGTCCCCCACCAAGGGAGGTTGC | 88 |

### [DddAtox G1397-N variant]

| Type | SEQ | No. |
|---|---|---|
| C1376A | GSYALGPYQISAPQLPAYNGQTVGTFYYVNDAGGLESKVFSSGGPTPYPNY ANAGHVEGQSALFMRDNGISEGLVFHNNPEGTCGFAVNMTETLLPENAKMT VVPPEG | 89 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCgcaGTGAATATGACTGAAACCCTTCTGCCCGAAAATGCCAAGA TGACTGTCGTCCCACCTGAAGGC | 90 |
| V1377A | GSYALGPYQISAPQLPAYNGQTVGTFYYVNDAGGLESKVFSSGGPTPYPNY ANAGHVEGQSALFMRDNGISEGLVFHNNPEGTCGFCANMTETLLPENAKM TVVPPEG | 91 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCTGTgcaAATATGACTGAAACCCTTCTGCCCGAAAATGCCAAGA TGACTGTCGTCCCACCTGAAGGC | 92 |
| T1380A | GSYALGPYQISAPQLPAYNGQTVGTFYYVNDAGGLESKVFSSGGPTPYPNY ANAGHVEGQSALFMRDNGISEGLVFHNNPEGTCGFCVNMAETLLPENAKM TVVPPEG | 93 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCTGTGTGAATATGgcaGAAACCCTTCTGCCCGAAAATGCCAAGA TGACTGTCGTCCCACCTGAAGGC | 94 |
| E1381A | GSYALGPYQISAPQLPAYNGQTVGTFYYVNDAGGLESKVFSSGGPTPYPNY ANAGHVEGQSALFMRDNGISEGLVFHNNPEGTCGFCVNMTATLLPENAKMT VVPPEG | 95 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCTGTGTGAATATGACTgcaACCCTTCTGCCCGAAAATGCCAAGA TGACTGTCGTCCCACCTGAAGGC | 96 |
| L1384A | GSYALGPYQISAPQLPAYNGQTVGTFYYVNDAGGLESKVFSSGGPTPYPNY ANAGHVEGQSALFMRDNGISEGLVFHNNPEGTCGFCVNMTETLAPENAKM TVVPPEG | 97 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCTGTGTGAATATGACTGAAACCCCTTacaCCCGAAAATGCCAAGA | 98 |
| | TGACTGTCGTCCCACCTGAAGGC | |
| K1389A | GSYALGPYQISAPQLPAYNGQTVGTFYYVNDAGGLESKVFSSGGPTPYPNY ANAGHVEGQSALFMRDNGISEGLVFHNNPEGTCGFCVNMTETLLPENAAM TVVPPEG | 99 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCTGTGTGAATATGACTGAAACCCTTCTGCCCGAAAATGCCgcaA TGACTGTCGTCCCACCTGAAGGC | 100 |
| M1390A | GSYALGPYQISAPQLPAYNGQTVGTFYYVNDAGGLESKVFSSGGPTPYPNY ANAGHVEGQSALFMRDNGISEGLVFHNNPEGTCGFCVNMTETLLPENAKAT VVPPEG | 101 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCTGTGTGAATATGACTGAAACCCTTCTGCCCGAAAATGCCAAG gcaACTGTCGTCCCACCTGAAGGC | 102 |
| T1391A | GSYALGPYQISAPQLPAYNGQTVGTFYYVNDAGGLESKVFSSGGPTPYPNY ANAGHVEGQSALFMRDNGISEGLVFHNNPEGTCGFCVNMTETLLPENAKM AVVPPEG | 103 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCTGTGTGAATATGACTGAAACCCTTCTGCCCGAAAATGCCAAG ATGgcaGTCGTCCCACCTGAAGGC | 104 |
| V1392A | GSYALGPYQISAPQLPAYNGQTVGTFYYVNDAGGLESKVFSSGGPTPYPNY ANAGHVEGQSALFMRDNGISEGLVFHNNPEGTCGFCVNMTETLLPENAKM TAVPPEG | 105 |
| | GGTAGCTACGCACTTGGTCCTTACCAGATTAGCGCACCCCAACTCCCCGC CTATAATGGTCAAACCGTCGGGACCTTTTACTACGTAAACGATGCTGGTG GGCTGGAATCCAAAGTATTCTCCTCAGGGGGCCCTACACCCTACCCCAAC TACGCCAATGCTGGTCATGTAGAAGGGCAGTCAGCACTGTTTATGCGCGA TAATGGTATAAGCGAGGGGTTGGTCTTCCATAACAACCCAGAGGGTACTT GTGGCTTCTGTGTGAATATGACTGAAACCCTTCTGCCCGAAAATGCCAAG ATGACTgcaGTCCCACCTGAAGGC | 706 |

### [DddAtox G1397-C variant]

| Type | SEQ | No. |
|---|---|---|
| K1410A | AIPVKRGATGETAVFTGNSNSPKSPTKGGC | 107 |
| | GCCATACCTGTGAAGCGGGGAGCAACAGGGGAGACAgcaGTGTTCACAG GCAACTCTAACAGTCCAAAGAGCCCCACCAAAGGCGGGTGT | 108 |
| V1411A | AIPVKRGATGETKAFTGNSNSPKSPTKGGC | 109 |
| | GCCATACCTGTGAAGCGGGGAGCAACAGGGGAGACAAAGgcaTTCACAG GCAACTCTAACAGTCCAAAGAGCCCCACCAAAGGCGGGTGT | 110 |
| F1412A | AIPVKRGATGETKVATGNSNSPKSPTKGGC | 111 |
| | GCCATACCTGTGAAGCGGGGAGCAACAGGGGAGACAAAGGTGgcaACAG GCAACTCTAACAGTCCAAAGAGCCCCACCAAAGGCGGGTGT | 112 |
| T1413A | AIPVKRGATGETKVFAGNSNSPKSPTKGGC | 113 |
| | GCCATACCTGTGAAGCGGGGAGCAACAGGGGAGACAAAGGTGTTCgcaG GCAACTCTAACAGTCCAAAGAGCCCCACCAAAGGCGGGTGT | 114 |

The cytosine deaminase variant according to the present invention may include at least one sequence selected from the group consisting of amino acid sequences described in the tables above. The cytosine deaminase variant according to the present invention shows the possibility of reducing unwanted editing on various bases in non-specific target sites.

### 3. Full-length cytosine deaminase

The inventors of the present application developed a new programmable cytosine deaminase using full-length DddA made by modifying the positively charged amino acid of a wild-type cytosine deaminase DddAₜₒₓ, which is used in a split form due to cytotoxicity thereof.

The present invention pertains to a fusion protein including (i) a DNA-binding protein and (ii) a cytosine deaminase or a variant thereof, in which the cytosine deaminase or the variant thereof is a non-toxic full-length cytosine deaminase.

At the C-terminus of DddAₜₒₓ, positively charged amino acids (KRKKK) are specifically clustered. Since DNA is negatively charged, it binds to the positively charged amino acid of a protein. By substituting the positively charged amino acid with an amino acid that is not charged, binding force of DddAₜₒₓ to DNA is weakened, thus reducing or eliminating cytotoxicity. Particularly, a non-toxic combination resulting from substitution of a positively charged amino acid with a non-polar amino acid enables cloning using *E. coli* to afford full-length DddA.

Wild-type DddAₜₒₓ is used in two split forms due to cytotoxicity thereof, which has many limitations in experiments. In particular, when Cas9 is used, orthogonal Cas9 variants that recognize other PAMs are used. As such, since PAM is limitedly present, it is often difficult to deaminate cytosine to thymine exactly at a desired position. In addition, the target window with the highest activity is a region 40-bp long between two Cas9 variants that bind to each other, and unwanted cytosine in this region is also deaminated. However, full-length DddA is not constrained by PAM because it is not isolated. Furthermore, it is most active in the TC motif within 10 bps from the target position, resulting in high accuracy.

It was confirmed that Cas9 deaminates cytosine in the ACA, GC, and CC motifs in the R-loop formed by binding to the target site to thymine. This is activity that has not been identified in an isolated form.

In full-length DddA, it is possible to substitute thymine for cytosine at a desired position using a TALE module or a zinc finger protein, as well as Cas9. The existing DddAₜₒₓ has to be delivered in pairs in an isolated form, but full-length DddA may use only one module of the TALE module or zinc finger protein, so that the target site may be selected without restriction. In addition, cytosine of specific DNA may be converted to thymine by targeting DNA in mitochondria, plant chloroplasts, or plastids, as well as genomic sites.

In addition, all constructs may be inserted into AAV, which is a viral vector used for gene therapy, due to a small size thereof. The existing CBE (cytosine base editor) substitutes thymine for cytosine in the R-loop formed by Cas9 binding to the target site, but the full-length DddA invented herein deaminates cytosine outside the R-loop. Therefore, it is possible to convert cytosine to thymine at positions where editing is restricted with conventional CBE.

Based thereon, in the non-toxic full-length cytosine deaminase, at least one, at least two, at least three, at least four, or at least five amino acids of the wild-type deaminase of SEQ ID NO: 1 may be substituted with a different amino acid.

Here, the "different amino acid" may be alanine, isoleucine, leucine, methionine, phenylalanine, proline, tryptophan, valine, asparagine, cysteine, glutamine, glycine, serine, threonine, tyrosine, aspartic acid, glutamic acid, arginine, histidine, or lysine, and may refer to an amino acid selected from among amino acids excluding amino acids at original mutation positions in a wild-type protein from all known variants of the amino acids described above. In an exemplary embodiment, the "different amino acid" may be alanine.

The non-toxic full-length DddA may include a sequence selected from the group consisting of SEQ ID NO: 12 to SEQ ID NO: 18 depending on the type thereof.
Wild type (SEQ ID No: 1) 
GSYALGPYQISAPQLPAYNGQTVGTFYYVNDAGGLESKVFSSGGPTPY
PNYANAGHVEGQSALFMRDNGISEGLVFHNNPEGTCGFCVNMTETLLPENAKMTVV
PPEGAIPVKRGATGETKVFTGNSNSPKSPTKGGC
A1341D KRKKA (SEQ ID No: 12) 
GSYALGPYQISAPQLPAYNGQTVGTFYYVNDAGGLESKVFSSGGPTPY
PNYDNAGHVEGQSALFMRDNGISEGLVFHNNPEGTCGFCVNMTETLLPENAKMTVV
PPEGAIPVKRGATGETKVFTGNSNSPKSPTAGGC
AAAAA (SEQ ID No: 13) 
GSYALGPYQISAPQLPAYNGQTVGTFYYVNDAGGLESKVFSSGGPTPY
PNYANAGHVEGQSALFMRDNGISEGLVFHNNPEGTCGFCVNMTETLLPENAKMTVV
PPEGAIPVAAGATGETAVFTGNSNSPASPTAGGC
AAAAK (SEQ ID No: 14) GSYALGPYQISAPQLPAYNGQTVGTFYYVNDAGGLESKVFSSGGPTPY 
PNYANAGHVEGQSALFMRDNGISEGLVFHNNPEGTCGFCVNMTETLLPENAKMTVV

PPEGAIPVAAGATGETAVFTGNSNSPASPTKGGC
AAKAA (SEQ ID No: 15) 
GSYALGPYQISAPQLPAYNGQTVGTFYYVNDAGGLESKVFSSGGPTPY
PNYANAGHVEGQSALFMRDNGISEGLVFHNNPEGTCGFCVNMTETLLPENAKMTVV
PPEGAIPVAAGATGETKVFTGNSNSPASPTAGGC
AAKAK (SEQ ID No: 16) 
GSYALGPYQISAPQLPAYNGQTVGTFYYVNDAGGLESKVFSSGGPTPY
PNYANAGHVEGQSALFMRDNGISEGLVFHNNPEGTCGFCVNMTETLLPENAKMTVV
PPEGAIPVAAGATGETKVFTGNSNSPASPTKGGC
KAAAA (SEQ ID No: 17) 
GSYALGPYQISAPQLPAYNGQTVGTFYYVNDAGGLESKVFSSGGPTPY
PNYANAGHVEGQSALFMRDNGISEGLVFHNNPEGTCGFCVNMTETLLPENAKMTVV
PPEGAIPVKAGATGETAVFTGNSNSPASPTAGGC
E1347A (SEQ ID No: 18)

GSYALGPYQISAPQLPAYNGQTVGTFYYVNDAGGLESKVFSSGGPTPY
PNYANAGHVAGQSALFMRDNGISEGLVFHNNPEGTCGFCVNMTETLLPENAKMTVV
PPEGAIPVKRGATGETKVFTGNSNSPKSPTKGGC

The full-length deaminase variant may include at least one substitution selected from the group consisting of the following in the amino acid sequence of SEQ ID NO: 1:
substitution of S at position 37 with G;
substitution of G at position 59 with S;
substitution of A at position 109 with V; and
substitution of S at position 129 with G.

In an embodiment, the full-length deaminase variant may include the sequence of SEQ ID NO: 19, including substitution of S at position 37 with G, substitution of G at position 59 with S, substitution of A at position 109 with V, and substitution of S at position 129 with G, in the amino acid sequence of SEQ ID NO: 1.

GSVG (SEQ ID No: 19)

GSYALGPYQISAPQLPAYNGQTVGTFYYVNDAGGLEGKVFSSGGPTPY

PNYANAGHVESQSALFMRDNGISEGLVFHNNPEGTCGFCVNMTETLLPENAKMTVV

PPEGVIPVKRGATGETKVFTGNSNGPKSPTKGGC

The full-length DddA GSVG enables cloning using general *E. coli.* It was confirmed in the human cell genomic context that the full-length DddA GSVG deaminates cytosine of the TC motif at the target site to thymine. The full-length DddA GSVG may be cloned to each of the N-terminus and C-terminus of Cas9. DddA GSVG linked to the N-terminus of Cas9 at the same target site may substitute thymine for cytosine. It was confirmed in the human cell that DddA GSVG linked to the C-terminus of Cas9 induces cytosine-to-thymine substitutions in the TC motif (guanine-to-adendine substitutions in the complementary sequence).

In an embodiment, the full-length deaminase variant may include a sequence selected from the group consisting of SEQ ID NOs: 20 to 22.
SSVG (SEQ ID No: 20) 
GSYALGPYQISAPQLPAYNGQTVGTFYYVNDAGGLESKVFSSGGPTPY
PNYANAGHVESQSALFMRDNGISEGLVFHNNPEGTCGFCVNMTETLLPENAKMTVV
PPEGVIPVKRGATGETKVFTGNSNGPKSPTKGGC
GSAG (SEQ ID No: 21) 
GSYALGPYQISAPQLPAYNGQTVGTFYYVNDAGGLEGKVFSSGGPTPY
PNYANAGHVESQSALFMRDNGISEGLVFHNNPEGTCGFCVNMTETLLPENAKMTVV
PPEGAIPVKRGATGETKVFTGNSNGPKSPTKGGC
GSVS (SEQ ID No: 22)

GSYALGPYQISAPQLPAYNGQTVGTFYYVNDAGGLEGKVFSSGGPTPY
PNYANAGHVESQSALFMRDNGISEGLVFHNNPEGTCGFCVNMTETLLPENAKMTVV
PPEGVIPVKRGATGETKVFTGNSNSPKSPTKGGC

### 4. DNA-binding protein

The DNA-binding protein may be, for example, a zinc finger protein, a TALE (transcription activator-like effector) protein, a CRISPR-associated nuclease, or combinations of two or more thereof.

The zinc finger motif of the zinc finger protein has a DNA-binding domain, and the c-terminal portion of the finger specifically recognizes a DNA sequence. The DNA-binding protein containing 3 to 6 zinc finger motifs recognizes the DNA sequence.

In an embodiment, each of the first split and the second split of the cytosine deaminase may be fused to the N-terminus or C-terminus of the zinc finger protein.

The C-terminus of the zinc finger protein (ZF-Left) is fused to the N-terminus of the first split of the cytosine deaminase and the C-terminus of the zinc finger protein (ZF-Right) is fused to the N-terminus of the second split of the cytosine deaminase (CC configuration).

The N-terminus of the zinc finger protein (ZF-Left) is fused to the C-terminus of the first split of the cytosine deaminase and the C-terminus of the zinc finger protein (ZF-Right) is fused to the N-terminus of the second split of the cytosine deaminase (NC configuration).

The C-terminus of the zinc finger protein (ZF-Left) is fused to the N-terminus of the first split of the cytosine deaminase and the N-terminus of the zinc finger protein (ZF-Right) is fused to the C-terminus of the second split of the cytosine deaminase (CN configuration).

The N-terminus of the zinc finger protein (ZF-Left) is fused to the C-terminus of the first split of the cytosine deaminase and the N-terminus of the zinc finger protein (ZF-Right) is fused to the C-terminus of the second split of the cytosine deaminase (NN configuration).

The ZF-Left may include the sequence of SEQ ID NO: 2 below:

[SEQ ID NO: 2]

GIHGVPAAMAERPFQCRICMRNFSDRSNLSRHIRTHTGEKPFACDICG

RKFAISSNLNSHTKIHTGSQKPFQCRICMRNFSRSDNLARHIRTHTGEKPFACDIC

GRKFATSGNLTRHTKIHLR.

The ZF-Right may include the sequence of SEQ ID NO: 3 below:

[SEQ ID NO: 3]

GIHGVPAAMAERPFQCRICMRNFSRSDNLSVHIRTHTGEKPFACDICG

RKFAQKINLQVHTKIHTGEKPFQCRICMRNFSRSDVLSEHIRTHTGEKPFACDICG

RKFAQRNHRTTHTKIHLR.

The sequence of the ZF may vary depending on the DNA target. ZFs may be custom-made depending on the DNA target sequence. Since a ZF recognizes 3-bp DNA, it is possible to construct a ZF combination that recognizes 9-18 bp DNA by connecting 3 to 6 ZFs. For example, it may be produced using a library that includes modules recognizing GNN, TNN, CNN, or ANN.

In some cases, the zinc finger protein may be linked to the deaminase through a linker. The linker may be a peptide linker including 2 to 40 amino acid residues. The linker may be, for example, a linker having a length of 2aa, 5aa, 10aa, 16aa, 24aa, or 32aa, but is not limited thereto.

In an embodiment, the linker may include:
2a.a linker: GS
5a.a linker: TGEKQ (SEQ ID NO: 8)
10a.a linker: SGAQGSTLDF (SEQ ID NO: 9)
16a.a linker: SGSETPGTSESATPES (SEQ ID NO: 10);
24a.a linker: SGTPHEVGVYTLSGTPHEVGVYTL (SEQ ID NO: 115); or
32a.a linker: GSGGSSGGSSGSETPGTSESATPESSGGSSGGS (SEQ ID NO: 11).

In a specific embodiment according to the present invention, the split deaminase and the zinc finger protein may be linked through a linker, the zinc finger protein is fused to the N-terminus of the split half deaminase including the first split, and the zinc finger protein is fused to the N-terminus of the half deaminase including the second split. Here, C-to-T base conversion may occur in the spacer between the left and right ZFP-binding sites. Both the left and right ZFPs were confirmed to show high editing efficiency when linked respectively to the half deaminase including the first split and the half deaminase including the second split through the 24a.a linker.

The TAL effector (TALE) is configured such that 33-34 amino acid sequences are repeated, and about nine RVDs (repeated variant domains) are repeated. It is able to recognize one nucleotide per domain and may bind to a specific DNA sequence depending on the 12^{th}-13^{th} amino acid sequences (HD->cytosine, NI->adenine, NG->thymine, NN->guanine). The TAL effector (TALE) recognizes single-stranded DNA within the target site. The distance between target sites may be 12-14 nucleotides.

The TALE domain is a protein domain that binds to a nucleotide in a sequence-specific manner by at least one TALE-repeat combination. It includes at least one TALE-repeat, particularly 1 to 30 TALE-repeats, but is not limited thereto. TALE-repeat is a domain that recognizes a specific nucleotide sequence in the TALE domain.

The TALE domain includes a region including the N-terminus of TALE and a region including the C-terminus of TALE as a backbone structure. The first TALE including the N-terminus of TALE may be encoded by SEQ ID NO: 4 or 5. The second TALE including the C-terminus of TALE may be encoded by SEQ ID NO: 6 or 7.

| Name | Sequence | No. |
|---|---|---|
| First TALE N-term | GATATAGCTGATTTGAGGACCCTCGGGTACTCACAGCAGCAACAAGAAAAAATCAAACCAA AGGTACGTAGCACGGTTGCCCAACATCATGAAGCCCTAGTTGGTCACGGATTTACTCATGC CCATATTGTTGCACTGAGTCAACACCCTGCTGCATTAGGCACAGTGGCTGTGAAATATCAA GATATGATTGCTGCTCTTCCTGAGGCCACACACGAGGCAATTGTGGGTGTTGGAAAACAAT GGTCTGGTGCGCGAGCGCTTGAAGCTCTCCTAACCGTAGCTGGAGAGTTGAGAGGCCCACC GCTTCAGCTTGACACAGGGCAGCTGTTGAAGATAGCAAAGAGGGGGGGTGTAACTGCTGTT GAAGCAGTCCATGCATGGAGAAATGCATTAACTGGAGCTCCA | 4 |
| | DIADLRTLGYSQQQQEKIKPKVRSTVAQHHEALVGHGFTHAHIVALSQHPAALGTVAVKYQ DMIAALPEATHEAIVGVGKQWSGARALEALLTVAGELRGPPLQLDTGQLLKIAKRGGVTAV EAVHAWRNALTGAP | 269 |
| Secon d TALE N-term | GATATTGCTGATCTTCGAACATTGGGTTATTCTCAGCAGCAACAAGAGAAAATCAAACCGA AAGTCCGTTCAACCGTAGCTCAACATCATGAAGCCCTTGTTGGTCATGGATTTACTCATGC TCACATTGTTGCTCTAAGTCAGCACCCTGCCGCGCTAGGCACAGTTGCCGTCAAGTATCAA GATATGATAGCTGCTTTACCTGAAGCAACCCACGAGGCAATAGTGGGGGTTGGGAAGAGGG GTGCAGGCGCCAGGGCACTGGAGGCACTCTTAACAGTAGCTGGAGAACTTCGGGGACCACC ATTGCAGCTTGATACTGGACAACTCTTGAAGATCGCAAAAAGAGGGGGTGTGACTGCTGTG GAAGCTGTTCATGCATGGAGAAATGCTTTGACGGGAGCACCCCTG | 5 |
| | DIADLRTLGYSQQQQEKIKPKVRSTVAQHHEALVGHGFTHAHIVALSQHPAALGTVAVKYQ DMIAALPEATHEAIVGVGKRGAGARALEALLTVAGELRGPPLQLDTGQLLKIAKRGGVTAV EAVHAWRNALTGAPL | 270 |
| First TALE C-term | AGCATAGTTGCTCAGCTAAGTAGACCTGATCCAGCACTTGCTGCATTGACGAACGATCATC TCGTAGCGCTGGCTTGTTTGGGCGGCAGGCCAGCCTTGGACGCCGTGAAGAAAGGATTAGG T | 6 |
| | SIVAQLSRPDPALAALTNDHLVALACLGGRPALDAVKKGLG | 271 |
| Secon d TALE C-term | TCGATAGTAGCACAATTAAGCAGGCCTGACCCTGCACTTGCTGCTCTTACAAATGATCATC TTGTTGCTCTGGCCTGTTTGGGTGGGCGTCCCGCGTTGGATGCTGTTAAGAAAGGACTTGG T | 7 |
| | SIVAQLSRPDPALAALTNDHLVALACLGGRPALDAVKKGLG | 271 |

Depending on the position to which the TALE domain binds based on the cleavage site, a single TALE array or each of a first TALE array and a second TALE array may bind thereto.

The first TALE (left TALE) may be fused to the first split of the cytosine deaminase and the second TALE (right TALE) may be fused to the second split of the cytosine deaminase. Respective constructs can be described as N'-TALE-first split-C' and N'-TALE-second split-C'.

When the cytosine deaminase is full-length, a single-module TALE may bind to the N-terminus of the cytosine deaminase. A single TALE module and a cytosine deaminase are included in the N-C direction. A dual module TALE may be included, in which the first TALE may be fused to the N-terminus of the full-length cytosine deaminase and the second TALE may be separately included. The first TALE module and the cytosine deaminase are included in the N-C direction, and constructs of N'-TALE-cytosine deaminase-C' and N'-TALE-C' are provided.

A TALE array may be custom-made depending on the target DNA sequence. The TALE array is configured such that modules composed of 33 to 35 amino acid residues are repeatedly arranged. These are derived from the plant pathogen Xanthomonas, and a module recognizes each of bases A, C, G, and T, which then binds to DNA. The base specificity of each module is determined by the 12^{th} and 13^{th} amino acid residues, so-called repeat variable diresidue (RVD). For example, a module in which RVD is NN recognizes G, and NI recognizes A, HD recognizes C, and NG recognizes T. The TALE array may be composed of at least 14 to 18 modules and may be designed to recognize a target DNA sequence 15-20 bp long.

Regarding the CRISPR-associated nuclease, two RNAs are encoded in the CRISPR array, one is crRNA (CRISPR RNA) and the remaining one is tracrRNA (transactivating CRISPR RNA). Also, crRNA is transcribed in the protospacer site and binds to tracrRNA to form a tertiary structure. Two types of RNA help recognize and cleave foreign DNA.

The Cas protein may include, but is not limited to, Cas1, Cas1B, Cas2, Cas3, Cas4, Cas5, Cas6, Cas7, Cas8, Cas9, Cas10, Cas12a, Cas12b, Cas12c, Cas12d, Cas12e, Cas12g, Cas12h, Cas12i, Cas12j, Cas13a, Cas13b, Cas13c, Cas13d, Cas14, Csy1, Csy2, Csy3, Cse1, Cse2, Csc1, Csc2, Csa5, Csn2, CsMT2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, or Csf4 endonuclease.

The Cas protein may be derived from the genus of microorganisms containing an ortholog of the Cas protein selected from the group consisting of Corynebacter, Sutterella, Legionella, Treponema, Filifactor, Eubacterium, Streptococcus (*Streptococcus pyogenes*), Lactobacillus, Mycoplasma, Bacteroides, Flaviivola, Flavobacterium, Azospirillum, Gluconacetobacter, Neisseria, Roseburia, Parvibaculum, Staphylococcus (*Staphylococcus aureus*)*,* Nitratifractor, Corynebacterium, and Campylobacter, and may be simply isolated therefrom or recombinant.

The Cas protein may be included in a mutated form, which may lose endonuclease activity. There is exemplified at least one selected from among mutation target-specific nucleases mutated to lose endonuclease activity and have nickase activity and forms mutated to lose both the endonuclease activity and the nickase activity.

When having nickase activity, simultaneously with base conversion by the cytosine deaminase (e.g. cytosine-to-uridine conversion) or sequentially regardless of the order, a nick may be introduced to the strand where base conversion occurs or the opposite strand (e.g. the strand opposite the strand where base conversion occurs) (e.g. a nick is introduced at a position between the 3^{rd} nucleotide and the 4^{th} nucleotide in a direction of the 5' end of the PAM sequence on the strand opposite the strand where PAM is located). Such mutations (e.g. amino acid substitutions, etc.) may occur in a catalytically active domain (e.g. a RuvC catalytic domain in Cas9). Also, *Streptococcus pyogenes*-derived Cas9 may include mutations in which at least one selected from the group consisting of a catalytically active aspartate residue (aspartic acid at position 10 (D10), etc.), glutamic acid at position 762 (E762), histidine at position 840 (H840), asparagine at position 854 (N854), asparagine at position 863 (N863), aspartic acid at position 986 (D986), and the like is substituted with any different amino acid. Here, any different amino acid that is substituted may be alanine, but is not limited thereto.

In some cases, the *Streptococcus pyogenes-*derived Cas9 protein may be mutated to recognize NGA (in which N is any base selected from among A, T, G, and C) that is different from the PAM sequence (NGG) of wild-type Cas9 by substituting at least one selected from among aspartic acid at position 1135 (D1135), arginine at position 1335 (R1335), and threonine at position 1337 (T1337), for example, all three, with a different amino acid.

For example, in the amino acid sequences of the *Streptococcus pyogenes*-derived Cas9 protein, amino acid substitution may occur at:
(1) D10, H840, or D10 + H840;
(2) D1135, R1335, T1337, or D1135 + R1335 + T1337; or
(3) both residues (1) and (2).

Here, the "different amino acid" may be alanine, isoleucine, leucine, methionine, phenylalanine, proline, tryptophan, valine, asparagine, cysteine, glutamine, glycine, serine, threonine, tyrosine, aspartic acid, glutamic acid, arginine, histidine, or lysine, and may refer to an amino acid selected from among amino acids excluding amino acids at original mutation positions in a wild-type protein from all known variants of the amino acids described above. In an exemplary embodiment, the "different amino acid" may be alanine, valine, glutamine, or arginine.

In some cases, guide RNA may be further included. The guide RNA may be, for example, at least one selected from the group consisting of CRISPR RNA (crRNA), transactivating crRNA (tracrRNA), and single guide RNA (sgRNA). Specifically, it may be a double-stranded crRNA:tracrRNA complex in which crRNA and tracrRNA bind to each other, or single-stranded guide RNA (sgRNA) in which crRNA or a portion thereof and tracrRNA or a portion thereof are linked by an oligonucleotide linker.

### 5. Addition of adenine deaminase

The invention pertains a fusion protein comprising three components: a DNA-binding protein, a cytosine deaminase or variant thereof, and an adenine deaminase. The cytosine deaminase or variant thereof is split into two parts, called "splits," which are derived from a non-toxic full-length cytosine deaminase or from a cytosine deaminase or variant thereof. Both splits are fused to the DNA-binding protein.

The inventors of the present application constructed a base editor capable of editing base A by linking an adenine deaminase capable of causing A-to-G conversion with a DddAtox cytosine deaminase to a TALE or ZFP protein capable of binding to DNA.

The deaminase (DdCBE) using existing DddAtox is a cytosine deaminase that uses a TALE repeat as a DNA-binding module. Unlike DdCBE, which only causes C-to-T conversion, DdABE may induce A-to-G conversion, and thus other mutation patterns may be created.

Since DdABE recognizes double-stranded DNA by itself and causes deamination, there is no additional component such as RNA. For mitochondria or chloroplasts, the delivery mechanism of RNA is not known, and thus the Crispr system cannot be applied. However, DdABE without such a component is able not only to target genomic DNA in cells, but also to target DNA in organelles such as mitochondria or chloroplasts, inducing A-to-G conversion of specific DNA.

Currently, DdCBE is only gene editing technology that targets mitochondria or organelles. Therefore, mutations that may be introduced through all conventional technologies may include C-to-T conversion alone, but DdABE may induce A-to-G conversion, and thus the spectrum that may introduce mutations becomes much more diverse. This makes it possible to create or treat mitochondrial disease models that have not been possible to date.

Existing DdCBE requires two TALE modules (attached to the left and right), and as such, it cannot be loaded on AAV, which is a viral vector with low gene capacity in gene therapy. However, since DdABE may be used as a single module capable of using only one TALE module, it may be loaded on AAV and is useful for gene therapy.

DdABE has high compatibility because it is able to use either a split DddAtox or a full-length DddAtox variant, as necessary.

The adenine deaminase may be selected from the group consisting of, for example, APOBEC1 (apolipoprotein B editing complex 1), AID (activation-induced deaminase), and tadA (tRNA-specific adenosine deaminase), and may be particularly tadA (tRNA-specific adenosine deaminase). The adenine deaminase may be, for example, a deoxy-adenine deaminase as a variant of *E*. *coli* TadA.

The adenine deaminase may be fused to the C-terminus of the zinc finger protein (ZF-left), the N-terminus or C-terminus of the first split of the cytosine deaminase, the N-terminus of the zinc finger protein (ZF-right), or the N-terminus or C-terminus of the second split of the cytosine deaminase in a construct in which cytosine deaminase is splitted, the DNA-binding protein is a zinc finger protein, the N-terminus of the zinc finger protein (ZF-left) is fused to the C-terminus of the first split of the cytosine deaminase, and the C-terminus of the zinc finger protein (ZF-right) is fused to the N-terminus of the second split of the cytosine deaminase (NC configuration).

Also, the adenine deaminase may be fused to the C-terminus of the zinc finger protein (ZF-left), the N-terminus or C-terminus of the first split of the cytosine deaminase, the N-terminus of the zinc finger protein (ZF-right), or the N-terminus or C-terminus of the second split of the cytosine deaminase even in constructs in which the C-terminus of the zinc finger protein (ZF-left) is fused to the N-terminus of the first split of the cytosine deaminase and the C-terminus of the zinc finger protein (ZF-right) is fused to the N-terminus of the second split of the cytosine deaminase (CC configuration); the C-terminus of the zinc finger protein (ZF-left) is fused to the N-terminus of the first split of the cytosine deaminase and the N-terminus of the zinc finger protein (ZF-right) is fused to the C-terminus of the second split of the cytosine deaminase (CN configuration); or the N-terminus of the zinc finger protein (ZF-left) is fused to the C-terminus of the first split of the cytosine deaminase and the N-terminus of the zinc finger protein (ZF-right) is fused to the C-terminus of the second split of the cytosine deaminase (NN configuration).

When the cytosine deaminase is included in a split form and the DNA-binding protein is TALE, the first TALE may be fused to the first split of the cytosine deaminase and the second TALE may be fused to the second split of the cytosine deaminase, and respective constructs may be described as N'-TALE-first split DddA-C' and N'-TALE-second split DddA-C'. The adenine deaminase may be fused to the N-terminus or C-terminus of the first split of the cytosine deaminase or to the N-terminus or C-terminus of the second split of the cytosine deaminase.

When the cytosine deaminase is included in a full-length form and the DNA-binding protein is TALE, a single TALE module may be N'-TALE-full length DDDA-C'. Here, the adenine deaminase may fuse to the N-terminus or C-terminus of the cytosine deaminase. Here, the adenine deaminase may be fused to the C-terminal direction of the single TALE module or to the N-terminus or C-terminus of the cytosine deaminase.

When the cytosine deaminase is included in a full-length form and the DNA-binding protein is TALE, a dual TALE module may be included, the first TALE module and the cytosine deaminase may be included in the N-C direction (N'-TALE-full length DDDA-C'), and the adenine deaminase and the second split including the second TALE may be further included (N'-TALE-adenine deaminase-C'). Here, the adenine deaminase may be fused to the N-terminus or C-terminus of TALE such as in constructs of N'-TALE-cytosine deaminase-C' and N'-TALE-adenine deaminase-C'.

In some cases, UGI (uracil DNA glycosylase inhibitor) capable of increasing base editing efficiency may be additionally included. UGI is able to increase base editing efficiency by inhibiting the activity of UDG (uracil DNA glycosylase), which is an enzyme that repairs mutant DNA through removal of U from DNA.

The present invention pertains to a composition for A-to-G base editing in prokaryotic or eukaryotic cells including the fusion protein or a nucleic acid encoding the same, in which the DNA-binding protein is a zinc finger protein, a TALE protein, or a CRISPR-associated nuclease, and the cytosine deaminase of the fusion protein or a variant thereof is derived from bacteria and is specific to double-stranded DNA.

The present invention pertains to a composition for A-to-G base editing in prokaryotic or eukaryotic cells including the fusion protein or a nucleic acid encoding the same, in which the DNA-binding protein is a zinc finger protein, a TALE protein, or a CRISPR-associated nuclease.
a cytosine deaminase of the fusion protein or a variant thereof is derived from bacteria and is specific to double-stranded DNA. The DNA-binding protein is fused to both the N-terminus and the C-terminus of the cytosine deaminase or the variant thereof. Similarly, the DNA-binding protein also is fused both the N-terminus and the C-terminus of the adenine deaminase of the fusion protein. In the context of the fusion protein including a DNA-binding protein, a cytosine deaminase or variant thereof, and an adenine deaminase, the adenine deaminase may be located at the N-terminus or C-terminus of the cytosine deaminase within the fusion protein, or may be present as a separate protein independent of other DNA-binding proteins.

The present invention pertains to a composition for C-to-T base editing in prokaryotic or eukaryotic cells including the fusion protein or a nucleic acid encoding the same and UGI (uracil glycosylase inhibitor), in which the DNA-binding protein is a zinc finger protein, a TALE protein, or a CRISPR-associated nuclease, and the cytosine deaminase of the fusion protein or a variant thereof is derived from bacteria and is specific to double-stranded DNA.

Specifically, the present invention pertains to a composition for A-to-G base editing (without UGI) in prokaryotic and eukaryotic cells including 1) a DNA-binding protein, 2) a full-length double-stranded DNA-specific bacterial cytosine deaminase or a variant thereof, and 3) a deoxy-adenine deaminase derived from *E*. *coli* TadA, in which the DNA-binding protein is a zinc finger protein (ZFP), a transcription activator-like effector (TALE) array, or a catalytically deficient CRISPR-Cas9 (nCas9 or dCas9) or Cas12a, and the full-length double-stranded DNA-specific bacterial cytosine deaminase is DddAtox derived from *Burkholderia cenocepacia.*

The present invention pertains to a composition for A-to-G base editing (without UGI) in prokaryotic and eukaryotic cells including 1) a left DNA-binding protein operably linked to a full-length double-stranded DNA-specific bacterial cytosine deaminase or a variant thereof and 2) a right DNA-binding protein operably linked to a deoxy-adenine deaminase derived from *E. coli* TadA, in which the left or right DNA-binding protein is a zinc finger protein (ZFP), a transcription activator-like effector (TALE) array, or a catalytically deficient CRISPR-Cas9 (nCas9 or dCas9) or Cas12a, and the full-length double-stranded DNA-specific bacterial cytosine deaminase is DddAtox derived from *Burkholderia cenocepacia.* The order of the left and right components in the fusion protein can be interchanged.

The present invention also pertains to a composition for A-to-G and C-to-T base editing in prokaryotic and eukaryotic cells including 1) a DNA-binding protein, 2) a full-length double-stranded DNA-specific bacterial cytosine deaminase or a variant thereof, 3) a deoxy-adenine deaminase derived from *E*. *coli* TadA, and 4) UGI (uracil glycosylase inhibitor), in which the DNA-binding protein is a zinc finger protein (ZFP), a transcription activator-like effector (TALE) array, or a catalytically deficient CRISPR-Cas9 (nCas9 or dCas9) or Cas12a, and the full-length double-stranded DNA-specific bacterial cytosine deaminase is DddAtox derived from *Burkholderia cenocepacia.*

The present invention also pertains to a composition for A-to-G base editing (without UGI) in prokaryotic and eukaryotic cells including 1) a DNA-binding protein, 2) a split double-stranded DNA-specific bacterial cytosine deaminase or a variant thereof, and 3) a deoxy-adenine deaminase derived from *E. coli* TadA, in which the DNA-binding protein is a zinc finger protein (ZFP), a transcription activator-like effector (TALE) array, or a catalytically deficient CRISPR-Cas9 (nCas9 or dCas9) or Cas12a, and the split double-stranded DNA-specific bacterial cytosine deaminase is DddAtox derived from *Burkholderia cenocepacia.*

The present invention also pertains to a composition for A-to-G and C-to-T base editing in prokaryotic and eukaryotic cells including 1) a DNA-binding protein, 2) a split double-stranded DNA-specific bacterial cytosine deaminase or a variant thereof, 3) a deoxy-adenine deaminase derived from *E. coli* TadA, and 4) UGI (uracil glycosylase inhibitor), in which the DNA-binding protein is a zinc finger protein (ZFP), a transcription activator-like effector (TALE) array, or a catalytically deficient CRISPR-Cas9 (nCas9 or dCas9) or Cas12a, and the split double-stranded DNA-specific bacterial cytosine deaminase is DddAtox derived from *Burkholderia cenocepacia.*

The present invention pertains to a method for A-to-G base editing in prokaryotic or eukaryotic cells including treating a prokaryotic cell or a eukaryotic cell with the fusion protein or a nucleic acid encoding the same, in which the DNA-binding protein is a zinc finger protein, a TALE protein, or a CRISPR-associated nuclease, and the cytosine deaminase of the fusion protein or a variant thereof is derived from bacteria and is specific to double-stranded DNA.

The present invention pertains to a method for A-to-G base editing in prokaryotic or eukaryotic cells including treating a prokaryotic cell or a eukaryotic cell with the fusion protein or a nucleic acid encoding the same, in which the DNA-binding protein is a zinc finger protein, a TALE protein, or a CRISPR-associated nuclease, a cytosine deaminase of the fusion protein or a variant thereof is derived from bacteria and is specific to double-stranded DNA. The DNA-binding protein is fused to both the N-terminus and the C-terminus of the cytosine deaminase or the variant thereof. Similarly, the DNA-binding protein also is fused both the N-terminus and the C-terminus of the adenine deaminase of the fusion protein. In the context of the fusion protein including a DNA-binding protein, a cytosine deaminase or variant thereof, and an adenine deaminase, the adenine deaminase may be located at the N-terminus or C-terminus of the cytosine deaminase within the fusion protein, or may be present as a separate protein independent of other DNA-binding proteins.

The present invention pertains to a method for C-to-T base editing in prokaryotic or eukaryotic cells including treating a prokaryotic cell or a eukaryotic cell with the fusion protein or a nucleic acid encoding the same and UGI (uracil glycosylase inhibitor), in which the DNA-binding protein is a zinc finger protein, a TALE protein, or a CRISPR-associated nuclease, and the cytosine deaminase of the fusion protein or a variant thereof is derived from bacteria and is specific to double-stranded DNA.

The specific sequences of components included in the composition or method according to the present invention are as follows.
ND1-ZFP-Right-1397C-AD (FIGs.61f-61g: SEQ ID No: **410)** 
MLGFVGRVAAAPASGALRRLTPSASLPPAQLLLRAAPTAVHPVRDYAA
QDYKDDDDKVDEMTKKFGTLTIHDTEKAAEFGIRIPGEKPFQCRICMRNFSDSGNL
 
RVHIRTHTGEKPYKCPDCGKSFSQSSSLIRHQRTHTGEKPYECDHCGKSFSQSSHL
NVHKRTHTGEKPYRCKYCDRSFSISSNLQRHVRNIHLRSGTPHEVGVYTLSGTPHE
VGVYTLAIPVKRGATGETKVFTGNSNSPKSPTKGGCSGSETPGTSESATPESSEVE
FSHEYWMRHALTLAKRARDEREVPVGAVLVLNNRVIGEGWNRAIGLHDPTAHAEIM
ALRQGGLVMQNYRLIDATLYVTFEPCVMCAGAMIHSRIGRVVFGVRNSKRGAAGSL
MNVLNYPGMNHRVEITEGILADECAALLCDFYRMPRQVFNAQKKAQSSIN
ND1-ZFP-Left-1397C-UGI (FIGs.61f-61g: SEQ ID No: **411)** 
MLGFVGRVAAAPASGALRRLTPSASLPPAQLLLRAAPTAVHPVRDYAA
QDYKDDDDKVDEMTKKFGTLTIHDTEKAAEFGIRIPGEKPFQCRICMRNFSDSGNL
RVHIRTHTGEKPYKCPDCGKSFSQSSSLIRHQRTHTGEKPYECDHCGKSFSQSSHL
NVHKRTHTGEKPYRCKYCDRSFSISSNLQRHVRNIHLRSGTPHEVGVYTLSGTPHE
VGVYTLAIPVKRGATGETKVFTGNSNSPKSPTKGGCSGGSTNLSDIIEKETGKQLV
IQESILMLPEEVEEVIGNKPESDILVHTAYDESTDENVMLLTSDAPEYKPWALVIQ
DSNGENKIKML
ND1-ZFP-Right-1397N-UGI (FIGs.61f-61g: SEQ ID **No: 412)** 
MLGFVGRVAAAPASGALRRLTPSASLPPAQLLLRAAPTAVHPVRDYAA
QYPYDVPDYAVDEMTKKFGTLTIHDTEKAAEFGIHGVPAAMGGSYALGPYQISAPQ
LPAYNGQTVGTFYYVNDAGGLESKVFSSGGPTPYPNYANAGHVEGQSALFMRDNGI
SEGLVFHNNPEGTCGFCVNMTETLLPENAKMTVVPPEGSGTPHEVGVYTLSGTPHE
VGVYTLYKCPECGKSFSTKNSLTEHQRTHTGEKPYKCPECGKSFSSKKALTEHQRT
HTGEKPYECNYCGKTFSVSSTLIRHQRIHTGEKPYRCKYCDRSFSISSNLQRHVRN
 
IHLRSGGSTNLSDIIEKETGKQLVIQESILMLPEEVEEVIGNKPESDILVHTAYDE

STDENVMLLTSDAPEYKPWALVIQDSNGENKIKML
**Trac-ZFP-LEFT-1397N-UGI (****FIG.61d****: SEQ ID No: 413)** 
MAPKKKRKVGIHGVPAAMGGSYALGPYQISAPQLPAYNGQTVGTFYYV
NDAGGLESKVFSSGGPTPYPNYANAGHVEGQSALFMRDNGISEGLVFHNNPEGTCG
FCVNMTETLLPENAKMTVVPPEGSGTPHEVGVYTLSGTPHEVGVYTLFQCRICMRK
FATSGSLTRHTKIHTGEKPFQCRICMRNFSRSDHLSTHIRTHTGEKPFACDICGRK
FATSSNRTKHTKIHTHPRAPIPKPFQCRICMRNFSRSDNLSEHIRTHTGEKPFACD
ICGRKFAWHSSLRVHTKIHLRSGGSTNLSDIIEKETGKQLVIQESILMLPEEVEEV
IGNKPESDILVHTAYDESTDENVMLLTSDAPEYKPWALVIQDSNGENKIKML
**Trac-ZFP-right-1397C-AD (****FIG.61d****: SEQ ID No: 414)** 
MAPKKKRKVGIHGVPAAMAERPFQCRICMRNFSRSDHLSTHIRTHTGE
KPFACDICGRKFADRSHLARHTKIHTGSQKPFQCRICMRKFALKQHLNEHTKIHTG
EKPFQCRICMRNFSQSGNLARHIRTHTGEKPFACDICGRKFAHNSSLKDHTKIHLR
SGTPHEVGVYTLSGTPHEVGVYTLAIPVKRGATGETKVFTGNSNSPKSPTKGGCSG
SETPGTSESATPESSEVEFSHEYWMRHALTLAKRARDEREVPVGAVLVLNNRVIGE
GWNRAIGLHDPTAHAEIMALRQGGLVMQNYRLIDATLYVTFEPCVMCAGAMIHSRI
GRVVFGVRNSKRGAAGSLMNVLNYPGMNHRVEITEGILADECAALLCDFYRMPRQV
FNAQKKAQSSIN
**ND1-Left-TALE-1397N-UGI (****FIG.62****: SEQ ID No: 415)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALLVGSGSYA
LGPYQISAPQLPAYNGQTVGTFYYVNDAGGLESKVFSSGGPTPYPNYANAGHVEGQ
SALFMRDNGISEGLVFHNNPEGTCGFCVNMTETLLPENAKMTVVPPEGSGGSTNLS
DIIEKETGKQLVIQESILMLPEEVEEVIGNKPESDILVHTAYDESTDENVMLLTSD
APEYKPWALVIQDSNGENKIKML
**ND1-Left-TALE-1397C-UGI (****FIG.62****: SEQ ID No: 416)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
 
GKQALETVQRLLPVLCQAHGLTPAQVVAIASNGGKQALETVQRLLPVLCQDHGLT

PAQVVAIASNIGGKQALETVQRLLPVLCQAHGLTPDQVVAIASNNGGKQALETVQR

LLPVLCQAHGLTPAQVVAIASHDGGKQALETVQRLLPVLCQDHGLTPDQVVAIASH

DGGKQALETVQRLLPVLCQAHGLTPEQVVAIASNNGGKQALETVQRLLPVLCQAHG

LTPAQVVAIASNGGGKQALETVQRLLPVLCQAHGLTPAQVVAIASNGGGKQALETV

QRLLPVLCQAHGLTPEQVVAIASNGGGKQALESIVAQLSRPDPALAALLVGSAIPV

KRGATGETKVFTGNSNSPKSPTKGGCSGGSTNLSDIIEKETGKQLVIQESILMLPE

EVEEVIGNKPESDILVHTAYDESTDENVMLLTSDAPEYKPWALVIQDSNGENKIKM

L
**ND1-Right-TALE-1397N-UGI (****FIG.62****: SEQ ID No: 417)** 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
IASHDGGKQALETVQRLLPVLCQAHGLTPDQVVAIASNIGGKQALETVQRLLPVLC
QAHGLTPAQVVAIASHDGGKQALETVQRLLPVLCQAHGLTPAQVVAIASHDGGKQA
LETVQRLLPVLCQDHGLTPAQVVAIASHDGGKQALETVQRLLPVLCQAHGLTPEQV
 
VAIASNGGGKQALESIVAQLSRPDPALAALLVGSGSYALGPYQISAPQLPAYNGQT

VGTFYYVNDAGGLESKVFSSGGPTPYPNYANAGHVEGQSALFMRDNGISEGLVFHN

NPEGTCGFCVNMTETLLPENAKMTVVPPEGSGGSTNLSDIIEKETGKQLVIQESIL

MLPEEVEEVIGNKPESDILVHTAYDESTDENVMLLTSDAPEYKPWALVIQDSNGEN

KIKML
**ND1-Right-TALE-1397C-UGI (****FIG.62****: SEQ ID No: 418)** 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
IASHDGGKQALETVQRLLPVLCQAHGLTPDQVVAIASNIGGKQALETVQRLLPVLC
QAHGLTPAQVVAIASHDGGKQALETVQRLLPVLCQAHGLTPAQVVAIASHDGGKQA
LETVQRLLPVLCQDHGLTPAQVVAIASHDGGKQALETVQRLLPVLCQAHGLTPEQV
VAIASNGGGKQALESIVAQLSRPDPALAALLVGSAIPVKRGATGETKVFTGNSNSP
KSPTKGGCSGGSTNLSDIIEKETGKQLVIQESILMLPEEVEEVIGNKPESDILVHT
AYDESTDENVMLLTSDAPEYKPWALVIQDSNGENKIKML
**ND4-Left-TALE-AD (****FIG.62b****: SEQ ID No: 419)** 
MALSRAVCGTSRQLAPVLGYLGSRQKHSLPDYPYDVPDYAGYPYDVPD
YAGYPYDVPDYAMDIADLRTLGYSQQQQEKIKPKVRSTVAQHHEALVGHGFTHAHI
VALSQHPAALGTVAVKYQDMIAALPEATHEAIVGVGKQWSGARALEALLTVAGELR
GPPLQLDTGQLLKIAKRGGVTAVEAVHAWRNALTGAPLNLTPEQVVAIASNNGGKQ
ALETVQALLPVLCQAHGLTPQQVVAIASHDGGKQALETVQRLLPVLCQAHGLTPQQ
VVAIASNGGGKQALETVQRLLPVLCQAHGLTPEQVVAIASNIGGKQALETVQALLP
VLCQAHGLTPEQVVAIASNNGGKQALETVQALLPVLCQAHGLTPEQVVAIASNGGG
KQALETVQALLPVLCQAHGLTPEQVVAIASNIGGKQALETVQRLLPVLCQAHGLTP
EQVVAIASNIGGKQALETVQALLPVLCQAHGLTPEQVVAIASHDGGKQALETVQRL
LPVLCQAHGLTPEQVVAIASHDGGKQALETVQALLPVLCQAHGLTPEQVVAIASNI
GGKQALETVQALLPVLCQAHGLTPEQVVAIASHDGGKQALETVQALLPVLCQAHGL
TPEQVVAIASNNGGKQALETVQRLLPVLCQAHGLTPQQVVAIASNGGGKQALETVQ
RLLPVLCQAHGLTPEQVVAIASNGGGKQALETVQALLPVLCQAHGLTPEQVVAIAS
HDGGKQALETVQALLPVLCQAHGLTPQQVVAIASNGGGRPALESIVAQLSRPDPAL
AALTNDHLVALACLGGRPALDAVKKGLGGSSGSETPGTSESATPESSEVEFSHEYW
MRHALTLAKRARDEREVPVGAVLVLNNRVIGEGWNRAIGLHDPTAHAEIMALRQGG
LVMQNYRLIDATLYVTFEPCVMCAGAMIHSRIGRVVFGVRNSKRGAAGSLMNVLNY
PGMNHRVEITEGILADECAALLCDFYRMPRQVFNAQKKAQSSIN
**ND4-Right-TALE-AD (****FIG.62b****: SEQ ID No: 420)** 
MASVLTPLLLRGLTGSARRLPVPRAKIHSLDYKDHDGDYKDHDIDYKD
DDDKMDIADLRTLGYSQQQQEKIKPKVRSTVAQHHEALVGHGFTHAHIVALSQHPA
ALGTVAVKYQDMIAALPEATHEAIVGVGKQWSGARALEALLTVAGELRGPPLQLDT
GQLLKIAKRGGVTAVEAVHAWRNALTGAPLNLTPEQVVAIASHDGGKQALETVQAL
LPVLCQAHGLTPQQVVAIASHDGGKQALETVQRLLPVLCQAHGLTPQQVVAIASNG
 
GGKQALETVQRLLPVLCQAHGLTPEQVVAIASNNGGKQALETVQALLPVLCQAHGL
TPEQVVAIASNGGGKQALETVQALLPVLCQAHGLTPEQVVAIASNIGGKQALETVQ
ALLPVLCQAHGLTPEQVVAIASNIGGKQALETVQRLLPVLCQAHGLTPEQVVAIAS
NNGGKQALETVQALLPVLCQAHGLTPEQVVAIASNGGGKQALETVQRLLPVLCQAH
GLTPEQVVAIASNIGGKQALETVQRLLPVLCQAHGLTPEQVVAIASNNGGKQALET
VQALLPVLCQAHGLTPEQVVAIASNNGGKQALETVQALLPVLCQAHGLTPEQVVAI
ASNIGGKQALETVQALLPVLCQAHGLTPEQVVAIASNNGGKQALETVQRLLPVLCQ
AHGLTPQQVVAIASNIGGKQALETVQRLLPVLCQAHGLTPQQVVAIASNNGGKQAL
ETVQRLLPVLCQAHGLTPQQVVAIASNGGGRPALESIVAQLSRPDPALAALTNDHL
VALACLGGRPALDAVKKGLGGSSGSETPGTSESATPESSEVEFSHEYWMRHALTLA
KRARDEREVPVGAVLVLNNRVIGEGWNRAIGLHDPTAHAEIMALRQGGLVMQNYRL
IDATLYVTFEPCVMCAGAMIHSRIGRVVFGVRNSKRGAAGSLMNVLNYPGMNHRVE
ITEGILADECAALLCDFYRMPRQVFNAQKKAQSSIN
**ND1-Left-TALE-1333N-AD (****FIG.63****: SEQ ID No: 421)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
 
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALLVGSGSYA
LGPYQISAPQLPAYNGQTVGTFYYVNDAGGLESKVFSSGGSGSETPGTSESATPES
SEVEFSHEYWMRHALTLAKRARDEREVPVGAVLVLNNRVIGEGWNRAIGLHDPTAH
AEIMALRQGGLVMQNYRLIDATLYVTFEPCVMCAGAMIHSRIGRVVFGVRNSKRGA
AGSLMNVLNYPGMNHRVEITEGILADECAALLCDFYRMPRQVFNAQKKAQSSIN
**ND1-Left-TALE-1333C-AD (****FIG.63****: SEQ ID No: 422)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALLVGSPTPY
PNYANAGHVEGQSALFMRDNGISEGLVFHNNPEGTCGFCVNMTETLLPENAKMTVV
PPEGAIPVKRGATGETKVFTGNSNSPKSPTKGGCSGSETPGTSESATPESSEVEFS
HEYWMRHALTLAKRARDEREVPVGAVLVLNNRVIGEGWNRAIGLHDPTAHAEIMAL
 
RQGGLVMQNYRLIDATLYVTFEPCVMCAGAMIHSRIGRVVFGVRNSKRGAAGSLMN

VLNYPGMNHRVEITEGILADECAALLCDFYRMPRQVFNAQKKAQSSIN
**ND1-Right-TALE-1333N-AD (****FIG.63****: SEQ ID No: 423)** 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
IASHDGGKQALETVQRLLPVLCQAHGLTPDQVVAIASNIGGKQALETVQRLLPVLC
QAHGLTPAQVVAIASHDGGKQALETVQRLLPVLCQAHGLTPAQVVAIASHDGGKQA
LETVQRLLPVLCQDHGLTPAQVVAIASHDGGKQALETVQRLLPVLCQAHGLTPEQV
VAIASNGGGKQALESIVAQLSRPDPALAALLVGSGSYALGPYQISAPQLPAYNGQT
VGTFYYVNDAGGLESKVFSSGGSGSETPGTSESATPESSEVEFSHEYWMRHALTLA
KRARDEREVPVGAVLVLNNRVIGEGWNRAIGLHDPTAHAEIMALRQGGLVMQNYRL
IDATLYVTFEPCVMCAGAMIHSRIGRVVFGVRNSKRGAAGSLMNVLNYPGMNHRVE
ITEGILADECAALLCDFYRMPRQVFNAQKKAQSSIN
**ND1-Right-TALE-1333C-AD (****FIG.63****: SEQ ID No: 424)** 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
IASHDGGKQALETVQRLLPVLCQAHGLTPDQVVAIASNIGGKQALETVQRLLPVLC
QAHGLTPAQVVAIASHDGGKQALETVQRLLPVLCQAHGLTPAQVVAIASHDGGKQA
LETVQRLLPVLCQDHGLTPAQVVAIASHDGGKQALETVQRLLPVLCQAHGLTPEQV
VAIASNGGGKQALESIVAQLSRPDPALAALLVGSPTPYPNYANAGHVEGQSALFMR
DNGISEGLVFHNNPEGTCGFCVNMTETLLPENAKMTVVPPEGAIPVKRGATGETKV
FTGNSNSPKSPTKGGCSGSETPGTSESATPESSEVEFSHEYWMRHALTLAKRARDE
REVPVGAVLVLNNRVIGEGWNRAIGLHDPTAHAEIMALRQGGLVMQNYRLIDATLY
VTFEPCVMCAGAMIHSRIGRVVFGVRNSKRGAAGSLMNVLNYPGMNHRVEITEGIL
ADECAALLCDFYRMPRQVFNAQKKAQSSIN
**ND1-Left-TALE-1397C-AD (****FIGs.62-63****: SEQ ID No: 425)** 
MALSRAVCGTSRQLAPVLGYLGSRQKHSLPDYPYDVPDYAGYPYDVPD

YAGYPYDVPDYAGIRIQDLRTLGYSQQQQEKIKPKVRSTVAQHHEALVGHGFTHAH
 
IVALSQHPAALGTVAVKYQDMIAALPEATHEAIVGVGKQWSGARALEALLTVAGEL

RGPPLQLDTGQLLKIAKRGGVTAVEAVHAWRNALTGAPLNLTPDQVVAIASHDGGK

QALETVQRLLPVLCQAHGLTPDQVVAIASNGGGKQALETVQRLLPVLCQDHGLTPA

QVVAIASNIGGKQALETVQRLLPVLCQDHGLTPDQVVAIASNNGGKQALETVQRLL

PVLCQAHGLTPDQVVAIASHDGGKQALETVQRLLPVLCQDHGLTPDQVVAIASHDG

GKQALETVQRLLPVLCQAHGLTPAQVVAIASNGGGKQALETVQRLLPVLCQDHGLT

PAQVVAIASNIGGKQALETVQRLLPVLCQAHGLTPDQVVAIASNNGGKQALETVQR

LLPVLCQAHGLTPAQVVAIASHDGGKQALETVQRLLPVLCQDHGLTPDQVVAIASH

DGGKQALETVQRLLPVLCQAHGLTPEQVVAIASNNGGKQALETVQRLLPVLCQAHG

LTPAQVVAIASNGGGKQALETVQRLLPVLCQAHGLTPAQVVAIASNGGGKQALETV

QRLLPVLCQAHGLTPEQVVAIASNGGGKQALESIVAQLSRPDPALAALTNDHLVAL

ACLGGRPALDAVKKGLLVGSAIPVKRGATGETKVFTGNSNSPKSPTKGGCSGSETP

GTSESATPESSEVEFSHEYWMRHALTLAKRARDEREVPVGAVLVLNNRVIGEGWNR

AIGLHDPTAHAEIMALRQGGLVMQNYRLIDATLYVTFEPCVMCAGAMIHSRIGRVV

FGVRNSKRGAAGSLMNVLNYPGMNHRVEITEGILADECAALLCDFYRMPRQVFNAQ

KKAQSSIN
**ND1-Right-TALE-1397C-AD (****FIGs.62-63****: SEQ ID No: 426)** 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
 
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
IASHDGGKQALETVQRLLPVLCQAHGLTPDQVVAIASNIGGKQALETVQRLLPVLC
QAHGLTPAQVVAIASHDGGKQALETVQRLLPVLCQAHGLTPAQVVAIASHDGGKQA
LETVQRLLPVLCQDHGLTPAQVVAIASHDGGKQALETVQRLLPVLCQAHGLTPEQV
VAIASNGGGKQALESIVAQLSRPDPALAALTNDHLVALACLGGRPALDAVKKGLGG
SGSAIPVKRGATGETKVFTGNSNSPKSPTKGGCSGSETPGTSESATPESSEVEFSH
EYWMRHALTLAKRARDEREVPVGAVLVLNNRVIGEGWNRAIGLHDPTAHAEIMALR
QGGLVMQNYRLIDATLYVTFEPCVMCAGAMIHSRIGRVVFGVRNSKRGAAGSLMNV
LNYPGMNHRVEITEGILADECAALLCDFYRMPRQVFNAQKKAQSSIN
**ND1-Left-TALE-1333N (****FIG.63****: SEQ ID No: 427)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
 
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALTNDHLVAL
ACLGGRPALDAVKKGLGGSGSGSYALGPYQISAPQLPAYNGQTVGTFYYVNDAGGL
ESKVFSSGG
**ND1-Left-TALE-1333C (****FIG.63****: SEQ ID No: 428)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALTNDHLVAL
ACLGGRPALDAVKKGLGGSGSPTPYPNYANAGHVEGQSALFMRDNGISEGLVFHNN
PEGTCGFCVNMTETLLPENAKMTVVPPEGAIPVKRGATGETKVFTGNSNSPKSPTK
GGC
**ND1-Left-TALE-1397N (****FIGs.62-63****: SEQ ID No: 429)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALTNDHLVAL
ACLGGRPALDAVKKGLGGSGSGSYALGPYQISAPQLPAYNGQTVGTFYYVNDAGGL
ESKVFSSGGPTPYPNYANAGHVEGQSALFMRDNGISEGLVFHNNPEGTCGFCVNMT
ETLLPENAKMTVVPPEG
**ND1-Right-TALE-1333N (****FIG.63****: SEQ ID No: 430)** 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
 
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
IASHDGGKQALETVQRLLPVLCQAHGLTPDQVVAIASNIGGKQALETVQRLLPVLC
QAHGLTPAQVVAIASHDGGKQALETVQRLLPVLCQAHGLTPAQVVAIASHDGGKQA
LETVQRLLPVLCQDHGLTPAQVVAIASHDGGKQALETVQRLLPVLCQAHGLTPEQV
VAIASNGGGKQALESIVAQLSRPDPALAALLVGSGSYALGPYQISAPQLPAYNGQT
VGTFYYVNDAGGLESKVFSSGG
**ND1-Right-TALE-1333C (****FIG.63****: SEQ ID No: 431)** 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
IASHDGGKQALETVQRLLPVLCQAHGLTPDQVVAIASNIGGKQALETVQRLLPVLC
QAHGLTPAQVVAIASHDGGKQALETVQRLLPVLCQAHGLTPAQVVAIASHDGGKQA
 
LETVQRLLPVLCQDHGLTPAQVVAIASHDGGKQALETVQRLLPVLCQAHGLTPEQV
VAIASNGGGKQALESIVAQLSRPDPALAALLVGSPTPYPNYANAGHVEGQSALFMR
DNGISEGLVFHNNPEGTCGFCVNMTETLLPENAKMTVVPPEGAIPVKRGATGETKV
FTGNSNSPKSPTKGGC
**ND1-Right-TALE-1397N (****FIGs.62-63****: SEQ ID No:** 432) 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
IASHDGGKQALETVQRLLPVLCQAHGLTPDQVVAIASNIGGKQALETVQRLLPVLC
QAHGLTPAQVVAIASHDGGKQALETVQRLLPVLCQAHGLTPAQVVAIASHDGGKQA
LETVQRLLPVLCQDHGLTPAQVVAIASHDGGKQALETVQRLLPVLCQAHGLTPEQV
VAIASNGGGKQALESIVAQLSRPDPALAALLVGSGSYALGPYQISAPQLPAYNGQT
VGTFYYVNDAGGLESKVFSSGGPTPYPNYANAGHVEGQSALFMRDNGISEGLVFHN
NPEGTCGFCVNMTETLLPENAKMTVVPPEG
**ND4-LEFT-TALE-1333N-AD (****FIG.63****: SEQ ID No: 433)** 
MALSRAVCGTSRQLAPVLGYLGSRQKHSLPDYPYDVPDYAGYPYDVPD
YAGYPYDVPDYAMDIADLRTLGYSQQQQEKIKPKVRSTVAQHHEALVGHGFTHAHI
VALSQHPAALGTVAVKYQDMIAALPEATHEAIVGVGKQWSGARALEALLTVAGELR
GPPLQLDTGQLLKIAKRGGVTAVEAVHAWRNALTGAPLNLTPEQVVAIASNNGGKQ
ALETVQALLPVLCQAHGLTPQQVVAIASHDGGKQALETVQRLLPVLCQAHGLTPQQ
VVAIASNGGGKQALETVQRLLPVLCQAHGLTPEQVVAIASNIGGKQALETVQALLP
VLCQAHGLTPEQVVAIASNNGGKQALETVQALLPVLCQAHGLTPEQVVAIASNGGG
KQALETVQALLPVLCQAHGLTPEQVVAIASNIGGKQALETVQRLLPVLCQAHGLTP
EQVVAIASNIGGKQALETVQALLPVLCQAHGLTPEQVVAIASHDGGKQALETVQRL
LPVLCQAHGLTPEQVVAIASHDGGKQALETVQALLPVLCQAHGLTPEQVVAIASNI
GGKQALETVQALLPVLCQAHGLTPEQVVAIASHDGGKQALETVQALLPVLCQAHGL
TPEQVVAIASNNGGKQALETVQRLLPVLCQAHGLTPQQVVAIASNGGGKQALETVQ
RLLPVLCQAHGLTPEQVVAIASNGGGKQALETVQALLPVLCQAHGLTPEQVVAIAS
HDGGKQALETVQALLPVLCQAHGLTPQQVVAIASNGGGRPALESIVAQLSRPDPAL
AALTNDHLVALACLGGRPALDAVKKGLGGSGSYALGPYQISAPQLPAYNGQTVGTF
YYVNDAGGLESKVFSSGGSGSETPGTSESATPESSEVEFSHEYWMRHALTLAKRAR
DEREVPVGAVLVLNNRVIGEGWNRAIGLHDPTAHAEIMALRQGGLVMQNYRLIDAT
LYVTFEPCVMCAGAMIHSRIGRVVFGVRNSKRGAAGSLMNVLNYPGMNHRVEITEG
ILADECAALLCDFYRMPRQVFNAQKKAQSSIN
**ND4-LEFT-TALE-1333C-AD (****FIG.63****: SEQ ID No: 434)** 
MALSRAVCGTSRQLAPVLGYLGSRQKHSLPDYPYDVPDYAGYPYDVPD
YAGYPYDVPDYAMDIADLRTLGYSQQQQEKIKPKVRSTVAQHHEALVGHGFTHAHI
VALSQHPAALGTVAVKYQDMIAALPEATHEAIVGVGKQWSGARALEALLTVAGELR
 
GPPLQLDTGQLLKIAKRGGVTAVEAVHAWRNALTGAPLNLTPEQVVAIASNNGGKQ
ALETVQALLPVLCQAHGLTPQQVVAIASHDGGKQALETVQRLLPVLCQAHGLTPQQ
VVAIASNGGGKQALETVQRLLPVLCQAHGLTPEQVVAIASNIGGKQALETVQALLP
VLCQAHGLTPEQVVAIASNNGGKQALETVQALLPVLCQAHGLTPEQVVAIASNGGG
KQALETVQALLPVLCQAHGLTPEQVVAIASNIGGKQALETVQRLLPVLCQAHGLTP
EQVVAIASNIGGKQALETVQALLPVLCQAHGLTPEQVVAIASHDGGKQALETVQRL
LPVLCQAHGLTPEQVVAIASHDGGKQALETVQALLPVLCQAHGLTPEQVVAIASNI
GGKQALETVQALLPVLCQAHGLTPEQVVAIASHDGGKQALETVQALLPVLCQAHGL
TPEQVVAIASNNGGKQALETVQRLLPVLCQAHGLTPQQVVAIASNGGGKQALETVQ
RLLPVLCQAHGLTPEQVVAIASNGGGKQALETVQALLPVLCQAHGLTPEQVVAIAS
HDGGKQALETVQALLPVLCQAHGLTPQQVVAIASNGGGRPALESIVAQLSRPDPAL
AALTNDHLVALACLGGRPALDAVKKGLGGSPTPYPNYANAGHVEGQSALFMRDNGI
SEGLVFHNNPEGTCGFCVNMTETLLPENAKMTVVPPEGAIPVKRGATGETKVFTGN
SNSPKSPTKGGCSGSETPGTSESATPESSEVEFSHEYWMRHALTLAKRARDEREVP
VGAVLVLNNRVIGEGWNRAIGLHDPTAHAEIMALRQGGLVMQNYRLIDATLYVTFE
PCVMCAGAMIHSRIGRVVFGVRNSKRGAAGSLMNVLNYPGMNHRVEITEGILADEC
AALLCDFYRMPRQVFNAQKKAQSSIN
**ND4-LEFT-TALE-1397C-AD (****FIG.63****: SEQ ID No: 435)** 
MALSRAVCGTSRQLAPVLGYLGSRQKHSLPDYPYDVPDYAGYPYDVPD
YAGYPYDVPDYAMDIADLRTLGYSQQQQEKIKPKVRSTVAQHHEALVGHGFTHAHI
VALSQHPAALGTVAVKYQDMIAALPEATHEAIVGVGKQWSGARALEALLTVAGELR
GPPLQLDTGQLLKIAKRGGVTAVEAVHAWRNALTGAPLNLTPEQVVAIASNNGGKQ
ALETVQALLPVLCQAHGLTPQQVVAIASHDGGKQALETVQRLLPVLCQAHGLTPQQ
VVAIASNGGGKQALETVQRLLPVLCQAHGLTPEQVVAIASNIGGKQALETVQALLP
 
VLCQAHGLTPEQVVAIASNNGGKQALETVQALLPVLCQAHGLTPEQVVAIASNGGG
KQALETVQALLPVLCQAHGLTPEQVVAIASNIGGKQALETVQRLLPVLCQAHGLTP
EQVVAIASNIGGKQALETVQALLPVLCQAHGLTPEQVVAIASHDGGKQALETVQRL
LPVLCQAHGLTPEQVVAIASHDGGKQALETVQALLPVLCQAHGLTPEQVVAIASNI
GGKQALETVQALLPVLCQAHGLTPEQVVAIASHDGGKQALETVQALLPVLCQAHGL
TPEQVVAIASNNGGKQALETVQRLLPVLCQAHGLTPQQVVAIASNGGGKQALETVQ
RLLPVLCQAHGLTPEQVVAIASNGGGKQALETVQALLPVLCQAHGLTPEQVVAIAS
HDGGKQALETVQALLPVLCQAHGLTPQQVVAIASNGGGRPALESIVAQLSRPDPAL
AALTNDHLVALACLGGRPALDAVKKGLGGSGSAIPVKRGATGETKVFTGNSNSPKS
PTKGGCSGSETPGTSESATPESSEVEFSHEYWMRHALTLAKRARDEREVPVGAVLV
LNNRVIGEGWNRAIGLHDPTAHAEIMALRQGGLVMQNYRLIDATLYVTFEPCVMCA
GAMIHSRIGRVVFGVRNSKRGAAGSLMNVLNYPGMNHRVEITEGILADECAALLCD
FYRMPRQVFNAQKKAQSSIN
**ND4-Right-TALE-1333N-AD (****FIG.63****: SEQ ID No: 436)** 
MASVLTPLLLRGLTGSARRLPVPRAKIHSLDYKDHDGDYKDHDIDYKD
DDDKMDIADLRTLGYSQQQQEKIKPKVRSTVAQHHEALVGHGFTHAHIVALSQHPA
ALGTVAVKYQDMIAALPEATHEAIVGVGKQWSGARALEALLTVAGELRGPPLQLDT
GQLLKIAKRGGVTAVEAVHAWRNALTGAPLNLTPEQVVAIASHDGGKQALETVQAL
LPVLCQAHGLTPQQVVAIASHDGGKQALETVQRLLPVLCQAHGLTPQQVVAIASNG
GGKQALETVQRLLPVLCQAHGLTPEQVVAIASNNGGKQALETVQALLPVLCQAHGL
TPEQVVAIASNGGGKQALETVQALLPVLCQAHGLTPEQVVAIASNIGGKQALETVQ
ALLPVLCQAHGLTPEQVVAIASNIGGKQALETVQRLLPVLCQAHGLTPEQVVAIAS
NNGGKQALETVQALLPVLCQAHGLTPEQVVAIASNGGGKQALETVQRLLPVLCQAH
GLTPEQVVAIASNIGGKQALETVQRLLPVLCQAHGLTPEQVVAIASNNGGKQALET
 
VQALLPVLCQAHGLTPEQVVAIASNNGGKQALETVQALLPVLCQAHGLTPEQVVAI

ASNIGGKQALETVQALLPVLCQAHGLTPEQVVAIASNNGGKQALETVQRLLPVLCQ

AHGLTPQQVVAIASNIGGKQALETVQRLLPVLCQAHGLTPQQVVAIASNNGGKQAL

ETVQRLLPVLCQAHGLTPQQVVAIASNGGGRPALESIVAQLSRPDPALAALTNDHL

VALACLGGRPALDAVKKGLGGSGSYALGPYQISAPQLPAYNGQTVGTFYYVNDAGG

LESKVFSSGGSGSETPGTSESATPESSEVEFSHEYWMRHALTLAKRARDEREVPVG

AVLVLNNRVIGEGWNRAIGLHDPTAHAEIMALRQGGLVMQNYRLIDATLYVTFEPC

VMCAGAMIHSRIGRVVFGVRNSKRGAAGSLMNVLNYPGMNHRVEITEGILADECAA

LLCDFYRMPRQVFNAQKKAQSSIN
ND4-Right-TALE-1333C-AD (FIG.63: SEQ ID No: 437) 
MASVLTPLLLRGLTGSARRLPVPRAKIHSLDYKDHDGDYKDHDIDYKD
DDDKMDIADLRTLGYSQQQQEKIKPKVRSTVAQHHEALVGHGFTHAHIVALSQHPA
ALGTVAVKYQDMIAALPEATHEAIVGVGKQWSGARALEALLTVAGELRGPPLQLDT
GQLLKIAKRGGVTAVEAVHAWRNALTGAPLNLTPEQVVAIASHDGGKQALETVQAL
LPVLCQAHGLTPQQVVAIASHDGGKQALETVQRLLPVLCQAHGLTPQQVVAIASNG
GGKQALETVQRLLPVLCQAHGLTPEQVVAIASNNGGKQALETVQALLPVLCQAHGL
TPEQVVAIASNGGGKQALETVQALLPVLCQAHGLTPEQVVAIASNIGGKQALETVQ
ALLPVLCQAHGLTPEQVVAIASNIGGKQALETVQRLLPVLCQAHGLTPEQVVAIAS
NNGGKQALETVQALLPVLCQAHGLTPEQVVAIASNGGGKQALETVQRLLPVLCQAH
GLTPEQVVAIASNIGGKQALETVQRLLPVLCQAHGLTPEQVVAIASNNGGKQALET
VQALLPVLCQAHGLTPEQVVAIASNNGGKQALETVQALLPVLCQAHGLTPEQVVAI
ASNIGGKQALETVQALLPVLCQAHGLTPEQVVAIASNNGGKQALETVQRLLPVLCQ
AHGLTPQQVVAIASNIGGKQALETVQRLLPVLCQAHGLTPQQVVAIASNNGGKQAL
ETVQRLLPVLCQAHGLTPQQVVAIASNGGGRPALESIVAQLSRPDPALAALTNDHL
 
VALACLGGRPALDAVKKGLGGSPTPYPNYANAGHVEGQSALFMRDNGISEGLVFHN
NPEGTCGFCVNMTETLLPENAKMTVVPPEGAIPVKRGATGETKVFTGNSNSPKSPT
KGGCSGSETPGTSESATPESSEVEFSHEYWMRHALTLAKRARDEREVPVGAVLVLN
NRVIGEGWNRAIGLHDPTAHAEIMALRQGGLVMQNYRLIDATLYVTFEPCVMCAGA
MIHSRIGRVVFGVRNSKRGAAGSLMNVLNYPGMNHRVEITEGILADECAALLCDFY
RMPRQVFNAQKKAQSSIN
**ND4-Right-TALE-1397C-AD (****FIG.63****: SEQ ID No: 438)** 
MASVLTPLLLRGLTGSARRLPVPRAKIHSLDYKDHDGDYKDHDIDYKD
DDDKMDIADLRTLGYSQQQQEKIKPKVRSTVAQHHEALVGHGFTHAHIVALSQHPA
ALGTVAVKYQDMIAALPEATHEAIVGVGKQWSGARALEALLTVAGELRGPPLQLDT
GQLLKIAKRGGVTAVEAVHAWRNALTGAPLNLTPEQVVAIASHDGGKQALETVQAL
LPVLCQAHGLTPQQVVAIASHDGGKQALETVQRLLPVLCQAHGLTPQQVVAIASNG
GGKQALETVQRLLPVLCQAHGLTPEQVVAIASNNGGKQALETVQALLPVLCQAHGL
TPEQVVAIASNGGGKQALETVQALLPVLCQAHGLTPEQVVAIASNIGGKQALETVQ
ALLPVLCQAHGLTPEQVVAIASNIGGKQALETVQRLLPVLCQAHGLTPEQVVAIAS
NNGGKQALETVQALLPVLCQAHGLTPEQVVAIASNGGGKQALETVQRLLPVLCQAH
GLTPEQVVAIASNIGGKQALETVQRLLPVLCQAHGLTPEQVVAIASNNGGKQALET
VQALLPVLCQAHGLTPEQVVAIASNNGGKQALETVQALLPVLCQAHGLTPEQVVAI
ASNIGGKQALETVQALLPVLCQAHGLTPEQVVAIASNNGGKQALETVQRLLPVLCQ
AHGLTPQQVVAIASNIGGKQALETVQRLLPVLCQAHGLTPQQVVAIASNNGGKQAL
ETVQRLLPVLCQAHGLTPQQVVAIASNGGGRPALESIVAQLSRPDPALAALTNDHL
VALACLGGRPALDAVKKGLGGSGSAIPVKRGATGETKVFTGNSNSPKSPTKGGCSG
SETPGTSESATPESSEVEFSHEYWMRHALTLAKRARDEREVPVGAVLVLNNRVIGE
GWNRAIGLHDPTAHAEIMALRQGGLVMQNYRLIDATLYVTFEPCVMCAGAMIHSRI
 
GRVVFGVRNSKRGAAGSLMNVLNYPGMNHRVEITEGILADECAALLCDFYRMPRQV

FNAQKKAQSSIN
**ND1-Left-TALE-AD-GSVG (****FIG.64****: SEQ ID No: 439)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALLVSGSETP
GTSESATPESSEVEFSHEYWMRHALTLAKRARDEREVPVGAVLVLNNRVIGEGWNR
AIGLHDPTAHAEIMALRQGGLVMQNYRLIDATLYVTFEPCVMCAGAMIHSRIGRVV
FGVRNSKRGAAGSLMNVLNYPGMNHRVEITEGILADECAALLCDFYRMPRQVFNAQ
KKAQSSINLVGSGSYALGPYQISAPQLPAYNGQTVGTFYYVNDAGGLEGKVFSSGG
PTPYPNYANAGHVESQSALFMRDNGISEGLVFHNNPEGTCGFCVNMTETLLPENAK
MTVVPPEGVIPVKRGATGETKVFTGNSNGPKSPTKGGC
**ND1-Left-TALE-AD-E1347A (****FIG.64****: SEQ ID No: 440)**
   MALSRAVCGTSRQLAPVLGYLGSRQKHSLPDYPYDVPDYAGYPYDVPD 
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALLVSGSETP
GTSESATPESSEVEFSHEYWMRHALTLAKRARDEREVPVGAVLVLNNRVIGEGWNR
AIGLHDPTAHAEIMALRQGGLVMQNYRLIDATLYVTFEPCVMCAGAMIHSRIGRVV
FGVRNSKRGAAGSLMNVLNYPGMNHRVEITEGILADECAALLCDFYRMPRQVFNAQ
KKAQSSINLVGSGSYALGPYQISAPQLPAYNGQTVGTFYYVNDAGGLESKVFSSGG
PTPYPNYANAGHVAGQSALFMRDNGISEGLVFHNNPEGTCGFCVNMTETLLPENAK
MTVVPPEGAIPVKRGATGETKVFTGNSNSPKSPTKGGC
**ND1-Left-TALE-AD-AAAAA (****FIG.64****: SEQ ID No: 441)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
 
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALLVSGSETP
GTSESATPESSEVEFSHEYWMRHALTLAKRARDEREVPVGAVLVLNNRVIGEGWNR
AIGLHDPTAHAEIMALRQGGLVMQNYRLIDATLYVTFEPCVMCAGAMIHSRIGRVV
FGVRNSKRGAAGSLMNVLNYPGMNHRVEITEGILADECAALLCDFYRMPRQVFNAQ
KKAQSSINLVGSGSYALGPYQISAPQLPAYNGQTVGTFYYVNDAGGLESKVFSSGG
PTPYPNYANAGHVEGQSALFMRDNGISEGLVFHNNPEGTCGFCVNMTETLLPENAK
MTVVPPEGAIPVAAGATGETAVFTGNSNSPASPTAGGC
**ND1-Right-TALE-AD (****FIG.64****: SEQ ID No: 442)** 
MASVLTPLLLRGLTGSARRLPVPRAKIHSLDYKDHDGDYKDHDIDYKD
DDDKGIRIQDLRTLGYSQQQQEKIKPKVRSTVAQHHEALVGHGFTHAHIVALSQHP
AALGTVAVKYQDMIAALPEATHEAIVGVGKQWSGARALEALLTVAGELRGPPLQLD
TGQLLKIAKRGGVTAVEAVHAWRNALTGAPLNLTPDQVVAIASNNGGKQALETVQR
LLPVLCQDHGLTPAQVVAIASNIGGKQALETVQRLLPVLCQAHGLTPEQVVAIASN
NGGKQALETVQRLLPVLCQAHGLTPDQVVAIASNGGGKQALETVQRLLPVLCQAHG
LTPAQVVAIASNGGGKQALETVQRLLPVLCQAHGLTPEQVVAIASNGGGKQALETV
QRLLPVLCQAHGLTPAQVVAIASNNGGKQALETVQRLLPVLCQDHGLTPAQVVAIA
SNIGGKQALETVQRLLPVLCQAHGLTPEQVVAIASNGGGKQALETVQRLLPVLCQA
HGLTPDQVVAIASNNGGKQALETVQRLLPVLCQAHGLTPAQVVAIASHDGGKQALE
TVQRLLPVLCQDHGLTPDQVVAIASNGGGKQALETVQRLLPVLCQAHGLTPDQVVA
 
IASHDGGKQALETVQRLLPVLCQAHGLTPDQVVAIASNIGGKQALETVQRLLPVLC

QAHGLTPAQVVAIASHDGGKQALETVQRLLPVLCQAHGLTPAQVVAIASHDGGKQA

LETVQRLLPVLCQDHGLTPAQVVAIASHDGGKQALETVQRLLPVLCQAHGLTPEQV

VAIASNGGGKQALESIVAQLSRPDPALAALLVSGSETPGTSESATPESSEVEFSHE

YWMRHALTLAKRARDEREVPVGAVLVLNNRVIGEGWNRAIGLHDPTAHAEIMALRQ

GGLVMQNYRLIDATLYVTFEPCVMCAGAMIHSRIGRVVFGVRNSKRGAAGSLMNVL

NYPGMNHRVEITEGILADECAALLCDFYRMPRQVFNAQKKAQSSIN
**ND1-Left-TALE-GSVG (****FIG.64****: SEQ ID No: 443)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALLVGSGSYA
LGPYQISAPQLPAYNGQTVGTFYYVNDAGGLEGKVFSSGGPTPYPNYANAGHVESQ
SALFMRDNGISEGLVFHNNPEGTCGFCVNMTETLLPENAKMTVVPPEGVIPVKRGA
TGETKVFTGNSNGPKSPTKGGC
**ND1-Left-TALE-E1347A (****FIG.64****: SEQ ID No: 444)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
PAQVVAIASNIGGKQALETVQRLLPVLCQAHGLTPDQVVAIASNNGGKQALETVQR
LLPVLCQAHGLTPAQVVAIASHDGGKQALETVQRLLPVLCQDHGLTPDQVVAIASH
DGGKQALETVQRLLPVLCQAHGLTPEQVVAIASNNGGKQALETVQRLLPVLCQAHG
LTPAQVVAIASNGGGKQALETVQRLLPVLCQAHGLTPAQVVAIASNGGGKQALETV
QRLLPVLCQAHGLTPEQVVAIASNGGGKQALESIVAQLSRPDPALAALLVGSGSYA
LGPYQISAPQLPAYNGQTVGTFYYVNDAGGLESKVFSSGGPTPYPNYANAGHVAGQ
SALFMRDNGISEGLVFHNNPEGTCGFCVNMTETLLPENAKMTVVPPEGAIPVKRGA
TGETKVFTGNSNSPKSPTKGGC
**ND1-Left-TALE-AAAAA (****FIG.64** **SEQ ID No: 445)** 
MALSRAVCGTSRQLAPVLGYLGSRQKHSLPDYPYDVPDYAGYPYDVPD
YAGYPYDVPDYAGIRIQDLRTLGYSQQQQEKIKPKVRSTVAQHHEALVGHGFTHAH
IVALSQHPAALGTVAVKYQDMIAALPEATHEAIVGVGKQWSGARALEALLTVAGEL
RGPPLQLDTGQLLKIAKRGGVTAVEAVHAWRNALTGAPLNLTPDQVVAIASHDGGK
QALETVQRLLPVLCQAHGLTPDQVVAIASNGGGKQALETVQRLLPVLCQDHGLTPA
QVVAIASNIGGKQALETVQRLLPVLCQDHGLTPDQVVAIASNNGGKQALETVQRLL
PVLCQAHGLTPDQVVAIASHDGGKQALETVQRLLPVLCQDHGLTPDQVVAIASHDG
GKQALETVQRLLPVLCQAHGLTPAQVVAIASNGGGKQALETVQRLLPVLCQDHGLT
 
PAQVVAIASNIGGKQALETVQRLLPVLCQAHGLTPDQVVAIASNNGGKQALETVQR

LLPVLCQAHGLTPAQVVAIASHDGGKQALETVQRLLPVLCQDHGLTPDQVVAIASH

DGGKQALETVQRLLPVLCQAHGLTPEQVVAIASNNGGKQALETVQRLLPVLCQAHG

LTPAQVVAIASNGGGKQALETVQRLLPVLCQAHGLTPAQVVAIASNGGGKQALETV

QRLLPVLCQAHGLTPEQVVAIASNGGGKQALESIVAQLSRPDPALAALLVGSGSYA

LGPYQISAPQLPAYNGQTVGTFYYVNDAGGLESKVFSSGGPTPYPNYANAGHVEGQ

SALFMRDNGISEGLVFHNNPEGTCGFCVNMTETLLPENAKMTVVPPEGAIPVAAGA

TGETAVFTGNSNSPASPTAGGC
**ND1-TALE-Left (SEQ ID No: 446)** 
DLRTLGYSQQQQEKIKPKVRSTVAQHHEALVGHGFTHAHIVALSQHPA
ALGTVAVKYQDMIAALPEATHEAIVGVGKQWSGARALEALLTVAGELRGPPLQLDT
GQLLKIAKRGGVTAVEAVHAWRNALTGAPLNLTPDQVVAIASHDGGKQALETVQRL
LPVLCQAHGLTPDQVVAIASNGGGKQALETVQRLLPVLCQDHGLTPAQVVAIASNI
GGKQALETVQRLLPVLCQDHGLTPDQVVAIASNNGGKQALETVQRLLPVLCQAHGL
TPDQVVAIASHDGGKQALETVQRLLPVLCQDHGLTPDQVVAIASHDGGKQALETVQ
RLLPVLCQAHGLTPAQVVAIASNGGGKQALETVQRLLPVLCQDHGLTPAQVVAIAS
NIGGKQALETVQRLLPVLCQAHGLTPDQVVAIASNNGGKQALETVQRLLPVLCQAH
GLTPAQVVAIASHDGGKQALETVQRLLPVLCQDHGLTPDQVVAIASHDGGKQALET
VQRLLPVLCQAHGLTPEQVVAIASNNGGKQALETVQRLLPVLCQAHGLTPAQVVAI
ASNGGGKQALETVQRLLPVLCQAHGLTPAQVVAIASNGGGKQALETVQRLLPVLCQ
AHGLTPEQVVAIASNGGGKQALESIVAQLSRPDPALAAL
**ND1-TALE-Right (SEQ ID No: 447)** 
DLRTLGYSQQQQEKIKPKVRSTVAQHHEALVGHGFTHAHIVALSQHPA
ALGTVAVKYQDMIAALPEATHEAIVGVGKQWSGARALEALLTVAGELRGPPLQLDT
GQLLKIAKRGGVTAVEAVHAWRNALTGAPLNLTPDQVVAIASNNGGKQALETVQRL
LPVLCQDHGLTPAQVVAIASNIGGKQALETVQRLLPVLCQAHGLTPEQVVAIASNN
GGKQALETVQRLLPVLCQAHGLTPDQVVAIASNGGGKQALETVQRLLPVLCQAHGL
TPAQVVAIASNGGGKQALETVQRLLPVLCQAHGLTPEQVVAIASNGGGKQALETVQ
RLLPVLCQAHGLTPAQVVAIASNNGGKQALETVQRLLPVLCQDHGLTPAQVVAIAS
NIGGKQALETVQRLLPVLCQAHGLTPEQVVAIASNGGGKQALETVQRLLPVLCQAH
GLTPDQVVAIASNNGGKQALETVQRLLPVLCQAHGLTPAQVVAIASHDGGKQALET
VQRLLPVLCQDHGLTPDQVVAIASNGGGKQALETVQRLLPVLCQAHGLTPDQVVAI
ASHDGGKQALETVQRLLPVLCQAHGLTPDQVVAIASNIGGKQALETVQRLLPVLCQ
AHGLTPAQVVAIASHDGGKQALETVQRLLPVLCQAHGLTPAQVVAIASHDGGKQAL
ETVQRLLPVLCQDHGLTPAQVVAIASHDGGKQALETVQRLLPVLCQAHGLTPEQVV
AIASNGGGKQALESIVAQLSRPDPALAAL
**ND4-TALE-left (SEQ ID No: 448)** 
MDIADLRTLGYSQQQQEKIKPKVRSTVAQHHEALVGHGFTHAHIVALS
QHPAALGTVAVKYQDMIAALPEATHEAIVGVGKQWSGARALEALLTVAGELRGPPL
QLDTGQLLKIAKRGGVTAVEAVHAWRNALTGAPLNLTPEQVVAIASNNGGKQALET
VQALLPVLCQAHGLTPQQVVAIASHDGGKQALETVQRLLPVLCQAHGLTPQQVVAI
ASNGGGKQALETVQRLLPVLCQAHGLTPEQVVAIASNIGGKQALETVQALLPVLCQ
AHGLTPEQVVAIASNNGGKQALETVQALLPVLCQAHGLTPEQVVAIASNGGGKQAL
ETVQALLPVLCQAHGLTPEQVVAIASNIGGKQALETVQRLLPVLCQAHGLTPEQVV
AIASNIGGKQALETVQALLPVLCQAHGLTPEQVVAIASHDGGKQALETVQRLLPVL
CQAHGLTPEQVVAIASHDGGKQALETVQALLPVLCQAHGLTPEQVVAIASNIGGKQ
 
ALETVQALLPVLCQAHGLTPEQVVAIASHDGGKQALETVQALLPVLCQAHGLTPEQ

VVAIASNNGGKQALETVQRLLPVLCQAHGLTPQQVVAIASNGGGKQALETVQRLLP

VLCQAHGLTPEQVVAIASNGGGKQALETVQALLPVLCQAHGLTPEQVVAIASHDGG

KQALETVQALLPVLCQAHGLTPQQVVAIASNGGGRPALESIVAQLSRPDPALAALT

NDHLVALACLGGRPALDAVKKGLG
**ND4-TALE-Right (SEQ ID No: 449)** 
MDIADLRTLGYSQQQQEKIKPKVRSTVAQHHEALVGHGFTHAHIVALS
QHPAALGTVAVKYQDMIAALPEATHEAIVGVGKQWSGARALEALLTVAGELRGPPL
QLDTGQLLKIAKRGGVTAVEAVHAWRNALTGAPLNLTPEQVVAIASHDGGKQALET
VQALLPVLCQAHGLTPQQVVAIASHDGGKQALETVQRLLPVLCQAHGLTPQQVVAI
ASNGGGKQALETVQRLLPVLCQAHGLTPEQVVAIASNNGGKQALETVQALLPVLCQ
AHGLTPEQVVAIASNGGGKQALETVQALLPVLCQAHGLTPEQVVAIASNIGGKQAL
ETVQALLPVLCQAHGLTPEQVVAIASNIGGKQALETVQRLLPVLCQAHGLTPEQVV
AIASNNGGKQALETVQALLPVLCQAHGLTPEQVVAIASNGGGKQALETVQRLLPVL
CQAHGLTPEQVVAIASNIGGKQALETVQRLLPVLCQAHGLTPEQVVAIASNNGGKQ
ALETVQALLPVLCQAHGLTPEQVVAIASNNGGKQALETVQALLPVLCQAHGLTPEQ
VVAIASNIGGKQALETVQALLPVLCQAHGLTPEQVVAIASNNGGKQALETVQRLLP
VLCQAHGLTPQQVVAIASNIGGKQALETVQRLLPVLCQAHGLTPQQVVAIASNNGG
KQALETVQRLLPVLCQAHGLTPQQVVAIASNGGGRPALESIVAQLSRPDPALAALT
NDHLVALACLGGRPALDAVKKGLG
**TRAC-ZFP-LEFT (SEQ ID No: 450)** 
FQCRICMRKFATSGSLTRHTKIHTGEKPFQCRICMRNFSRSDHLSTHI
RTHTGEKPFACDICGRKFATSSNRTKHTKIHTHPRAPIPKPFQCRICMRNFSRSDN
 LSEHIRTHTGEKPFACDICGRKFAWHSSLRVHTKIHLR
**TRAC-ZFP-Right (SEQ ID No: 451)** 
FQCRICMRNFSRSDHLSTHIRTHTGEKPFACDICGRKFADRSHLARHT
KIHTGSQKPFQCRICMRKFALKQHLNEHTKIHTGEKPFQCRICMRNFSQSGNLARH
IRTHTGEKPFACDICGRKFAHNSSLKDHTKIHLR
**ND1-ZFP-Left (SEQ ID No: 452)** 
FQCRICMRNFSDSGNLRVHIRTHTGEKPYKCPDCGKSFSQSSSLIRHQ
RTHTGEKPYECDHCGKSFSQSSHLNVHKRTHTGEKPYRCKYCDRSFSISSNLQRHV
RNIHLR
**ND1-ZFP-Right (SEQ ID No: 453)** 
YKCPECGKSFSTKNSLTEHQRTHTGEKPYKCPECGKSFSSKKALTEHQ
RTHTGEKPYECNYCGKTFSVSSTLIRHQRIHTGEKPYRCKYCDRSFSISSNLQRHV
RNIHLR
**G1333N-Ddd.Atox (SEQ ID No: 454)**
   GSGSYALGPYQISAPQLPAYNGQTVGTFYYVNDAGGLESKVFSSGG
**G1333C-DddAtox (SEQ ID No: 455)** 
GSPTPYPNYANAGHVEGQSALFMRDNGISEGLVFHNNPEGTCGFCVNM
TETLLPENAKMTVVPPEGAIPVKRGATGETKVFTGNSNSPKSPTKGGC
**G1397N-Ddd.Atox (SEQ ID No: 456)** 
GSGSYALGPYQISAPQLPAYNGQTVGTFYYVNDAGGLESKVFSSGGPT
PYPNYANAGHVEGQSALFMRDNGISEGLVFHNNPEGTCGFCVNMTETLLPENAKMT
VVPPEG
**G1397C-Ddd.Atox (SEQ ID No: 457)**
   GSAIPVKRGATGETKVFTGNSNSPKSPTKGGC
**Adenine deaminase (AD: ABE 8e) (SEQ ID No: 458)** 
SEVEFSHEYWMRHALTLAKRARDEREVPVGAVLVLNNRVIGEGWNRAI
GLHDPTAHAEIMALRQGGLVMQNYRLIDATLYVTFEPCVMCAGAMIHSRIGRVVFG
VRNSKRGAAGSLMNVLNYPGMNHRVEITEGILADECAALLCDFYRMPRQVFNAQKK
AQSSIN
**Full-length DddAtox variant GSVG (SEQ ID No:** 459) 
GSGSYALGPYQISAPQLPAYNGQTVGTFYYVNDAGGLEGKVFSSGGPT
PYPNYANAGHVESQSALFMRDNGISEGLVFHNNPEGTCGFCVNMTETLLPENAKMT
VVPPEGVIPVKRGATGETKVFTGNSNGPKSPTKGGC
**Full-length DddAtox variant E1347A (SEQ ID No:** 460) 
GSGSYALGPYQISAPQLPAYNGQTVGTFYYVNDAGGLESKVFSSGGPT
PYPNYANAGHVAGQSALFMRDNGISEGLVFHNNPEGTCGFCVNMTETLLPENAKMT
VVPPEGAIPVKRGATGETKVFTGNSNSPKSPTKGGC
**Full-length DddAtox variant AAAAA (SEQ ID No:** 461) 
GSGSYALGPYQISAPQLPAYNGQTVGTFYYVNDAGGLESKVFSSGGPT
PYPNYANAGHVEGQSALFMRDNGISEGLVFHNNPEGTCGFCVNMTETLLPENAKMT
VVPPEGAIPVAAGATGETAVFTGNSNSPASPTAGGC
**UGI (SEQ ID No: 462)** 
TNLSDIIEKETGKQLVIQESILMLPEEVEEVIGNKPESDILVHTAYDE

STDENVMLLTSDAPEYKPWALVIQDSNGENKIKML
**Single module**
   **(TALE)-linker-AD-GSVG (SEQ ID No: 463)** 
SGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALL
CDFYRMPRQVFNAQKKAQSSINLVGSGSYALGPYQISAPQLPAYNGQTVGTFYYVN
DAGGLEGKVFSSGGPTPYPNYANAGHVESQSALFMRDNGISEGLVFHNNPEGTCGF
CVNMTETLLPENAKMTVVPPEGVIPVKRGATGETKVFTGNSNGPKSPTKGGC
**(TALE)-linker-AD-E1347A (SEQ ID No: 464)** 
SGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALL
CDFYRMPRQVFNAQKKAQSSINLVGSGSYALGPYQISAPQLPAYNGQTVGTFYYVN
DAGGLESKVFSSGGPTPYPNYANAGHVAGQSALFMRDNGISEGLVFHNNPEGTCGF
CVNMTETLLPENAKMTVVPPEGAIPVKRGATGETKVFTGNSNSPKSPTKGGC
**(TALE)-linker-AD-AAAAA (SEQ ID No: 465)** 
SGSETPGTSESATPESSEVEFSHEYWMRHALTLAKRARDEREVPVGAV
LVLNNRVIGEGWNRAIGLHDPTAHAEIMALRQGGLVMQNYRLIDATLYVTFEPCVM
CAGAMIHSRIGRVVFGVRNSKRGAAGSLMNVLNYPGMNHRVEITEGILADECAALL
CDFYRMPRQVFNAQKKAQSSINLVGSGSYALGPYQISAPQLPAYNGQTVGTFYYVN
DAGGLESKVFSSGGPTPYPNYANAGHVEGQSALFMRDNGISEGLVFHNNPEGTCGF
 CVNMTETLLPENAKMTVVPPEGAIPVAAGATGETAVFTGNSNSPASPTAGGC
**Dual module**
   **(TALE)-linker-AD (SEQ ID No: 466)** 
SGSETPGTSESATPESSEVEFSHEYWMRHALTLAKRARDEREVPVGAV

LVLNNRVIGEGWNRAIGLHDPTAHAEIMALRQGGLVMQNYRLIDATLYVTFEPCVM

CAGAMIHSRIGRVVFGVRNSKRGAAGSLMNVLNYPGMNHRVEITEGILADECAALL

CDFYRMPRQVFNAQKKAQSSIN
**(TALE)-GSVG (SEQ ID No: 467)** 
GSGSYALGPYQISAPQLPAYNGQTVGTFYYVNDAGGLEGKVFSSGGPT
PYPNYANAGHVESQSALFMRDNGISEGLVFHNNPEGTCGFCVNMTETLLPENAKMT
VVPPEGVIPVKRGATGETKVFTGNSNGPKSPTKGGC
**(TALE)-E1347A (SEQ ID No: 468)** 
GSGSYALGPYQISAPQLPAYNGQTVGTFYYVNDAGGLESKVFSSGGPT
PYPNYANAGHVAGQSALFMRDNGISEGLVFHNNPEGTCGFCVNMTETLLPENAKMT
VVPPEGAIPVKRGATGETKVFTGNSNSPKSPTKGGC
**(TALE) -AAAAA (SEQ ID No: 469)** 
GSGSYALGPYQISAPQLPAYNGQTVGTFYYVNDAGGLESKVFSSGGPT
PYPNYANAGHVEGQSALFMRDNGISEGLVFHNNPEGTCGFCVNMTETLLPENAKMT
VVPPEGAIPVAAGATGETAVFTGNSNSPASPTAGGC
**(TALE)-1397C-AD (SEQ ID No: 470)** 
GSAIPVKRGATGETKVFTGNSNSPKSPTKGGCSGSETPGTSESATPES
SEVEFSHEYWMRHALTLAKRARDEREVPVGAVLVLNNRVIGEGWNRAIGLHDPTAH
AEIMALRQGGLVMQNYRLIDATLYVTFEPCVMCAGAMIHSRIGRVVFGVRNSKRGA
AGSLMNVLNYPGMNHRVEITEGILADECAALLCDFYRMPRQVFNAQKKAQSSIN
**(TALE)-1333N-AD (SEQ ID No: 471)** 
GSGSYALGPYQISAPQLPAYNGQTVGTFYYVNDAGGLESKVFSSGGSG

SETPGTSESATPESSEVEFSHEYWMRHALTLAKRARDEREVPVGAVLVLNNRVIGE

GWNRAIGLHDPTAHAEIMALRQGGLVMQNYRLIDATLYVTFEPCVMCAGAMIHSRI

GRVVFGVRNSKRGAAGSLMNVLNYPGMNHRVEITEGILADECAALLCDFYRMPRQV

FNAQKKAQSSIN
**(TALE)-1333C-AD (SEQ ID No: 472)** 
GSPTPYPNYANAGHVEGQSALFMRDNGISEGLVFHNNPEGTCGFCVNM
TETLLPENAKMTVVPPEGAIPVKRGATGETKVFTGNSNSPKSPTKGGCSGSETPGT
SESATPESSEVEFSHEYWMRHALTLAKRARDEREVPVGAVLVLNNRVIGEGWNRAI
GLHDPTAHAEIMALRQGGLVMQNYRLIDATLYVTFEPCVMCAGAMIHSRIGRVVFG
VRNSKRGAAGSLMNVLNYPGMNHRVEITEGILADECAALLCDFYRMPRQVFNAQKK
AQSSIN
Linker (AA stands for amino acid)
   8AA: SGGGLGST (SEQ ID No: 473)
   16AA: SGSETPGTSESATPES (SEQ ID No: 474)
   32AA: SGGSSGGSSGSETPGTSESATPESSGGSSGGS (SEQ ID No: 475)
SOD2 MTS-3xHA: 
MALSRAVCGTSRQLAPVLGYLGSRQKHSLPDYPYDVPDYAGYPYDVPDYAGYPYDV

PDYA (SEQ ID No: 476)
COX8A MTS-3xFLAG:

MASVLTPLLLRGLTGSARRLPVPRAKIHSLDYKDHDGDYKDHDIDYKD

DDDK (SEQ ID No: 477)

### 6. Delivery

The fusion protein according to the present invention may be delivered to cells by various methods known in the art, for example, microinjection, electroporation, DEAE-dextran treatment, lipofection, nanoparticle-mediated transfection, protein transduction domain-mediated introduction, and PEG-mediated transfection, but the present invention is not limited thereto.

Another aspect of the present invention pertains to a nucleic acid encoding the fusion protein.

The nucleic acid may be used interchangeably with "polynucleotide", "nucleotide", "nucleotide sequence", and "oligonucleotide". It may include a nucleotide of any length in a polymeric form, deoxyribonucleotide or ribonucleotide, or analogues thereof. A polynucleotide may have any three-dimensional structure and may perform any function, known or unknown. A polynucleotide may include at least one modified nucleotide, such as a methylated nucleotide and nucleotide analogues. Modification to the nucleotide structure may be possible before or after polymer assembly.

The polynucleotide may have an RNA sequence, a DNA sequence, or a combination thereof (RNA-DNA combination sequence).

In order to express the fusion protein, known expression vectors such as plasmid vectors, cosmid vectors, bacteriophage vectors, and the like may be used, and vectors may be easily constructed by those skilled in the art according to any known method using DNA recombination technology.

The vector may be a plasmid vector or a viral vector, and particularly, examples of the viral vector may include, but are not limited to, adenoviral, adeno-associated viral, lentiviral, and retroviral vectors.

A recombinant expression vector may contain a nucleic acid in a form suitable for expression of the nucleic acid in a host cell, and may include at least one regulatory element selected on a host cell basis so that the recombinant expression vector is used for expression, namely is operably linked to a nucleic acid sequence to be expressed.

Within a recombinant expression vector, "operably linked" means that the nucleotide sequence of interest is connected to the regulatory element in a manner that allows for expression of the nucleotide sequence (e.g. in an *in-vitro* transcription/translation system or in a host cell when the vector is introduced into the host cell).

The recombinant expression vector may be provided in a form suitable for messenger RNA synthesis, including a T7 promoter, which means to include at least one regulatory element to enable *in-vitro* mRNA synthesis, namely messenger RNA synthesis by a T7 polymerase.

The "regulatory element" may include promoters, enhancers, internal ribosome entry sites (IRES), and other expression control elements (e.g. transcription termination signals such as polyadenylation signals and poly-U sequences). The regulatory element includes elements that direct the induction or constitutive expression of a nucleotide sequence in many types of host cells, and elements that direct the expression of a nucleotide sequence only in certain host cells (e.g. tissue-specific regulatory sequences). A tissue-specific promoter may direct expression primarily in a desired tissue of interest such as a muscle, neuron, bone, skin, blood, specific organ (e.g. liver, pancreas), or specific cell type (e.g. lymphocyte). The regulatory element may also direct expression in a transientdependent manner, such as in a cell-cycle- or developmental-stage-dependent manner, which may be specific to tissues or cells or not.

In some cases, the vector includes at least one pol III promoter, at least one pol II promoter, at least one pol I promoter, or combinations thereof. Examples of the pol III promoter include, but are not limited to, U6 and H1 promoters. Examples of the pol II promoter include, but are not limited to, retroviral Rous Sarcoma virus (RSV) LTR promoters (optionally with RSV enhancer), cytomegalovirus (CMV) promoters (optionally with CMV enhancer) (e.g. Boshart et al. al (1985) Cell 41:521-530), SV40 promoters, dihydrofolate reductase promoters, β-actin promoters, phosphoglycerol kinase (PGK) promoters, and EF1α promoters.

The "regulatory element" may include an enhancer such as WPRE; CMV enhancer; R-U5' segment in LTR of HTLV-I; SV40 enhancer; and an intronic sequence between exons 2 and 3 of rabbit β-globin. It will be appreciated by those skilled in the art that design of the expression vector may be dependent on factors, such as choice of a host cell to be transformed, the level of expression desired, and the like. The vector may be introduced into a host cell to form a transcript, protein, or peptide including a fusion protein or peptide encoded by the nucleic acid as described herein (e.g. clustered regularly interspaced short palindromic repeat (CRISPR) transcripts, proteins, enzymes, mutants thereof, fusion proteins thereof, etc.). Useful vectors may include lentiviral and adeno-associated viral vectors, and these types of vectors may also be selected to target certain types of cells.

The vector may be delivered *in vivo* or into cells through microinjection (e.g., direct injection into a lesion or target site), electroporation, lipofection, viral vector, nanoparticles, PTD (protein translocation domain) fusion protein method, etc.

The nucleic acid may be injected in the form of ribonucleic acid, for example, messenger ribonucleic acid mRNA, so that gene base editing of cells, such as animal cells or plant cells, is possible without limitation.

The nucleic acid according to the present invention may be in the form of mRNA, and when delivered in the form of mRNA, compared to delivery in the form of a vector using DNA, the transcription process into mRNA is unnecessary, and thus gene editing may be initiated quickly. There is a high possibility of transient protein expression.

The inventors of the present application found that, when a cytosine base editor was injected into plant cells in the form of ribonucleic acid, for example, messenger ribonucleic acid, for plant organelle gene editing, the off-target effect was reduced compared to delivery with a plasmid. In plant organelle gene editing, when the cytosine base editor was transformed into plant cells in the form of mRNA, it was demonstrated for the first time that there is an advantage in off-target effect compared to plasmids.

The mRNA may be delivered directly or through a carrier. In some cases, mRNA of the nuclease and/or the cleavage factor may be chemically modified or directly delivered in the form of synthetic self-replicative RNA.

Methods of delivering mRNA molecules into cells *in vitro* or *in vivo* are contemplated, including methods of delivering mRNA to cells or methods of delivering mRNA to cells of organisms such as humans or animals *in vivo.* For example, mRNA molecules may be delivered into cells using lipids (e.g. liposomes, micelles, etc.), nanoparticles or nanotubes, or cationic compounds (e.g. polyethyleneimine or PEI). In some cases, a biolistic method, such as a gene gun or biolistic particle delivery system, may be used to deliver mRNA into cells.

Examples of the carrier may include, but are not limited to, cell penetrating peptides (CPPs), nanoparticles, and polymers.

The CPP is a short peptide that facilitates cellular uptake of various molecular cargoes (from nanoscale particles to small chemical molecules and large fragments of DNA).

Regarding the nanoparticles, the composition according to the present invention may be delivered via polymer nanoparticles, metal nanoparticles, metal/inorganic nanoparticles, or lipid nanoparticles. The polymer nanoparticles may be, for example, DNA nanoclews or thread-like DNA nanoparticles synthesized by rolling circle amplification. DNA nanoclews or thread-like DNA nanoparticles may be loaded with mRNA and coated with PEI to improve endosomal escape. These complexes bind to cell membranes, are internalized, and then delivered to the nucleus via endosomal escape.

Regarding the metal nanoparticles, gold particles may be connected and complexed with a cationic endosomal disruptive polymer and thus delivered to cells. The cationic endosomal disruptive polymer may include, for example, polyethylene imine, poly(arginine), poly(lysine), poly(histidine), poly-[2-{(2-aminoethyl)amino}-ethyl-aspartamide (pAsp(DET)), block copolymer of poly(ethylene glycol) (PEG) and poly(arginine), block copolymer of PEG and poly(lysine), or block copolymer of PEG and poly{N-[N-(2-aminoethyl)-2-aminoethyl]aspartamide} (PEG-pAsp(DET)).

Regarding the metal/inorganic nanoparticles, mRNA may be encapsulated through, for example, zeolitic imidazolate framework-8 (ZIF-8).

In some cases, mRNA, which is negatively charged, may be coupled with cationic materials to form nanoparticles, which may penetrate cells through receptor-mediated endocytosis or phagocytosis.

Examples of the cationic polymer may include polyallylamine (PAH); polyethyleneimine (PEI); poly(L-lysine) (PLL); poly(L-arginine) (PLA); polyvinylamine homopolymers or copolymers; poly(vinylbenzyl-tri-C1-C4-alkylammonium salts); polymers of aliphatic or alicyclic dihalides and aliphatic N,N,N',N'-tetra-C1-C4-alkylalkylenediamines; poly(vinylpyridine) or poly(vinylpyridinium salt); poly(N,N-diallyl-N,N-di-C1-C4-alkyl-ammonium halide); homopolymers or copolymers of quaternized di-C1-C4-alkyl-aminoethyl acrylates or methacrylates; POLYQUAD^{™}; polyaminoamide, and the like.

The cationic lipids may include cationic liposomal formulations. The lipid bilayer of liposomes may protect encapsulated nucleic acids from degradation and may prevent specific neutralization by antibodies capable of binding to nucleic acids. During endosomal maturation, endosome membranes and liposomes are fused, enabling efficient endosomal escape of cationic lipidnucleases. Examples of the cationic lipids may include polyethyleneimine, starburst polyamidoamine (PAMAM) dendrimers, Lipofectin (combination of DOTMA and DOPE), lipofectase, LIPOFECTAMINE^{®} (e.g. Lipofectamine^{®} 2000, Lipofectamine^{®} 3000, Lipofectamine^{®} RNAiMAX, Lipofectamine^{®} LTX), SAINT-RED (Synvolux Therapeutics, Groningen, The Netherlands), DOPE, Cytofectin (Gilead Sciences, Foster City, California), and Eufectin (JBL, San Luis Obispo, California). Representative cationic liposomes may be prepared from N-[1-(2,3-dioleoloxy)-propyl]-N,N,N-trimethylammonium chloride (DOTMA), N-[1-(2,3-dioleoloxy)-propyl]-N,N,N-trimethylammonium methylsulfate (DOTAP), 3β-[N-(N',N'-dimethylaminoethane)carbamoyl]cholesterol (DC-Chol), 2,3-dioleyloxy-N-[2(sperminecarboxamido)ethyl]-N,N-dimethyl-1-propanaminium trifluoroacetate (DOSPA), 1,2-dimyristyloxypropyl-3-dimethyl-hydroxyethyl ammonium bromide, or dimethyldioctadecylammonium bromide (DDAB).

Regarding the lipid nanoparticles, they may be delivered using liposomes as carriers. Liposomes are spherical vesicular structures composed of a unilamellar or multilamellar lipid bilayer surrounding an inner aqueous compartment and a relatively impermeable outer lipophilic phospholipid bilayer. Liposomal formulations may contain primarily natural phospholipids and lipids such as 1,2-distearoyl-sn-glycero-3-phosphatidylcholine (DSPC), sphingomyelin, phosphatidylcholine, or monosialoganglioside. In some cases, cholesterol or 1,2-dioleoyl-sn-glycero-3-phosphoethanolamine (DOPE) may be added to the lipid membrane in order to resolve instability in plasma. The addition of cholesterol reduces the rapid release of encapsulated bioactive compounds into plasma or 1,2-dioleoyl-sn-glycero-3-phosphoethanolamine (DOPE) increases stability.

### 7. Base editing

Still another aspect of the present invention pertains to a composition for base editing including the fusion protein or the nucleic acid.

Yet another aspect of the present invention pertains to a base editing method including treating cells with the composition.

After binding of a DNA-binding protein such as a TALE or ZFP (zinc finger protein) to the target DNA, the cytosine deaminase of the fusion protein hydrolyzes the amino group of cytosine to convert the same into uracil. Since uracil may form a base pair with adenine, a cytosine-guanine base pair may be ultimately edited to a thymine-adenine base pair via a uracil-adenine base pair during intracellular DNA replication. In addition, the adenine deaminase of the fusion protein hydrolyzes the amino group of adenine to convert the same to hypoxanthine. Since hypoxanthine may form a base pair with cytosine, similarly, an adenine-thymine base pair may be edited to a guanine-cytosine base pair via a hypoxanthine-cytosine base pair during intracellular DNA replication.

The cells may be eukaryotic cells (e.g. fungi such as yeast, eukaryotic animals, and/or eukaryotic plant-derived cells (e.g. embryonic cells, stem cells, somatic cells, germ cells, etc.), etc.), eukaryotic animals (e.g. primates such as humans, monkeys, dogs, pigs, cattle, sheep, goats, mice, rats, etc.), or eukaryotic plants (e.g. algae such as green algae, corn, soybeans, wheat, rice, etc.), but are not limited thereto.

### (1) Base editing of plant cell DNA

The present invention pertains to a composition or method for base editing of plant cell DNA. The composition for base editing in plant cells includes the fusion protein or a nucleic acid encoding the same; and a nuclear localization signal (NLS) peptide, a chloroplast transit peptide, a mitochondrial targeting signal (MTS), a nuclear export signal, or a nucleic acid encoding the same.

The present invention also provides a composition for base editing in plant cells including the fusion protein or the nucleic acid; and a nuclear localization signal (NLS) peptide or a nucleic acid encoding the same.

The present invention also provides a composition for base editing in plant cells including the fusion protein or the nucleic acid; and a chloroplast transit peptide or a nucleic acid encoding the same.

The present invention also provides a composition for base editing in plant cells including the fusion protein or the nucleic acid; and a mitochondrial targeting signal (MTS) or a nucleic acid encoding the same.

In some cases, the present invention also provides a composition for base editing in plant cells, further including a nuclear export signal or a nucleic acid encoding the same.

Specifically, the present invention pertains to a composition or method for base editing of nuclear DNA, mitochondrial DNA, or chloroplast DNA in plant cells.

Specifically, the fusion protein may be delivered to plant cells through the following:
injection using a gene gun (bombardment);
PEG-mediated protoplast transfection;
protoplast transfection through electroporation; or
protoplast injection through microinjection.

The polynucleotide sequence encoding the fusion protein according to the present invention may be an RNA sequence, a DNA sequence, or a combination thereof (RNA-DNA combination sequence).

The polynucleotide encoding the fusion protein may be delivered to plant cells through the following:
transformation using Agrobacterium, such as *Agrobacterium tumefaciens, Agrobacterium rhizogene,* etc.
   - binary vector,
   - viral vector: Geminivirus, tobacco rattle virus (TRV), tomato mosaic virus (ToMV), foxtail mosaic virus (FoMV), barley yellow striate mosaic virus (BYSMV), Sonchus yellow net rhabdovirus (SYNV), etc.;
transfection using a virus;
injection (bombardment, gene gun);
PEG-mediated protoplast transfection;
protoplast transfection through electroporation; or
protoplast injection through microinjection.

Examples of the virus may include geminivirus, tobacco rattle virus (TRV), tomato mosaic virus (ToMV), foxtail mosaic virus (FoMV), barley yellow striate mosaic virus (BYSMV), Sonchus yellow net rhabdovirus (SYNV), and the like in viral vectors.

The vector may be delivered into cells through microinjection (e.g. direct injection into a lesion or target site), electroporation, lipofection, viral vector, nanoparticles, PTD (protein translocation domain) fusion protein method, etc.

Regarding the protein transported to plant organelles or the nucleic acid encoding the same, the plant organelles may be mitochondria, chloroplasts, or plastids (leucoplasts, chromoplasts).

The protein transported to plant organelles may be, for example, a chloroplast transit peptide or a mitochondrial targeting signal (MTS).

For example, the chloroplast transit peptide (CTP) or mitochondrial targeting signal (MTS) binds and is then delivered to chloroplasts or mitochondria in plant cells. When delivered to chloroplasts or mitochondria, the remaining portion except for the N-terminal CTP or MTS is delivered into chloroplasts or mitochondria in the form of preprotein. In the process of entering inside of chloroplasts or mitochondria, the delivered protein portion is separated, and targets chloroplasts or mitochondria to induce site-specific base editing.

In addition to the fusion protein or the nucleic acid encoding the same, a chloroplast transit peptide (CTP) or a nucleic acid encoding the same or a mitochondrial targeting signal (MTS) or a nucleic acid encoding the same can be fused and is delivered to plant cells, enabling base editing of plant mitochondrial, chloroplast, chromoplast or leucoplast DNA.

Base editing may be achieved with higher efficiency when the nuclear export signal is attached to the base editing protein during mitochondrial gene editing. The nuclear export signal may be derived from, for example, MVM (minute virus of mice), but the present invention is not limited thereto. The nuclear export signal may include, for example, the amino acid sequence of SEQ ID NO: 31, but is not limited thereto.
VDEMTKKFGTLTIHDTEK (SEQ ID NO: 31)

The present invention further includes a TAL (transcription activator-like) effector (TALE)- FokI nuclease, or a nucleic acid encoding the same, that cleaves a wild-type DNA sequence but does not cleave an edited base sequence, or ZFN (zinc finger nuclease) or a nucleic acid encoding the same, particularly mitoTALEN (mitochondrial TALE nuclease), which is a mitochondrial nuclease, or a nucleic acid encoding the same, or ZFN (zinc finger nuclease) or a nucleic acid encoding the same, thereby expecting mitochondrial base editing with higher efficiency even when using a mitochondrial sequence cleavage protein simultaneously.

### (2) Base editing of animal cell DNA

The present invention pertains to a composition or method for base editing of animal cell DNA. The composition for base editing in animal cells includes the fusion protein or a nucleic acid encoding the same; and a nuclear localization signal (NLS) peptide, a mitochondrial targeting signal (MTS), a nuclear export signal, or a nucleic acid encoding the same.

The present invention also provides a composition for base editing in animal cells, including the fusion protein or the nucleic acid; and a nuclear localization signal (NLS) peptide or a nucleic acid encoding the same.

The present invention also provides a composition for base editing in animal cells, including the fusion protein or the nucleic acid; and a mitochondrial targeting signal (MTS) or a nucleic acid encoding the same.

In some cases, the present invention also provides a composition for base editing in animal cells, further including a nuclear export signal or a nucleic acid encoding the same.

The animal cells are non-human animal cells, and treatment with a nuclear export signal or a nucleic acid encoding the same and/or a mitochondrial targeting signal (MTS) or a nucleic acid encoding the same enables base editing of mitochondrial DNA in non-human animal cells.

For example, in addition to the fusion protein or the nucleic acid encoding the same, a mitochondrial targeting signal (MTS) binds and is delivered to mitochondria. When delivered to mitochondria, the remaining portion except for the N-terminal MTS is delivered into mitochondria in the form of preprotein. In the process of entering inside of mitochondria, the delivered protein portion is separated, and targets mitochondrial DNA to induce site-specific base editing.

The present invention pertains to a composition or method for base editing of mitochondrial DNA in non-human animal cells in which a nuclear export signal (NES) or a nuclear acid encoding the same is fused to a TALE-DdCBE (TALE DddA-derived cytosine base editor) including a mitochondrial targeting signal, TAL effector, and cytosine deaminase (DddAₜₒₓ), or a nucleic acid encoding the same. The addition of the nuclear export signal in a fusion protein can reduce nuclear DNA base editing at sites with similar DNA sequences.

According to the present invention, more efficient animal mitochondrial DNA base editing may be achieved by including a nuclear export signal or a nucleic acid encoding the same. Moreover, nuclear DNA base editing for mitochondrial-nuclear-like sequences may be reduced by virtue of the nuclear export signal, so that only mitochondrial DNA may be edited.

The nuclear export signal may be derived from, for example, MVM (minute virus of mice), but the present invention is not limited thereto. The nuclear export signal may include, for example, the amino acid sequence of VDEMTKKFGTLTIHDTEK (SEQ ID NO: 31), but is not limited thereto.

Before editing simultaneously or sequentially with (1) a nuclear export signal or a nucleic acid encoding the same and (2) a DNA-binding protein, a deaminase or a variant thereof, or a nucleic acid encoding the same, a TAL (transcription activator-like) effector (TALE)-FokI nuclease that cleaves a wild-type DNA bsae sequence but does not cleave an edited base sequence or a nucleic acid encoding the same or ZFN (zinc finger nuclease) or a nucleic acid encoding the same may be injected into eukaryotic cells.

In particular, with regard to base editing of mitochondrial genes in eukaryotic cells, a nuclear export signal or a nucleic acid encoding the same and/or a mitochondrial targeting signal (MTS) or a nucleic acid encoding the same may be included.

According to the present invention, when the nuclear export signal is attached to the base editing protein during animal mitochondrial gene editing, base editing is possible with higher efficiency, and in animal embryos, non-specific base editing of homologous sequences in the nucleus is also inhibited.

In the present invention, by further including a mitochondrial nuclease, namely mitoTALEN (mitochondrial TALE nuclease), or a nucleic acid encoding the same, mitochondrial base editing may be achieved with higher efficiency even when the mitochondrial nuclease is used simultaneously. Mitochondrial DNA may be cleaved using mitoTALEN (mitochondrial TALE nuclease), which is a mitochondrial DNA nuclease, and wild-type mitochondrial genomes may be cleaved to obtain base-edited genomes in animals with high efficiency.

In the present invention, by further including mitoTALEN (mitochondrial TALE nuclease), which is a mitochondrial nuclease, or a nucleic acid encoding the same, mitochondrial base editing may be achieved with higher efficiency even when the mitochondrial nuclease is used simultaneously. Specifically, a fusion protein (mitoTALEN), including a TAL effector domain to which a mitochondrial targeting signal and the FokI nuclease are attached or ZFN or a nucleic acid encoding the same, or a nucleic acid encoding the same may be included.

Mitochondrial DNA may be cleaved using mitoTALEN (mitochondrial TALE nuclease), which is a mitochondrial DNA nuclease, and wild-type mitochondrial genomes may be cleaved to obtain base-edited genomes in animals with high efficiency.

In some cases, UGI (uracil DNA glycosylase inhibitor) capable of increasing base editing efficiency may be additionally included. UGI is capable of increasing base editing efficiency by inhibiting the activity of UDG (uracil DNA glycosylase), which is an enzyme that repairs mutant DNA by catalyzing the removal of U from DNA.

Specifically, DddA-derived cytosine base editors (DdCBEs) composed of the split interbacterial toxin DddAtox, a transcription activator-like effector (TALE) designed to bind to DNA, and a uracil glycosylase inhibitor (UGI) enabled targeted cytosine-thymine base editing in mitochondrial DNA. According to embodiments, high-efficiency mitochondrial DNA editing was possible in mouse embryos. Among mitochondrial genes, MT-ND5 (ND5), which encodes the subunit of a NADH dehydrogenase that catalyzes NADH dehydration and electron transfer to ubiquinone, was targeted, cauing mutations associated with human mitochondrial diseases, such as m.G12918A, and mutations that create early stop codons, such as m.C12336T. Thereby, it was possible to construct a mitochondrial disease model in mice, suggesting the possibility of treating mitochondrial diseases.

(2) A DNA-binding protein, a deaminase or a variant thereof, or a nucleic acid encoding the same may be linked to (1) a nuclear export signal or a nucleic acid encoding the same, and (3) mitoTALEN (mitochondrial TALE nuclease), which is a nuclease, or a nucleic acid encoding the same may be linked to (1). In order to deliver (1) to (3), a single delivery vehicle or a plurality of delivery vehicles may be used in combination with the same or different configurations.

The (1) may be included in the first delivery vehicle, the (2) may be included in the second delivery vehicle, and the (3) may be included in the third delivery vehicle. These individual delivery systems may be viral delivery vehicles simultaneously, may be viral and non-viral delivery vehicles, or may be non-viral delivery vehicles simultaneously.

The nuclear export signal, DdCBE, and mitoTALEN of (1) to (3) may be mixed and delivered.

At least one selected from among (1) to (3) may be delivered to a nuclear export signal, DdCBE, or mitoTALEN, and some may be delivered by locating DNA sequences encoding (1) to (3) on a vector.

The DNA sequences encoding the (1) to (3) above may be located on the same vector and delivered simultaneously through one vector, or may be located on different vectors and delivered.

The animals according to the present invention may include human or non-human animals. Examples of the non-human transgenic animals may be insects, annelids, mollusks, brachiopods, nematodes, coelenterates, sponges, chordates, and vertebrates, the vertebrates may be fish, amphibians, reptiles, birds, or mammals, the insects may be *Drosophila,* the nematodes may be *C*. *elegans,* the fish may be zebrafish, the mammals may be a primate, carnivora, insectivora, rodentia, artiodactyla, perissodactyla, or proboscidea, and the rodentia may include rats or mice.

A base-edited animal may be produced by introducing the composition according to the present invention into an embryo of a human or non-human animal, transferring the embryo into a surrogate mother, and conceiving the embryo. The composition according to the present invention may be introduced into the fertilized egg of the animal and cultured.

The fertilized egg thus obtained may be transferred to a surrogate mother and delivered. Confirming whether or not the non-human transgenic animal is transgenic after delivery may be further included. The non-human transgenic animals may be mated to produce progeny transgenic animals.

The "progeny" means all viable offspring transgenic animals resulting from mating of non-human transgenic animals, and more specifically, may be the F1 generation produced by mating the transgenic animals to each other as parents or mating the same with normal animals, the F2 generation produced by mating the animals of the F1 generation with normal animals, and subsequent generations, but the present invention is not limited thereto.

The mating may be characterized by mating of the transgenic animals or normal animals. The present invention may include cells, tissues, and byproducts isolated from the transgenic animals or progeny transgenic animals. The byproduct may include any material derived from the transgenic rabbits, and is preferably selected from the group consisting of blood, serum, urine, feces, saliva, organs, and skin, but is not limited thereto.

### Examples

A better understanding of the present invention may be obtained through the following examples. These examples are merely set forth to illustrate the present invention, and are not to be construed as limiting the scope of the present invention, as will be apparent to those skilled in the art.

### Example 1. Zinc finger deaminase (ZFD)

Base editing of nuclear DNA or mitochondrial DNA is widely useful in biomedical research, medicine, and biotechnology. A ZFD platform includes a DNA-binding protein, a split interbacterial toxin deaminase DDDAtox, and a uracil glycosylase inhibitor (UGI). Here, a ZFD catalyzes targeted C-to-T base conversion without inducing unwanted small insertions and deletions (indels) in human cells. Using publicly available zinc finger resources, plasmids encoding ZFDs were constructed, achieving base editing at frequencies of up to 60% in nuclear DNA and 30% in mitochondrial DNA. Unlike CRISPR-based base editing, ZFD does not create single- or double-stranded breaks through DNA cleavage, so that unwanted insertions and deletions (indels) caused by error-prone non-homologous end joining are not produced at target sites. Moreover, recombinant ZFD proteins purified from *E. coli* penetrate human cells spontaneously to induce targeted base conversions. This demonstrates the proof-of-principle of gene-free gene therapy.

Technologies for genome editing in eukaryotic cells and organisms include zinc finger nucleases (ZFNs), transcription activator-like effector (TALE) nucleases (TALENs), TALE-linked split interbacterial deaminase toxin DddA-derived cytosine base editors (a.k.a. DdCBEs), CRISPR-Cas9, and Cas9-linked deaminases without cleaving activity (a.k.a. base editors), but are not limited thereto. These tools are in principle composed of two functional units, namely a DNA-binding moiety and a catalytic moiety. Thus, a zinc finger array or TALE array functions as a DNA-binding moiety, whereas a nuclease (FokI in ZFNs and TALENs) or deaminase (split DddAₜₒₓ in DdCBE and APOBEC1 in CBE) functions as a catalytic unit. Crispr-cas9 has both a nuclease function and an RNA-guided DNA-binding protein function. Custom-designed programmable nucleases such as ZFNs, TALENs, and Cas9 cleave DNA, producing double-strand breaks, the repair of which gives rise to gene knock-out and knock-in in a targeted manner. However, programmable nuclease-induced double-strand breaks may cause unwanted large gene deletions at target sites, p53 activation, and chromosomal rearrangements during repair of two concurrent DSBs at on-target and off-target sites. In contrast, programmable base editors, including cytosine and adenine base editors (CBEs and ABEs), do not produce DSBs, avoiding these unwanted events in cells, and efficiently catalyze single nucleotide conversions without a repair template or donor DNA. However, CBEs or ABEs containing Cas9 nickase variants cleave the target DNA strand to produce nicks or singlestrand breaks, resulting in unwanted indels at gene target sites.

CBEs catalyze C-to-T base conversions in nuclear DNA and mitochondrial DNA in cells. We used custom-designed DdCBEs to demonstrate mitochondrial DNA editing in mice and chloroplast DNA editing in plants. We also created zinc finger deaminases (ZFDs) for indel-free, precision base editing in human and other eukaryotic cells by linking split DddAtox to custom-designed zinc finger proteins. Since zinc finger arrays (a 2x0.3-0.6 k base pair) are smaller in size than TALE arrays (a 2x1.7-2 k base pair) or *S. pyogenes* Cas9 (a 4.1 k base pair), ZFD-encoding genes may be readily packaged in a viral vector with a limited cargo space such as AAV for *in vivo* studies and gene therapy applications. Unlike TALE arrays, zinc finger arrays lack bulky domains at the C-terminus or N-terminus, making them engineering friendly. The split DddAtox halves may be fused to either the C-terminus or the N-terminus of the zinc finger protein. In addition, zinc finger proteins having an intrinsic ability to penetrate cells enable nucleic acid-free gene editing in human cells. These properties make zinc finger proteins an ideal platform as a DNA-binding module for base editing in the nucleus or other organelles.

### 1-1. Materials and methods

### Plasmid construction

The p3s-ZFD plasmids for mammalian expression were created by modifying the p3s-ABE7.10 plasmid (Addgene, #113128) after digestion with HindIII and XhoI (NEB) enzymes. The digested p3s plasmid and synthesized insert DNAs were assembled using a HiFi DNA assembly kit (NEB). All insert DNAs encoding MTS, ZFP (Toolgen, Sangamo, and Barbas module), split DddA, or UGI were synthesized by IDT. The pTarget plasmids were designed to determine the optimal length of the spacer sequence for ZFD activity. Each pTarget plasmid, containing two ZFP-binding sites with a spacer of various lengths between, was constructed by inserting the ZFP-binding sequences and a spacer sequence into the pRGS-CCR5-NHEJ reporter plasmid digested with two enzymes (EcoRI and BamHI, NEB). The pET-ZFD plasmids for protein production in *E. coli* were created by modifying the pET-Hisx6-rAPOBEC1-XTEN-nCas9-UGI-NLS plasmid (Addgene, #89508) after digestion with NcoI and XhoI (NEB). The ZFD sequences were amplified from the p3s-ZFD plasmid using PCR, and Hisx6 tag and GST tag sequences were synthesized as oligonucleotides (Macrogen). All plasmids were generated using a HiFi DNA Assembly Kit (NEB) to insert sequences encoding the ZFD and tag for protein purification into the digested pET plasmid. Chemically competent DH5α *E. coli* cells were used for transformation of plasmids, and plasmids were purified with an AccuPrep Plasmid Mini Extraction Kit (Bioneer) according to the manufacturer's protocol. After identifying the entire sequence with Sanger sequencing, desired plasmids were selected.

### HEK293T cell culture and transfection

HEK 293T cells (ATCC CRL-11268) were cultured in Dulbecco's Modified Eagle Medium (Welgene) supplemented with 10% fetal bovine serum (Welgene) and 1% antibiotic-antimycotic solution (Welgene). HEK 293T cells (7.5×10⁴) were seeded into 48-well plates. After 18-24 hours, the cells were transfected at 70-80% confluency with plasmids encoding left and right ZFDs (500 ng each), or together with a pTarget plasmid (10 ng), using Lipofectamine 2000 (1.5 pl, Invitrogen). The cells were harvested 96 hours after transfection, and then lysed by incubation at 55°C for 1 hour and then at 95°C for 10 minutes in 100 pl of cell lysis buffer (50 mM Tris-HCl, pH 8.0 (Sigma-Aldrich), 1 mM EDTA (Sigma-Aldrich), 0.005% sodium dodecyl sulfate (Sigma-Aldrich)) supplemented with 5 pl of Proteinase K (Qiagen). For whole mtDNA sequencing, HEK 293T cells were transfected with serially diluted concentrations of plasmids or mRNA encoding ND1- or ND2-targeted mitoZFD pairs. The amounts of constructs (ng) that were delivered per 7.5×10⁴ cells are indicated. 96 hours after transfection, mtDNA was isolated from the cells.

### K562 cell culture and transfection

K562 cells were cultured in RPMI 1640 supplemented with 10% fetal bovine serum (Welgene) and 1% antibiotic-antimycotic solution (Welgene). For ZFD delivery into K562 cells by electroporation, an Amaxa 4D-Nucleofector^{™} X Unit system with program FF-120 (Lonza) was used. The maximum volume of substrate solution added to each sample was 2 pl when using a 16-well Nucleocuvette^{™} Strip. K562 cells (1×10⁵) were transfected with 220 pmol (for maximum capacity) or 110 pmol (for half of the maximum capacity) of each of the left and right ZFD proteins or 500 ng of plasmids encoding left and right ZFDs. 96 hours after treatment, the cells were harvested by centrifugation at 100 g for 5 minutes, and lysed by incubation at 55°C for 1 hour and then at 95°C for 10 minutes in 100 pl of cell lysis buffer (50 mM Tris-HCl, pH 8.0 (Sigma-Aldrich), 1 M EDTA (Sigma-Aldrich), 0.005% sodium dodecyl sulfate (Sigma-Aldrich)) supplemented with 5 pl of Proteinase K (Qiagen). For direct delivery of ZFDs or ZFD-encoding plasmids into K562 cells, reference was made to methods previously used for direct delivery of ZFNs. A mixture of left and right ZFD proteins (at a final concentration of 50 µM) or a mixture of plasmids encoding left and right ZFDs (500 ng each) was diluted with serum-free medium at a pH of 7.4 containing 100 mM L-arginine and 90 µM ZnCl₂ to a final volume of 20 pl. K562 cells (1×10⁵) were centrifuged at 100 g for 5 minutes and the supernatant was discarded. The cells were then resuspended in a diluted ZFD solution and incubated for 1 hour at 37°C. After incubation, the cells were centrifuged at 100 g for 5 minutes and then resuspended in fresh culture medium. The cells were maintained at 30°C (for a transient hypothermic condition) or 37°C for 18 hours and then allowed to grow at 37°C for two more days. Some cells were treated twice according to the above procedure. The cells were analyzed 96 hours after treatment.

### ZFD protein expression and purification

The plasmids encoding each pair of ZFDs, each with a C-terminal GST tag, were transformed into Rosetta (DE3) competent cells, which were then cultured in LB-agar plates containing kanamycin. After culture overnight, a single colony was picked and cultured overnight (preculture) in liquid medium containing 50 µg/ml kanamycin and 100 µM ZnCl₂ at 37°C. The next day, a portion of the preculture was transferred to a large volume of liquid medium, followed by culture at 37°C until absorbance A600 nm was about 0.5-0.70. The cultures were placed on ice for about 1 hour, after which ZFD protein expression was induced by the addition of 0.5 mM isopropyl β-D-1-thiogalactopyranoside (IPTG; GoldBio) and the cultures were incubated at 18°C for 14 hours.

In the protein purification process, the cells were resuspended in lysis buffer (50 mM Tris-HCl (Sigma-Aldrich), 500 mM NaCl (Sigma-Aldrich), 1 mM MgCl₂ (Sigma-Aldrich), 10 mM 1,4-dithiothreitol (DTT; GoldBio), 1% Triton X-10 (Sigma-Aldrich), 10% glycerol, 1 mM phenylmethylsulfonyl fluoride (Sigma-Aldrich), 1 mg/ml lysozyme from chicken egg white (Sigma-Aldrich), 100 µM ZnCl₂ (Sigma-Aldrich), 100 mM arginine (Sigma-Aldrich), pH 8.0), followed by sonication (3 min total, 5 s on, 10 s off) for further lysis. Thereafter, the solution was centrifuged (13,000 rpm) to extract only the supernatant. The supernatant was incubated for 1 hour by adding Glutathione Sepharose 4B (GE Healthcare) thereto. After incubation, the resin-lysate mixture was placed in a column, followed by washing three times with wash buffer (50 mM Tris-HCl (Sigma-Aldrich), 500 mM NaCl (Sigma-Aldrich), 10 mM DTT (GoldBio), 1 mM MgCl₂ (Sigma-Aldrich), 100 µM ZnCl₂ (Sigma-Aldrich), 10% glycerol, 100 mM arginine (Sigma-Aldrich), pH 8.0). The proteins attached to the resin were eluted from the resin using elution buffer (50 mM Tris-HCl (Sigma-Aldrich), 500 mM NaCl (Sigma-Aldrich), 1 mM MgCl₂ (Sigma-Aldrich), 40 mM glutathione (Sigma-Aldrich), 10% glycerol, 1 mM DTT (GoldBio), 100 µM ZnCl₂ (Sigma-Aldrich), 100 mM arginine (Sigma-Aldrich), pH 8.0). Finally, the eluted proteins were concentrated to a concentration of about 15 ng/µl (200-240 pmol/µl, depending on protein size).

### In vitro deamination of PCR amplicons by ZFD

An amplicon containing the TRAC site was prepared using PCR. 8 µg of the amplicon was incubated with 2 µg of each ZFD protein (left-G1397N and right-G1397C) in NEB3.1 buffer containing 100 µM ZnCl₂ for 1-2 hours at 37°C. After reaction, ZFD proteins were removed by incubation with 4 pl of Proteinase K solution (Qiagen) at 55°C for 30 minutes, and the amplicon was purified using a PCR purification kit (MGmed). 1 µg of the purified amplicon was incubated with 2 units of USER enzyme (NEB) for 1 hour at 37°C. The amplicon was then incubated with 4 µl of Proteinase K solution (Qiagen) and purified again using a PCR purification kit (MGmed). The purified PCR product was subjected to electrophoresis on an agarose gel and imaged.

### Targeted deep sequencing

In order to analyze the base editing ratio of on-target and off-target sites, the target sites were subjected to overlapping primary PCR, secondary PCR amplification, and tertiary PCR using TruSeq HT Dual index-containing primers using PrimeSTAR^{®} GXL DNA polymerase (TAKARA), so that deep sequencing libraries were produced. The libraries were paired-end sequenced using an Illumina MiniSeq.

### mRNA preparation

DNA templates containing a T7 RNA polymerase promoter upstream of the ZFD sequence were generated by PCR using forward and reverse primers (forward: 5'-CATCAATGGGCGTGGATAG-3' SEQ ID No: 116, reverse: 5'-CATCAATGGGCGTGGATAG-3' SEQ ID No: 117, reverse: 5'-GACACCTACTCAGACAATGC-3' SEQ ID No: 118). Then, mRNAs were synthesized *in vitro* using a mMESSAGE mMACHINE^{™} T7 ULTRA transcription kit (Thermo Fisher). *In-vitro* transcribed mRNAs were purified using a MEGAclear^{™} Transcription Clean-Up Kit (Thermo Fisher) according to the manufacturer's protocol.

### Whole mitochondrial genome sequencing

For whole mitochondrial genome sequencing, three steps are required. 1. Extraction of mtDNA from isolated mitochondria: 3×10⁵ HEK293T cells were trypsinized and collected by centrifugation (500 g, 4 minutes, 4°C) 96 hours after transfection with ND1- or ND2-targeted mitoZFD pairs. The cells were then washed with phosphate-buffered saline (Welgene) and collected again by centrifugation. The supernatant was removed and mitochondria were isolated from the cultured cells using the reagent-based method of the Mitochondria Isolation Kit for Cultured Cells (Thermo Fisher) according to the manufacturer's protocol. Then, mtDNA was extracted from the isolated mitochondria using a DNeasy Blood & Tissue Kit (Qiagen). 2. Production of NGS library: an Illumina DNA Prep kit with Nextera^{™} DNA CD Indexes (Illumina) was used to produce an NGS library from the extracted mtDNA. 3. NGS: the libraries were pooled and loaded onto a MiniSeq sequencer (Illumina). The average sequencing depth was >50.

### Analysis of mitochondrial genome-wide DNA editing

In order to analyze NGS data from whole mitochondrial genome sequencing, the Fastq files were aligned to the GRCh38.p13 (release v102) reference genome using BWA and BAM files with SAMtools (v.1.9) were generated by fixing read pairing information and flags. Then, the REDItoolDenovo.py script from REDItools (v.1.2.1) was used to identify positions with base editing rates of 1% or more among all cytosines and guanines in the mitochondrial genome. Positions with base editing rates of 50% or more were regarded as single-nucleotide variations in the cell lines and were excluded from all samples. For off-target analysis, the target site of each ZFD was excluded. The remaining positions with an editing frequency of ≥1% were regarded as off-target sites and the number of edited C/G nucleotides was counted. In order to calculate the average C/G to T/A base editing frequency for all C/Gs in the mitochondrial genome, the editing rates in the off-target sites were averaged. Specificity ratios were calculated by dividing the average on-target editing frequency by the average off-target editing frequency. Mitochondrial genome-wide graphs were created by plotting the base editing rates at on-target and off-target sites.

### 1-2. Optimization of ZFD construct

In order to develop ZFDs for base editing in human and other eukaryotic cells, the lengths of the amino acid linkers and spacers of zinc finger proteins (ZFPs) attached to split-DddAtox halves were optimized. C-to-T base conversion was induced in the spacer between the left and right ZFP-binding sites. A well-characterized ZFN pair targeting the human CCR5 gene was selected. Using the same, ZFDs with various linkers of 2, 5, 10, 16, 24, and 32 amino acids were made and a series of target plasmids with various spacers ranging in length from 1 to 24 base pairs with left and right ZFP-binding sites of ZFDs and repetitive TC sequences were constructed (FIGs. 1a and 1b and Table 1).

**[Table 1] pTarget library sequences**

| **Spacer Length (bp)** | **Left-ZFD DNA binding region** | **Spacer sequence** | **Right-ZFD DNA binding region** |
|---|---|---|---|
| **1** | GTCATCCTCATC (SEQ ID NO.: 119) | C | AAACTGCAAAAG(SEQ ID NO.: 120) |
| **2** | GTCATCCTCATC | TC | AAACTGCAAAAG |
| **3** | GTCATCCTCATC | TCG (SEQ ID NO.: 121) | AAACTGCAAAAG |
| **4** | GTCATCCTCATC | CTGA (SEQ ID NO.: 122) | AAACTGCAAAAG |
| **5** | GTCATCCTCATC | CTGAT (SEQ ID NO.: 123) | AAACTGCAAAAG |
| **6** | GTCATCCTCATC | CTGATC (SEQ ID NO.: 124) | AAACTGCAAAAG |
| **7** | GTCATCCTCATC | CTGATCG (SEQ ID NO.: 125) | AAACTGCAAAAG |
| **8** | GTCATCCTCATC | CTGATCGA (SEQ ID NO.: 126) | AAACTGCAAAAG |
| **9** | GTCATCCTCATC | CTGATCGAT (SEQ ID NO.: 127) | AAACTGCAAAAG |
| **10** | GTCATCCTCATC | CTGATCGATC (SEQ ID NO.: 128) | AAACTGCAAAAG |
| **11** | GTCATCCTCATC | CTGATCGATCG (SEQ ID NO.: 129) | AAACTGCAAAAG |
| **12** | GTCATCCTCATC | CTGATCGATCGA (SEQ ID NO.: 130) | AAACTGCAAAAG |
| **13** | GTCATCCTCATC | CTGATCGATCGAT (SEQ ID NO.: 131) | AAACTGCAAAAG |
| **14** | GTCATCCTCATC | CTGATCGATCGATC (SEQ ID NO.: 132) | AAACTGCAAAAG |
| **15** | GTCATCCTCATC | CTGATCGATCGATCG (SEQ ID NO.: 133) | AAACTGCAAAAG |
| **16** | GTCATCCTCATC | CTGATCGATCGATCGA (SEQ ID NO.: 134) | AAACTGCAAAAG |
| **17** | GTCATCCTCATC | CTGATCGATCGATCGAT (SEQ ID NO. 135) | AAACTGCAAAAG |
| **18** | GTCATCCTCATC | CTGATCGATCGATCGATC (SEQ ID NO.: 136) | AAACTGCAAAAG |
| **19** | GTCATCCTCATC | CTGATCGATCGATCGATCG (SEQ ID NO.: 137) | AAACTGCAAAAG |
| **20** | GTCATCCTCATC | CTGATCGATCGATCGATCGA (SEQ ID NO.: 138) | AAACTGCAAAAG |
| **21** | GTCATCCTCATC | CTGATCGATCGATCGATCGAT (SEQ ID NO.: 139) | AAACTGCAAAAG |
| **22** | GTCATCCTCATC | CTGATCGATCGATCGATCGATC (SEQ ID NO.: 140) | AAACTGCAAAAG |
| **23** | GTCATCCTCATC | CTGATCGATCGATCGATCGATCG (SEQ ID NO.: 141) | AAACTGCAAAAG |
| **24** | GTCATCCTCATC | CTGATCGATCGATCGATCGATCGA (SEQ ID NO.: 142) | AAACTGCAAAAG |

DddAtox may be split at two positions (G1333 and G1397) and each half may be fused to either the left or right ZFP. The base editing efficiencies of the resulting 24 (= 6 linkers × 2 split positions × ZFP fusion positions (left or right)) ZFD constructs were measured for each of the 24 target plasmids with spacers. Measurement was performed with deep sequencing on day 4 of transfection in Hek293T cells.

### [Constructs]

* Left-ZFD: SV40 NLS-ZFP(S162-left)-linker-DddAtox half-4aa linker-UGI
* Right-ZFD: SV40 NLS-ZFP(S162-right)-linker-DddAtox half-4aa linker-UGI

- SV40 NLS:
   PKKKRKV (SEQ ID No: 478)
- ZFP (S162-left) 
GIHGVPAAMAERPFQCRICMRNFSDRSNLSRHIRTHTGEKPFACDICG
RKFAISSNLNSHTKIHTGSQKPFQCRICMRNFSRSDNLARHIRTHTGEKPFACDIC
GRKFATSGNLTRHTKIHLR (SEQ ID NO: 2)
- ZFP (S162-right) 
GIHGVPAAMAERPFQCRICMRNFSRSDNLSVHIRTHTGEKPFACDICG
RKFAQKINLQVHTKIHTGEKPFQCRICMRNFSRSDVLSEHIRTHTGEKPFACDICG
RKFAQRNHRTTHTKIHLR (SEQ ID NO: 3)
- Linker between zinc finger protein and DddAtox half:
   - 2aa: GS
   - 5aa: TGEKP (SEQ ID No: 479)
   - 10aa: SGAQGSTLDF (SEQ ID No: 9)
   - 16aa: SGSETPGTSESATPES (SEQ ID No: 10)
   - 24aa: SGTPHEVGVYTLSGTPHEVGVYTL (SEQ ID No: 115)
   - 32aa: GSGGSSGGSSGSETPGTSESATPESSGGSSGGS (SEQ ID No: 11)
   - Split-DddAtox G1333-N
      GSYALGPYQISAPQLPAYNGQTVGTFYYVNDAGGLESKVFSSGG (SEQ ID No: 27)
   - Split-DddAtox G1333-C 
PTPYPNYANAGHVEGQSALFMRDNGISEGLVFHNNPEGTCGFCVNMTE
TLLPENAKMTVVPPEGAIPV KRGATGETKVFTGNSNSPKSPTKGGC (SEQ ID
No: 272)
   - Split-DddAtox G1397-N 
GSYALGPYQISAPQLPAYNGQTVGTFYYVNDAGGLESKVFSSGGPTPY
PNYANAGHVEGQSALFMRDN
GISEGLVFHNNPEGTCGFCVNMTETLLPENAKMTVVPPEG (SEQ ID No:
273)
   - Split-DddAtox G1397-C
      AIPVKRGATGETKVFTGNSNSPKSPTKGGC (SEQ ID No: 26)
   - 4aa linker
      SGGS (SEQ ID NO: 480)
   - UGI

TNLSDIIEKETGKQLVIQESILMLPEEVEEVIGNKPESDILVHTAYDE
STDENVMLLTSDAPEYKPWA LVIQDSNGENKIKML (SEQ ID NO: 481)

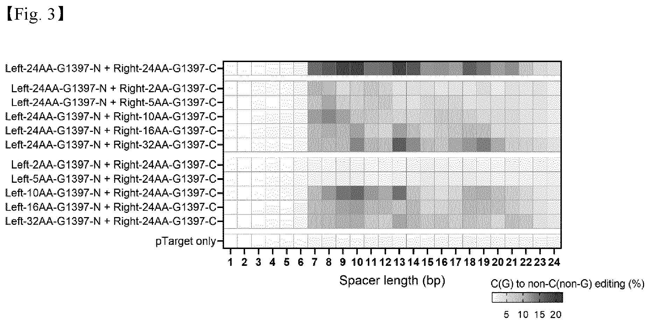

ZFDs with short linkers (2 and 5 amino acid (AA) linkers) have low or no efficiency. In contrast, in ZFDs with linkers of 10AA or more, the C-to-T base editing efficiency in the spacer of 4 base pairs or more ranged from 1% to 24% (FIGs. 1c, 2a, and 2c). Among the ZFD pairs, the ZFD pair with the 24AA linker showed the highest editing efficiency. In order to determine the best linker combination, ZFD combinations in which the left ZFD of a ZFD pair was fixed with the 24AA linker and linkers of various lengths were used for the right ZFD were constructed, or *vice versa,* and the editing efficiencies thereof were measured (FIGs. 1d and 3). It was found that the use of the same 24AA linker at both sites was most efficient. It was also found that DddAtox split at G1397 was more efficient than DddAtox split at G1333 (FIGs. 1c, 2a, and 2b). Thus, cytosines were edited by these most efficient ZFD pairs with the highest efficiency of >6.7% in spacer regions of 7-21 bps in length (FIGs. 1c, 2a, and 2c).

### 1-3. Base editing in nuclear DNA target in vivo

Whether ZFDs with 24AA linkers in human cells could catalyze C-to-T base editing at chromosomal target positions *in vivo* was investigated. 22 pairs of ZFDs targeting 11 sites (a pair of two ZFDs per site) in a total of eight genes were constructed (FIG. 4). There among, 14 pairs of ZFDs were assembled using a publicly available zinc finger resource. The other 8 pairs of ZFDs were created by adapting previously-characterized ZFNs (specific to CCR5 and TRAC) . The reason is that a ZFN cleaves the target DNA in a spacer of 5-7 bps in length but a ZFD works in a spacer of at least 7 bps, so that ZFDs capable of functioning by attaching or detaching one or two zinc fingers to or from the ZFN pairs were created. Since ZFNs with four different configurations may be constructed by fusing the FokI nuclease to the N-terminus or C-terminus of ZFP, two pairs of ZFDs with different configurations (Trac-NC in FIG. 4b) were constructed and whether the split-DddAtox halves could be fused to the N-terminus of ZFP as well as the C-terminus of existing ZFP was tested (NC configurations shown in FIGs. 4a and 5).

### [Constructs]

* C type: SV40 NLS-Zinc finger protein-24aa linker-DddAₜₒₓ half-4aa linker-UGI
* N type: SV40 NLS-DddAₜₒₓ half-24aa linker-Zinc finger protein-4aa linker-UGI

- SV40 NLS
   PKKKRKV (SEQ ID No: 478)
- 24aa linker
   SGTPHEVGVYTLSGTPHEVGVYTL
- Split-DddAₜₒₓ G1397-N 
GSYALGPYQISAPQLPAYNGQTVGTFYYVNDAGGLESKVFSSGGPTPYPNY
ANAGHVEGQSALFMRDN GISEGLVFHNNPEGTCGFCVNMTETLLPENAKMTVVPPEG
- Split-DddAₜₒₓ G1397-C
   AIPVKRGATGETKVFTGNSNSPKSPTKGGC
- 4aa linker
   SGGS
- UGI 
TNLSDIIEKETGKQLVIQESILMLPEEVEEVIGNKPESDILVHTAYDESTD

ENVMLLTSDAPEYKPWA LVIQDSNGENKIKML
- ZFP
   CCR5-1 Left (C type) [S162 ZFN-Left]

GIHGVPAAMAERPFQCRICMRNFSDRSNLSRHIRTHTGEKPFACDICGRKF
AISSNLNSHTKIHTGSQKPFQCRICMRNFSRSDNLARHIRTHTGEKPFACDICGRKFAT
SGNLTRHTKIHLR

CCR5-1 Right (C type) [S162 ZFN-Right] 
GIHGVPAAMAERPFQCRICMRNFSRSDNLSVHIRTHTGEKPFACDICGRKF
AQKINLQVHTKIHTGEKPFQCRICMRNFSRSDVLSEHIRTHTGEKPFACDICGRKFAQR
NHRTTHTKIHLR
CCR5-2 Left (C type) [S162 ZFN-Left] 
GIHGVPAAMAERPFQCRICMRNFSDRSNLSRHIRTHTGEKPFACDICGRKF
AISSNLNSHTKIHTGSQKPFQCRICMRNFSRSDNLARHIRTHTGEKPFACDICGRKFAT
SGNLTRHTKIHLR (SEQ ID NO: 2)
CCR5-2 Right (C type) [Modifying S162 ZFN-Right with additional ZF using the Barbas set of zinc finger modules] 
GIHGVPAAMAERPFQCRICMRNFSQSGDLRRHIRTHTGEKPFACDICGRKF
ARSDNLSVHTKIHTGSQKPFQCRICMRNFSQKINLQVHIRTHTGEKPFACDICGRKFAR
SDVLSEHTKIHLR (SEQ ID NO: 482)
TRAC-Left (C type) [Adapted from Paschon, D.E. et al., 2019] 
GIHGVPAAMAERPFQCRICMRNFSDQSNLRAHIRTHTGEKPFACDICGRKF
ATSSNRKTHTKIHTGSQKPFQCRICMRNFSLQQTLADHIRTHTGEKPFACDICGRKFAQ
SGNLARHTKIHLR (SEQ ID NO: 483)
TRAC-Left (N type) [Adapted from Paschon, D.E. et al., 2019] 
FQCRICMRKFATSGSLTRHTKIHTGEKPFQCRICMRNFSRSDHLSTHIRTH
TGEKPFACDICGRKFATSSNRTKHTKIHTHPRAPIPKPFQCRICMRNFSRSDNLSEHIR
THTGEKPFACDICGRKFAWHSSLRVHTKIHLR (SEQ ID NO: 484)
TRAC-Right (C type) [From Paschon, D.E. et al., 2019] 
GIHGVPAAMAERPFQCRICMRNFSRSDHLSTHIRTHTGEKPFACDICGRKF
ADRSHLARHTKIHTGSQKPFQCRICMRKFALKQHLNEHTKIHTGEKPFQCRICMRNFSQ
SGNLARHIRTHTGEKPFACDICGRKFAHNSSLKDHTKIHLR (SEQ ID NO: 485)
MFAP1 Left (C type) [De novo designed using the Toolgen set of zinc finger modules] 
GIRIPGEKPYSCGICGKSFSDSSAKRRHCILHTGEKPYTCSDCGKAFRDKS
CLNRHRRTHTGEKPYKCMECGKAFNRRSHLTRHQRIHTGEKPYECNYCGKTFSVSSTLI
RHQRIHLR (SEQ ID NO: 486)
MFAP1 Right (C type) [Modifying S162 ZFN-Right with additional ZF using the Barbas set of zinc finger modules] 
GIRERPYACPVESCDRRFSTSGSLVRHIRIHTGQKPFQCRICMRNFSRSDE
LTRHIRTHTGEKPFACDICGRKFARSDHLTTHTKIHTGEKPFQCRICMRKFAQSSNLVR
HTKIHLR (SEQ ID NO: 487)
CCDC28B Left (C type) [Modifying S162 ZFN-Right with additional ZF using the Barbas set of zinc finger modules] 
GIRERPYACPVESCDRRFSDPGHLVRHIRIHTGQKPFQCRICMRNFSRSDE
LTRHIRTHTGEKPFACDICGRKFARSDHLTTHTKIHTGEKPFQCRICMRKFARSDKLVR
HTKIHLR (SEQ ID NO: 488)
CCDC28B Right (C type) [De novo designed using the Toolgen set of zinc finger modules] 
GIRIPGEKPYECNYCGKTFSVSSTLIRHQRIHTGEKPYHCDWDGCGWKFAR
SDELTRHYRKHTGEKPFECKDCGKAFIQKSNLIRHQRTHTGEKPYHCDWDGCGWKFARS
DELTRHYRKHLR (SEQ ID NO: 489)
KDM4B Left (C type) [Modifying S162 ZFN-Right with additional ZF using the Barbas set of zinc finger modules] 
GIRERPYACPVESCDRRFSDCRDLARHIRIHTGQKPFQCRICMRNFSRSDE
LTRHIRTHTGEKPFACDICGRKFARSDHLTTHTKIHTGEKPFQCRICMRKFARSDKLVR
HTKIHLR (SEQ ID NO: 490)
KDM4B Right (C type) [De novo designed using the Toolgen set of zinc finger modules] 
GIRIPGEKPFECKDCGKAFIQKSNLIRHQRTHTGEKPYRCEECGKAFRWPS
NLTRHKRIHTGEKPYRCEECGKAFRWPSNLTRHKRIHTGEKPYSCGICGKSFSDSSAKR
RHCILHLR (SEQ ID NO: 491)
NUMBL Left (C type) [Modifying S162 ZFN-Right with additional ZF using the Barbas set of zinc finger modules] 
GIRERPYACPVESCDRRFSDCRDLARHIRIHTGQKPFQCRICMRNFSRSDE
LTRHIRTHTGEKPFACDICGRKFARSDHLTTHTKIHTGEKPFQCRICMRKFARSDKLVR
HTKIHLR (SEQ ID NO: 492)
NUMBL Right (C type) [De novo designed using the Toolgen set of zinc finger modules] 
GIRIPGEKPYTCSDCGKAFRDKSCLNRHRRTHTGEKPYKCGQCGKFYSQVS
HLTRHQKIHTGEKPFECKDCGKAFIQKSNLIRHQRTHTGEKPYHCDWDGCGWKFARSDE
LTRHYRKHLR (SEQ ID NO: 493)
INPP5D-1 Left (C type) [Modifying S162 ZFN-Right with additional ZF using the Barbas set of zinc finger modules] 
GIRERPYACPVESCDRRFSRSDKLVRHIRIHTGQKPFQCRICMRNFSRSDE
LTRHIRTHTGEKPFACDICGRKFARSDHLTTHTKIHTGEKPFQCRICMRKFARSDKLVR
HTKIHLR (SEQ ID NO: 494)
INPP5D-1 Right (C type) [De novo designed using the Toolgen set of zinc finger modules] 
GIRIPGEKPYTCSDCGKAFRDKSCLNRHRRTHTGEKPYECNYCGKTFSVSS
TLIRHQRIHTGEKPYTCSDCGKAFRDKSCLNRHRRTHTGEKPYTCSDCGKAFRDKSCLN
RHRRTHLR (SEQ ID NO: 495)
INPP5D-2 Left (C type) [Modifying S162 ZFN-Right with additional ZF using the Barbas set of zinc finger modules] 
GIRERPYACPVESCDRRFSRSDKLVRHIRIHTGQKPFQCRICMRNFSRSDE
LTRHIRTHTGEKPFACDICGRKFARSDHLTTHTKIHTGEKPFQCRICMRKFARSDKLVR
HTKIHLR (SEQ ID NO: 496)
INPP5D-2 Right (C type) [De novo designed using the Toolgen set of zinc finger modules] 
GIRIPGEKPYECNYCGKTFSVSSTLIRHQRIHTGEKPYTCSDCGKAFRDKS
CLNRHRRTHTGEKPYTCSDCGKAFRDKSCLNRHRRTHTGEKPYHCDWDGCGWKFARSDE
LTRHYRKH (SEQ ID NO: 497)
DVL3 Left (C type) [De novo designed using Barbas zinc finger modules] 
GIHGVPAAMAERPFQCRICMRNFSTSGHLVRHIRTHTGEKPFACDICGRKF
ATSGHLVRHTKIHTGEKPFQCRICMRNFSTSGELVRHIRTHTGEKPFACDICGRKFAQS
SNLVRHTKIHLR (SEQ ID NO: 498)
DVL3 Right (C type) [S162 ZFN-left]

GIHGVPAAMAERPFQCRICMRNFSDRSNLSRHIRTHTGEKPFACDICGRKF
AISSNLNSHTKIHTGSQKPFQCRICMRNFSRSDNLARHIRTHTGEKPFACDICGRKFAT
SGNLTRHTKIHLR (SEQ ID NO: 499)

In Hek 293T cells, the C-to-T base editing efficiency of ZFDs, including ZFDs with NC configurations, was 1.0% to 60%. On the other hand, insertion-deletion (indel) was <0.4% and thus rarely occurred (FIGs. 4b and 6). As seen in the plasmid-based experiment described above, the ZFD targeting CCR5 with a 5-bp long spacer was very inefficient. In targets with a spacer of at least 7 bps, the other 20 ZFD pairs showed an average editing efficiency of 12.013.4%, which is comparable to an average of 8.3±2.2% of cas9-derived base editor2. Additionally, C-to-T base editing occurred not only in the TC context but also in AC or GCC (FIGs. 4c-4f). Respective C base editing efficiencies of AC at C₆ of *NUMBL* and GCC at C₇ of *INPP5D-2* were 4.58% and 1.85%.

| | | Left-ZFD binding region | Spacer region | Right-ZFD binding region | | ZFD configuration |
|---|---|---|---|---|---|---|
| MFAP1 | 5'- | GACGGCCCCAGC | CGTAGACT | GAATGGGCGGTT | -3' | C-C |
| | 3'- | CTGCCGGGCTCG | GCATCTGA | CTTACCCGCCAA | -5' | |
| CCDC28B | 5'- | TGCCGCCCAGTC | CTGGCTGGAG | GTGGAAGCGGCT | -3' | C-C |
| | 3'- | ACGGCGGGTCAG | GACCGACCTC | CACCTTCGCCGA | -5' | |
| KDM4B | 5'- | GGCCGCCCACCC | GGTCCCCACT | GTCGGTGGTGAA | -3' | C-C |

| | | | | | | |
|---|---|---|---|---|---|---|
| | 3'- | CCGGCGGGTGGG | CCAGGGGTGA | CAGCCACCACTT | -5' | |
| NUMBL | 5'- | GGCCGCCCACCC | CCAGACAG | GCGGAAGGAGCC | -3' | C-C |
| | 3'- | CCGGCGGGTGGG | GGTCTGTC | CGCCTTCCTCGG | -5' | |
| INPP5D-1 | 5'- | CCCCGCCCACCC | CGACGCCGCG | GCCGCCGCTGCC | -3' | C-C |
| | 3'- | GGGGCGGGTGGG | GCTGCGGCGC | CGGCGGCGACGG | -5' | |
| INPP5D-2 | 5'- | CCCCGCCCACCC | CGACGCC | GCGGCCGCCGCT | -3' | C-C |
| | 3'- | GGGGCGGGTGGG | GCTGCGG | CGCCGGCGGCGA | -5' | |
| DVL3 | 5'- | ACCACCAGCTTC | TTTGACTCA | GATGAGGATGAC | -3' | C-C |
| | 3'- | TGGTGGTCGAAG | AAACTGAGT | CTACTCCTACTG | -5' | |
| CCR5-1 | 5'- | GTCATCCTCATC | CTGAT | AAACTGCAAAAG | -3' | C-C |
| | 3'- | CAGTAGGAGTAG | GACTA | TTTGACGTTTTC | -5' | |
| CCR5-2 | 5'- | GTCATCCTCATC | CTGATAAA | CTGCAAAAGGCT | -3' | C-C |
| | 3'- | CAGTAGGAGTAG | GACTATTT | GACGTTTTCCGA | -5' | |
| TRAC-CC | 5'- | GTGATTGGGTTC | CGAATCCTCCT | CCTGAAAGTGGCCGG | -3' | C-C |
| | 3'- | CACTAACCCAAG | GCTTAGGAGGA | GGACTTTCACCGGCC | -5' | |
| TRAC-NC | 5'- | TGTCAGTGATTGGGTT | CCGAATCCTCCT | CCTGAAAGTGGCCGG | -3' | N-C |
| | 3'- | ACAGTCACTAACCCAA | GGCTTAGGAGGA | GGACTTTCACCGGCC | -5' | |

### 1-4. Direct delivery of purified ZFD protein to human cells

Delivering the purified gene editing protein into cells, rather than delivering plasmid DNA encoding the gene editing protein, is capable of reducing off-target effects, avoiding the innate immune response by foreign DNA, and preventing the insertion of foreign plasmid DNA into the genome *in vivo.* Other groups showed that ZFPs may pass spontaneously through mammalian cells both *in vitro* and *in vivo.* In order to demonstrate protein-mediated base editing of ZFDs, ZFD pairs targeting the TRAC gene with high efficiency were selected, and recombinant ZFD proteins with one or four nuclear localization signals (NLSs) were purified from *E*. *coli.* First, the base editing efficiency of the ZFD protein was tested *in vitro* using a PCR amplicon having a TRAC site, confirming very high efficiency. Efficiency was confirmed based on gene cleavage using a uracil-specific excision reagent (USER), which is a mixture of uracil DNA glycosylase and DNA glycosylase-lyase Endonuclease VIII (FIG. 7). TRAC-NC ZFD protein was delivered to human leukemia K562 cells, which are difficult to transfect, in two ways, electroporation or direct delivery without electroporation. ZFD proteins were very efficient. The C-to-T base editing efficiencies were 26.5% (electroporation) and 17% (direct delivery) (FIG. 4g). Therefore, these results show that plasmids encoding the ZFDs or purified recombinant ZFD proteins are usable for base editing of nuclear DNA in human cells.

### 1-5. Mitochondrial DNA base editing with ZFDs

Unlike CRISPR-based systems, the split-DddAtox system fused to custom-designed DNA-binding proteins may be used to edit organelle DNA, including mitochondrial DNA, a major advantage of the DddA system over the CRISPR systems. In order to deliver ZFDs to mitochondria, mitoZFDs were constructed by linking the mitochondrial targeting signal (MTS) and nuclear export signal (NES) to the N-terminal portions of nine ZFDs designed to target mitochondrial genes (FIG. 8). The ZFP segments for ZFDs were assembled using a publicly available zinc finger resource. The ZFDs were configured such that a spacer was 7-15 bps in length and both the left- and right-DNA-binding sites were 12-bp long.

### [Constructs]

* C type: MTS-FLAG tag-NES-Zinc finger protein-24aa linker-DddAtox half-4aa linker-UGI
* N type: MTS-HA tag-NES-DddAtox half-24aa linker-Zinc finger protein-4aa linker-UGI
- MTS (Mitochondrial Targeting Sequence of human mitochondrial ATP synthase F1β subunit) 
MLGFVGRVAAAPASGALRRLTPSASLPPAQLLLRAAPTAVHPVRDYAA
Q (SEQ ID No:274)
- FLAG tag (C type)
   DYKDDDDK (SEQ ID No:275)
- HA tag (N type)
   YPYDVPDYA (SEQ ID No:276)
- NES (Nuclear export signal)
   VDEMTKKF (Minute virus if mice; MVM NES)
- Split-DddAtox G1397-N 
GSYALGPYQISAPQLPAYNGQTVGTFYYVNDAGGLESKVFSSGGPTPY
PNYANAGHVEGQSALFMRDN
GISEGLVFHNNPEGTCGFCVNMTETLLPENAKMTVVPPEG
- Split-DddAtox G1397-C
   AIPVKRGATGETKVFTGNSNSPKSPTKGGC
- 4aa linker
   SGGS
- UGI 
TNLSDIIEKETGKQLVIQESILMLPEEVEEVIGNKPESDILVHTAYDE

STDENVMLLTSDAPEYKPWA LVIQDSNGENKIKML
- ZFP

* Zinc fingers are linked by TGEKP linkers. (ZF1-linker-ZF2-linker-ZF3-linker-ZF4).
* ND2-targeted mitoZFD has a QQ variant. This variant is a substitution of Q for an R or K amino acid. It is marked with the red letter.

| | Type | ZF1 | No. | ZF2 | No. | ZF3 | No. | ZF4 | No. |
|---|---|---|---|---|---|---|---|---|---|
| ND1_Left | C | FQCRICM RNFSDSG NLRVHIRT H | 500 | YKCPDCG KSFSQSS SLIRHQRT H | 518 | YECDHCG KSFSQSS HLNVHKR TH | 536 | YRCKYCD RSFSISSN LQRHVRNI H | 554 |
| ND1_Right | N | YKCPECG KSFSTKN SLTEHQRT H | 501 | YKCPECG KSFSSKK ALTEHQRT H | 519 | YECNYCG KTFSVSST LIRHQRIH | 537 | YRCKYCD RSFSISSN LQRHVRNI H | 555 |
| ND2_Left | C | YKCPECG KSFSRED NLHTHQRT H | 502 | YHCDWD GCGWKF ARSDELT RHYRKH | 520 | YKCPECG KSFSRED NLHTHQRT H | 538 | YRCKYCD RSFSISSN LQRHVRNI H | 556 |
| ND2_Right | N | YECDHCG KSFSQSS HLNVHKR TH | 503 | YHCDWD GCGWKF ARSDELT RHYRKH | 521 | YKCPDCG KSFSQSS SLIRHQRT H | 539 | YKCGQCG KFYSQVS HLTRHQKI H | 557 |
| Cox1_Left | C | YECHDCG KSFRQST HLTQHRRI H | 504 | YSCGICG KSFSDSS AKRRHCIL H | 522 | YECHDCG KSFRQST HLTQHRRI H | 540 | YKCPDCG KSFSQSS SLIRHQRT H | 558 |
| Cox1_Right | N | YKCPDCG KSFSQSS SLIRHQRT H | 505 | YKCGQCG KFYSQVS HLTRHQKI H | 523 | YKCPDCG KSFSQSS SLIRHQRT H | 541 | YECDHCG KSFSQSS HLNVHKR TH | 559 |
| Cox2_Left | C | YKCPECG KSFSDPG HLVRHQR TH | 506 | YRCKYCD RSFSISSN LQRHVRNI H | 524 | YRCKYCD RSFSISSN LQRHVRNI H | 542 | YKCPECG KSFSRED NLHTHQRT H | 560 |
| Cox2_Right | N | YGCHLCG KAFSKSS NLRRHEMI H | 507 | FQCKTCQ RKFSRSD HLKTHTRT H | 525 | YKCGQCG KFYSQVS HLTRHQKI H | 543 | YKCPECG KSFSRED NLHTHQRT H | 561 |
| ND4L_Left | C | YKCPECG KSFSDPG HLVRHQR TH | 508 | YKCPECG KSFSRED NLHTHQRT H | 526 | YKCPECG KSFSRED NLHTHQRT H | 544 | YRCKYCD RSFSDSS NLQRHVR NIH | 562 |
| ND4L_Righ t | C | YKCPECG KSFSDPG HLVRHQR TH | 509 | FHCGYCE KSFSVKD YLTKHIRT H | 527 | YKCPECG KSFSSPA DLTRHQRT H | 545 | FQCRICM RNFSDSG NLRVHIRT H | 563 |
| ND4_Left | C | FHCGYCE KSFSVKD YLTKHIRT H | 510 | YECVQCG KGFTQSS NLITHQRV H | 528 | YTCSYCG KSFTQSN TLKQHTRI H | 546 | YRCKYCD RSFSISSN LQRHVRNI H | 564 |
| ND4_Right | C | FHCGYCE KSFSVKD YLTKHIRT H | 511 | YKCPECG KSFSTHLD LIRHQRTH | 529 | YKCPECG KSFSSKK ALTEHQRT H | 547 | FQCRICM RNFSDSG NLRVHIRT H | 565 |
| ND5_Left | C | FHCGYCE KSFSVKD YLTKHIRT H | 512 | YECDHCG KSFSQSS HLNVHKR TH | 530 | FQCRICM RNFSDSG NLRVHIRT H | 548 | YECDHCG KSFSQSS HLNVHKR TH | 566 |
| ND5_Right | N | FACPECP KRFMRSD NLTQHIKT H | 513 | YECNYCG KTFSVSST LIRHQRIH | 531 | YECDHCG KAFSVSS NLNVHRRI H | 549 | YECDHCG KAFSVSS NLNVHRRI H | 567 |
| ND6_Left | C | YECHDCG KSFRQST HLTQHRRI H | 514 | YECHDCG KSFRQST HLTQHRRI H | 532 | YKCGQCG KFYSQVS HLTRHQKI H | 550 | YHCDWD GCGWKF ARSDELT RHYRKH | 568 |
| ND6_Right | N | YRCEECG KAFRWPS NLTRHKRI H | 515 | YKCPECG KSFSRED NLHTHQRT H | 533 | YGCHLCG KAFSKSS NLRRHEMI H | 551 | YKCPECG KSFSRED NLHTHQRT H | 569 |
| CYB_Left | C | FECKDCG KAFIQKSN LIRHQRTH | 516 | FHCGYCE KSFSVKD YLTKHIRT H | 534 | YTCSDCG KAFRDKS CLNRHRR TH | 552 | YTCSYCG KSFTQSN TLKQHTRI H | 570 |
| CYB_Right | C | YTCSYCG KSFTQSN TLKQHTRI H | 517 | YKCDECG KNFTQSS NLIVHKRIH | 535 | YKCPECG KSFSDPG HLVRHQR TH | 553 | YECDHCG KSFSQSS HLNVHKR TH | 571 |

| | Left-ZFD binding region | | Spacer region | Right-ZFD binding region | | ZFD configuration |
|---|---|---|---|---|---|---|
| ND1 | 5'- | GTTTACTCAATC | CTCTGATC | AGGGTGAGCATC | -3' | C-N |
| | 3'- | CAAATGAGTTAG | GAGACTAG | TCCCACTCGTAG | -5' | |
| ND2 | 5'- | CTACGCCTAATC | TACTCCACCTCAA | TCACACTACTCC | -3' | C-N |
| | 3'- | GATGCGGATTAG | ATGAGGTGGAGTT | AGTGTGATGAGG | -5' | |
| Cox1 | 5'- | TCTGACTCTTAC | CTCCCTCTCTCC | TACTCCTGCTCG | -3' | C-N |
| | 3'- | AGACTGAGAATG | GAGGGAGAGAGG | ATGAGGACGAGC | -5' | |
| Cox2 | 5'- | GCCATCATCCTA | GTCCTCATCGCC | CTCCCATCCCTA | -3' | C-N |
| | 3'- | CGGTAGTAGGAT | CAGGAGTAGCGG | GAGGGTAGGGAT | -5' | |
| ND4L | 5'- | GCCCTACTAGTC | TCAATCTCC | AACACATATGGC | -3' | C-C |
| | 3'- | CGGGATGATCAG | AGTTAGAGG | TTGTGTATACCG | -5' | |
| ND4 | 5'- | ATATTTTATATC | TTCTTCGA | AACCACACTTAT | -3' | C-C |
| | 3'- | TATAAAATATAG | AAGAAGCT | TTGGTGTGAATA | -5' | |
| ND5 | 5'- | ATATCGGTTTCA | TCCTCGC | CTTAGCATGATT | -3' | C-N |
| | 3'- | TATAGCCAAAGT | AGGAGCG | GAATCGTACTAA | -5' | |
| ND6 | 5'- | TCTTCTTCCCAC | TCATCCTA | ACCCTACTCCTA | -3' | C-N |
| | 3'- | AGAAGAAGGGTG | AGTAGGAT | TGGGATGAGGAT | -5' | |
| CYB | 5'- | TTCATAGGCTAT | GTCCTCCCG | TGAGGCCAAATA | -3' | C-C |
| | 3'- | AAGTATCCGATA | CAGGAGGGC | ACTCCGGTTTAT | -5' | |

Mitochondrial DNA base editing efficiency by mitoZFD in HEK293T cells ranged from 2.6% to 30% (average 1413%) (FIG. 9a). Also, mitoZFD with NC configuration (1614.5%, n=6) was as efficient as that with CC configuration (8.312.7%, n=3). Most cytosines with TC context in the spacer region were base-edited with various efficiencies (FIGs. 9b-9g). Furthermore, cytosines with the ACC (C₈ and C₉) context in the *ND2* site also showed efficiencies of about 7.4% and 19.9%, respectively (FIG. 9b). This shows that ZFD-mediated C-to-T base editing is not limited to the TC motif.

In addition, single-cell-derived clonal populations were isolated from mitochondrial DNA (mtDNA) mutant cells to prove that mitoZFDs are not cytotoxic and that mtDNA mutations are also maintained in clonal populations. Among 30 single-cell-derived clonal populations isolated from HEK293T cells treated with *ND1*-specific mitoZFD, five clonal populations showed *ND1* gene base editing efficiencies of 35% to 98% (FIG. 10a). Likewise, among 36 single-cell-derived clonal populations isolated from HEK293T cells treated with ND2-specific mitoZFD, seven clonal populations showed *ND2* gene base editing efficiencies of 26% to 76% (FIG. 10b). For all of the other clonal populations, low efficiencies of 0.4%-1.0% were observed, most likely resulting from sequencing errors. Similar efficiency was obtained in cells not treated with ZFD (FIG. 10c). These results show that mitoZFDs induced heteroplasmic mutations unevenly in cell populations. Most ZFD-treated cells are of wild-type, whereas cells with heteroplasmic mutations have mutation rates of up to 98%. These mutations are maintained even after clonal expansion (FIGs. 11 and 12).

### 1-6. mitoZFDs and TALE-based DdCBEs

It was found that the mutation patterns of the constructed *ND1*-specific mitoZFDs were different from those of TALE-based DdCBEs targeting the same gene (FIGs. 9f-9h). The two mitoZFDs catalyzed C-to-T base editing at C₅ or C₈ (FIG. 9f), whereas the DdCBEs induced base editing at C₈, C₉, and C₁₁ positions (FIG. 9g) . Consequently, amino-acid changes caused by mitoZFDs were completely different from those caused by DdCBEs (FIG. 9h). The left and right sites, to which mitoZFDs were attached, were separated by an 8-bp spacer, but in the case of DdCBEs, such sites were separated by a 16-bp spacer, which is likely responsible for differential mutation patterns. These results show that mizoZFDs and DdCBEs may create various mutations in a complementary manner in mitochondrial DNA.

In order to create more mutation patterns, whether a ZFD monomer and a DdCBE monomer were mixed to form a hybrid pair was tested. 10 hybrid pairs targeting the *ND1* gene showed good activity with an average base editing efficiency of 17±3.4% in HEK293T cells (FIG. 13). In fact, one of the hybrid pairs (TALE-L/ZFD-R1) showed better efficiency than two DdCBE pairs and ten ZFD pairs targeting the same site, with the highest base editing efficiency of 41% (FIG. 13b). Moreover, hybrid pairs yielded different mutation patterns from those obtained with DdCBEs and mitoZFDs (FIG. 13c). A few hybrid pairs (e.g. ZFD-L1/TALE-R and ZFD-L2/TALE-R) induced C-to-T conversions at single positions without bystander edits. In contrast, most mitoZFD pairs and DdCBE pairs induced C-to-T conversions at multiple positions in spacer regions. These results show that the ZFD/DdCBE hybrid pairs may create unique mutation patterns and produce certain mutations that cannot be obtained using ZFD pairs or DdCBE pairs.

### 1-7. Mitochondrial genome-wide target specificity of mitoZFDs

In order to confirm whether mitoZFDs cause off-target editing, mitochondrial DNA was extracted from cells treated with each pair of mitoZFDs targeting *ND1* or *ND2* genes, followed by whole mitochondrial genome sequencing. Various amounts (5-500 ng) of mRNA or plasmids encoding the mitoZFD pairs were transfected into HEK293T cells. As expected, on-target editing efficiency was dose-dependent. High concentrations (100, 200, and 500 ng) of mRNA or plasmids yielded on-target efficiency of >30%, but also caused hundreds of off-target edits of >1% (FIGs. 14-17). Low concentrations (5 and 10 ng) largely avoided these off-target edits but significantly reduced on-target efficiencies. A medium concentration (50 ng) of mRNA was most appropriate. High on-target efficiency was maintained without causing hundreds of off-target edits. In order to further eliminate remaining off-target edits, R(-5)Q mutations were introduced into each zinc finger to eliminate nonspecific DNA contacts. The resulting ZFD variants (shown as QQ in FIG. 18) retained high on-target activity and showed exquisite specificity with almost no off-target edits, compared to mtDNA of cells not treated with ZFDs (FIGS. 18a and 18b).

Base editing is a relatively new method capable of editing targeted bases without causing DNA double-strand breaks or a DNA repair template. Base editing enables C-to-T or A-to-G conversions in cells, animals, and plants, allowing study of the functional effects of single nucleotide polymorphisms (SNPs) and correction of disease-causing point mutations for therapeutic applications. Two types of base editing technology have been developed, namely CRISPR-based adenine and cytosine base editors and DddA-based base editing technology. The CRISPR-based base-editors are composed of catalytically-impaired Cas9 or Cas12a as DNA-binding units and single-strand DNA-specific deaminases originating from rats or *E. coli.* On the other hand, DdCBEs are composed of TALE DNA-binding arrays and double-strand DNA-specific DddAtox.

Compared to DdCBEs, ZFDs are smaller in size. This is because the zinc finger proteins in ZFDs are compact, whereas the TALE arrays in DdCBEs are bulky. Thus, a ZFD pair-encoding gene, rather than a DdCBE pair-encoding gene, may be readily packaged in an AAV vector with a small cargo space. Additionally, compact ZFPs are engineering friendly, making it possible to fuse split-DddAtox halves to either the N-terminus or the C-terminus of ZFPs, creating ZFDs that operate either upstream or downstream of the ZFP-binding site. Moreover, recombinant ZFD proteins may spontaneously penetrate human cells without electroporation or lipofection, enabling gene-free gene therapy. The ZFD pairs or ZFD/DdCBE hybrid pairs may create unique mutation patterns, which cannot be obtained using DdCBEs alone. These properties make ZFDs a powerful new platform for modeling and treating mitochondrial diseases.

### Example 2. Plant chloroplast and mitochondrial gene editing by TALE-DdCBE

Plant organelles, including mitochondria and chloroplasts, each have their own genomes that encode many genes essential for respiration and photosynthesis. Plant organellar gene editing, an unmet need for plant genetics and biotechnology, has been limited due to lack of appropriate tools to target the DNA in these organelles. A Golden Gate cloning system composed of 16 expression plasmids (8 for delivery of the resulting protein to the mitochondria, and the other 8 for delivery to the chloroplast) and 424 TALE subarray plasmids was developed in order to assemble a DddA-derived cytosine base editing plasmid (DdCBE), and the completed DdCBE plasmids were used to induce efficiently point mutations in mitochondria and chloroplasts. DdCBE base editing induced mutations with efficiencies of up to 25% (mitochondria) and 38% (chloroplasts) in lettuce or rapeseed calli. In order to avoid off-target mutations caused by the DdCBE-encoding plasmid, DdCBE messenger RNA was transfected into lettuce protoplasts, demonstrating DNA-free base editing in chloroplasts. In addition, streptomycin- or spectinomycin-resistant lettuce calli and shoots with editing efficiencies of up to 99% were created by introducing point mutations in the chloroplast 16S rRNA gene.

DdCBEs are heterodimers including an isolated non-toxic domain derived from the bacterial cytosine deaminase toxin DddAtox, a TALE array designed for a specific position, and a UGI, and induce cytosine-to-thymine substitution in the spacer between TALE protein binding sites in the target DNA. Our results with DdCBEs demonstrated highly efficient organelle base editing in plants.

### 2-1. Methods

### Construction of expression plasmids for plant protoplast experiments

DdCBE Golden Gate destination vectors were constructed using a Gibson assembly method. Sequences encoding the TAL N-terminal domain, HA tag, FLAG tag, TAL C-terminal domain, split DddAtox, and UGI were codon-optimized for expression in dicotyledonous plants (*Arabidopsis thaliana*) and synthesized by integrated DNA technology. The sequences encoding the CTP from AtinfA and AtRbcS and the MTS from ATPase delta subunit and ATPase gamma subunit were amplified from *Arabidopsis thaliana* cDNAs. For plant expression, the PcUbi promoter and pea3A terminator were used to replace the mammalian CMV promoter in a backbone plasmid. In order to construct the vector for *in-vitro* DdCBE mRNA transcription, a T7 promoter cassette was cloned into the DdCBE Golden Gate destination vector between the PcUbi promoter and the DdCBE-encoding region.

TALE array genes were constructed by one-way Golden Gate assembly. The DdCBE expression plasmids were constructed by BsaI digestion and T4 ligation of Golden Gate assembly using 424 TALE array plasmids and destination vectors. One-way Golden Gate cloning was performed using the following steps: 20 cycles of 37°C and 50°C for 5 minutes each, followed by final reaction at 50°C for 15 minutes and then at 80°C for 5 minutes. All vectors for plant protoplast transfection were purified using a Plasmid Plus midi-prep Kit (Qiagen). The DNA and amino acid sequences used in vector construction are as follows.

**[Table 2]**

| Name | Sequence | No. |
|---|---|---|
| Parsley ubiquiti n promoter | CTAGCAACGATTGTACAATTGCTTCTTTAAAAAAGGAAGAAAGAAAGAAAGAAAA GAATCAACATCAGCGTTAACAAACGGCCCCGTTACGGCCCAAACGGTCATATAGA GTAACGGCGTTAAGCGTTGAAAGACTCCTATCGAAATACGTAACCGCAAACGTGT CATAGTCAGATCCCCTCTTCCTTCACCGCCTCAAACACAAAAATAATCTTCTACA GCCTATATATACAACCCCCCCTTCTATCTCTCCTTTCTCACAATTCATCATCTTT CTTTCTCTACCCCCAATTTTAAGAAATCCTCTCTTCTCCTCTTCATTTTCAAGGT AAATCTCTCTCTCTCTCTCTCTCTCTGTTATTCCTTGTTTTAATTAGGTATGTAT TATTGCTAGTTTGTTAATCTGCTTATCTTATGTATGCCTTATGTGAATATCTTTA TCTTGTTCATCTCATCCGTTTAGAAGCTATAAATTTGTTGATTTGACTGTGTATC TACACGTGGTTATGTTTATATCTAATCAGATATGAATTTCTTCATATTGTTGCGT TTGTGTGTACCAATCCGAAATCGTTGATTTTTTTCATTTAATCGTGTAGCTAATT GTACGTATACATATGGATCTACGTATCAATTGTTCATCTGTTTGTGTTTGTATGT ATACAGATCTGAAAACATCACTTCTCTCATCTGATTGTGTTGTTACATACATAGA TATAGATCTGTTATATCATTTTTTTTATTAATTGTGTATATATATATGTGCATAG ATCTGGATTACATGATTGTGATTATTTACATGATTTTGTTATTTACGTATGTATA TATGTAGATCTGGACTTTTTGGAGTTGTTGACTTGATTGTATTTGTGTGTGTATA TGTGTGTTCTGATCTTGATATGTTATGTATGTGCAGC | 162 |
| Pea3A terminat or | CAGGCCTCCCAGCTTTCGTCCGTATCATCGGTTTCGACAACGTTCGTCAAGTTCA ATGCATCAGTTTCATTGCCCACACACCAGAATCCTACTAAGTTTGAGTATTATGG CATTGGAAAAGCTGTTTTCTTCTATCATTTGTTCTGCTTGTAATTTACTGTGTTC TTTCAGTTTTTGTTTTCGGACATCAAAATGCAAATGGATGGATAAGAGTTAATAA ATGATATGGTCCTTTTGTTCATTCTCAAATTATTATTATCTGTTGTTTTTACTTT AATGGGTTGAATTTAAGTAAGAAAGGAACTAACAGTGTGATATTAAGGTGCAATG TTAGACATATAAAACAGTCTTTCACCTCTCTTTGGTTATGTCTTGAATTGGTTTG TTTCTTCACTTATCTGTGTAATCAAGTTTACTATGAGTCTATGATCAAGTAATTA TGCAATCAAGTTAAGTACAGTATAGGCTT | 163 |
| T7 promoter | TAATACGACTCACTATAGG | 164 |

The specific amino acid sequences for DdCBEs and the specific amino acid sequences for TALE repeats are as follows.

**[Table 3]**

| Name | Sequence | No. |
|---|---|---|
| AtinfA CTP-3xHA-N terminal domain | MLQLCSTFRPQLLLPCQFRFTNGVLIPQINYVASNSVVNIRPMIRCQRASGGRGGA NRSKPAKPQVKEGSNKTVIEGLVTESLPNGMFRVDLENGDNILGYICGKIRKNFIR ILPGDKVKVEMSVYDSTKGRIIFRMSSRDYPYDVPDYAGYPYDVPDYAGYPYDVPD YAMDIADLRTLGYSQQQQEKIKPKVRSTVAQHHEALVGHGFTHAHIVALSQHPAAL GTVAVKYQDMIAALPEATHEAIVGVGKQWSGARALEALLTVAGELRGPPLQLDTGQ LLKIAKRGGVTAVEAVHAWRNALTGAPL | 165 |
| AtRbcS CTP-3xFLAG-N terminal domain | MASSMLSSATMVASPAQATMVAPFNGLKSSAAFPATRKANNDITSITSNGGRVNCM QVWPPIGKKKFETLSYLPDLTDSEDYKDHDGDYKDHDIDYKDDDDKMDIADLRTLG YSQQQQEKIKPKVRSTVAQHHEALVGHGFTHAHIVALSQHPAALGTVAVKYQDMIA ALPEATHEAIVGVGKRGAGARALEALLTVAGELRGPPLQLDTGQLLKIAKRGGVTA VEAVHAWRNALTGAPL | 166 |
| AtATPase gamma subunit MTS-3xHA-N terminal domain | MAMAVFRREGRRLLPSIAARPIAAIRSPLSSDQEEGLLGVRSISTQVVRNRMKSVK NIQKITKAMKMVAASKLRAVQYPYDVPDYAGYPYDVPDYAGYPYDVPDYAMDIADL RTLGYSQQQQEKIKPKVRSTVAQHHEALVGHGFTHAHIVALSQHPAALGTVAVKYQ DMIAALPEATHEAIVGVGKQWSGARALEALLTVAGELRGPPLQLDTGQLLKIAKRG GVTAVEAVHAWRNALTGAPL | 167 |
| AtATPase delta subunit MTS-3xFLAG-N terminal domain | MFKQASRLLSRSVAAASSKSVTTRAFSTELPSTLDSDYKDHDGDYKDHDIDYKDDD DKMDIADLRTLGYSQQQQEKIKPKVRSTVAQHHEALVGHGFTHAHIVALSQHPAAL GTVAVKYQDMIAALPEATHEAIVGVGKRGAGARALEALLTVAGELRGPPLQLDTGQ LLKIAKRGGVTAVEAVHAWRNALTGAPL | 168 |
| Half **(NG)** domain-C-terminal domain-G1333-N-UGI | GLTPEQVVAIASNGGGKQALESIVAQLSRPDPALAALTNDHLVALACLGGRPALDA VKKGLGGSGSYALGPYQISAPQLPAYNGQTVGTFYYVNDAGGLESKVFSSGGSGGS TNLSDIIEKETGKQLVIQESILMLPEEVEEVIGNKPESDILVHTAYDESTDENVML LTSDAPEYKPWALVIQDSNGENKIKML* | 169 |
| Half **(NG)** domain-C-terminal domain-G1333-C-UGI | GLTPEQVVAIASNGGGKQALESIVAQLSRPDPALAALTNDHLVALACLGGRPALDA VKKGLGGSPTPYPNYANAGHVEGQSALFMRDNGISEGLVFHNNPEGTCGFCVNMTE TLLPENAKMTVVPPEGAIPVKRGATGETKVFTGNSNSPKSPTKGGCSGGSTNLSDI IEKETGKQLVIQESILMLPEEVEEVIGNKPESDILVHTAYDESTDENVMLLTSDAP EYKPWALVIQDSNGENKIKML* | 170 |
| Half **(NG)** domain-C-terminal domain-G1397-N-UGI | GLTPEQVVAIASNGGGKQALESIVAQLSRPDPALAALTNDHLVALACLGGRPALDA VKKGLGGSGSYALGPYQISAPQLPAYNGQTVGTFYYVNDAGGLESKVFSSGGPTPY PNYANAGHVEGQSALFMRDNGISEGLVFHNNPEGTCGFCVNMTETLLPENAKMTVV PPEGSGGSTNLSDIIEKETGKQLVIQESILMLPEEVEEVIGNKPESDILVHTAYDE STDENVMLLTSDAPEYKPWALVIQDSNGENKIKML* | 171 |
| Half **(NG)** domain-C-terminal domain-G1397-C-UGI | GLTPEQVVAIASNGGGKQALESIVAQLSRPDPALAALTNDHLVALACLGGRPALDA VKKGLGGSAIPVKRGATGETKVFTGNSNSPKSPTKGGCSGGSTNLSDIIEKETGKQ LVIQESILMLPEEVEEVIGNKPESDILVHTAYDESTDENVMLLTSDAPEYKPWALV IQDSNGENKIKML* | 172 |
| 16s rDNA Left TALE repeat - GGGGAGTA CGTTCGCA AG | NLTPAQVVAIASNNGGKQALETVQRLLPVLCQAHGLTPDQVVAIASNNGGKQALET VQRLLPVLCQDH | 173 |
| | GLTPEQVVAIASNNGGKQALETVQRLLPVLCQAHGLTPDQVVAIASNNGGKQALET VQRLLPVLCQDH | 174 |
| | GLTPAQVVAIASNIGGKQALETVQRLLPVLCQAHGLTPEQVVAIASNNGGKQALET VQRLLPVLCQAH | 175 |
| | GLTPEQVVAIASNGGGKQALETVQRLLPVLCQAHGLTPDQVVAIASNIGGKQALET VQRLLPVLCQAH | 176 |
| | GLTPAQVVAIASHDGGKQALETVQRLLPVLCQAHGLTPAQVVAIASNNGGKQALET VQRLLPVLCQDH | 177 |
| | GLTPEQVVAIASNGGGKQALETVQRLLPVLCQAHGLTPAQVVAIASNGGGKQALET VQRLLPVLCQDH | 178 |
| | GLTPAQVVAIASHDGGKQALETVQRLLPVLCQAHGLTPDQVVAIASNNGGKQALET VQRLLPVLCQDH | 179 |
| | GLTPDQVVAIASHDGGKQALETVQRLLPVLCQDHGLTPAQVVAIASNIGGKQALET VQRLLPVLCQDH | 180 |
| | GLTPDQVVAIASNIGGKQALETVQRLLPVLCQDHGLTPDQVVAIASNNGGKQALET VQRLLPVLCQAH | 181 |
| 16s rDNA Right TALE repeat - TGTGCGGG CCCCCGTC AA | NLTPAQVVAIASNGGGKQALETVQRLLPVLCQAHGLTPDQVVAIASNNGGKQALET VQRLLPVLCQAH | 182 |
| | GLTPAQVVAIASNGGGKQALETVQRLLPVLCQDHGLTPAQVVAIASNNGGKQALET VQRLLPVLCQAH | 183 |
| | GLTPDQVVAIASHDGGKQALETVQRLLPVLCQDHGLTPAQVVAIASNNGGKQALET VQRLLPVLCQAH | 184 |
| | GLTPEQVVAIASNNGGKQALETVQRLLPVLCQAHGLTPAQVVAIASNNGGKQALET VQRLLPVLCQDH | 185 |
| | GLTPDQVVAIASHDGGKQALETVQRLLPVLCQAHGLTPAQVVAIASHDGGKQALET VQRLLPVLCQAH | 186 |
| | GLTPDQVVAIASHDGGKQALETVQRLLPVLCQDHGLTPDQVVAIASHDGGKQALET VQRLLPVLCQDH | 187 |
| | GLTPDQVVAIASHDGGKQALETVQRLLPVLCQDHGLTPDQVVAIASNNGGKQALET VQRLLPVLCQDH | 188 |
| | GLTPAQVVAIASNGGGKQALETVQRLLPVLCQAHGLTPAQVVAIASHDGGKQALET VQRLLPVLCQDH | 189 |
| | GLTPEQVVAIASNIGGKQALETVQRLLPVLCQAHGLTPAQVVAIASNIGGKQALET VQRLLPVLCQDH | 190 |
| psbA Left TALE repeat -GCAACAGG AGCTGAAT AT | NLTPAQVVAIASNNGGKQALETVQRLLPVLCQAHGLTPDQVVAIASHDGGKQALET VQRLLPVLCQDH | 191 |
| | GLTPAQVVAIASNIGGKQALETVQRLLPVLCQAHGLTPDQVVAIASNIGGKQALET VQRLLPVLCQDH | 192 |
| | GLTPDQVVAIASHDGGKQALETVQRLLPVLCQDHGLTPAQVVAIASNIGGKQALET VQRLLPVLCQAH | 193 |
| | GLTPAQVVAIASNNGGKQALETVQRLLPVLCQAHGLTPDQVVAIASNNGGKQALET VQRLLPVLCQDH | 194 |
| | GLTPAQVVAIASNIGGKQALETVQRLLPVLCQAHGLTPDQVVAIASNNGGKQALET VQRLLPVLCQAH | 195 |
| | GLTPAQVVAIASHDGGKQALETVQRLLPVLCQDHGLTPDQVVAIASNGGGKQALET VQRLLPVLCQAH | 196 |
| | GLTPEQVVAIASNNGGKQALETVQRLLPVLCQAHGLTPDQVVAIASNIGGKQALET VQRLLPVLCQDH | 197 |
| | GLTPDQVVAIASNIGGKQALETVQRLLPVLCQAHGLTPDQVVAIASNGGGKQALET VQRLLPVLCQAH | 198 |
| | GLTPDQVVAIASNIGGKQALETVQRLLPVLCQDHGLTPDQVVAIASNGGGKQALET VQRLLPVLCQAH | 199 |
| psbA Right TALE repeat - AGTTTCCG TCTGGGTA TG | NLTPAQVVAIASNIGGKQALETVQRLLPVLCQAHGLTPEQVVAIASNNGGKQALET VQRLLPVLCQAH | 200 |
| | GLTPDQVVAIASNGGGKQALETVQRLLPVLCQDHGLTPDQVVAIASNGGGKQALET VQRLLPVLCQDH | 201 |
| | GLTPEQVVAIASNGGGKQALETVQRLLPVLCQAHGLTPDQVVAIASHDGGKQALET VQRLLPVLCQAH | 202 |
| | GLTPDQVVAIASHDGGKQALETVQRLLPVLCQDHGLTPDQVVAIASNNGGKQALET VQRLLPVLCQDH | 203 |
| | GLTPAQVVAIASNGGGKQALETVQRLLPVLCQAHGLTPAQVVAIASHDGGKQALET VQRLLPVLCQAH | 204 |
| | GLTPAQVVAIASNGGGKQALETVQRLLPVLCQDHGLTPDQVVAIASNNGGKQALET VQRLLPVLCQDH | 205 |
| | GLTPAQVVAIASNNGGKQALETVQRLLPVLCQDHGLTPAQVVAIASNNGGKQALET VQRLLPVLCQDH | 206 |
| | GLTPDQVVAIASNGGGKQALETVQRLLPVLCQAHGLTPEQVVAIASNIGGKQALET VQRLLPVLCQAH | 207 |
| | GLTPAQVVAIASNGGGKQALETVQRLLPVLCQAHGLTPAQVVAIASNNGGKQALET VQRLLPVLCQDH | 208 |
| psbB Left TALE repeat - AAATGAAT TCCAAAAA TC | NLTPDQVVAIASNIGGKQALETVQRLLPVLCQAHGLTPAQVVAIASNIGGKQALET VQRLLPVLCQDH | 209 |
| | GLTPAQVVAIASNIGGKQALETVQRLLPVLCQAHGLTPAQVVAIASNGGGKQALET VQRLLPVLCQAH | 210 |
| | GLTPAQVVAIASNNGGKQALETVQRLLPVLCQDHGLTPAQVVAIASNIGGKQALET VQRLLPVLCQDH | 211 |
| | GLTPAQVVAIASNIGGKQALETVQRLLPVLCQAHGLTPDQVVAIASNGGGKQALET VQRLLPVLCQAH | 212 |
| | GLTPDQVVAIASNGGGKQALETVQRLLPVLCQAHGLTPAQVVAIASHDGGKQALET VQRLLPVLCQDH | 213 |
| | GLTPDQVVAIASHDGGKQALETVQRLLPVLCQAHGLTPDQVVAIASNIGGKQALET VQRLLPVLCQAH | 214 |
| | GLTPDQVVAIASNIGGKQALETVQRLLPVLCQAHGLTPAQVVAIASNIGGKQALET VQRLLPVLCQDH | 215 |
| | GLTPAQVVAIASNIGGKQALETVQRLLPVLCQAHGLTPDQVVAIASNIGGKQALET VQRLLPVLCQDH | 216 |
| | GLTPAQVVAIASNGGGKQALETVQRLLPVLCQAHGLTPAQVVAIASHDGGKQALET VQRLLPVLCQDH | 217 |
| psbB Right TALE repeat -GAACGTAC AGGAAAAC C | NLTPEQVVAIASNNGGKQALETVQRLLPVLCQAHGLTPDQVVAIASNIGGKQALET VQRLLPVLCQDH | 218 |
| | GLTPDQVVAIASNIGGKQALETVQRLLPVLCQAHGLTPDQVVAIASHDGGKQALET VQRLLPVLCQDH | 219 |
| | GLTPDQVVAIASNNGGKQALETVQRLLPVLCQDHGLTPAQVVAIASNGGGKQALET VQRLLPVLCQAH | 220 |
| | GLTPDQVVAIASNIGGKQALETVQRLLPVLCQDHGLTPDQVVAIASHDGGKQALET VQRLLPVLCQDH | 221 |
| | GLTPAQVVAIASNIGGKQALETVQRLLPVLCQAHGLTPAQVVAIASNNGGKQALET VQRLLPVLCQAH | 222 |
| | GLTPDQVVAIASNNGGKQALETVQRLLPVLCQDHGLTPAQVVAIASNIGGKQALET VQRLLPVLCQAH | 223 |
| | GLTPDQVVAIASNIGGKQALETVQRLLPVLCQAHGLTPAQVVAIASNIGGKQALET VQRLLPVLCQDH | 224 |
| | GLTPAQVVAIASNIGGKQALETVQRLLPVLCQAHGLTPAQVVAIASHDGGKQALET VQRLLPVLCQDH | 225 |
| | GLTPAQVVAIASHDGGKQALETVQRLLPVLCQAH | 226 |
| ATP6 (lettuce, rapeseed ) Left TALE repeat - TATTGGCA TTACTATA G | NLTPDQVVAIASNGGGKQALETVQRLLPVLCQAHGLTPDQVVAIASNIGGKQALET VQRLLPVLCQDH | 227 |
| | GLTPDQVVAIASNGGGKQALETVQRLLPVLCQAHGLTPEQVVAIASNGGGKQALET VQRLLPVLCQAH | 228 |
| | GLTPAQVVAIASNNGGKQALETVQRLLPVLCQDHGLTPDQVVAIASNNGGKQALET VQRLLPVLCQDH | 229 |
| | GLTPAQVVAIASNGGGKQALETVQRLLPVLCQAHGLTPEQVVAIASNGGGKQALET VQRLLPVLCQAH | 230 |
| | GLTPDQVVAIASNIGGKQALETVQRLLPVLCQAHGLTPAQVVAIASHDGGKQALET VQRLLPVLCQDH | 231 |
| | GLTPDQVVAIASNGGGKQALETVQRLLPVLCQAHGLTPDQVVAIASNIGGKQALET VQRLLPVLCQDH | 232 |
| | GLTPDQVVAIASNGGGKQALETVQRLLPVLCQAHGLTPDQVVAIASNIGGKQALET VQRLLPVLCQDH | 233 |
| | GLTPAQVVAIASNNGGKQALETVQRLLPVLCQDHGLTPAQVVAIASNNGGKQALET VQRLLPVLCQDH | 234 |
| | GLTPDQVVAIASNNGGKQALETVQRLLPVLCQDH | 235 |
| ATP6 (lettuce ) Right TALE repeat - GAAAAAAT GAAGCCCA | NLTPEQVVAIASNGGGKQALETVQRLLPVLCQAHGLTPAQVVAIASNIGGKQALET VQRLLPVLCQDH | 236 |
| | GLTPAQVVAIASNIGGKQALETVQRLLPVLCQAHGLTPDQVVAIASNIGGKQALET VQRLLPVLCQAH | 237 |
| | GLTPAQVVAIASNIGGKQALETVQRLLPVLCQDHGLTPAQVVAIASNIGGKQALET VQRLLPVLCQAH | 238 |
| | GLTPEQVVAIASNIGGKQALETVQRLLPVLCQAHGLTPAQVVAIASNGGGKQALET VQRLLPVLCQAH | 239 |
| | GLTPAQVVAIASNNGGKQALETVQRLLPVLCQAHGLTPAQVVAIASNIGGKQALET VQRLLPVLCQDH | 240 |
| | GLTPDQVVAIASNIGGKQALETVQRLLPVLCQDHGLTPDQVVAIASNNGGKQALET VQRLLPVLCQAH | 241 |
| | GLTPAQVVAIASHDGGKQALETVQRLLPVLCQAHGLTPDQVVAIASHDGGKQALET VQRLLPVLCQDH | 242 |
| | GLTPDQVVAIASHDGGKQALETVQRLLPVLCQDHGLTPDQVVAIASNIGGKQALET VQRLLPVLCQAH | 243 |
| ATP6 (rapesee d) Right TALE repeat - TAAAAAAT GAAGCCCA | NLTPEQVVAIASNNGGKQALETVQRLLPVLCQAHGLTPDQVVAIASNIGGKQALET VQRLLPVLCQDH | 244 |
| | GLTPDQVVAIASNIGGKQALETVQRLLPVLCQAHGLTPDQVVAIASNIGGKQALET VQRLLPVLCQAH | 245 |
| | GLTPAQVVAIASNIGGKQALETVQRLLPVLCQDHGLTPAQVVAIASNIGGKQALET VQRLLPVLCQAH | 246 |
| | GLTPEQVVAIASNIGGKQALETVQRLLPVLCQAHGLTPAQVVAIASNGGGKQALET | 247 |
| | VQRLLPVLCQAH | |
| | GLTPAQVVAIASNNGGKQALETVQRLLPVLCQAHGLTPAQVVAIASNIGGKQALET VQRLLPVLCQDH | 248 |
| | GLTPDQVVAIASNIGGKQALETVQRLLPVLCQDHGLTPDQVVAIASNNGGKQALET VQRLLPVLCQAH | 249 |
| | GLTPAQVVAIASHDGGKQALETVQRLLPVLCQDHGLTPAQVVAIASHDGGKQALET VQRLLPVLCQAH | 250 |
| | GLTPAQVVAIASHDGGKQALETVQRLLPVLCQDHGLTPDQVVAIASNIGGKQALET VQRLLPVLCQAH | 251 |
| RPS14 Left TALE repeat - CGTTCTGT ATCTGAG | NLTPDQVVAIASHDGGKQALETVQRLLPVLCQDHGLTPDQVVAIASNNGGKQALET VQRLLPVLCQDH | 252 |
| | GLTPAQVVAIASNGGGKQALETVQRLLPVLCQAHGLTPEQVVAIASNGGGKQALET VQRLLPVLCQAH | 253 |
| | GLTPDQVVAIASHDGGKQALETVQRLLPVLCQDHGLTPDQVVAIASNGGGKQALET VQRLLPVLCQAH | 254 |
| | GLTPDQVVAIASNNGGKQALETVQRLLPVLCQAHGLTPAQVVAIASNGGGKQALET VQRLLPVLCQDHG | 255 |
| | LTPAQVVAIASNIGGKQALETVQRLLPVLCQAHGLTPEQVVAIASNGGGKQALETV QRLLPVLCQAH | 256 |
| | GLTPDQVVAIASHDGGKQALETVQRLLPVLCQDHGLTPDQVVAIASNGGGKQALET VQRLLPVLCQAH | 257 |
| | GLTPAQVVAIASNNGGKQALETVQRLLPVLCQAHGLTPAQVVAIASNIGGKQALET VQRLLPVLCQAH | 258 |
| | GLTPDQVVAIASNNGGKQALETVQRLLPVLCQAH | 259 |
| RPS14 Right TALE repeat - AATCCACG AAAAACGA | NLTPDQVVAIASNIGGKQALETVQRLLPVLCQAHGLTPDQVVAIASNIGGKQALET VQRLLPVLCQDH | 260 |
| | GLTPDQVVAIASNGGGKQALETVQRLLPVLCQAHGLTPAQVVAIASHDGGKQALET VQRLLPVLCQDH | 261 |
| | GLTPDQVVAIASHDGGKQALETVQRLLPVLCQAHGLTPDQVVAIASNIGGKQALET VQRLLPVLCQAH | 262 |
| | GLTPEQVVAIASHDGGKQALETVQRLLPVLCQAHGLTPAQVVAIASNNGGKQALET VQRLLPVLCQAH | 263 |
| | GLTPAQVVAIASNIGGKQALETVQRLLPVLCQAHGLTPDQVVAIASNIGGKQALET VQRLLPVLCQAH | 264 |
| | GLTPAQVVAIASNIGGKQALETVQRLLPVLCQDHGLTPAQVVAIASNIGGKQALET VQRLLPVLCQAH | 265 |
| | GLTPDQVVAIASNIGGKQALETVQRLLPVLCQDHGLTPDQVVAIASHDGGKQALET VQRLLPVLCQAH | 266 |
| | GLTPAQVVAIASNNGGKQALETVQRLLPVLCQDHGLTPDQVVAIASNIGGKQALET VQRLLPVLCQAH | 267 |

### mRNA in-vitro transcription

DdCBE DNA templates were prepared by PCR using a Phusion DNA polymerase (Thermo Fisher). DdCBE mRNAs were synthesized and purified using an *in-vitro* mRNA synthesis kit (Enzynomics).

### Protoplast isolation and transduction

Lettuce seeds were surface-sterilized in 70% ethanol for 30 seconds and 0.4% hypochlorite solution for 15 minutes, and then washed three times with sterile distilled water. The lettuce seeds were germinated on 0.5x MS medium supplemented with 2% sucrose at 25°C under 16-hour light and 8-hour dark conditions. Rapeseed seeds were surface-sterilized in 70% ethanol for 3 minutes and 1.0% hypochlorite solution for 30 minutes, and then washed three times with sterile distilled water. The rapeseed seeds were germinated on 1x MS medium supplemented with 3% sucrose under 16-hour light and 8-hour dark conditions at 25°C.

Protoplast isolation and transduction are performed as described previously. Cotyledons from 7-day-old lettuce and 14-day-old rapeseed plants were digested with enzyme solution for 3 hours under dark conditions with shaking (40 rpm). The protoplast-enzyme mixture was washed with an equal volume of W5 solution and then intact protoplasts were obtained from a sucrose solution by centrifugation at 80 g for 7 minutes. The protoplasts were treated with W5 solution at 4°C for 1 hour, followed by transfection using polyethylene glycol.

The lettuce protoplasts and rapeseed protoplasts resuspended in MMG solution were transfected with plasmids or mRNA using PEG, followed by culture at room temperature for 20 minutes. The PEG-protoplast mixture was washed three times with an equal volume of W5 solution with gentle inverting, followed by culture for 10 minutes. The protoplasts were then pelleted by centrifugation at 100 g for 5 minutes.

### Protoplast culture

The lettuce protoplasts transfected with DdCBE-encoding plasmids were resuspended in lettuce protoplast culture medium (LPCM). The protoplasts in the medium were mixed at 1:1 with medium containing 2.4% low-melting-point agarose, and then immediately placed in a 6-well plate. After the mixture was solidified, the embedded protoplasts were overlaid with 1 ml of liquid medium, followed by culture at 25°C under dark conditions for 1 week. After initial culture, the overlaid liquid medium was replaced with fresh medium every week, and the embedded protoplasts were cultured for 1 week under 16-hour light and 8-hour dark conditions, and then cultured for 2 weeks under 16-hour light and 8-hour dark conditions. Microcalli induced from the protoplasts were cultured in regeneration medium at 25°C under 16-hour light and 8-hour dark conditions for 4 weeks. In preparation for analysis of base editing efficiency, the protoplasts were cultured for 1 week under dark conditions at 25°C in liquid medium without embedding. In order to test antibiotic resistance, microcalli embedded for one month were cultured in regeneration medium containing 50 mg/L streptomycin or 50 mg/L spectinomycin at 25°C under 16-hour light and 8-hour dark conditions for 4 weeks. After 4 weeks, the antibiotic-resistant green calli or adventitious shoots were transferred to fresh regeneration medium containing 200 mg/L streptomycin or 50 mg/L spectinomycin.

The rapeseed protoplasts transfected with the DdCBE-encoding plasmids were resuspended in rapeseed culture medium. The protoplast-medium mixture was transferred into a 6-well plate and cultured for 2 weeks under dark conditions at 25°C. After 2 weeks, the protoplasts were cultured for 3 weeks under 16-hour light and 8-hour dark conditions. The medium was replaced with very fresh medium.

### DNA and RNA extraction

Total DNA or RNA was extracted from cultured cells in liquid medium or transgenic calli using a DNeasy Plant Mini Kit or RNeasy Plant Mini Kit. The cultured cells or calli were harvested by centrifugation at 10,000 rpm for 1 minute. Then, cDNA from total RNA was reverse-transcribed using RNA to cDNA EcoDry Premix (TaKaRa).

### Deep sequencing

Target regions were amplified using fusion enzymes and appropriate primers (Supplementary Table 1). In order to create a DNA sequencing library, three rounds of PCR (first, nested PCR; second, PCR; and third, indexing PCR) were performed. Equal amounts of DNAs were pooled and then sequenced using a MiniSeq system (Illumina). The paired-end sequencing files were analyzed by a Cas-analyzer and source code of the computer program.

### 2-2. Results

A Golden Gate assembly system was developed to construct chloroplast-targeting DdCBE (cp-DdCBE) and mitochondria-targeting DdCBE (mt-DdCBE) (FIG. 19). Expression plasmids encoded fusion proteins composed of a chloroplast transit peptide or mitochondrial targeting sequence, N- or C-terminal domains of TALE, split-DddAtox halves (G1333N, G1333C, G1397N, and G1397C), and UGI, which were optimized for expression in dicotyledonous plants under the control of the parsley ubiquitin (PcUbi) promoter and pea3A terminator. DdCBE plasmids with custom-designed TALE DNA-binding sequences were constructed in a single subcloning step by mixing an expression vector and six TALE subarray plasmids in an E-tube. A total of 424 (6x64 tripartite + 2x16 bipartite + 2x4 monopartite) modular TALE subarray plasmids are available for making cp-DdCBEs and mt-DdCBEs that recognize sequences of 16-20 bps in length, including conserved T at the 5' end. Consequently, DdCBE heterodimers functionally recognize 32-40 bp DNA sequences.

In order to determine whether DdCBEs are able to promote base editing in chloroplasts, four pairs of cp-DdCBE plasmids suitable for the chloroplast 16S rRNA gene encoding the RNA component of the 30S ribosomal subunit were constructed, and each pair was co-transfected into lettuce and rapeseed protoplasts. After 7 days, base editing efficiency was measured through deep sequencing (FIGs. 20a and 20b). The cp-DdCBE pair (left-G1397-N + right-G1397-C) with the highest efficiency induced C*G-to-T*A conversions in the 15-bp spacer region between the two TALE binding sites with efficiencies of 30% in lettuce protoplasts and 15% in rapeseed protoplasts (FIG. 20b). Like previous results in mammalian cells and mice, cytosines (C9 and C13) in a 5'-TC motif were preferentially converted to thymine by cp-DdCBE. Interestingly, cytosine (C7) in a 5'-AC context was changed to thymine with an efficiency of 4.2% by another cp-DdCBE (left-G1333-N + right-G1333-C) in lettuce protoplasts. In addition, persistence of base editing by cp-DdCBE in lettuce protoplasts during 14-day culture was observed (FIG. 24). The editing efficiency continuously increased for up to 10 days and was maintained throughout the period of culture.

Base editing was tested in two additional chloroplast genes, psbA and psbB, encoding D1 and CP-47 photosynthetic proteins, respectively, of photosystem II (FIGs. 20c, 20d, and 25). Among cp-DdCBEs targeting the Psb gene, the most active one (left-G1397-C + right-G1397-N) was able to induce C*G-to-T*A conversions in lettuce protoplasts with an efficiency of up to 25% (FIG. 20d). Only the two cytosines (C11 and C12) in a 5'-TCC context were efficiently converted to thymines by the base editors. It is possible that 5'-TCC was first converted to 5'-TTC and then to 5'-TTT. In rapeseed protoplasts, the other combination (left-G1333-N + right-G1333-C) showed the highest efficiency at four cytosine positions (C3, C4, C11, and C12), with efficiencies of up to 3.5% (C3). C3 and C4 are in a 5'-TCC context in the rapeseed gene, whereas they are in a 5'-ACC context in the lettuce counterpart due to a single nucleotide polymorphism, which is responsible for efficient editing of the two cytosines (C3 and C4) in the rapeseed gene but not in the lettuce gene by DdCBE. Likewise, the cp-DdCBE combination targeting the psbB gene catalyzed the conversion of two cytosines in a TCC context with an efficiency of 0.36% to 4.1% in rapeseed protoplasts (FIG. 25). Taken together, these results show that the editing efficiency is determined by cytosine positions and sequences, including the DddAtox split positions (G1333 vs G1397) and orientations (left-G1333-N vs left-G1333-C), and that cp-DdCBEs are capable of efficient base editing in the chloroplast genome in plants.

In addition, attempts were made to achieve base editing in plant mitochondrial DNA using custom-designed mt-DdCBEs. To this end, mt-DdCBE-encoding plasmids (using the Golden Gate cloning system) targeting the atp6 gene in lettuce and rapeseed and the rps14 gene in rapeseed were constructed, and the plasmids were introduced into lettuce and rapeseed protoplasts. 7 days after introduction, base editing efficiency was measured through deep sequencing (FIGs. 20e, 20f, and 26). The most efficient mt-DdCBE combination (left-G1397-N + right-G1397-C in lettuce and left-G1397-C + right-G1397-N in rapeseed) catalyzed C*G-to-T*A conversions with 23% efficiency in lettuce protoplasts and 23% efficiency in rapeseed protoplasts at the atp6 gene target site (FIG. 20). Also, the mt-DdCBE combination induced C*G-to-T*A conversions with 11% efficiency in rapeseed protoplasts at the rps14 target site. These results suggest that mitochondrial DNA in plants is amenable to base editing with mt-DdCBEs.

In order to investigate whether editing of cpDNA and mtDNA by DdCBE was maintained during regeneration, regenerated lettuce and rapeseed calli were collected from DdCBE-treated protoplasts 4 weeks after introduction (FIG. 21a), and the base editing efficiency of each callus was measured using deep sequencing and Sanger sequencing (FIGs. 21b and 27). Chloroplast or mitochondrial genes, base editing of which was induced by DdCBE, exhibited respective efficiencies of up to 38% and 25% in 22 out of 26 lettuce calli and 7 out of 14 rapeseed calli (FIG. 21c). Also, base editing of the chloroplast psbA gene showed an efficiency of up to 3.9% in lettuce calli (FIG. 27). Likewise, mitochondrial base editing in rapeseed calli was measured with efficiencies of up to 25% and 1.9% at atp6 and rps14, respectively (FIG. 27). These results showed that DdCBE expression in plant protoplasts could be tolerated and organelle base editing was induced by DdCBEs during regeneration in protoplasts.

In addition, attempts were made to demonstrate DNA-free base editing in organelles using *in-vitro* transcribed cp-DdCBE mRNA rather than plasmids. After introducing an *in-vitro* transcript encoding cp-DdCBE targeting the 16S rRNA gene into lettuce protoplasts, base editing efficiency at the target site was analyzed (FIG. 21a). C-to-T mutations in the protoplasts were measured with an efficiency of up to 25% (FIGs. 21d and 28). As expected, DdCBE mRNA and DNA sequences were absent 7 days after introduction into the protoplasts (FIG. 29). This method may avoid potential integration of plasmid DNA fragments into the host genome.

By virtue of stable maintenance of organelle editing in calli regenerated from the protoplasts, resistance to streptomycin and spectinomycin antibiotics that inhibit protein synthesis by irreversibly binding to the 16S rRNA gene through 16S rRNA gene editing in chloroplast DNA was measured. Several single nucleotide polymorphisms in the 16S rRNA gene are commonly observed in streptomycin-resistant prokaryotes and eukaryotes, and in particular, the 16S rRNA C860T (*E. coli* coordinate C912) mutation result in streptomycin resistance in tobacco. The C860T point mutation in tobacco is equivalent to the C9 position in lettuce (FIGs. 20a, 20b, 21b, and 21d). Lettuce calli regenerated from DdCBE-treated protoplasts were transferred to medium supplemented with streptomycin and spectinomycin. The Mock-treated group turned white when exposed to antibiotics, indicating protoplast dysfunction of calli. In contrast, DdCBE-treated calli remained greenish, showing resistance to such antibiotics. The editing efficiency by DdCBE was analyzed in lettuce calli and plantlets with resistance. Like the C860T mutation, C-to-T conversion at position C9 was observed with an efficiency of up to 98.6% in calli and shoots obtained after drug treatment (FIGs. 21e and 21f). Interestingly, C-to-T editing at nearby position C13 showed an efficiency of up to 20% in the absence of spectinomycin, but not at all in the presence of antibiotics, demonstrating selection against this mutation upon drug treatment. Taken together, these results suggest that plant organelle mutations induced by DdCBEs in protoplasts may be maintained even after cell division and plant development, and that homoplasmy of chloroplast editing may be achieved by drug selection.

In addition, off-target activity of TALE deaminase targeting the 16S rRNA site was analyzed in protoplasts, calli, and shoots. No off-target mutations were detected in antibiotic-resistant calli or shoots, which were derived from single cells, in the vicinity (50 base pairs on both sides) of the target site (FIG. 31) or at the top five candidate off-target sites (FIG. 32) in the chloroplast genome, which were chosen on the basis of sequence homology. In contrast, when plasmids encoding DdCBEs were introduced into protoplasts, off-target TC-to-TT mutations were induced at three out of five candidate off-target sites with low efficiencies of 1.2% to 4.1%. Off-target efficiencies in protoplasts were greatly reduced when using *in-vitro* transcripts (mRNA) instead of plasmids encoding TALE deaminase (FIG. 22). These results show that overexpression or prolonged plasmid-based expression of DdCBEs increases off-target mutations, and that transient mRNA-based expression using mRNA is preferable to avoid off-target base editing.

In summary, the Golden Gate cloning system using 424 TALE subarray plasmids and 16 expression plasmids was developed to assemble DdCBE-encoding plasmids for organelle base editing in plants. Custom-designed DdCBEs targeting three genes in chloroplast DNA and two genes in mitochondrial DNA achieved C-to-T conversions with high efficiencies in lettuce and rapeseed protoplasts. In particular, editing in plant organelles was maintained during cell division and plant development. Moreover, antibiotic-resistant lettuce calli and plantlets were obtained with near homoplasmy (99%) through a mutation in the chloroplast 16S rRNA gene. Without antibiotic selection, the editing efficiency was 25% in mitochondria and 38% in chloroplasts. It is expected that the Golden Gate cloning system will be a valuable resource for organelle DNA editing in plants.

### Example 3. Animal DNA editing by TALE-DdCBE

DddA-derived cytosine base editors (DdCBEs), composed of the split interbacterial toxin DddAtox, a transcription activator-like effector (TALE) designed to bind to DNA, and a uracil glycosylase inhibitor (UGI), enabled desired cytosine-to-thymine base editing in mitochondrial DNA. Also, high-efficiency mitochondrial DNA editing was possible in mouse embryos. Among mitochondrial genes, MT-ND5 (ND5), which encodes a subunit of NADH dehydrogenase that catalyzes NADH dehydration and electron transfer to ubiquinone, was targeted, including mutations associated with human mitochondrial diseases, such as m.G12918A, and mutations that create early stop codons, such as m.C12336T. Thereby, it was possible to generate a mitochondrial disease model in mice, suggesting the possibility of treating mitochondrial diseases.

### 3-1. Methods

Plasmid assembly. The TALEN (transcription activator-like effector nuclease) system was employed to construct an expression plasmids containing the DddA half and a final TALE-DddAtox construct. In the expression plasmid of the TALEN system, the nuclear localization signal and monomers of the FokI dimer were substituted with the mitochondrial targeting signal (MTS), the DddA deaminase half, and the uracil glycosylase inhibitor (UGI). Sequences encoding MTS, DddA, and UGI were synthesized by IDT. In order to construct an expression vector, DNA fragments required for Gibson assembly were amplified using Q5 DNA polymerase (NEB) and then purified. The purified gene fragments were assembled using a HiFi DNA assembly kit (NEB), chemically transformed into *E. coli* DH5α (Enzynomics), and then identified by Sanger sequencing. Thus, eight different expression plasmids were obtained, in which the BsaI restriction site for Golden Gate cloning was located between sequences encoding the N-terminal and C-terminal domains. In order to assemble the DdCBE plasmid, expression plasmids were loaded with module vectors (each encoding a TALE sequence), BsaI-HFv2 (10 U), T4 DNA ligase (200 U), and reaction buffer in one tube. Thereafter, restriction enzyme and ligase reaction was carried out in a thermocycler for 20 cycles of 5 minutes at 37°C and 5 minutes at 50°C, followed by further reaction for 15 minutes at 50°C and 5 minutes at 80°C. The conjugated plasmid was introduced into *E*. *coli* DH5α via chemical transformation, and the final construct was identified by Sanger sequencing. For cell line introduction, plasmids were midi-prepped.

Mammalian cell line culture and transfection. The NIH3T3 (CRL-1658, American Type Culture Collection (ATCC)) cell line was cultured at 37°C in a 5% CO₂ environment. The cell line grew without antibiotics in DMEM (Gibco) supplemented with 10% (v/v) fetal bovine serum and was not tested for mycoplasma. For lipofection, cells were seeded 18-24 hours before transfection at a cell density of 1.5×10⁴ in 12-well cell culture plates (SPL, Seoul, Korea). A total of 1,000 ng of plasmid DNA was introduced using 500 ng of each DdCBE split using Lipofectamine 3000 (Invitrogen). The cells were harvested 4 days after transfection.

mRNA preparation. The mRNA template was amplified by PCR using Q5 DNA polymerase (NEB), and the following primers were used (F: 5'-CATCAA TGGGCGTGGATAG-3' SEQ ID No: 268, R: 5'-GACACCTACTCAGACAATGC-3 SEQ ID No: 269). DdCBE mRNA was synthesized using an *in-vitro* RNA transcription kit (mMESSAGE mMACHINE T7 Ultra kit, Ambion) and then purified using a MEGAclear kit (Ambion).

Animals. All experiments involving mice were conducted with the approval of the Animal Care and Use Committee of the Institute for Basic Science. Superovulated C57BL/6J females were mated with C57BL/6J males, and ICR strain females were used as surrogate mothers. Mice were housed in a specific pathogen-free facility under 12-hour day-night cycle conditions and constant temperature and humidity conditions (20-26°C, 40-60%) .

Microinjection into mouse zygotes. Superovulation, embryo collection, and microinjection, which are processes immediately before microinjection, were performed as described previously. For microinjection, a mixture of left DdCBE mRNA (300 ng/ul) and right DdCBE mRNA (300 ng/ul) was diluted with DEPC-treated injection buffer (0.25 mM EDTA, 10 mM Tris, pH 7.4), and injected into the zygote cytoplasm using a Nikon ECLIPSE Ti micromanipulator and a FemtoJet 4i microinjector (Eppendorf). After microinjection, the embryos were placed in KSOM + AA (Millipore) microdrops, followed by culture for 4 days at 37°C under 5% CO₂ conditions. 2-cell stage embryos were transferred into the oviduct of a 0.5-d.p.c. pseudo-pregnant surrogate mother.

Genotyping. Embryos at the blastocyst stage and tissues were placed in digestion buffer (25 mM NaOH, 0.2 mM EDTA, pH 10), followed by incubation at 95°C for 20 minutes, after which the pH was adjusted to 7.4 so that a final concentration was 50 mM using HEPES (free acid, without pH adjustment). Genomic DNA was isolated from mouse offspring using DNeasy Blood & Tissue Kits (Qiagen), and analyzed by Sanger sequencing and targeted deep sequencing.

Mitochondrial DNA isolation for high-throughput sequencing. In order to isolate mitochondria from cultured NIH3T3 cells in a 12-well plate, the cell culture medium was removed and then 200 µl of mitochondrial isolation buffer A (ScienCell) was added to the culture plate. The cells were scraped using a cell lifter and then placed in a microtube, and the cells were ground using a disposable pestle. After grinding 15 times, the well-ground homogenate was centrifuged for 5 minutes at 1,000x g and 4°C. The supernatant was placed in a new microtube and centrifuged at 10,000x g, 4°C for 20 minutes. The precipitate was resuspended in 20 pl of lysis buffer (25 mM NaOH, 0.2 mM EDTA, pH 10) and then boiled at 95°C for 20 minutes. In order to lower the pH, 2 pl of 1 M HEPES (free acid, without pH adjustment) was added to the mitochondrial lysate. 1 pl of the solution was used in a PCR template strand for high-throughput sequencing.

High-throughput sequencing. In order to prepare a deep sequencing library, nested primary PCR and secondary PCR were performed using Q5 DNA Polymerase, and then a final index sequence was added. The library was used for paired-end read sequencing using a MiniSeq (Illumina). For whole mitochondrial genome analysis, isolated mitochondrial DNA was prepared using a tagmentation DNA prep kit (Illumina) according to the manufacturer's protocol. Paired-end sequencing results from all analyses were merged into one fastq-join file and analyzed using CRISPR RGEN Tools (http://www.rgenome.net/) .

Data analysis and display. Microsoft Excel (2019) and PowerPoint (2019) were used to create figures, graphs, and tables. Geneious (version 2021.0.1) and Snapgene 5.2.3 were used for genome sequence alignment, primer construction, and cloning design, and NC_005089 was used as a reference sequence.

### 3-2. Results

DdCBE plasmid assembly. In order to facilitate assembly of the custom-designed TALE sequences in DdCBE, expression plasmids encoding the split-DddAtox halves were constructed, and the Golden Gate cloning system using a total of 424 (6x64 tripartite + 2x16 bipartite + 2x4 monopartite) plasmids was used (FIG. 33a). As shown in Table 4 below, six TALE module plasmids and expression plasmids were mixed in the same tube to construct ready-to-use DdCBE plasmids, with 15.5-18.5 repeat variable diresidue sequences (FIG. 38).

**[Table 4]**

| | **Left TALE** | **Right TALE** |
|---|---|---|
| | **Target Sequence** | **Target Sequence** |
| **ND5 Silent Mutation** | **5'-T TTTCCTACTGGTCCGAT T-3'** | **5'-T TAAAATAAAGTTATTAT T-3'** |
| | SEQ ID NO: 277 | SEQ ID NO: 280 |
| **ND5 G12918A Mutation** | **5'-T TGCAGGTATTAATTGCT T-T** | **5'-T TCCTMCAGGGTTCTAC T-3'** |
| | SEQ ID NO: 278 | SEQ ID NO: 281 |
| **ND5 Nonsense Mutation** | **5'-T TCCCTAAACATAAACTCA T-3'** | **5'-T TGTTGTTGGAGAATA T-3'** |
| | SEQ ID NO: 279 | SEQ ID NO: 282 |

The sequences for the DdCBE constructs are shown in Table 5 below. Consequently, DdCBE recognizes 17-20 DNA sequences, including a conserved thymine sequence at the 5' end. Thus, a functional DdCBE pair recognizes a total of 32-40 DNA sequences.

**[Table 5]**

| |
|---|
| **Left-SOD2** MTS-3×HA-N terminal domain |
| MALSRAVCGTSRQLAPVLGYLGSRQKHSLPDYPYDVPDYAGYPYDVPDYAGYPY DVPDYAGIRIQDLRTLGYSQQQQEKIKPKVRSTVAQHHEALVGHGFTHAHIVALSQ HPAALGTVAVKYQDMIAALPEATHEAIVGVGKQWSGARALEALLTVAGELRGPPLQ LDTGQLLKIAKRGGVTAVEAVHAWRNALTGAPL SEQ ID No: 283 |
| Right-COX8A MTS-3×FLAG-N terminal domain |
| MASVLTPLLLRGLTGSARRLPVPRAKIHSLDYKDHDGDYKDHDIDYKDDDDKGIRIQ DLRTLGYSQQQQEKIKPKVRSTVAQHHEALVGHGFTHAHIVALSQHPAALGTVAVK YQDMIAALPEATHEAIVGVGKQWSGARALEALLTVAGELRGPPLQLDTGQLLKIAKR GGVTAVEAVHAWRNALTGAPL SEQ ID No: 284 |
| **C terminal half domain (NG)-G1333-N**-UGI |
| GLTPEQVVAIASNGGGKQALESIVAQLSRPDPALAALTNDHLVALACLGGRPALDA VKKGLGGSGSGSYALGPYQISAPQLPAYNGQTVGTFYYVNDAGGLESKVFSSGGS GGSTNLSDIIEKETGKQLVIQESILMLPEEVEEVIGNKPESDILVHTAYDESTDENVML LTSDAPEYKPWALVIQDSNGENKIKML SEQ ID No: 285 |
| **C terminal half domain (NG)-G1333-C**-UGI |
| GLTPEQVVAIASNGGGKQALESIVAQLSRPDPALAALTNDHLVALACLGGRPALDA VKKGLGGSGSPTPYPNYANAGHVEGQSALFMRDNGISEGLVFHNNPEGTCGFCV NMTETLLPENAKMTVVPPEGAIPVKRGATGETKVFTGNSNSPKSPTKGGCSGGST NLSDIIEKETGKQLVIQESILMLPEEVEEVIGNKPESDILVHTAYDESTDENVMLLTSD APEYKPWALVIQDSNGENKIKML SEQ ID No: 286 |
| **C terminal half domain (NG)-G1397-N**-UGI |
| GLTPEQVVAIASNGGGKQALESIVAQLSRPDPALAALTNDHLVALACLGGRPALDA VKKGLGGSGSGSYALGPYQISAPQLPAYNGQTVGTFYYVNDAGGLESKVFSSGGP TPYPNYANAGHVEGQSALFMRDNGISEGLVFHNNPEGTCGFCVNMTETLLPENAK MTVVPPEGSGGSTNLSDIIEKETGKQLVIQESILMLPEEVEEVIGNKPESDILVHTAY DESTDENVMLLTSDAPEYKPWALVIQDSNGENKIKML SEQ ID No: 287 |
| **C terminal half domain (NG)-G1397-C**-UGI |
| GLTPEQVVAIASNGGGKQALESIVAQLSRPDPALAALTNDHLVALACLGGRPALDA VKKGLGGSGSAIPVKRGATGETKVFTGNSNSPKSPTKGGCSGGSTNLSDIIEKETG KQLVIQESILMLPEEVEEVIGNKPESDILVHTAYDESTDENVMLLTSDAPEYKPWALV IQDSNGENKIKML SEQ ID No: 288 |
| **ND5 silent mutation Left TALE repeat** |
| NLTPDQVVAIASNGGGKQALETVQRLLPVLCQAHGLTPAQVVAIASNGGGKQALET VQRLLPVLCQAHGLTPEQVVAIASNGGGKQALETVQRLLPVLCQAHGLTPDQVVAI ASHDGGKQALETVQRLLPVLCQAHGLTPAQVVAIASHDGGKQALETVQRLLPVLC QDHGLTPAQVVAIASNGGGKQALETVQRLLPVLCQDHGLTPEQVVAIASNIGGKQA LETVQRLLPVLCQAHGLTPDQVVAIASHDGGKQALETVQRLLPVLCQAHGLTPDQV VAIASNGGGKQALETVQRLLPVLCQAHGLTPAQVVAIASNNGGKQALETVQRLLPV LCQDHGLTPAQVVAIASNNGGKQALETVQRLLPVLCQDHGLTPDQVVAIASNGGG KQALETVQRLLPVLCQAHGLTPAQVVAIASHDGGKQALETVQRLLPVLCQDHGLTP DQVVAIASHDGGKQALETVQRLLPVLCQAHGLTPEQVVAIASNNGGKQALETVQRL LPVLCQAHGLTPEQVVAIASNIGGKQALETVQRLLPVLCQAHGLTPDQVVAIASNGG GKQALETVQRLLPVLCQD SEQ ID No: 289 |
| **ND5 silent mutation Right TALE repeat** |
| NLTPEQVVAIASNGGGKQALETVQRLLPVLCQAHGLTPAQVVAIASNIGGKQALETV QRLLPVLCQDHGLTPAQVVAIASNIGGKQALETVQRLLPVLCQAHGLTPDQVVAIAS NIGGKQALETVQRLLPVLCQAHGLTPDQVVAIASNIGGKQALETVQRLLPVLCQDH GLTPDQVVAIASNGGGKQALETVQRLLPVLCQAHGLTPDQVVAIASNIGGKQALET VQRLLPVLCQAHGLTPAQVVAIASNIGGKQALETVQRLLPVLCQDHGLTPAQVVAIA SNIGGKQALETVQRLLPVLCQAHGLTPAQVVAIASNNGGKQALETVQRLLPVLCQD HGLTPEQVVAIASNGGGKQALETVQRLLPVLCQAHGLTPAQVVAIASNGGGKQALE TVQRLLPVLCQDHGLTPAQVVAIASNIGGKQALETVQRLLPVLCQAHGLTPDQVVAI ASNGGGKQALETVQRLLPVLCQAHGLTPDQVVAIASNGGGKQALETVQRLLPVLC QAHGLTPEQVVAIASNIGGKQALETVQRLLPVLCQAHGLTPDQVVAIASNGGGKQA LETVQRLLPVLCQD SEQ ID No: 290 |
| **ND5 point mutation Left TALE repeat** |
| NLTPAQVVAIASNGGGKQALETVQRLLPVLCQAHGLTPDQVVAIASNNGGKQALET VQRLLPVLCQAHGLTPAQVVAIASHDGGKQALETVQRLLPVLCQAHGLTPAQVVAI ASNIGGKQALETVQRLLPVLCQAHGLTPAQVVAIASNNGGKQALETVQRLLPVLCQ DHGLTPAQVVAIASNNGGKQALETVQRLLPVLCQAHGLTPDQVVAIASNGGGKQAL ETVQRLLPVLCQAHGLTPDQVVAIASNIGGKQALETVQRLLPVLCQDHGLTPDQVV AIASNGGGKQALETVQRLLPVLCQAHGLTPEQVVAIASNGGGKQALETVQRLLPVL CQAHGLTPAQVVAIASNIGGKQALETVQRLLPVLCQDHGLTPAQVVAIASNIGGKQA LETVQRLLPVLCQAHGLTPEQVVAIASNGGGKQALETVQRLLPVLCQAHGLTPAQV VAIASNGGGKQALETVQRLLPVLCQAHGLTPDQVVAIASNNGGKQALETVQRLLPV LCQDHGLTPDQVVAIASHDGGKQALETVQRLLPVLCQAHGLTPAQVVAIASNGGG KQALETVQRLLPVLCQD SEQ ID No: 291 |
| **ND5 point mutation Right TALE repeat** |
| NLTPAQVVAIASNGGGKQALETVQRLLPVLCQDHGLTPAQVVAIASHDGGKQALET VQRLLPVLCQAHGLTPEQVVAIASHDGGKQALETVQRLLPVLCQAHGLTPEQVVAI ASNGGGKQALETVQRLLPVLCQAHGLTPAQVVAIASNIGGKQALETVQRLLPVLCQ DHGLTPAQVVAIASNIGGKQALETVQRLLPVLCQAHGLTPEQVVAIASHDGGKQAL ETVQRLLPVLCQAHGLTPAQVVAIASNIGGKQALETVQRLLPVLCQAHGLTPAQVV AIASNNGGKQALETVQRLLPVLCQAHGLTPAQVVAIASNNGGKQALETVQRLLPVL CQDHGLTPAQVVAIASNNGGKQALETVQRLLPVLCQDHGLTPDQVVAIASNGGGK QALETVQRLLPVLCQAHGLTPEQVVAIASNGGGKQALETVQRLLPVLCQAHGLTPD QVVAIASHDGGKQALETVQRLLPVLCQDHGLTPDQVVAIASNGGGKQALETVQRLL PVLCQAHGLTPEQVVAIASNIGGKQALETVQRLLPVLCQAHGLTPAQVVAIASHDG GKQALETVQRLLPVLCQD SEQ ID No: 292 |
| **ND5 nonsense mutation Left TALE repeat** |
| NLTPAQVVAIASNGGGKQALETVQRLLPVLCQDHGLTPAQVVAIASHDGGKQALET VQRLLPVLCQAHGLTPEQVVAIASHDGGKQALETVQRLLPVLCQAHGLTPDQVVAI ASHDGGKQALETVQRLLPVLCQAHGLTPDQVVAIASNGGGKQALETVQRLLPVLC QDHGLTPAQVVAIASNIGGKQALETVQRLLPVLCQDHGLTPDQVVAIASNIGGKQAL ETVQRLLPVLCQAHGLTPDQVVAIASNIGGKQALETVQRLLPVLCQAHGLTPAQVV AIASHDGGKQALETVQRLLPVLCQAHGLTPAQVVAIASNIGGKQALETVQRLLPVLC QDHGLTPAQVVAIASNGGGKQALETVQRLLPVLCQDHGLTPDQVVAIASNIGGKQA LETVQRLLPVLCQDHGLTPDQVVAIASNIGGKQALETVQRLLPVLCQAHGLTPDQV VAIASNIGGKQALETVQRLLPVLCQAHGLTPAQVVAIASHDGGKQALETVQRLLPVL CQAHGLTPAQVVAIASNGGGKQALETVQRLLPVLCQDHGLTPEQVVAIASHDGGK QALETVQRLLPVLCQAHGLTPAQVVAIASNIGGKQALETVQRLLPVLCQD SEQ ID No: 293 |
| |
| **ND5 nonsense mutation Right TALE repeat** |
| NLTPAQVVAIASNGGGKQALETVQRLLPVLCQAHGLTPDQVVAIASNNGGKQALET VQRLLPVLCQAHGLTPAQVVAIASNGGGKQALETVQRLLPVLCQDHGLTPAQVVAI ASNGGGKQALETVQRLLPVLCQAHGLTPDQVVAIASNNGGKQALETVQRLLPVLC QAHGLTPAQVVAIASNGGGKQALETVQRLLPVLCQDHGLTPEQVVAIASNGGGKQ ALETVQRLLPVLCQAHGLTPAQVVAIASNNGGKQALETVQRLLPVLCQDHGLTPDQ VVAIASNNGGKQALETVQRLLPVLCQAHGLTPDQVVAIASNIGGKQALETVQRLLPV LCQDHGLTPAQVVAIASNNGGKQALETVQRLLPVLCQDHGLTPAQVVAIASNIGGK QALETVQRLLPVLCQAHGLTPEQVVAIASNIGGKQALETVQRLLPVLCQAHGLTPD QVVAIASNGGGKQALETVQRLLPVLCQDHGLTPDQVVAIASNIGGKQALETVQRLL PVLCQA SEQ ID No: 294 |

Mitochondrial base editing *in vitro.* In order to attempt mitochondrial DNA editing *in vivo* using the Golden Gate cloning system, the ND5 gene encoding Mus musculus mitochondrial NADH-ubiquinone oxidoreductase chain 5 protein was selected. The ND5 protein is the key subunit of NADH dehydrogenase (ubiquinone) and catalyzes the transfer of electrons from NADH to the respiratory chain. In humans, ND5 gene mutations are known to be associated with MELAS (mitochondrial encephalomyopathy, lactic acidosis, and stroke-like episodes), also with some symptoms of Leigh syndrome or LHON (Leber's hereditary optic neuropathy). Attempts were made to create a mouse model with a genetic alteration in the mitochondrial gene so as to mimic the dysfunction in humans.

First, several DdCBE plasmids were assembled, which were designed to give rise to two silent mutations, m.C12539T and m.G12542A. These plasmids were transfected into the NIH3T3 mouse cell line, and the base editing frequency was measured after 3 days. As expected, cytosine bases in the target range were edited to thymine with an efficiency of up to 19% (FIG. 34a). DddAtox was previously reported to exclusively deaminate only cytosines in the "TC" sequence, but based on experimental results, only two cytosines in the TC context were edited. Indels or other types of point mutations were not significantly generated in the editing target range.

Mitochondrial base editing *in vivo.* The most potent DdCBE pair (left-G1397-N and right-G1397-C) was used for *in-vivo* experiments. Four days after microinjection of *in vitro* transcripts encoding this DdCBE pair into the 1-cell stage of C57BL6/J embryos, 9 out of 32 embryos were successfully edited (28%, Table 6) .

**[Table 6]**

| **Type of mutagenesis** | **Number of examined embryos** | **Number of blastocysts (%)** | **Number of transferred embryos** | **Number of offspring (%)** | **Number of edited/total blastocysts (%)** | **Number of edited/total offspring (%)** |
|---|---|---|---|---|---|---|
| N/A (buffer injection) | 20 | 11 (55) | NA | NA | 0/11 (0) | NA |
| *ND5* silent | 56 | 32 (57) | 30 | 4(13) | 9 (28) | 3 (75) |
| *ND5* G12918A | 79 | 44 (55) | 50 | 11 (22) | 11 (25) | 4 (36) |
| *ND5* STOP | 68 | 37 (54) | 120 | 27(23) | 19 (51) | 9 (33) |

The TALE-DddAtox deaminase efficiently generated C*G-to-T*A base conversions, with efficiencies of 2.2-25% at m.C12539 and 0.63-5.8% at m.G12542. Next, the embryos injected with DdCBE were transferred to surrogate mothers to obtain offspring with m.C12539T and m.G12542T (FIG. 39). Three out of four pups (F0) showed C*G-to-T*A editing, with an efficiency ranging from 1 to 27% (FIG. 34c). The two pups showed similar mutation levels in the toes and tails and maintained the efficiency 14 days after birth. Moreover, these mitochondrial DNA mutations were detected in various tissues of adult F0 mice 50 days after birth (FIG. 34d). These results suggest that the DdCBE-induced heteroplasmy of mitochondrial DNA in 1-cell stage zygotes is maintained during development and differentiation.

In order to determine whether the DdCBE-induced mutations were passed on to the next generation, F1 offspring were generated by mating female F0 mice with wild-type C57BL6/J males. The m.C12539T and m.G12542T mutations were observed with efficiencies of 6-26% in two pups. In addition, similar mitochondrial editing was observed in 11 different tissues (FIG. 35b).

DdCBE-mediated MT-ND5 G12918A mutation. Attempts were made to create the m.G12918A mutation, which also causes mitochondrial diseases in humans. This mutation causes various mitochondrial diseases, such as Leigh syndrome, MELAS syndrome, and LHON syndrome. Since the cytosine base at this position has adjacent thymine, base editing using DdCBE is possible (FIG. 36a). Four pairs of DdCBEs were assembled, confirming that editing was possible with an efficiency of up to 6.4% in NIH3T3 (FIG. 36b). Then, the most efficient DdCBE combination was microinjected into mouse zygotes, and efficiency thereof was observed in the blastocysts. 11 out of 44 embryos (25%) carried the m.G12918A mutation, with an efficiency of 0.25 to 23% (FIG. 36c). In addition, DdCBE-microinjected embryos were transferred to surrogate mothers to obtain offspring with the G12918A mutation (FIG. 39b). It was confirmed that 4 out of 11 newborn mice had this mutation in a range of about 3.9-31.6% (FIG. 36d). Although there was no phenotype immediately after birth, presumably because the offspring are very young and also wild-type mitochondrial DNA and mutant DNA co-exist in a heterozygous state, these results suggest that DdCBE may create an animal model with mitochondrial disease.

MT-ND5 nonsense mutation. Finally, whether the ND5 loss-of-function mutation could be maintained in mice was confirmed by creating a nonsense mutation in genes. Using m.C12336 as the target cytosine, an early stop codon was introduced at position 199 of the ND5 protein (Q199*; FIG. 37a). Specifically, four DdCBE combinations were transfected into the NIH3T3 cell line to confirm base editing efficiency, indicating that the most effective DdCBE pair caused a nonsense mutation with an efficiency of about 5.7% (FIG. 37b). It was confirmed that this DdCBE caused cytosine-to-thymine editing, and also that a mutation (Q200Q) that produces a silent mutation m.G12341A, although slightly less efficient, was edited in the target range. In 19 out of 37 mouse embryos (=51%), the m.C12336T and m.G12341A mutations were confirmed with respective efficiencies of 32% and 23% (FIG. 37c).

Based on such results, mouse embryos were transferred to surrogate mothers to obtain offspring with m.C12336T and m.G12341A mutations (FIG. 39c). 9 out of 27 F0 mice (23%) showed C*G-to-T*A editing, with an efficiency ranging from 0.22 to 57% (FIGs. 37d and 37e), which showed that the nonsense mutation of ND5 did not cause embryo culling.

### Example 4. Mitochondrial DNA editing in animals

### 4-1. Construction of expression vector for base editing of animal mitochondria with a nuclear export signal

A vector (FIG. 40) that expresses a protein with a nuclear export signal fused to TALE-DdCBE in animal cells was constructed. A cytomegalovirus promoter (CMV promoter) was used for the vector. The vector includes a mitochondrial targeting signal, protein purification/detection tag, TALE array N-terminal domain, repeat region, C-terminal domain, DddA cytosine deaminase split half, uracil glycosylase inhibitor, and nuclear export signal (FIG. 40a). For the nuclear export signal, for example, NS2 protein derived from MVM (minute virus of mice) may be used, but other sequences may also be used. The protein expressed thereby is released outside the nucleus and then transferred to the mitochondria and thus subjected to base editing. Here, target DNA sites were chosen in a mitochondrial ND5 gene-chromosome 4 ND5-like gene, a mitochondrial TrnA-chromosome 5, and a mitochondrial Rnr2-chromosome 6 (FIG. 40b) .

### 4-2. DdCBE-NES in animal cell line

The NIH3T3 cell line (ATCC CRL-1658) was dispensed into a 12-well plate containing 1 ml of cell growth medium (DMEM + 10% bovine calf serum) at 1.5×10⁴/well in the afternoon the day before transfection. The next morning, the cells were transfected with experiment groups with DNA-untreated Mock, DdCBE, and DdCBE-MVM NES plasmids added thereto using Lipofectamine 3000 according to the manufacturer's protocol. After culture in an incubator (37°C, 5% CO₂) for three days, the cells were harvested and total DNA was purified using a Qiagen Blood & Tissue Kit, followed by amplification using mitochondrial gene-specific PCR primers and then next-generation sequencing using an Illumina MiniSeq system, after which base editing efficiency was determined using a Cas-analyzer (www.rgenome.net).

FIGs. 40c, 40d, and 40e show mutations in mouse mitochondrial genes ND5, TrnA, and Rnr2 resulting from transfection of the NIH3T3 mouse cell line with DdCBE-NES, indicating that the efficiency varied depending on DdCBE combinations.

### 4-3. mitoTALEN in animal cell line

TALEN recognizing the sequence shown in FIG. 40f was constructed, and MTS was linked to this construct to introduce the same into mitochondria along with DdCBE. The experimental method was the same as in Example 4-2. A mismatch was intentionally given to the TALE recognition site so that 1 mismatch with wild-type mtDNA and 2 mismatches with mutant mtDNA occurred. This is because TALE may not distinguish a 1-nucleotide mismatch. As a result, it was confirmed that the efficiency was further increased in the +2 mismatch experimental group when treated with both DdCBE and TALEN compared to when treated with DdCBE alone.

### 4-4. DdCBE-NES in animal embryos

Using the DdCBE expression vector or the DdCBE-NES expression vector as a template, PCR amplicon containing the T7 promoter and the DdCBE or DdCBE-NES expression site was obtained. Using this PCR amplicon as a template, mRNA was synthesized using T7 polymerase.

A DdCBE mRNA pair or a DdCBE-NES mRNA pair, in a microinjection solution, was microinjected into mouse fertilized eggs.

After fertilized eggs cultured for four days became blastocysts, the blastocysts were lysed. Using the same as a template, a portion of the target site in mitochondrial DNA, which is distinct from nuclear DNA, was PCR-amplified, and then the index and sequencing adapter were amplified through additional PCR. High-throughput sequencing was performed using an Illumina MiniSeq system, after which base editing efficiency was analyzed using a Cas-analyzer (www.rgenome.net). In addition, DNA in the nucleus having a sequence similar to the mitochondrial target region was amplified using PCR and sequenced.

As a result, DdCBE induced mutations not only in mitochondrial DNA but also in a similar DNA sequence in the nucleus (mitochondria: 13.1%, nuclear: 3.20). With DdCBE-NES, the mitochondrial target mutation efficiency was increased to 18.2% while the nuclear DNA mutation efficiency was lowered to 0.2% (FIG. 41a). For other targets, TrnA or Rnr2, nuclear DNA mutation did not occur, whereas, in the mitochondrial DNA, the base editing efficiency was increased statistically significantly (*p<0.05, **p<0.01, n.s.: not significant).

### 4-5: DdCBE and mitoTALEN in animal embryos

TALEN, which cleaves the unedited mitochondrial DNA sequence, was also injected in addition to the ND5 gene-specific DdCBE in order to increase the proportion of the edited mitochondrial DNA in the cells after C-toT conversion. The microinjection method and the sequencing identification method were the same as in Examples 4-2 and 4-4. The group microinjected with DdCBE alone showed an editing efficiency of 11%, and when treated with both DdCBE and mitoTALEN, the efficiency was increased to 33.3%, resulting in a statistically significant increase in editing efficiency. In addition, the group microinjected with DdCBE-NES alone showed an editing efficiency of 20.5%, and when treated with both DdCBE-NES and mitoTALEN, an efficiency of 36.8% was observed, which was also statistically significant (FIG. 41b).

Likewise, DdCBE showed an editing efficiency of 10.9% in newly born mouse pups by transferring microinjected fertilized eggs into surrogate mothers, but an efficiency of 23.4% was obtained when both DdCBE-NES and mitoTALEN were used (FIG. 41c).

When the nuclear export signal was attached to the base editing protein during animal mitochondrial gene editing, base editing was achieved with higher efficiency, and in animal embryos, non-specific base editing of the similar sequence in the nucleus was also suppressed. In addition, higher efficiency of mitochondrial base editing can be expected when the mitochondrial sequence cleavage protein simultaneously was co-injected.

### Example 5. Split-DddAₜₒₓ deaminase variant

A high-precision DddA-derived cytosine base editor capable of reducing the off-target effect of DdCBE was provided. This off-target base editing effect is a phenomenon caused by spontaneous assembly of the DddAtox deaminase splits independent of the interaction between TALE and DNA. Therefore, HF-DdCBE was constructed by substituting an amino acid residue located on the surface between DddAtox splits with alanine. HF-DdCBE prevented a pair of two deaminases linked to TALE from functioning properly when not bound to DNA. Through whole mitochondrial genome analysis, it was confirmed that HF-DdCBE was very efficient and precise, unlike conventional DdCBE that causes numerous unwanted off-target C-to-T conversions in human mitochondrial DNA.

### 5-1. Methods

Plasmid construction. A point mutation was introduced into the DdCBE expression plasmid. Plasmids were amplified using mutagenesis primers for Q5 Site-Directed Mutagenesis (NEB) (Table 7), and the results thereof were confirmed by Sanger sequencing.

**[Table 7]**

| **Name** | **Sequence** | **No.** |
|---|---|---|
| Left-G1333-N_F1315A_F | AGTGGGGACCGCATACTATGTCAACGAC | 295 |
| Left-G1333-N_F1315A_R | GTCTGACCGTTGTAGGCG | 296 |
| Left-G1333-N_F1329A_F | ATCCAAGGTTGCATCTAGCGGTGGGTCCGG | 297 |
| Left-G1333-N_F1329A_R | TCCAATCCCCCGGCGTCG | 298 |
| Left-G1333-N_G1313A_F | CAGACAGTGGCAACCTTTTACTATGTCAACGAC | 299 |
| Left-G1333-N_G1313A_R | ACCGTTGTAGGCGGGGAG | 300 |
| Left-G1333-N_I1299A_F | TCCATATCAGGCAAGTGCTCCCCAACTCCC | 341 |
| Left-G1333-N_I1299A_R | CCCAGGGCGTAGGAACCA | 302 |
| Left-G1333-N_K1327A_F | ATTGGAATCCGCAGTTTTCTCTAGCGGTGGGTCCGG | 303 |
| Left-G1333-N_K1327A_R | CCCCCGGCGTCGTTGACA | 304 |
| Left-G1333-N_L1294A_F | CTCTGGTFCGTACGCCGCAGGTCCATATCAGATTAG | 305 |
| Left-G1333-N_L1294A_R | CTAATCTGATATGGACCTGCGGCGTAGGAACCAGAG | 306 |
| Left-G1333-N_S1300A_F | ATATCAGATTGCAGCTCCCCAACTCCCCGC | 307 |
| Left-G1333-N_S1300A_R | GGACCCAGGGCGTACGGAA | 308 |
| Left-G1333-N_S1330A_F | CAAGGTTTTCGCAAGCGGTGGGTC | 309 |
| Left-69333-N_S1330A_R | GATTCCAATCCCCCGGCG | 310 |
| Left-G1333-N_T1314A_F | GGTCAGACAGTGGGGGCATTTTACTATGTC | 311 |
| Left-G1333-N_T1314A_R | GACATAGTAAAATGCCCCCACTGTCTGACC | 312 |
| Left-G1333-N_V1312A_F | GGTCAGACAGCAGGGACCTTTTACTATG | 310 |
| Left-G1333-N_V1312A_R | GTTGTAGGCGGGGAGTTG | 314 |
| Left-G1333-N_V1318A_F | TTTTACTATGCAAACGACGCCGGGG | 315 |
| Left-G1333-N_V1318A_R | GGTCCCCACTGTCTGACC | 316 |
| Left-G1333-N_Y1292A_F | CTCTGGTTCCGCAGCCCTGGGTCCATATCAGATTAGTGCTCC | 317 |
| Left-G1333-N_Y1292A_R | CCAGAGCCCCCCAACCCC | 318 |
| Left-G1333-N_Y1316A_F | CAGTGGGGACCTTTGCATATGTCAACGACGC | 319 |
| Left-G1333-N_Y1316A_R | GCGTCGTTGACATATGCAAAGGTCCCCACTG | 320 |
| Left-G1333-N_Y1317A_F | GACCTTTTACGCAGTCAACGACGCCGGG | 321 |
| Left-G1333-N_Y1317A_R | CCCACTGTCTGACCGTTG | 322 |
| Left-G139T-N_C1376A_F | TGTGGCTTCGCAGTGAATATGACTGAAACCCTTCTG | 323 |
| Left-G1397-N_C1376A_R | CATATTCACTGCGAAGCCACAAGTACCCTCTG | 324 |
| Left-G1397-N_E1381A_F | GTGTGAATATGACTGCAACCCTTCTGCCCGAAAATG | 325 |
| Left-G1397-N_E1381A_R | CATTTTCGGGCAGAAGGGTTGCAGTCATATTCACAC | 326 |
| Left-G1397-N_K1389A_F | GAAAATGCCGCAATGACTGTCGTCCCACCTGAAGG | 327 |
| Left-G1397-N_K1389A_R | GACAGTCATTGCGGCATTTTCGGGCAGAAGG | 328 |
| Left-G1397-N_L1384A_F | GAAACCCTTGCACCCGAAAATGCCAAGATGAC | 329 |
| Left-G1397-N_L1384A_R | ATTTTCGGGTGCAAGGGTTTCAGTCATATTCACACAGAAG | 330 |
| Left-G1397-N_M1390A_F | AATGCCAAGGCAACTGTCGTCCCACCTGAAGG | 331 |
| Left-G1397-N_M1390A_R | GACGACAGTTGCCTTGGCATTTTCGGGCAGAAGG | 332 |
| Left-G1397-N_T1380A_F | GTGAATATGGCAGAAACCCTTCTGCCCGAAAATG | 333 |
| Left-G1397-N_T1380A_R | AAGGGTTTCTGCCATATTCACACAGAAGCCACAAG | 334 |
| Left-G1397-N_T1391A_F | CGAAAATGCCAAGATGGCAGTCGTCCCACCTGAAG | 335 |
| Left-G1397-N_T1391A_R | CTTCAGGTGGGACGACTGCCATCTTGGCATTTTCG | 336 |
| Left-G1397-N_V1377A_F | GGTACTTGTGGCTTCTGTGCAAATATGACTGAAACC | 337 |
| Left-G1397-N_V1377A_R | GGTTTCAGTCATATTTGCACAGAAGCCACAAGTACC | 338 |
| Left-G1397-N_V1392A_F | GAAAATGCCAAGATGACTGCAGTCCCACCTGAAGG | 339 |
| Left-G1397-N_V1392A_R | CCTTCAGGTGGGACTGCAGTCATCTTGGCATTTTC | 340 |
| Righl-G1333-C_E1361A_F | GGCATTAGCGCAGGGCTTGTGTTCCATAATAATCC | 341 |
| Right-G1333-C_E1361A_R | CACAAGCCCTGCGCTAATGCCGTTATCTCTCATAAAAAGAGC | 342 |
| Right-G1333-C_F1353A_F | GTCAGCTCTTGCAATGAGAGATAACGGCATTAGCGAAGGG | 343 |
| Right-G1333-C_F1353A_R | TGCCCCTCGACGTGCCCG | 344 |
| Right-G1333-C_F1365A_F | GGGCTTGT6GCACATAATAATCCTGAGGGCAGCTG | 345 |
| Right-G1333-C_F1365A_R | GATTATTATGTGCCACAAGCCCTTCGCTAATG | 346 |
| Right-G1333-C_G1362A_F | CATTAGCGAAGCACTTGTGTTCC | 347 |
| Right-G1333-C_G1362A_R | CCGTTATCTCTCATAAAAAG | 348 |
| Right-G1333-C_H1366A_F | GCTTGTGTTCGCAAATAATCCTGAGGGCAC | 349 |
| Right-G1333-C_H1366A_R | CCTTCGCTAATGCCGTTA | 350 |
| Right-G1333-C_K1389A_F | TGAGAACGCTGCAATGACTGTCG | 351 |
| Right-G1333-C_K1389A_R | GGCAGAAGTGTTTCGGTC | 352 |
| Right-G1333-C_L1363A_F | TAGCGAAGGGGCAGTGTTCCATAATAATCCTGAGGGC | 353 |
| Right-G1333-C_L1363A_R | ATGGAACACTGCCCCTTCGCTAATGCCGTTATCTC | 354 |
| Right-G1333-C_M1354A_F | AGCTCTTTTTGCAAGAGATAACGGCATTAGCGAAGG | 355 |
| Right-G1333-G_M1354A_R | GACTGCCCCTCGACGTGC | 356 |
| Right-G1333-C_M1390A_F | GAACGCTAAAGCAACTGTCGTACCAC | 357 |
| Right-G1333-C_M1390A_R | TCAGGCAGAAGTGTTTCG | 358 |
| Right-G1333-C_Q1349A_F | CGTCGAGGGGGCATCAGCTCTTTTTATGAGAGATAACGGC | 359 |
| Right-G1333-C_Q1349A_R | TGCCCGGCGTTAGCGTAG | 360 |
| Right-G1333-C_S1350A_F | CGAGGGGCAGGCAGCTCTTTTTATG | 361 |
| Right-G1333-C_S1350A_R | ACGTGCCCGGCGTTAGCG | 362 |
| Right-G1333-C_T1391A_F | CGCTAAAATGGCAGTCGTACCAC | 363 |
| Right-G1333-C_T1391A_R | TTCTCAGGCAGAAGTGTTTC | 364 |
| | | |
| Right-G1333-C_V1346A_F | GCCGGGCACGCAGAGGGGCAGT | 365 |
| Right-G1333-C_V1346A_R | GTTAGCGTAGTTAGGATAAGGTGTTG | 366 |
| Right-G1333-C_V1364A_F | CGAAGGGCTTGCATTCCATAATAATCC | 367 |
| Right-G1333-C_V1364A_R | CTAATGCCGTTATCTCTC | 368 |
| Right-G1333-C_V1393A_F | AATGACTGTCGCACCACCCGAAG | 369 |
| Right-G1333-C_V1393A_R | TTAGCGTTCTCAGGCAGAAG | 370 |
| Right-G1397-C_F1412A_F | ACAAAGGTGGCAACAGGCAACTCTAACAGTCCAAAG | 371 |
| Right-G1397-C_F1412A_R | GTTGCCTGTTGCCACCTTTGTCTCCCCTGTTGCTCC | 372 |
| Right-G1397-C_K1410A_F | CAACAGGGGAGACAGCAGTGTTCACAGGCAA | 373 |
| Right-G1397-C_K1410A_R | TTGCCTGTGAACACTGCTGTCTCCCCTGTTG | 374 |
| Right-G1397-C_T1413A_F | GGAGACAAAGGTGTTCGCAGGCAACTCTAACAGTCC | 375 |
| Right-G1397-C_T1413A_R | GGACTGTTAGAGTTGCCTGCGAACACCTTTGTCTCC | 316 |
| Right-G1397-C_V1411A_F | GAGACAAAGGCATTCACAGGCAACTCTAACAGTC | 377 |
| Right-G397-C_V1411A_R | GCCTGTGAATGCCTTTGTCTCCCCTGTTGCTCC | 378 |

For assembly of interface mutants, miniprepped mutant expression plasmids were mixed with module vectors (each encoding a TALE sequence), BsaI-HFv2 (10 U), T4 DNA ligase (200 U), and reaction buffer in one tube. Thereafter, restriction enzyme and ligase reaction was carried out in a thermocycler for 20 cycles of 5 minutes at 37°C and 20 minutes at 50°C, followed by further reaction for 15 minutes at 50°C and 5 minutes at 80°C. The ligated plasmids were introduced into *E. coli* DH5α by chemical transformation, and the final construct was identified by Sanger sequencing. For introduction into cell lines, plasmids were midiprepped.

Mammalian cell line culture and transfection. The HEK 293T/17 (CRL-11268, American Type Culture Collection (ATCC)) cell line was cultured at 37°C in a 5% CO₂ environment. The cell line grew without antibiotics in DMEM supplemented with 10% (v/v) fetal bovine serum (Gibco) and was not tested for mycoplasma. For lipofection, growth of the cells started 18-24 hours before transfection at a cell density of 1×10⁵ in 24-well cell culture plates (SPL, Seoul, Korea). A total of 1,000 ng of plasmid DNA was introduced using 500 ng of each DdCBE split using Lipofectamine 2000 (Invitrogen). The cells were harvested 4 days after transfection.

Genomic and mitochondrial DNA isolation for high-throughput sequencing. After removing the cell culture medium to isolate genomic DNA, lysis buffer containing Proteinase K of a DNeasy Blood & Tissue Kit (Qiagen) was added to a cell culture plate in order to separate the cells from the bottom of the plate. Genomic DNA was then isolated according to the manufacturer's protocol. For whole mitochondrial genome sequencing, 200 pl of mitochondrial isolation buffer A (ScienCell) was added to the culture plate from which the cell culture medium was removed. The cells were scraped using a cell lifter and then placed in a microtube, followed by cell grinding using a disposable pestle. After grinding 20 times, the well-ground homogenate was centrifuged at 1,000x g and 4°C for 5 minutes. The supernatant was placed in a new microtube and centrifuged at 10,000x g and 4°C for 20 minutes. The precipitate was resuspended in 10 pl of lysis buffer (25 mM NaOH, 0.2 mM EDTA, pH 10) and then boiled at 95°C for 20 minutes. In order to lower the pH, 1 pl of 1 M HEPES (free acid, without pH adjustment) was added to the mitochondrial lysate. 1 pl of the solution thus prepared was used in a PCR template strand for high-throughput sequencing.

High-throughput sequencing. In order to construct a deep sequencing library, nested primary PCR and secondary PCR were performed using Q5 DNA polymerase, and a final index sequence was added. The library was employed in paired-end read sequencing using a MiniSeq (Illumina). For whole mitochondrial genome analysis, isolated mitochondrial DNA was prepared using a tagmentation DNA prep kit (Illumina) according to the manufacturer's protocol. Paired-end sequencing results from all analyses were merged using one fastq-join file and analyzed using CRISPR RGEN Tools (http://www.rgenome.net/) .

### 5-2. Results

When chloroplast editing was attempted in plants, off-target base mutations appeared on the chloroplast genome, raising questions about the accuracy of DdCBE. There were two reasons for off-target base editing of DdCBE. The first was non-specific binding between TALE protein and DNA, and the second was unintentional, spontaneous interaction between DddAtox halves (FIG. 42a) . This study focused on the split-DddAtox halves and engineered the interface of the two protein splits to prevent unwanted assembly of the DddAtox halves.

Specifically, we examined whether each subunit (left-TALE or right-TALE) targeting the mitochondrial ND1 (mtND1) gene binds to DNA and interacts with the other half of TALE-free DddAtox to cause cytosine-to-thymine base editing. A DdCBE pair (left-TALE:G1397N (the N-terminal G1397 DddAtox half fused to the C-terminus of the left-TALE array recognizing and the left half site) + right-TALE:G1397C (the C-terminal G1397 DddAtox half fused to the C-terminus of the right-TALE array reconizing the right half site)) targeting the human mitochondrial ND1 (*mtND1*) gene in the human kidney embryonic cell line (HEK293T) effectively edited C11 of the target sequence, converting cytosine to thymine with an efficiency of 60.7% (FIG. 43a) . Moreover, **each subunit alone (Left-TALE:G1397N or Right-TALE-G1397C), paired with the other TALE-free DddAₜₒₓ half, also induced base editing, albeit less efficiently than did the original pair.** Thus, each of left-TALE and right-TALE bound to the target ND1 sequence, paired with the other TALE-free DddAtox half induced base editing with an efficiency of 31% or 8.1% (FIG. 43) . **In other words, the DdCBE pair with two TALE fusions was more efficient than unmatched pairs with only one TALE fusion by merely 2.0-fold (60.7%/31%) or 7.5-fold (60.7%/8.1%). Apparently, the DddAₜₒₓ N-terminal moiety fused to a TALE array bound to a half-site can recruit the DddAₜₒₓ C-terminal moiety with no TALE array or vice versa to reconstitute a functional deaminase.**

Since DddAtox can be split at two positions (G1333 and G1397), a DdCBE pair targeting the mtND1 gene at position G1333 was also constructed (left-TALE:G1333-N and right-TALE:G1333-C) to test whether the left-TALE:G1333-N and right-TALE:G1333-C constructs were able to recruit a TALE-free DddAtox half and to induce C-to-T editing. As expected, each TALE fusion, paired with the other TALE-fee DddAtox half, showed a base editing efficiency of 32.7% (left TALE conjugate) or 18.1% (right TALE conjugate) at position C8, compared to 56.1% for the original DdCBE pair. Thus, **Thus, the original pair with two TALE fusions was more efficient than the unmatched pairs with one TALE fusion by merely 1.7-fold (56.1%/32.7%) or 3.1-fold (56.1%/18.1%). Taken together, these results suggest that DdCBEs can cause unwanted off-target mutations at sites where only one TALE array can bind. Because TALE proteins can bind to sites with a few mismatches, DdCBE pairs probably induce many off-target mutations in the organelle or nuclear genome.**

**We sought to develop high fidelity-DdCBEs that would not exhibit such off-target editing caused by spontaneous assembly of the split DddAₜₒₓ halves. We reasoned that the split dimer interface could be engineered to inhibit or prevent self-assembly.** To this end, we used a Python script (InterfaceResidues.py) in PyMOL software to identify amino acid residues in the interface of the two splits DddAtox (split at G1333 and G1397) within a range of 1 square angstrom. As a result, we found 9 amino acid residues in G1397-N (which is the N-terminal DddAtox half split at position G1397), 4 residues in G1397-C (which is the C-terminal DddAtox half split at position G1397), 14 amino acid residues in G1333-N (which is the N-terminal DddAtox half split at position G1333), and 15 amino acid residues in G1333-C (which is the C-terminal DddAtox half split at position G1333) (FIGs. 42b and 42c).

Subsequently, we created various mutant DddAtox halves by substituting each of these amino acid residues with alanine. **Then we measured the editing frequencies of these interface mutant DdCBEs in combination with a wild-type DdCBE partner or a TALE-free DddAₜₒₓ half in HEK293T cells. Many G1397-split DddAₜₒₓ variants, containing interface mutations such as C1376A, M1390A and F1412A, failed to induce C-to-T conversions in the spacer region between the two TALE-binding sites, even when combined with the wild-type partner, suggesting that these mutants cannot interact with other wild-type DddAₜₒₓ half nearby at the target site. Other DddAₜₒₓ variants, such as those containing V1377A and E1381A, induced C-to-T edits at high frequencies in partnership with the TALE-free half, comparable to the wild-type DdCBE pair, showing that these mutations are neutral and do not prevent split dimer interactions.**

Importantly, several mutations, such as K1389A, K1410A, and T1413A, showed high activity when paired with the wild-type DdCBE partner, but low activity when paired with the TALE-free half. For example, the K1410A mutation showed an efficiency of 53.2%, which was similar to that when paired with the wild-type DdCBE partner (60.7%), but showed an efficiency of 0.9% when paired with the TALE-free half, resulting in a 59.1-fold difference (= 53.2%/0.9%). As described above, the wild-type pair showed a 7.5-fold (= 60.7%/8.1%) difference. In addition, these variants edited bases more selectively than the wild-type DdCBE pair. **Thus, these variants edited C₁₁ preferentially over C₈, C₉ and C₁₃ in the editing window, whereas the wild-type DdCBE pair was much less discriminatory, editing all four cytosines with high frequencies of >6.7%** (FIG. 43b).

In addition, screening of 29 mutations at G1333 (14 mutations in G1333N and 15 mutations in G1333C) yielded several desirable interface mutations (FIG. 44). **Variants containing most of these mutations (such as I1299A, Y1316A, Y1317A and F1329A) were either poorly active even in combination with the wild-type partner or, in the case of other mutations (such as S1300A and T1314A), undesirably active in combination with the TALE-free partner. Notably, variants containing several mutations, including K1389A, T1391A and V1393A, were highly active when paired with the wild-type partner but inefficient when paired with the TALE-free partner.** For example, K1389A showed a 38-fold difference (= 45.4%/1.2%), whereas the wild-type DdCBE pair showed only a 3.1-fold difference (= 56.1%/18.1%). **Furthermore, the pair containing the K1389A variant was more selective than the wild-type pair. Thus, this variant edited C₈ preferentially over C₉, C₁₁ and C₁₃, whereas the wild-type pair was promiscuous, editing all four cytosines with high frequencies of >19%.** Also noteworthy was that K1410A in G1397-C preferentially edited C8, whereas K1389A in G1333-C selectively edited C11 with higher efficiency. In contrast, the wild-type DdCBE pairs (G1333 or G1397 DddAₜₒₓ splits) showed poor selectivity. These results show that the interface mutants described above have the potential to reduce unwanted editing of multiple bases within the target site, often observed with DdCBE.

### Example 6. Full-length deaminase

The DddA-derived cytosine base editor (DdCBE), composed of a split interbacterial toxin DddAtox, a TALE array, and a uracil glycosylase inhibitor (UGI), enables target cytosine in eukaryotic nuclear DNA, mitochondrial DNA (mtDNA), and plant chloroplast DNA to be converted to thymine. DddAtox, which is toxic to bacteria, is an enzyme derived from *Burkholderia cenocepacia* and deaminates cytosine in double-stranded DNA. In order to avoid host-cell toxicity, DddAtox is split into inactive halves, each fused to the TALE DNA-binding protein to form a DdCBE pair. **A functional deaminase is reconstituted only when the two inactive halves are brought together on target DNA by two adjacently bound TALE proteins. C-to-T base conversions are induced in a spacer region of 14-18 base pairs (bp)** between the two TALE binding sites.

Unlike CRISPR-derived base editors that cannot edit organelle DNA, DdCBE enables targeted base editing in both nuclear and organelle DNA, but has the disadvantage of requiring two TALE constructs, rather than one construct, to induce such editing. The first drawback is that TALE has to bind to the target DNA site with thymine at both the 5' and 3' ends, so that the use of two TALE arrays limits the targetable sites. Second, the delivery of two TALE constructs instead of one is often inefficient and challenging. The viral vectors with limited capacities, such as adeno-associated virus (AAV) vectors (capacity: about 4.7 kbps) widely used in gene therapy, cannot accommodate split DdCBE-encoding sequences because the dimeric DdCBE combination is too large (2x4.1 kbps, including promoter and polyA signal). Furthermore, cloning two TALE array-encoding DNA segments into a single larger-capacity vector may become difficult due to high similarity of the two TALE array sequences. Lastly, using two TALE arrays instead of one may exacerbate the off-target effect. In order to overcome these limitations of dimeric DdCBEs with DddAtox splits, **we present non-toxic, full-length DddAₜₒₓ-fused DdCBEs, termed mDdCBEs (monomeric DdCBEs), for targeted C-to-T conversions in nuclear and organelle DNA.**

### 6-1. Methods

Plasmid construction. A DddA variant was amplified by PCR using the synthesized full-length DddAtox (gBlock, IDT) as a template and using the primers in Table 8 below and Q5 DNA polymerase (NEB). These PCR products were cloned using Gibson assembly (NEB) at the p3s-BE3 site where Apobec1 was digested with BamHI and Sma I (NEB). TALE-DddAtox (Addgene #158093, #158095, #157842, #157841) digested the plasmid with BamHI and Sma I, and the DddA variant was amplified by PCR using the primers in Table 8 and then cloned using Gibson assembly. The plasmid thus obtained was transformed into chemically prepared *E. coli* DH5α by a heat shock method, and the plasmid sequence of the surviving colony was analyzed by a Sanger sequencing method. The final plasmids were midiprepped (Macherey-Nagel) for cell transfection.

**[Table 8]**

| Name | Sequence | No. |
|---|---|---|
| DddAF | cccaagcttgccaccatgggatccggcagctacgccctgggtccgtatcag | 380 |
| DddAR | cccgggagtctcgctgccgctgcaaccgcctttggtcgggctcttcggg | 381 |
| K1402AR | cccgggagtctcgctgccgctgcaaccgcctttggtcgggctcttcgggctgttgctgttaccggtaaacac tttggtttcaccggtagcgccgcgCGCcactggaatgg | 382 |
| R1403A R | cccgggagtctcgctgccgctgcaaccgcctttggtcgggctcttcgggctgttgctgttaccggtaaacac tttggtttcaccggtagcgccGGCcttcttcactgg | 383 |
| K1402A R1403A R | cccgggagtctcgctgccgctgcaaccgcctttggtcgggctcttcgggctgttgctgttaccggtaaacac tttggtttcaccggtagcgccGGCCGCcactggaatgg | 384 |
| K1410AR | cccgggagtctcgctgccgctgcaaccgcctttggtcgggctcttcgggctgttgctgttaccggtaaacac GGCggtttcacc | 385 |
| K1420AR | cccgggagtctcgctgccgctgcaaccgcctttggtcgggctGGCcgggctgttgctg | 386 |
| K1424AR | cccgggagtctcgctgccgctgcaaccgccGGCggtcgggctcttggg | 387 |
| E1347 F | ctatgccaatgccggtcatgtgGCCggtcagagcgccctgttcatg | 388 |
| E1347 R | catgaacagggcgctctgaccGGCcacatgaccggcattggcatag | 389 |
| error_prone PCRF | tgagccagctgggcggcgacagcggcagcgagactcccgggacctcagagtccgccacacccgaaag tggcagctacgccctgggtcc | 394 |
| error_prone PCRR | aatagggccctctagatgcatgctcgagttagcaaccgcctttggtcggg | 391 |
| TAIL Right DddAF | tgaagaaaggtctgggcggatccggcagctacgcctgggtccgtatcag | 392 |
| TAIL_Left_D ddA_F | tgaagaaaggcctgggtggatccggcagctacgcctgggtccgtatcag | 393 |
| TAIL DddA R | tcagattagttgagccgccagagcaaccgcctttggtcgggctcttcggg | 394 |
| TAIL UGI F | tctggcggctcaactaatctgagcgacatcattg | 395 |
| TAIL UGI R | cagatccgaaaatggatatacaagctccc | 396 |

Random mutagenesis. Error-prone PCR was performed using the synthesized full-length DddAtox (gBlock, IDT) as a template by use of a GeneMorph II Random mutagenesis kit (Agilent) according to the manufacturer's protocol. In summary, random mutations of 0-16 mutations/kb were introduced using 1 ng, 100 ng, and 700 ng of DddAtox DNAs as templates. The full-length DddAtox gBlock was amplified by PCR in advance using the primers in Table 8. All PCR products were combined and cloned into p3s-UGI-Cas9 (H840A) digested with Sma1 and Xho1 using Gibson assembly (NEB). Chemically prepared *E. coli* DH5α was transformed with the plasmid by a heat shock method, and the plasmid sequence of the surviving colony was analyzed by Sanger sequencing. Among the analyzed plasmids, the p3s-UGI-nCas9(H840A)-DddAtox plasmid having a coding frame was transfected along with sgRNA into HEK293T cells, and editing activity was then determined by targeted deep sequencing.

Mammalian cell culture and transfection. HEK293T (ATCC, CRL-11268) cells and HeLa (ATCC, CCL-2) cells were cultured at 37°C in 5% CO₂. The cells were cultured in DMEM supplemented with 10% (v/v) fetal bovine serum (Welgene) and 1% penicillin/streptomycin (Welgene). The cells were seeded into 48-well plates (Corning) at densities of 3×10⁵ cells (HEK293T) and 4×10⁴ cells (HeLa) 24 hours before transfection, and then transfected with a Cas9-fused DddA plasmid (750 ng) and sgRNA (250 ng) using Lipofectamine 2000 (Invitrogen). TALE-DddA was transfected into HEK293T cells using 200 ng of the plasmid and Lipofectamine 2000. Here, sgRNA sequences are shown in Table 9 below.

**[Table 9]**

| Name | sgRNA sequence 5' to 3' | No. |
|---|---|---|
| HEK3 | GGCCCAGACTGAGCACGTGA | 397 |
| TYRO3 | GGCCACACTAGCGTTGCTGC | 398 |
| ROR1 site1 | GCCATAGATGGTGGACCGAA | 399 |
| ROR1 site2 | CCATCTATGGCTCTCGGCTG | 400 |
| ROR1 site3 | CCGCAGCCGAGAGCCATAGA | 401 |
| FANCF | GGAATCCCTTCTGCAGCACC | 402 |
| HBB | CTTGCCCCACAGGGCAGTAA | 403 |
| EMX1-1 | TGCCCCTCCCTCCCTGGCCC | 404 |
| EMX1-2 | CCCTGGCCCAGGTGAAGGTG | 405 |
| EMX1-3 | GTGAAGGTGTGGTTCCAGAAC | 406 |
| EMX1-4 | AAAGTACAAACGGCAGAAGC | 407 |
| TRAC5 site1 | GTGGTAGCGGAACTCACTAAG | 408 |
| TRAC5 site2 | CACCCAGCCTGCTCTGCCTT | 409 |

Genomic and mitochondrial DNA preparation. The cells transfected with the Cas9-fused DddA variant were harvested 2 days after transfection, and the cells transfected with TALE-DddA were harvested 3 days after transfection. Genomic and mitochondrial DNAs were isolated using a DNeasy Blood and Tissue Kit (Qiagen). For large-scale analysis, DNA was extracted using 100 pl of cell lysis buffer containing 5 µl of proteinase K (Qiagen) (50 mM Tris-HCl, pH 8.0 (Sigma-Aldrich), 1 mM EDTA (Sigma-Aldrich), 0.005% sodium dodecyl sulfate (Sigma-Aldrich)). The lysate was allowed to react at 55°C for 1 hour and then at 95°C for 10 minutes.

### 6-2. Results

The amino acid sequences of the wild-type and new full-length DddA variants were compared. The altered amino acids were indicated as gray boxes in FIG. 45.

As shown in FIG. 46, DddA was linked upstream of the N-terminus of Cas9 using a linker composed of 16 amino acids, whereas UGI (uracil glycosylase inhibitor) and NLS (nuclear localization signal) were linked to the C-terminus using a linker composed of 4 amino acids. Conversely, DddA was linked downstream of the C-terminus of Cas9 using a linker composed of 16 amino acids, wheras UGI and NLS were linked to the N-terminus using a linker composed of 4 amino acids.

In the present invention, we constructed and used DddA-Cas9(D10A, D10A, and H840A)-UGI. A full-length, single DddA module fused to a zinc finger protein or a TALE module enables cytosine-to-thymine editig. Current split systems require two modules, but full-length DddA requires only one module. These two DNA-binding proteins can be linked to NLS (nuclear localization signal), MTS (mitochondrial targeting sequence), or CTP (chloroplast transit peptide), making it possible to substitute cytosine with thymine not only in the nuclear genome, but also in mitochondrial and plant chloroplast genomes, which cannot be edited using Cas9. As shown in FIG. 47, in the human cell genomic context ROR1 site (a), HEK3 site (b), and TYRO3 site (c), the activity of substituting cytosine in the TC motif with thymine was confirmed. The activity of substituting cytosine in the TC motif 25 bps away from the target position with thymine was confirmed (a). For A1341D KRKKA, the activity of substituting the second cytosine in the CC motif with thymine was confirmed (a, b). For E1347A, which is a catalytic mutant, the activity of substituting cytosine in the TC motif with thymine was also confirmed (a, b, c). The red underline indicates the binding site of Cas9. The efficiency was represented as a percentage of cytosine-to-thymine conversion in indel-free reads among the total sequencing reads. Moreover, the ratio of indels among the total sequencing reads was represented as a percentage.

As shown in FIG. 48, the red square box indicates the part where the activity of split DddAtox was confirmed, and the activity was measured by dividing the same part into three target sites using full-length DddA. For splits, cytosine between two Cas9s is converted to thymine using orthogonal Cas9 with a different PAM. As such, exact substitution of the desired cytosine with thymine is difficult. However, for full-length DddA, the part where Cas9 binds to the same target site may be divided into three and targeted, enabling precise substitution of the desired cytosine with thymine. The efficiency was represented as a percentage of cytosine-to-thymine conversion in indel-free reads among the total sequencing reads. Also, the ratio of indels among the total sequencing reads was represented as a percentage.

As shown in FIG. 49, the activity of full-length DddA was measured in the human cell genomic context TRAC site 1 (a), TRAC site 2 (b), FANCF (c), and HBB (d). The red underline indicates the binding site of Cas9. The efficiency was represented as a percentage of cytosine-to-thymine conversion in indel-free reads among the total sequencing reads. Also, the ratio of indels among the total sequencing reads was represented as a percentage.

As shown in FIG. 50, the activity of DddA was measured in the human cell genomic context TYRO3 (a), ROR1 (b), HEK3 (c), EMX1 site 2 (d), TRAC site 1 (e), and HBB (f) using DddA-dCas9(D10A, H840A)-UGI. The efficiency was represented as a percentage of cytosine-to-thymine conversion among the total sequencing reads. Indels were not observed.

In order to obtain a non-toxic full-length DddAtox variant useful for base editing, two methods were used: structure-based site-specific mutagenesis and random mutagenesis. In the first approach, a DddAtox variant with reduced DNA binding or lowered catalytic activity was fused to an inactive CRISPR-Cas9 (dCas9) or nickase (nCas9) variant to develop a new base editor in which target cytosine was substituted with thymine in cultured human cells. To this end, the positively charged amino acid of DddAtox was substituted with alanine and subcloned into an expression vector (FIG. 51a). It was assumed that these variants could potentially avoid toxicity by attenuating binding to the negatively charged dsDNA. Most alanine-substituted variants failed to form *E. coli* transformants (FIG. 51b). Based on sequencing analysis of plasmid DNA isolated from the resulting transformants, various frameshift mutations were induced in the protein-coding region. This full-length DddAtox variant, although under the control of a mammalian promoter, was weakly expressed in *E. coli,* resulting in cell death. Fortunately, it was possible to obtain several triple, quadruple, or quintuple (referred to as "AAAAA") alanine-substituted variants without frameshift mutations. The active site mutation E1347A was also successfully cloned.

In addition, we investigated whether the AAAAA variant fused to D10A nCas9 or dCas9 and UGI could induce base editing in human embryonic kidney 293T (HEK293T) cells (FIGs. 51c and 51d). The base editor 2 (or 3) composed of rat APBEC1 deaminase, uracil glycosylase inhibitor (UGI), and dCas9 (or D10A nCas9) was active in a narrow region within the protospacer, whereas the AAAAA variant induced cytosine-to-thymine conversion with an efficiency of up to 43% 5' upstream of the protospacer. Unexpectedly, the E1347A mutation induced base editing at the same C-3 position at a frequency of 37% (nCas9 fusion) or 16% (dCas9 fusion) (FIG. 51c), **which suggests that the E1347A mutation did not completely inactivate the deaminase activity of DddAₜₒₓ and that the residual deaminase activity of the E1347A mutant was high enough to achieve base editing in human cells.** However, the E1347A variant bound to the quintuple AAAAAA mutation failed to induce base editing. In addition, it was confirmed that E1347A, AAAAA, and other alanine-substituted variants (FIG. 53), without frameshift mutations fused to dCas9 or nCas9 and UGI, induced editing at positions a maximum of 25 bases away from upstream of the protospacer, and also exhibited an editing efficiency of up to 26% in a variety of different sites (FIG. 54). Also, editing was very efficient in HeLa cells at a frequency of up to 60% (FIG. 55). Base editing induced by the fusion protein was maintained in cells for up to 21 days, suggesting that such base editing is not cytotoxic (FIG. 56).

In order to change the editing window of the cytosine base editor, attempts were made to fuse the alanine-substituted variants to the C-terminus of H840A nCas9. Unexpectedly, intact constructs without frameshift mutations were not obtained. Therefore, error-prone PCR was performed to introduce random mutations into the DddAtox coding sequence, and non-toxic full-length DddAtox variants with four point mutations S1326G, G1348S, A1398V, and S1418G (referred to as "GSVG") were obtained (for S1326G, G1348S, A1398V, and S1418G, in the amino acid sequence of SEQ ID NO: 269, S at position 37 was substituted with G; G at position 59 was substituted with S; A at position 109 was substituted with V; and S at position 129 was substituted with G, including the sequence of SEQ ID NO: 276, FIG. 52a). Also, these variants were fused to the C terminus of dCas9, D10A nCas9, and Cas9 and to the N terminus of dCas9, nCas9, and Cas9. In human cells, these fusion proteins, except for wild-type Cas9, induced cytosine-to-thymine conversions with efficiencies of up to 38% at various sites (FIGs. 52b, 57, and 58). Interestingly, fusion proteins containing GSVG variants fused to the C-terminus of dCas9, D10A nCas9, and H840A nCas9 caused cytosine base editing 3' downstream of the protospacer-adjacent motif (PAM), whereas fusion proteins dCas9 and nCas9 containing the same variant fused to the N-terminus thereof induced base editing 5' upstream of the protospacer (FIG. 52c). As expected, fusion proteins containing Cas9 caused indels rather than base substitutions.

In order to find out which mutations are important in the GSVG variant, attempts were made to construct four revertants of SSVG, GGVG, GSAG, and GSVS through site-directed mutagenesis. SSVG, GSAG, and GSVS revertants were obtained, but the GGVG variant fused to the C-terminus of nCas9 was not obtained. G1348 is right next to E1347, which is the key of the catalytic site. The G1348S mutation reduced catalytic activity, avoiding cytotoxicity in *E. coli.* The editing frequencies of the three revertants and the GSVG variant at two target sites in the transfected cells for up to 21 days were measured. The frequency of cytosine-to-thymine editing induced by GSAG and GSVS was gradually decreased to about half from day 3 to day 21 after transfection, and thus these two revertants were somewhat cytotoxic while GSVG and SSVG were retained (FIG. 59). These results suggest that, in the GSVG variant, G1348S is essential and S1326G is neutral, while A1398V and S1418G reduce cytotoxicity.

**Taken together, our results show that non-toxic, fu**ll-length **DddAₜₒₓ variants with reduced affinity for dsDNA (AAAAA), attenuated deaminase activity (E1347A and possibly GSVG), or reduced cytotoxicity (GSVG) can be fused to dCas9 or nCas9 to create novel base editors with altered editing windows.** These base editors, termed dCas9-mDdBE (a DddA-derived base editor composed of a full-length monomeric DddAtox variant fused to the C-terminus of dCas9), nCas9-mDdBE, mDdCE-dCas9, and mDdCE-nCas9, can **be used for base editing at positions upstream or downstream of a protospacer region, which is beyond the reach of BE2 or BE3 .**

We also investigated whether the non-toxic full-length DddAtox variant could be used for mitochondrial DNA editing. Among various variants, only two variants, GSVG and E1347A, were successfully fused to the C-terminus of the TALE array designed to bind to mitochondrial genes ND4 and ND6. Monomeric DdCBE (mDdCBE) including the GSVG variant achieved base editing at target nucleotide positions with efficiencies of up to 31% (ND4) (FIG. 60a) and 27% (ND6) (FIG. 60b) equivalent to the originally split DdCBE pair. Also, mDdCBE containing E1347A converted the target cytosine to thymine, although the efficiency was reduced, with editing rates of up to 7.2% (ND4) and 8.9% (ND6). Interestingly, the original DdCBE pair (G1333 split) specific to the ND4 gene had an editing efficiency of 0.8% at position C4, whereas the two mDdCBEs containing GSVG showed high editing efficiencies of 26% and 31%. These results show that split-dimeric DdCBE and mDdCBE have different mutation patterns, suggesting that mDdCBE may be complementary to dimeric DdCBE, inducing various mutations at given target sites.

One potential advantage of mDdCBE over split-dimeric DdCBE is that the off-target effect due to non-specific TALE-DNA interactions is halved compared to dimeric DdCBE. Dimeric DdCBE with split-DddAtox is able to operate at the half site to which only one subunit may bind, resulting in unwanted off-target mutations. The inactive DddAtox half of the DdCBE pair may recruit the other inactive half to form a functional deaminase. In order to confirm this hypothesis, HEK293T cells were co-transfected with a plasmid encoding one subunit of dimeric DdCBE and a plasmid encoding the TALE-free DddAtox half and editing frequency was measured at two mitochondrial target sites. As expected, cytosine-to-thymine editing was observed at the target sites with a frequency of 0.7 to 3.6% (FIGs. 60c-60f). These results suggest that unwanted off-target mutations may be caused by interactions of the split-DddAtox halves in the DdCBE pair with each other at the half sites, and that mDdCBE may avoid half of the off-target mutations caused by dimeric DdCBE.

### Example 7. High-efficiency A-to-G base editing in human cells using DdABE

Mitochondrial DNA base editing by DddA-derived cytosine base editors (DdCBEs) have enabled the creation of disease models in various cell lines and animals, opening a new way to treat mitochondrial genetic diseases. However, since DdCBE causes almost exclusively TC-to-TT base editing, it is able to cover only about 1/8 of all cases. Therefore, TALE-linked deaminase (TALED) was developed by linking two types of deaminase to TALE (transcription activator-like effector). Here, TALE was custom-designed to bind to a desired DNA moiety, and was fused to a DddAtox cytosine deaminase variant without catalytic activity, and a TadA protein, which is a DNA adenine deaminase derived from *E*. *coli.* TALED enables base editing for A-to-G conversion, unlike conventional base editing technology in which cytosine base editing was only possible for the TC context in human mitochondria. In fact, the custom-made TALED was able to induce adenine base editing with high efficiency (up to about 50%) at various targets in human cells.

In order to develop new base editing technology, a TadA variant (TadA*) of ABE8e was selected from among various TadA variants. This is because such a variant is able to induce adenine editing with high efficiency and is improved to be compatible with various DNA binding proteins and is thus efficiently compatible with a TALE or ZFP (zinc finger protein) upon real-world application.

TadA* and MTS (mitochondrial targeting sequence) were fused to TALE custom-made for a ND1 or ND4 target site, and whether base editing could actually occur in mitochondrial DNA was tested. Based on results of targeted deep sequencing, it was found that the adenosine base editing efficiency of the fusion protein was very low but detectable. Adenine base editing was induced with efficiencies of up to 1.2% (FIG. 67a) at the ND1 site and 0.6% (FIG. 67b) at the ND4 site. While TadA* is known to act specifically only on singlestranded DNA, it was found that, when TadA* was fused to TALE, base editing in double-stranded target DNA was also induced, although the efficiency was very low.

With the result that adenine base editing may occur in mitochondrial DNA, we sought to enhance the efficiency by fusing the DddAtox protein. The DddAtox protein is an interbacterial toxin derived from *Burkholderia cenocepacia* that deaminates cytosine. This protein works on double-stranded DNA, and thus may help TadA*adenine deaminase better access the target DNA. For existing DdCBE using DddAtox, the DddAtox protein is split into two halves, which are then fused respectively to left-TALE (L-TALE) recongizing the left-half DNA site and right-TALE (R-TALE) recognizing the right-half DNA site, and to a uracil glycosylase inhibitor (UGI) that increases cytosine base editing efficiency (TALE-split DddAtox-UGI). The reason why DddAtox is used in the form of splits is that the use of the full-length protein causes cytotoxicity. Specifically, TadA* was attached instead of UGI to either side of DdCBE targeting the ND1 site, and L-TALE-split DddAtox-TadA* and R-TALE-split DddAtox-UGI, or L-TALE-split DddAtox-UGI and R-TALE-1397C-TadA* forms were made and tested. Curiously, it was confirmed that both A-to-G and C-to-T conversions occurred when TadA* on one side and 1397N and UGI on the other side were paired and transferred to human cells (FIG. 62c). In the conventional DdCBE, cytosine base editing occurred with an efficiency of about 20%, and no adenine base editing occurred at all, whereas when UGI was replaced with TadA* on either side, cytosine base editing was reduced to about half and adenine base editing occurred with an efficiency of about 10% (FIG. 62c). Briefly, TALE deaminases created by fusing the TadA variant to a split DddAtox half in DdCBEs could induce simultaneous A-to-G and C-to-T edits in human mtDNA (FIG. 62c). In the conventional DdCBE, cytosine base editing occurred with an efficiency of about 20%, and no adenine base editing occurred at all, whereas when TadA* was provided on either side, cytosine base editing was reduced to about half and adenine base editing occurred with an efficiency of about 10% (FIG. 62c) . Briefly, adenine base editing and cytosine base editing efficiencies were similar (FIG. 62c) .

Simultaneous cytosine base editing and adenine base editing may be useful for random mutagenesis, but in treating diseases, especially mitochondrial genetic diseases such as LOHN and MEALS caused by C-to-T mutations, it is desirable to induce adenine base editing exclusively. Therefore, in order to eliminate this concurrent cytosine base editing, UGI was removed. In DdCBE, when the cytosine deaminase DddAtox deaminates C to U, in order to prevent U from being repaired again by uracil glycosylase, which is a repair protein in cells during the DNA repair process, UGI is fused as a uracil glycosylase inhibitor. Hence, it was thought that, if such UGI was removed, adenine base editing efficiency would be maintained and cytosine base editing could be suppressed. Surprisingly, it was confirmed that the ND1-targeting TALE deaminase pair without UGI hardly caused cytosine base editing (<0.5%) and induced adenine base editing alone with high efficiency (about 50%) (FIGs. 63a and 63c), much higher than that with UGI . This was also confirmed in the ND4-targeting TALE deaminase pair. Only adenine editing was detected with high efficiency (about 35%) as in targeting ND1 (FIGs. 63b and 63d). In this way, TALED, which is a new adenine deaminase acting on double-stranded DNA in which the DddAtox system and TadA* were fused, was developed, and adenine base editing was possible in human mitochondria for the first time. Also, adenine base editing was ultimately induced with about 50-fold higher efficiency compared to TALE fused to TadA* alone.

In addition, attempts were made to induce adenine base editing using the full-length E1347A DddAtox variant in which catalytic activity was eliminated or the variants (AAAAA and GSVG) in which catalytic activity was maintained but cytotoxicity was eliminated. Since adenine base editing, rather than cytosine base editing, is intended to occur in singlestranded DNA, the full-length E1347A DddAtox variant, which lacks cytosine base editing activity, could still be utilized to enhance A-to-G editing efficiency by facilitating the accessibility of double-stranded DNA to TadA.Also, based on results in which cytosine base editing was ineffective in the absence of UGI, the variants that eliminated only cytotoxicity could be used. Two types of TALEDs containing the full-length variant were made (FIG. 64a). The first type was configured such that both TadA* (AD) and the full-length DddAtox variant were contained in one TALE (mTALED), the second type was configured such that TadA* (AD) and the full-length DddAtox variant were separately fused to respective TALEs (dTALD), and these two types were tested (FIG. 64a). Surprisingly, it was confirmed that both types of TALED targeting ND1 induced adenine base editing with high efficiency (FIG. 64b). Here, mTALED exhibited an efficiency of up to about 45%, and dTALED also showed an adenine base editing efficiency of about 50% (FIG. 64b). Similar experimentation was also performed at the ND4 site, in addition to the ND1 site, and adenine base editing was induced with similarly high efficiencies (FIG. 64c). Also, when using the full-length E1347A DddAtox variant without cytosine base editing activity, adenine base editing was induced with high efficiency (FIGs. 64b and 64c). This is deemed to be because the role in helping TadA* access to double-stranded DNA sufficiently is retained even in the absence of cytosine deamination activity. When the above results were reviewed in detail at the single nucleotide level (FIGs. 65 and 66), adenine base editing was induced in the immediate vicinity of TALE binding to DNA. Moreover, when two TALEs were used, base editing was induced only therebetween (spacer), and curiously, it was found that the target length was similar even in mTALED using one TALE (FIGs. 65 and 66).

In addition, we investigated whether the system would work with the zinc finger protein (ZFP) system in nuclear DNA. Therefore, an NC-type ZFP targeting nuclear DNA was created, and split-DddAtox and TadA* were fused thereto (FIG. 61a). Here, TadA* was fused to various positions of the ZFP (FIG. 61b). Among various constructs, a construct capable of inducing adenine base editing with an efficiency of up to 10% in nuclear DNA was created (FIG. 61d). Since UGI was present on either side, cytosine base editing efficiency was also high (FIG. 61c). The ZFP-DddAtox-TadA* system worked in human cell nuclear DNA, and furthermore, whether it also worked in mitochondria was tested. As such, reference was made to the construct that worked with the highest efficiency in nuclear DNA. Experimentation was performed in a manner in which, instead of a nuclear localization signal (NLS) toward nuclear DNA, a mitochondrial targeting sequence (MTS) toward mitochondria was attached thereto, and 1397N was fused to the Right ZFP of a ZFP targeting the ND1 site and TadA* and 1397C were fused to the Left ZFP. Consequently, adenine base editing was induced with an efficiency of about 3% (FIG. 61f). Although the adenine base editing efficiency was lower than that of TALED, if various conditions such as the linker that connects proteins and the like are optimized, this ZFP system will also be able to induce adenine base editing with good efficiency.

To date, gene editing technology has made remarkable progress. CRISPR-based genetic scissors (CRISPR Cas9, base editor, prime editor, etc.) have been developed in various ways by improving off-target editing and increasing efficiency. However, despite these many advances, limitations are imposed on treatment of mitochondrial genetic diseases. This is because, in CRISPR-based technology including a catalytic protein and gRNA that serves as a guide to the target, methods of transferring gRNA to mitochondria are absent, unlike proteins. Thus, there is no technology for handling mitochondrial genes other than eliminating mitochondrial DNA by cleaving DNA. David R. Liu's group in the United States first introduced DdCBE capable of inducing base editing in mitochondria. Since DdCBE contains the cytosine deaminase DddAtox, which acts on double-stranded DNA, it is fused to the DNA-binding protein TALE to induce base editing. However, since DdCBE causes limited base editing only in the TC context, there are many limitations in creating disease models or treating genetic diseases with real-world applications. Accordingly, TALEDs capable of inducing adenine base editing in mitochondria was created for the first time. TALEDs had a high efficiency of up to 50% and induced base editing of various types of adenines at the target sites. Moreover, TALEDs may induce both cytosine base editing and adenine base editing in the presence of UGI and is thus useful for random mutagenesis, whereas it may be utilized as a specific adenine base editing technique because cytosine base editing does not occur and only adenine base editing is induced in the absence of UGI. It is also applicable to the ZFP system, and adenine base editing is possible for nuclear DNA. The development of TALED will provide solutions to many mitochondrial genetic diseases, making it possible to create disease models corresponding thereto, and TALEDs will be useful for many mitochondrial gene-related studies that have not yet been pioneered.

Although specific embodiments of the present invention have been disclosed in detail above, it will be obvious to those skilled in the art that the description is merely of preferable exemplary embodiments and is not to be construed as limiting the scope of the present invention. Therefore, the substantial scope of the present invention will be defined by the appended claims and equivalents thereto.

### [Industrial Applicability]

According to the present invention, it is possible to reduce non-selectivity of unwanted cytosine deaminase by substituting specific amino acid residues in the interface of cytosine deaminase splits during in DdCBEs.

Regarding a full-length cytosine deaminase, it is possible to edit a portion that is difficult to edit with a conventional cytosine base editor. Apobec1, which is used as a deaminase in current cytosine base editors, is known to be an oncogene, and the use thereof for therapeutic purposes is limited, but the full-length deaminase developed herein may have no such problems.

The present invention is as small as about 2.5 kb, including a DNA-binding protein, and is thus useful for gene therapy using AAV vectors, facilitates delivery of mRNA and RNP, and enables production of useful materials using prokaryotes.

### [Sequence List Free Text]

An electronic file is attached.

## Claims

1. A fusion protein comprising:
(i) a DNA-binding protein; and
(ii) a first split and a second split derived from a cytosine deaminase or a variant thereof,
wherein each of the first split and the second split is fusedto the DNA-binding protein.

2. A fusion protein comprising:
(i) a DNA-binding protein; and
(ii) a non-toxic full-length cytosine deaminase derived from a cytosine deaminase or a variant thereof.

3. The fusion protein according to claim 1, wherein each of the first split and the second split has no cytosine deaminase activity.

4. The fusion protein according to claim 1, wherein the first split comprises the amino acid sequence starting from the N-terminal residue to at least one residue selected from the group consisting of G33, G44, A54, N68, G82, N98, and G108 of SEQ ID NO: 1.

5. The fusion protein according to claim 1 or 2, wherein the cytosine deaminase is derived from a double-stranded DNA deaminase (DddA) or an orthologue thereof.

6. The fusion protein according to claim 1, wherein the second split comprises the amino acid sequence starting from at least one residue selected from the group consisting of G34, P45, G55, N69, T83, A99, and A109 to the C-terminal residue of SEQ ID NO: 1.

7. The fusion protein according to claim 1, wherein the variant of the cytosine deaminase has at least one amino acid substitution at residues 3, 5, 10, 11, 13, 14, 15, 16, 17, 18, 19, 28, 30, and 31 in the first split comprising the amino acid sequence starting from the N-terminal residue to G44 of SEQ ID NO: 1 with a different amino acid.

8. The fusion protein according to claim 1, wherein the variant of the cytosine deaminase has at least one amino acid substitution at residues 13, 16, 17, 20, 21, 28, 29, 30, 31, 32, 33, 56, 57, 58, and 60 in the second split comprising the amino acid sequence starting from P45 to the C-terminal residue of SEQ ID NO: 1 with a different amino acid.

9. The fusion protein according to claim 1, wherein the variant of the cytosine deaminase has at least one amino acid substitution at residues 87, 88, 91, 92, 95, 100, 101, 102, and 103 in the first split comprising the amino acid sequence starting from the N-terminal residue to G108 of SEQ ID NO: 1 with a different amino acid.

10. The fusion protein according to claim 1, wherein the variant of the cytosine deaminase has at least one amino acid substitution at residues 13, 14, 15, and 16 in the second split comprising the amino acid sequence starting from A109 to the C-terminal residue of SEQ ID NO: 1 with a different amino acid.

11. The fusion protein according to claim 2, wherein the non-toxic full-length cytosine deaminase has at least one amino acid substitution at residues 37, 59, 109, and 129 in a wild-type cytosine deaminase of SEQ ID NO: 1 with a different amino acid.

12. The fusion protein according to any one of claims 7 to 11, wherein the different amino acid is alanine.

13. The fusion protein according to claim 2, wherein the non-toxic full-length cytosine deaminase is at least one selected from the group consisting of SEQ ID NOs: 12 to 22.

14. The fusion protein according to claim 1 or 2, wherein the DNA-binding protein is a zinc finger protein, a TALE protein, or a CRISPR-associated nuclease.

15. The fusion protein according to claim 1 or 2, wherein the DNA-binding protein is fused to a cytosine deaminase or a variant thereof through a peptide linker comprising 2 to 40 amino acid residues.

16. The fusion protein according to claim 15, wherein the linker comprises:
2a.a linker: GS;
5a.a linker: TGEKQ (SEQ ID NO: 8);
10a.a linker: SGAQGSTLDF (SEQ ID NO: 9);
16a.a linker: SGSETPGTSESATPES (SEQ ID NO: 10);
24a.a linker: SGTPHEVGVYTLSGTPHEVGVYTL (SEQ ID NO: 115); or
32a.a linker: GSGGSSGGSSGSETPGTSESATPESSGGSSGGS (SEQ ID NO: 11).

17. The fusion protein according to claim 1, wherein each of the first split and the second split is fused to the N-terminus or C-terminus of a zinc finger protein.

18. The fusion protein according to claim 1 or 2, wherein a single TALE array or each of a first TALE array and a second TALE array is fused to the cytosine deaminase.

19. The fusion protein according to claim 1 or 2, further comprising (iii) an adenine deaminase.

20. The fusion protein according to claim 19, wherein the adenine deaminase is a deoxy-adenine deaminase that is a variant of *E. coli* TadA.

21. The fusion protein according to claim 19, wherein the adenine deaminase is fused to the N terminus or C terminus of the DNA-binding protein or the cytosine deaminase or the variant thereof.

22. A nucleic acid encoding the fusion protein according to any one of claims 1 to 21.

23. The nucleic acid according to claim 22, which is ribonucleic acid or DNA.

24. A composition for base editing comprising the fusion protein according to any one of claims 1 to 21 or the nucleic acid according to claim 22.

25. The composition according to claim 24, further comprising a uracil glycosylase inhibitor (UGI).

26. A composition for base editing in a eukaryotic cell comprising the fusion protein according to any one of claims 1 to 21 or the nucleic acid according to claim 22.

27. A composition for base editing in a plant cell comprising the fusion protein according to any one of claims 1 to 21 or the nucleic acid according to claim 22, and a nuclear localization signal (NLS) peptide or a nucleic acid encoding the same.

28. A composition for base editing in a plant cell comprising the fusion protein according to any one of claims 1 to 21 or the nucleic acid according to claim 22, and a chloroplast transit peptide or a nucleic acid encoding the same.

29. A composition for base editing in a plant cell comprising the fusion protein according to any one of claims 1 to 21 or the nucleic acid according to claim 22, and a mitochondrial targeting signal (MTS) or a nucleic acid encoding the same.

30. The composition according to claim 29, further comprising a nuclear export signal (NES) or a nucleic acid encoding the same.

31. The composition according to any one of claims 27 to 30, wherein the fusion protein is delivered to a plant cell through injection using a gene gun (bombardment), PEG-mediated protoplast transfection, protoplast transfection by electroporation, or protoplast injection by microinjection.

32. The composition according to claim 31, wherein the nucleic acid is delivered to a plant cell through transformation using Agrobacterium (for example, *Agrobacterium tumefaciens* or *Agrobacterium rhizogene)*, viral transfection, injection using a gene gun (bombardment), PEG-mediated protoplast transfection, protoplast transfection by electroporation; or protoplast injection by microinjection.

33. The composition according to any one of claims 27 to 30, which is for base editing in mitochondria, chloroplasts, or plastids (leucoplasts, chromoplasts) in plants.

34. The composition according to any one of claims 27 to 30, further comprising a transcription activator-like effector (TALE)-FokI nuclease or zinc finger nuclease (ZFN) that cleaves a wild-type DNA sequence but does not cleave an edited base sequence, or a nucleic acid encoding the same.

35. A method for base editing in eukaryotic nuclear, mitochondrial, or plastid DNA comprising performing treatment with the composition according to any one of claims 27 to 30.

36. The method according to claim 35, wherein base editing efficiency is increased by further comprising a TALEN or ZFN that cleaves a wild-type DNA sequence but does not cleave an edited base sequence, or a nucleic acid encoding the same.

37. A method for base editing in a plant cell comprising treating a plant cell with the composition according to any one of claims 27 to 30.

38. A method for base editing in a plant cell comprising treating a plant cell with the fusion protein according to any one of claims 1 to 21 or the nucleic acid according to claim 22, and a nuclear localization signal (NLS) peptide or a nucleic acid encoding the same.

39. A method for base editing in a plant cell comprising treating a plant cell with the fusion protein according to any one of claims 1 to 21 or the nucleic acid according to claim 22, and a chloroplast transit peptide or a nucleic acid encoding the same.

40. A method for base editing in a plant cell comprising treating a plant cell with the fusion protein according to any one of claims 1 to 21 or the nucleic acid according to claim 22, and a mitochondrial targeting signal (MTS) or a nucleic acid encoding the same.

41. A composition for base editing in an animal cell comprising the fusion protein according to any one of claims 1 to 21 or the nucleic acid according to claim 22, and a nuclear localization signal (NLS) peptide or a nucleic acid encoding the same.

42. A composition for base editing in an animal cell comprising the fusion protein according to any one of claims 1 to 21 or the nucleic acid according to claim 22, and a mitochondrial targeting signal (MTS) or a nucleic acid encoding the same.

43. The composition according to claim 42, further comprising a nuclear export signal or a nucleic acid encoding the same.

44. The composition according to claim 42, further comprising a transcription activator-like effector (TALE)-FokI nuclease or ZFN that cleaves a wild-type DNA sequence but does not cleave an edited base sequence, or a nucleic acid encoding the same.

45. A method for base editing in an animal cell comprising treating an animal cell with the composition according to claim 41 or 42.

46. A method for base editing in an animal cell comprising treating an animal cell with the fusion protein according to any one of claims 1 to 21 or the nucleic acid according to claim 22, and a nuclear localization signal (NLS) peptide or a nucleic acid encoding the same.

47. A method for base editing in an animal cell comprising treating an animal cell with the fusion protein according to any one of claims 1 to 21 or the nucleic acid according to claim 22, and a mitochondrial targeting signal (MTS) or a nucleic acid encoding the same.

48. The method according to claim 46 or 47, wherein base editing efficiency is increased by further comprising a TALEN or ZFN that cleaves a wild-type DNA sequence but does not cleave an edited base sequence, or a nucleic acid encoding the same.

49. A composition for A-to-G base editing in a prokaryotic cell or a eukaryotic cell comprising the fusion protein according to claim 19 or a nucleic acid encoding the same, wherein a DNA-binding protein is a zinc finger protein, a TALE protein, or a CRISPR-associated nuclease, and a cytosine deaminase or a variant thereof of the fusion protein is derived from bacteria and is specific to double-stranded DNA.

50. A composition for A-to-G base editing in a prokaryotic cell or a eukaryotic cell comprising the fusion protein according to claim 19 or a nucleic acid encoding the same, wherein a DNA-binding protein is a zinc finger protein, a TALE protein, or a CRISPR-associated nuclease, a cytosine deaminase or a variant thereof of the fusion protein is derived from bacteria and is specific to double-stranded DNA, the DNA-binding protein is fused to the N-terminus of the cytosine deaminase or the variant thereof, and the DNA-binding protein is fusedto the C-terminus of an adenine deaminase of the fusion protein.

51. A composition for C-to-T base editing in a prokaryotic cell or a eukaryotic cell comprising the fusion protein according to claim 19 or a nucleic acid encoding the same and a uracil glycosylase inhibitor (UGI), wherein a DNA-binding protein is a zinc finger protein, a TALE protein, or a CRISPR-associated nuclease, and a cytosine deaminase or a variant thereof of the fusion protein is derived from bacteria and is specific to double-stranded DNA.

52. A method for A-to-G base editing in a prokaryotic cell or a eukaryotic cell comprising treating a prokaryotic cell or a eukaryotic cell with the fusion protein according to claim 19 or a nucleic acid encoding the same, wherein a DNA-binding protein is a zinc finger protein, a TALE protein, or a CRISPR-associated nuclease, and a cytosine deaminase or a variant thereof of the fusion protein is derived from bacteria and is specific to double-stranded DNA.

53. A method for A-to-G base editing in a prokaryotic cell or a eukaryotic cell comprising treating a prokaryotic cell or a eukaryotic cell with the fusion protein according to claim 19 or a nucleic acid encoding the same, wherein a DNA-binding protein is a zinc finger protein, a TALE protein, or a CRISPR associated nuclease, a cytosine deaminase or a variant thereof of the fusion protein is derived from bacteria and is specific to double-stranded DNA, the DNA-binding protein is fusedto the N terminus of the cytosine deaminase or the variant thereof, and the DNA-binding protein is fusedto the C terminus of an adenine deaminase of the fusion protein.

54. A method for C-to-T base editing in a prokaryotic cell or a eukaryotic cell comprising treating a prokaryotic cell or a eukaryotic cell with the fusion protein according to claim 19 or a nucleic acid encoding the same and a uracil glycosylase inhibitor (UGI), wherein a DNA-binding protein is a zinc finger protein, a TALE protein, or a CRISPR-associated nuclease, and a cytosine deaminase or a variant thereof of the fusion protein is derived from bacteria and is specific to double-stranded DNA.
